# EUROPÄISCHE PATENTSCHRIFT

(11) **EP 2 121 639 B1**
(45) Veröffentlichungstag und Bekanntmachung des Hinweises auf die Patenterteilung: **16.10.2013**
(21) Anmeldenummer: 07856396.2
(22) Anmeldetag: 06.12.2007
(51) Int. Cl.: C07D 251/18, A01N 43/68, C07C 279/26

(54) **SUBSTITUIERTE 2,4-DIAMINO-1,3,5-TRIAZINE, VERFAHREN ZU DEREN HERSTELLUNG UND DEREN VERWENDUNG ALS HERBIZIDE UND PFLANZENWACHSTUMSREGULATOREN**
SUBSTITUTED 2,4-DIAMINO-1,3,5-TRIAZINES, METHODS FOR THE PRODUCTION THEREOF, AND USE THEREOF AS HERBICIDES AND PLANT GROWTH REGULATORS
2,4-DIAMINO-1,3,5-TRIAZINES SUBSTITUÉES, PROCÉDÉ DE PRODUCTION DE CELLES-CI ET UTILISATION DE CELLES-CI COMME HERBICIDES ET RÉGULATEURS DE CROISSANCE VÉGÉTALE

(30) Priorität: 19.12.2006 DE 102006059941
(43) Veröffentlichungstag der Anmeldung: 25.11.2009
(73) Patentinhaber: Bayer CropScience AG, 40789 Monheim (DE)
(72) Erfinder: AHRENS, Hartmut, 63329 Egelsbach (DE); DIETRICH, Hansjörg, 65835 Liederbach (DE); AULER, Thomas, 42799 Leichlingen (DE); HILLS, Martin, 65510 Idstein (DE); KEHNE, Heinz, 65719 Hofheim (DE); FEUCHT, Dieter, 65760 Eschborn (DE); HERRMANN, Stefan, 40764 Langenfeld (DE); KATHER, Kristian, 40764 Langenfeld (DE); LEHR, Stefan, 65835 Liederbach (DE)
(86) Internationale Anmeldenummer: PCT/EP2007/010589
(87) Internationale Veröffentlichungsnummer: WO 2008/074403

(56) Entgegenhaltungen:
- EP-A- 1 484 324
- WO-A-00/32580
- WO-A-01/44208
- WO-A-99/46249
- DE-A1- 2 258 243
- DE-A1- 19 604 191
- DE-A1- 19 641 692
- DE-A1- 19 711 825

## Beschreibung

Die Erfindung betrifft das technische Gebiet der Herbizide und Pflanzenwachstumsregulatoren, insbesondere der Herbizide zur Bekämpfung von Unkräutern und Ungräsern in Nutzpflanzenkulturen und zur generellen Kontrolle von unerwünschtem Pflanzenwachstum.

2,4-Diamino-s-triazine mit Resten aus der Gruppe Halogen, Alkoxy, Alkylthio und anderen über Heteroatome gebundenen Resten am Triazinring sind vielfach als Herbizide bekannt. Diese Verbindungen weisen meist N,N-dialkylsubstituierte Aminogruppen auf; siehe sogenannte "Triazin-herbizide" wie Atrazin, Simazin etc.

Es ist weiterhin bekannt, dass bestimmte Verbindungen aus der Reihe der alkylsubstituierten 2,4-Diamino-s-triazine und analoger Verbindungen herbizide und pflanzenwachstumsregulierende Eigenschaften besitzen; siehe beispielsweise Verbindungen vom Typ der 2-Amino-4-alkylamino-6-halogenalkyl-1,3,5-triazine [WO-A-90/09378 (EP-A-411153), WO-A-88/02368 (EP-A-283522), WO-A-94/24086, EP-A-509544, EP-A-492,615] und der 2-Amino-4-bicyclylamino-1,3,5-triazine [WO 97/31904, DE-A-19607450, WO-A-97/19936, WO-A-2004/069814]. Derartige Verbindungen besitzen in der Regel einen gegebenenfalls substituierten Alkyl- oder Cycloalkylrest am Triazinring und eine (hetero)aromatische Gruppe, die über gegebenenfalls verbrückte aliphatische Brücken an einer Aminogruppe des 2,4-Diamino-s-triazins gebunden ist. Diese Herbizide unterscheiden sich von denen der "Triazin-herbizide" in der Regel hinsichtlich der Wirksamkeit als auch der Anwendungscharakteristika.

Aus WO 00/32580, WO 99/46249 und DE 2258243 sind jeweils herbizid wirksame 2,4-Diamino-s-triazine bekannt, welche an mindestens einer der beiden Aminogruppen verschiedene Reste, insbesondere verzweigte Alkyl- und Cycloalkyl-Reste, tragen.

Die Anwendung der Derivate des letztgenannten Typs als Herbizide zur Schadpflanzenbekämpfung in verschiedenen Nutzpflanzenkulturen oder im Nichtkulturland ist jedoch nicht unter allen gewünschten Bedingungen möglich. So erfordert in manchen Fällen die Schließung von Wirkstofflücken eine zu hohe Aufwandmenge, bei der Schäden an Kulturpflanzen oder Plantagenkulturen (inklusive Obstplantagen) auftreten, oder die Wirkung hängt zu stark von Umweltbedingungen, wie Wetter- und Bodenverhältnissen ab. Es besteht deshalb weiterhin ein Bedarf nach alternativen wirkungsstarken Herbiziden für die selektive Anwendung in Pflanzenkulturen oder Nichtkulturland.

Überraschenderweise wurden nun neue herbizide Wirkstoffe aus der letztgenannten Gruppe der alkylsubstituierten 2,4-Diamino-1,3,5-triazine gefunden, welche im Vergleich zu bekannten, strukturell verwandten Wirkstoffen aus dieser Gruppe mit Vorteilen als Herbizide oder Pflanzenwachstumsregulatoren eingesetzt werden können.

Gegenstand der Erfindung sind herbizide Verbindungen der Formel (I) oder deren Salze, worin 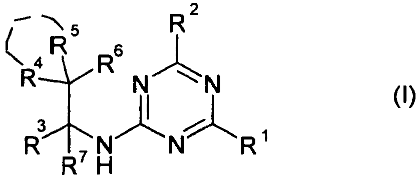
- R¹: einen Rest der Formel -NH₂, -NH(B¹-D¹), -N(B¹-D¹)(B²-D²), worin jeweils B¹, B², D¹ und D² wie unten definiert sind, oder eine Gruppe der Formel 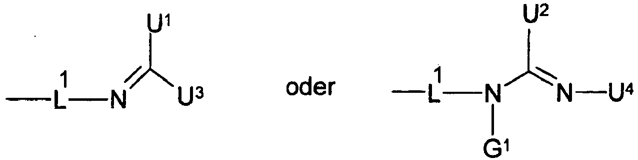 wobei
L¹ eine direkte Bindung, -O-, -S- oder eine Gruppe der Formel -NG²-, vorzugweise eine direkte Bindung bedeutet,
U¹, U² unabhängig voneinander eine Gruppe der Formel G³, OG⁴, SG⁵, NG⁶G⁷, NG⁸NG⁹G¹⁰, NG¹¹OG¹² oder NG¹¹SG¹² bedeuten,
U³ eine Gruppe der Formel G¹³, OG¹⁴, SG¹⁵, NG¹⁶G¹⁷, NG¹⁸NG¹⁹G²⁰, NG²¹OG²² oder NG²³SG²⁴ bedeutet,
U⁴ eine Gruppe der Formel G²⁵, OG²⁶, SG²⁷ oder NG²⁸G²⁹ bedeutet, wobei die Reste G¹ bis G²⁹ unabhängig voneinander Wasserstoff, Aryl, das unsubstituiert oder substituiert ist und inklusive Substituenten 6 bis 30 C-Atome aufweist, oder (C₄-C₉)Cycloalkyl, das unsubstituiert oder substituiert ist und inklusive Substituenten 3 bis 30 C-Atome aufweist, oder Heterocyclyl, das substituiert oder unsubstituiert ist und inklusive Substituenten 2 bis 30 C-Atome aufweist, oder (C₁-C₆)Alkyl, (C₂-C₆)Alkenyl oder (C₂-C₆)Alkinyl bedeuten, wobei jeder der letztgenannten 3 Reste unsubstituiert oder durch einen oder mehrere Reste aus der Gruppe bestehend aus Halogen, Hydroxy, Cyano, Nitro, Thiocyanato, (C₁-C₄)Alkoxy, (C₁-C₄)Haloalkoxy, (C₂-C₄)Alkenyloxy, (C₂-C₄)Haloalkenyloxy, (C₁-C₄)Alkylthio, (C₁-C₄)Haloalkylthio, Acyl, (C₄-C₉)Cycloalkyl, das unsubstituiert oder substituiert ist, Phenyl, das unsubstituiert oder substituiert ist, und Heterocyclyl, das unsubstituiert oder substituiert ist, substituiert ist und inklusive Substituenten 1 bis 30 C-Atome aufweist,
oder die Reste U¹ und U³ oder U² und U⁴ oder U² und G¹ oder U⁴ und G¹ paarweise mit den sie verbindenden Atomen jeweils einen carbocyclischen bzw. heterocyclischen Ring mit 4 bis 7 Ringatomen bedeuten, wobei der Ring unsubstituiert oder substituiert ist und inklusive Substituenten bis zu 30 C-Atome aufweist,
B¹, B² jeweils unabhängig voneinander eine divalente Gruppe der Formeln -C(=Z*)-, -C(=Z*)-Z**-, -C(=Z*)-NH- oder -C(=Z*)-NR*-bedeuten, wobei Z* ein Sauerstoff- oder Schwefelatom, Z** ein Sauerstoff- oder Schwefelatom und R* (C₁-C₆)Alkyl, Aryl, Aryl-(C₁-C₆)alkyl, (C₄-C₉)Cycloalkyl oder (C₄-C₉)Cycloalkyl-(C₁-C₆)alkyl, wobei jeder der 5 letztgenannten Reste unsubstituiert oder substituiert ist und inklusive Substituenten bis zu 30 C-Atome aufweist, bedeuten,
D¹, D² jeweils unabhängig voneinander Wasserstoff, (C₁-C₆)Alkyl, Aryl, Aryl-(C₁-C₆)alkyl, (C₄-C₉)Cycloalkyl oder (C₄-C₉)Cycloalkyl-(C₁-C₆)alkyl bedeuten, wobei jeder der 5 letztgenannten Reste unsubstituiert oder substituiert ist und inklusive Substituenten bis zu 30 C-Atome aufweist, wobei die Gruppe R¹ vorzugsweise bis zu 30 C-Atome, insbesondere bis zu 20 C-Atome, ganz besonders bis zu 12 C-Atome aufweist,
- R²: Wasserstoff, (C₁-C₆)Alkyl, (C₂-C₆)Alkenyl oder (C₂-C₆)Alkinyl, wobei jede der drei letztgenannten Gruppen unsubstituiert oder durch einen oder mehrere Reste aus der Gruppe, welche aus Halogen, Hydroxy, Cyano, (C₁-C₆)Alkoxy, (C₁-C₆)Alkylthio, (C₁-C₆)Haloalkoxy, (C₁-C₄)Alkoxy-(C₁-C₄)alkoxy und gegebenenfalls durch halogen-, cyano-, (C₁-C₄)alkyl- oder (C₁-C₄)haloalkylsubstituiertes (C₄-C₆)Cycloalkyl besteht, substituiert ist, oder (C₄-C₆)Cycloalkyl, das unsubstituiert oder durch einen oder mehrere Reste aus der Gruppe bestehend aus Halogen, Cyano, (C₁-C₄)Alkyl und (C₁-C₄)Haloalkyl substituiert ist,
- R³: Cyclopropyl oder Cyclobutyl, wobei jeder der letztgenannten beiden Reste unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist, und
- R⁴ und R⁵: unabhängig voneinander jeweils (C₁-C₆)Alkyl, (C₂-C₆)Alkenyl oder (C₂-C₆)Alkinyl, wobei jeder der letztgenannten drei Reste unsubstituiert oder durch einen oder mehrere Reste aus der Gruppe bestehend aus Halogen, (C₁-C₄)Alkoxy, (C₁-C₄)Haloalkoxy und (C₁-C₄)Alkylthio substituiert ist, oder Cyclopropyl, das unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist, oder
- R⁴ und R⁵: gemeinsam mit dem an sie gebundenen C-Atom einen 4- bis 9-gliedrigen carbocyclischen Ring, der unsubstituiert oder durch einen oder mehrere Reste aus der Gruppe bestehend aus Halogen, (C₁-C₄)Alkyl, (C₁-C₄)Haloalkyl, (C₁-C₄)Alkoxy-(C₁-C₄)Alkyl, (C₁-C₄)Alkoxy, (C₁-C₄)Haloalkoxy und (C₁-C₄)Alkylthio substituiert ist, und
- R⁶: Wasserstoff oder (C₁-C₄)Alkyl, das unsubstituiert oder durch einen oder mehrere Reste aus der Gruppe bestehend aus Halogen, (C₁-C₄)Alkoxy, (C₁-C₄)Haloalkoxy und (C₁-C₄)Alkylthio substituiert ist, und
- R⁷: Wasserstoff, Methyl, Ethyl oder Cyclopropyl, das unsubstituiert oder durch einen oder mehrere Reste aus der Gruppe bestehend aus Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist,
bedeuten.

Mit dem C-Atom in Position 2 eines Cyclus (siehe Bedeutungen von R³, R⁴, R⁵) ist ein Ring-C-atom bezeichnet, das dem C-Atom in Position 1 benachbart ist, wobei letzteres das C-Atom mit der "yl-Position" bezeichnet. Die "yl-Position" eines Alkylrestes bezeichnet das C-Atom mit der freien Bindung.

Wenn nicht näher angegeben, sind divalente Reste, z.B. B¹ = -C(=Z*)-Z**-, so definiert, dass in den zusammengesetzten Gruppen, z. B. - B¹-D¹, diejenige Bindung des divalenten Restes mit der Gruppe D¹ verbunden ist, die in der Formel für den divalenten Rest rechts geschrieben ist, d. h. - B¹-D¹ ist eine Gruppe der Formel -C(=Z*)-Z**-D¹; Entsprechendes gilt für analoge divalente Reste.

Die Verbindungen der Formel (I) können durch Anlagerung einer geeigneten anorganischen oder organischen Säure, wie beispielsweise HCl, HBr, H₂SO₄ oder HNO₃, aber auch Oxalsäure oder Sulfonsäuren an eine basische Gruppe, wie z.B. Amino oder Alkylamino, Salze bilden. Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden. Salze können ebenfalls dadurch gebildet werden, dass bei geeigneten Substituenten, wie z.B. Sulfonsäuren oder Carbonsäuren, der Wasserstoff durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze.

In der Formel (I) und allen nachfolgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein.
Der Ausdruck "(C₁-C₄)Alkyl" bedeutet eine Kurzschreibweise für Alkyl mit einem bis 4 Kohlenstoffatomen, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)Alkyl" umfassen entsprechend auch geradkettige oder verzweigte Alkylreste mit einer größeren Zahl von C-Atomen, d. h. gemäß Beispiel auch die Alkylreste mit 5 und 6 C-Atomen. Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,4-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Vinyl, Allyl, 1-Methyl-2-propenyl, 2-Methyl-2-propenyl, 2-Butenyl, Pentenyl, 2-Methylpentenyl oder Hexenyl group, vorzugsweise Allyl, 1-Methylprop-2-en-1-yl, 2-Methyl-prop-2-en-1-yl, But-2-en-1-yl, But-4-en-1-yl, 1-Methyl-but-4-en-1-yl oder 1-Methyl-but-2-en-1-yl. Alkenyl schließt insbesondere auch geradkettige oder verweigte Kohlenwasserstoffreste mit mehr als einer Doppelbindung ein, wie 1,4-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,4-Pentatrienyl;

Alkinyl bedeutet z.B. Propargyl, But-2-in-1-yl, But-4-in-1-yl, 1-Methyl-but-4-in-1-yl. Alkinyl schließt insbesondere auch geradkettige oder verweigte Kohlenwasserstoffreste mit mehr als einer Dreifachbindung oder auch mit einer oder mehreren Dreifachbindungen und einer oder mehreren Doppelbindungen ein, wie beispielsweise 1,4-Butatrienyl bzw. 4-Penten-1-in-1-yl.

Ein 4- bis 9-gliedriger carbocyclischer Ring bedeutet (C₄-C₉)Cycloalkyl oder (C₅-C₉)Cycloalkenyl.
(C₄-C₉)Cycloalkyl bedeutet ein carbocyclisches, gesättigtes Ringsystem mit vorzugsweise 4-9 C-Atomen, z.B. Cyclopropyl, Cyclobutyl, Cyclopentyl, Cyclohexyl, Cycloheptyl, Cyclooctyl oder Cyclononyl. Im Falle von substituiertem Cycloalkyl werden cyclische Systeme mit Substituenten umfaßt, wobei die Substituenten auch mit einer Doppelbindung am Cycloalkylrest, z. B. eine Alkylidengruppe wie Methyliden, gebunden sein können.
(C₅-C₉)Cycloalkenyl bedeutet ein carbocyclisches, nicht aromatisches, partiell ungesättigtes Ringsystem mit 5-9 C-Atomen, z.B. 1-Cyclobutenyl, 2-Cyclobutenyl, 1-Cyclopentenyl, 2-Cyclopentenyl, 4-Cyclopentenyl, oder 1-Cyclohexenyl, 2-Cyclohexenyl, 4-Cyclohexenyl, 1,4-Cyclohexadienyl oder 1,4-Cyclohexadienyl. Im Falle von substituiertem Cycloalkenyl gelten die Erläuterungen für substituiertes Cycloalkyl entsprechend

Alkyliden, z. B. auch in der Form (C₁-C₁₀)Alkyliden, bedeutet den Rest eines geradkettigen oder verzweigten Alkans, der über eine Zweifachbindung gebunden ist, wobei die Position der Bindungsstelle noch nicht festgelegt ist. Im Falle eines verzweigten Alkans kommen naturgemäß nur Positionen in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅.

Halogen bedeutet beispielsweise Fluor, Chlor, Brom oder Iod. Haloalkyl, -alkenyl und -alkinyl bedeuten durch gleiche oder verschiedene Halogenatome, vorzugsweise aus der Gruppe Fluor, Chlor und Brom, insbesondere aus der Gruppe Fluor und Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; Entsprechendes gilt für Haloalkenyl und andere durch Halogen substituierte Reste.

Aryl bedeutet ein mono-, bi- oder polycyclisches aromatisches System, beispielsweise Phenyl; Naphthyl, Tetrahydronaphthyl, Indenyl, Indanyl, Pentalenyl, Fluorenyl und ähnliches, vorzugsweise Phenyl.

Ein heterocyclischer Rest oder Ring (Heterocyclyl) kann gesättigt, ungesättigt oder heteroaromatisch sein; wenn nicht anders definiert, enthält er vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält. Vorzugsweise ist er ein heteroaromatischer Ring mit einem Heteroatom aus der Gruppe N, O und S, beispielsweise Pyridyl, Pyrrolyl, Thienyl oder Furyl; weiterhin bevorzugt ist er ein entsprechender heteroaromatischer Ring mit 2 oder 3 Heteroatomen, z. B. Pyrimidinyl, Pyridazinyl, Pyrazinyl, Triazinyl, Thiazolyl, Thiadiazolyl, Oxazolyl, Isoxazolyl, Pyrazolyl, Imidazolyl und Triazolyl. Weiterhin bevorzugt ist er ein partiell oder vollständig hydrierter heterocyclischer Rest mit einem Heteroatom aus der Gruppe N, O und S, beispielsweise Oxiranyl, Oxetanyl, Oxolanyl (= Tetrahydrofuryl), Oxanyl, Pyrrolinyl, Pyrrolidyl oder Piperidyl,

Weiterhin bevorzugt ist er ein partiell oder vollständig hydrierter heterocyclischer Rest mit 2 Heteroatomen, aus der Gruppe N, O und S, beispielsweise Piperazinyl, Dioxolanyl, Oxazolinyl, Isoxazolinyl, Oxazolidinyl, Isoxazolidinyl und Morpholinyl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Bevorzugte Beispiele für Heterocyclyl sind ein heterocyclischer Rest mit 3 bis 6 Ringatomen aus der Gruppe Pyridyl, Thienyl, Furyl, Pyrrolyl, Oxiranyl, 2-Oxetanyl, 4-Oxetanyl, Oxolanyl (= Tetrahydrofuryl), Pyrrolidyl, Piperidyl, insbesondere Oxiranyl, 2-Oxetanyl, 4-Oxetanyl oder Oxolanyl, oder ist ein heterocyclischer Rest mit zwei oder drei Heteroatomen, beispielsweise Pyrimidinyl, Pyridazinyl, Pyrazinyl, Triazinyl, Thienyl, Thiazolyl, Thiadiazolyl, Oxazolyl, Isoxazolyl, Pyrazolyl, Triazolyl, Piperazinyl, Dioxolanyl, Oxazolinyl, Isoxazolinyl, Oxazolidinyl, Isoxazolidinyl oder Morpholinyl.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Substituierte Reste, wie ein substituierter Alkyl-, Alkenyl-, Alkinyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Alkylsulfonyl und, im Falle cyclischer Reste, auch Alkyl, Haloalkyl, Alkylthio-alkyl, Alkoxy-alkyl, gegebenfalls substituiertes Mono- und Dialkylaminoalkyl und Hydroxy-alkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden substituiert sind.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bevorzugt Substituenten für die Subtituentenebenen sind beispielsweise

Amino, Hydroxy, Halogen, Nitro, Cyano, Mercapto, Carboxy, Carbonamid, SF₅, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxy-carbonyl, Alkenyloxy-carbonyl, Alkinyloxy-carbonyl, Aryloxycarbonyl, Alkanoyl, Alkenyl-carbonyl, Alkinyl-carbonyl, Aryl-carbonyl, Alkylthio, Cycloalkylthio, Alkenylthio, Cycloalkenylthio, Alkinylthio, Alkylsulfinyl, Alkylsulfonyl, Monoalkyl-aminosulfonyl, Dialkyl-aminosulfonyl, N-Alkylaminocarbonyl, N,N-Dialkyl-aminocarbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino und Benzylamino.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Mono- und Dialkyl-amino, Mono- und Diarylamino, Acylamino, N-Alkyl-N-arylamino, N-Alkyl-N-acylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Acyl bedeutet einen Rest einer organischen Säure, der formal durch Abtrennen einer Hydroxygruppe an der Säurefunktion entsteht, wobei der organische Rest in der Säure auch über ein Heteroatom mit der Säurefunktion verbunden sein kann. Beispiele für Acyl sind der Rest -CO-R einer Carbonsäure HO-CO-R und Reste davon abgeleiteter Säuren wie der Thiocarbonsäure, gegebenenfalls N-substituierten Iminocarbonsäuren oder der Rest von Kohlensäuremonoestern, N-substituierter Carbaminsäure, Sulfonsäuren, Sulfinsäuren, N-substituierter Sulfonamidsäuren, Phosphonsäuren oder Phosphinsäuren.
Acyl bedeutet beispielsweise Formyl, Alkylcarbonyl wie [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren. Dabei können die Reste jeweils im Alkyl- oder Phenylteil noch weiter substituiert sein, beispielsweise im Alkylteil durch einen oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.
Acyl bedeutet vorzugsweise einen Acylrest im engeren Sinne, d. h. einen Rest einer organischen Säure, bei der die Säuregruppe direkt mit dem C-Atom eines organischen Restes verbunden ist, beispielsweise Formyl, Alkylcarbonyl wie Acetyl oder [(C₁-C₄)Alkyl]-carbonyl, Phenylcarbonyl, Alkylsulfonyl, Alkylsulfinyl und andere Reste von organischen Säuren.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Vor allem aus den Gründen der höheren herbiziden Wirkung, besseren Selektivität und/oder besseren Herstellbarkeit sind erfindungsgemäße Verbindungen der genannten Formel (I) oder deren Salze von besonderem Interesse, worin einzelne Reste eine der bereits genannten oder im folgenden genannten bevorzugten Bedeutungen haben, oder insbesondere solche, worin eine oder mehrere der bereits genannten oder im Folgenden genannten bevorzugten Bedeutungen kombiniert auftreten.
Unabhängig von den jeweils anderen Resten aus der Gruppe R¹, R², R³, R⁴, R⁵, R⁶, R⁷ und der den allgemeinen Resten entsprechenden Unterbedeutungen, und vorzugsweise in Kombination mit bevorzugten Bedeutungen von einem oder mehreren dieser Reste sind erfindungsgemäße Verbindungen mit nachfolgend aufgeführten bevorzugten Bedeutungen der betreffenden Reste von besonderem Interesse.
Vorzugsweise bedeutet
- R¹: einen Rest der Formel -NH₂, -NH(B¹-D¹), -N(B¹-D¹)(B²-D²), worin jeweils B¹, B², D¹ und D² wie unten definiert sind, oder eine Gruppe der Formel 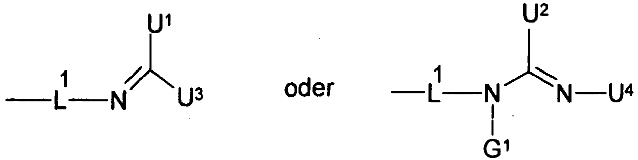 wobei
L¹ eine direkte Bindung, -O-, -S- oder eine Gruppe der Formel -NG²-, vorzugsweise eine direkte Bindung bedeutet,
U¹, U² unabhängig voneinander eine Gruppe der Formel G³, OG⁴, SG⁵, NG⁶G⁷, NG⁸NG⁹G¹⁰, NG¹¹OG¹² oder NG¹¹SG¹² bedeuten,
U³ eine Gruppe der Formel G¹³, OG¹⁴, SG¹⁵, NG¹⁶G¹⁷, NG¹⁸NG¹⁹G²⁰, NG²¹OG²² oder NG²³SG²⁴ bedeutet,
U⁴ eine Gruppe der Formel G²⁵, OG²⁶, SG²⁷ oder NG²⁸G²⁹ bedeutet, wobei die Reste G¹ bis G²⁹ unabhängig voneinander Wasserstoff, Phenyl, das unsubstituiert oder substituiert ist und inklusive Substituenten vorzugsweise 6 bis 30 C-Atome aufweist, oder (C₄C₉)Cycloalkyl, das unsubstituiert oder substituiert ist und inklusive Substituenten vorzugsweise 3 bis 30 C-Atome aufweist, oder Heterocyclyl, das substituiert oder unsubstituiert ist und inklusive Substituenten vorzugsweise 2 bis 30 C-Atome aufweist, oder (C₁-C₆)Alkyl, (C₂-C₆)Alkenyl oder (C₂-C₆)Alkinyl bedeuten, wobei jeder der letztgenannten 3 Reste unsubstituiert oder durch einen oder mehrere Reste aus der Gruppe Halogen, Hydroxy, Cyano, Nitro, Thiocyanato, (C₁-C₄)Alkoxy, (C₁-C₄)Haloalkoxy, (C₂-C₄)Alkenyloxy, (C₂-C₄)Haloalkenyloxy, (C₁-C₄)Alkylthio, (C₁-C₄)Alkylsulfinyl, (C₁-C₄)Alkylsulfonyl, (C₁-C₄)Haloalkylthio, (C₁-C₄)Haloalkylsulfinyl, (C₁-C₄)Haloalkylsulfonyl, (C₄-C₉)Cycloalkyl, das unsubstituiert oder substituiert ist, Phenyl, das unsubstituiert oder substituiert ist, und Heterocyclyl, das unsubstituiert oder substituiert ist, und Reste der Formeln R'-C(=Z')-, R'-C(=Z')-Z-, R'-Z-C(=Z')-, R'R"N-C(=Z')-, R'-Z-C(=Z')-O-, R'R"N-C(=Z')-Z-, R'-Z-C(=Z')-NR"- und R'R"N-C(=Z')-NR"'-,
worin R', R" und R"' jeweils unabhängig voneinander (C₁-C₆)Alkyl, Aryl, Aryl-(C₁-C₆)alkyl, (C₄-C₉)Cycloalkyl oder (C₄-C₉)Cycloalkyl-(C₁-C₆)alkyl, wobei jeder der 5 letztgenannten Reste unsubstituiert oder substituiert ist, bedeuten und worin Z und Z' unabhängig voneinander jeweils ein Sauerstoff- oder Schwefelatom sind,
substituiert ist,
oder die Reste U¹ und U³ oder U² und U⁴ oder U² und G¹ oder U⁴ und G¹ paarweise mit den sie verbindenden Atomen jeweils einen carbocyclischen bzw. heterocyclischen Ring mit 4 bis 7 Ringatomen bedeuten, wobei der Ring unsubstituiert oder substituiert ist,
B¹ und B² jeweils unabhängig voneinander eine divalente Gruppe der Formeln -C(=Z*)-, -C(=Z*)-Z**-, -C(=Z*)-NH- oder -C(=Z*)-NR*-bedeuten, wobei Z* ein Sauerstoff- oder Schwefelatom, Z** ein Sauerstoff- oder Schwefelatom und R* (C₁-C₆)Alkyl, Phenyl, Phenyl-(C₁-C₆)alkyl, (C₄-C₉)Cycloalkyl oder (C₄-C₉)Cycloalkyl-(C₁-C₆)alkyl, wobei jeder der 5 letztgenannten Reste unsubstituiert oder substituiert ist und inklusive Substituenten vorzugsweise bis zu 20 C-Atome aufweist, bedeuten,
D¹ und D² jeweils unabhängig voneinander Wasserstoff, (C₁-C₆)Alkyl, Phenyl, Phenyl-(C₁-C₆)alkyl, (C₄-C₉)Cycloalkyl oder (C₄-C₉)Cycloalkyl-(C₁-C₆)alkyl bedeuten, wobei jeder der 5 letztgenannten Reste unsubstituiert oder substituiert ist und inklusive Substituenten vorzugsweise bis zu 20 C-Atome aufweist,
wobei der Rest vorzugsweise eine Aminogruppe darstellt, welche vorzugsweise unsubstituiert oder einen oder zwei unter chemischen oder biologischen Bedingungen leicht abspaltbare Substituenten trägt.
Der Rest R¹ erlaubt in der Regel eine Steuerung und Beeinflussung der physikochemischen Eigenschaften. Daher kann der Wirkstoff beispielsweise schneller oder langsamer von den zu kontrollierenden, ungewünscht wachsenden Pflanzen aufgenommen werden. Je nach Anwendung und Struktur des Restes R¹ kann die Gruppe über einen definierten Zeitraum abgespalten werden, so dass im Ergebnis der Wirkstoff mit der freien Aminogruppe nach und nach freigesetzt wird und die Wirkdauer verlängert werden kann, ohne dass Wirkstoffe vergleichbarer Wirkstärke mit aus ökologischer Sicht ungewünscht langsamem Wirkstoffabbau eingesetzt werden müssen. Dieses Verfahren ist vorteilhaft, da es erlaubt, die für die Anwendung erforderliche Menge Wirkstoff über einen längeren Zeitraum zur Verfügung zu stellen. Daher können Mehrfachapplikationen vermieden werden. Da sich die Anforderungen an das Wirkprofil bei unterschiedlichen Anwendungen unterscheidet, kann mittels geeigneter Abgangsgruppen das Profil verändert und somit an die Anforderungen angepasst werden. Die Verbindungen dieser Reihe weisen ein vorteilhaftes Wirksamkeitsprofil und gute Kulturpflanzenverträglichkeit auf.

Weiter bevorzugt bedeutet
- R¹: einen Rest der Formel -NH₂, -NH(B¹-D¹), -N(B¹-D¹)(B²-D²), worin jeweils B¹, B², D¹ und D² wie unten definiert sind, oder eine Gruppe der Formel 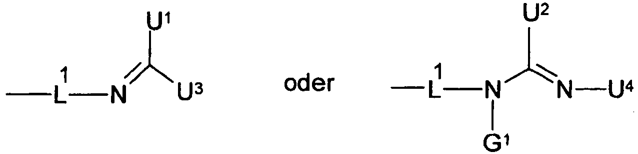 wobei
L¹ eine direkte Bindung, -O-, -S- oder eine Gruppe der Formel -NG²-, vorzugsweise eine direkte Bindung bedeutet,
U¹, U² unabhängig voneinander eine Gruppe der Formel G³, OG⁴, SG⁵, NG⁶G⁷, NG⁸NG⁹G¹⁰, NG¹¹OG¹² oder NG¹¹SG¹² bedeuten,
U³ eine Gruppe der Formel G¹³, OG¹⁴, SG¹⁵, NG¹⁶G¹⁷, NG¹⁸NG¹⁹G²⁰, NG²¹OG²² oder NG²³SG²⁴ bedeutet,
U⁴ eine Gruppe der Formel G²⁵, OG²⁶, SG²⁷ oder NG²⁸G²⁹ bedeutet, wobei die Reste G¹ bis G²⁹ unabhängig voneinander Wasserstoff, Phenyl, das unsubstituiert oder substituiert ist und inklusive Substituenten vorzugsweise 6 bis 30 C-Atome, insbesondere 6 bis 22 C-Atome aufweist, oder (C₄-C₉)Cycloalkyl, das unsubstituiert oder substituiert ist und inklusive Substituenten vorzugsweise 3 bis 30 C-Atome, insbesondere 6 bis 22 C-Atome aufweist, oder Heterocyclyl, das substituiert oder unsubstituiert ist und inklusive Substituenten vorzugsweise 2 bis 30 C-Atome, insbesondere 2 bis 20 C-Atome aufweist, oder
(C₁-C₆)Alkyl, (C₂-C₆)Alkenyl oder (C₂-C₆)Alkinyl bedeuten, wobei jeder der letztgenannten 3 Reste unsubstituiert oder durch einen oder mehrere Reste aus der Gruppe Halogen, Hydroxy, Cyano, Nitro, Thiocyanato, (C₁-C₄)Alkoxy, (C₁-C₄)Haloalkoxy, (C₂-C₄)Alkenyloxy, (C₂-C₄)Haloalkenyloxy, (C₁-C₄)Alkylthio, (C₁-C₄)Alkylsulfinyl, (C₁-C₄)Alkylsulfonyl, (C₁-C₄)Haloalkylthio, (C₁-C₄)Haloalkylsulfinyl, (C₁-C₄)Haloalkylsulfonyl, (C₄-C₉)Cycloalkyl, das unsubstituiert oder substituiert ist, Phenyl, das unsubstituiert oder substituiert ist, und Heterocyclyl, das unsubstituiert oder substituiert ist, und Reste der Formeln R'-C(=Z')-, R'-C(=Z')-Z-, R'-Z-C(=Z')-, R'R"N-C(=Z')-, R'-Z-C(=Z')-O-, R'R"N-C(=Z')-Z-, R'-Z-C(=Z')-NR"- und R'R"N-C(=Z')-NR"'-,
worin R', R" und R'" jeweils unabhängig voneinander (C₁-C₆)Alkyl, Phenyl, Phenyl-(C₁-C₆)alkyl, (C₄-C₉)Cycloalkyl oder (C₄-C₉)Cycloalkyl-(C₁-C₆)alkyl, wobei jeder der 5 letztgenannten Reste unsubstituiert oder substituiert ist, bedeuten und worin Z und Z' unabhängig voneinander jeweils ein Sauerstoff- oder Schwefelatom sind,
substituiert ist,
oder die Reste U¹ und U³ oder U² und U⁴ oder U² und G¹ oder U⁴ und G¹ paarweise mit den sie verbindenden Atomen jeweils einen carbocyclischen bzw. heterocyclischen Ring mit 4 bis 7 Ringatomen bedeuten, wobei der Ring unsubstituiert oder substituiert ist,
B¹ und B² jeweils unabhängig voneinander eine divalente Gruppe der Formeln -C(=Z*)-, -C(=Z*)-Z**-, -C(=Z*)-NH- oder -C(=Z*)-NR*-bedeuten, wobei Z* ein Sauerstoff- oder Schwefelatom, Z** ein Sauerstoff- oder Schwefelatom und R* (C₁-C₆)Alkyl, Phenyl, Phenyl-(C₁-C₆)alkyl, (C₄-C₉)Cycloalkyl oder (C₄-C₉)Cycloalkyl-(C₁-C₆)alkyl, wobei jeder der 5 letztgenannten Reste unsubstituiert oder substituiert ist und inklusive Substituenten vorzugsweise bis zu 20 C-Atome aufweist, bedeuten,
D¹ und D² jeweils unabhängig voneinander Wasserstoff, (C₁-C₆)Alkyl, Phenyl, Phenyl-(C₁-C₆)alkyl, (C₄-C₉)Cycloalkyl oder (C₄-C₉)Cycloalkyl-(C₁-C₆)alkyl bedeuten, wobei jeder der 5 letztgenannten Reste unsubstituiert oder substituiert ist und inklusive Substituenten vorzugsweise bis zu 20 C-Atome aufweist.

In den obengenannten Resten, die allgemein unsubstituiert oder substituiert sind, sind die möglichen Substituenten bei acyclischen Grundkörpern vorzugsweise ausgewählt aus Halogen und (C₁-C₄)Alkoxy; bei cyclischen Grundkörpern sind sie vorzugsweise ausgewählt aus Halogen, (C₁-C₄)Alkyl und (C₁-C₄)Alkoxy. Bevorzugt sind darüber hinaus die jeweils unsubstituierten Grundkörper.
- R¹: bedeutet beispielsweise einen Rest der Formel -NH₂, -NH(B¹-D¹) oder -N(B¹-D¹)(B²-D²), wobei B¹, B², D¹ und D² wie bereits definiert oder vorzugsweise wie weiter unten definiert sind, oder bedeutet vorzugweise einen Rest der Gruppe R⁸, wobei R⁸ einen Rest der Formeln (R⁸-a) bis (R⁸-d) bedeutet, 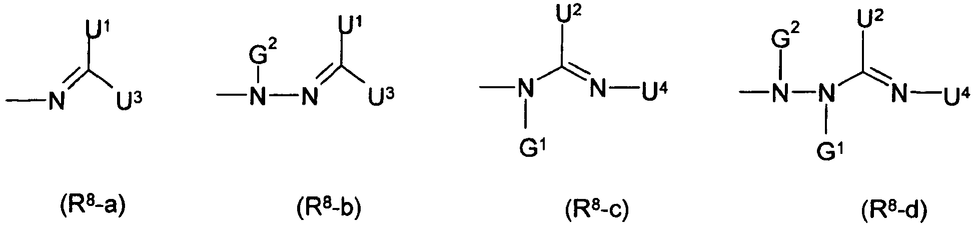 wobei in den Formeln die Gruppen U¹, U³, U⁴, G¹ und G² wie oben bei R¹ definiert sind. Die Gruppe R⁸ entspricht dabei Resten der Gruppe R¹, worin L¹ = direkte Bindung oder -NG²- bedeutet. Weiter bevorzugt sind Verbindungen (I) mit Resten, welche aus Untergruppen zu den letztgenannten Formeln zu R⁸ ausgewählt sind, beispielsweise mit Resten der nachfolgenden Formeln: 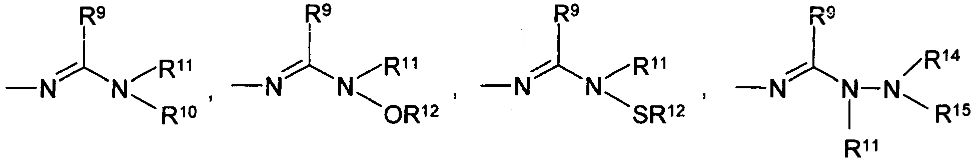 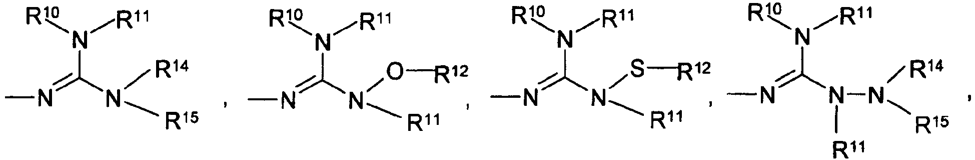 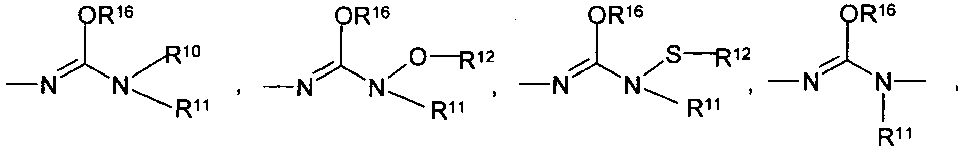 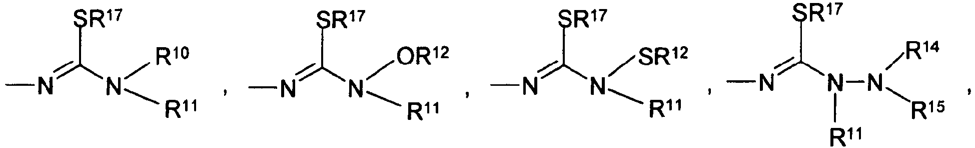 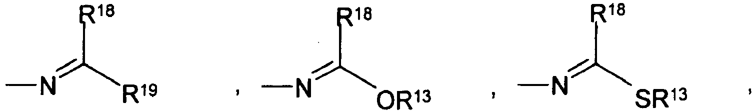 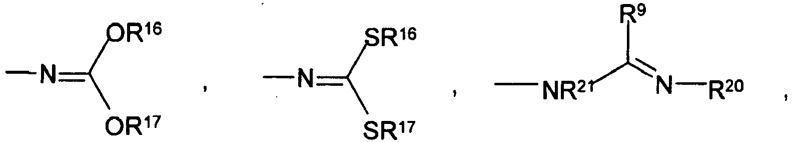 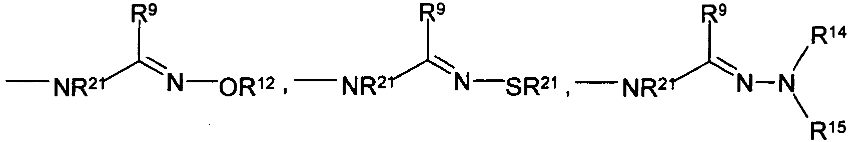 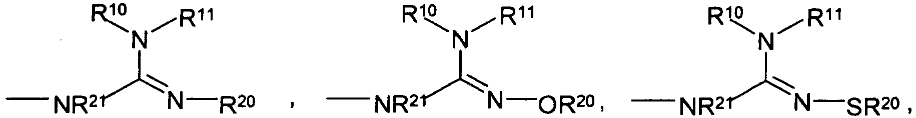 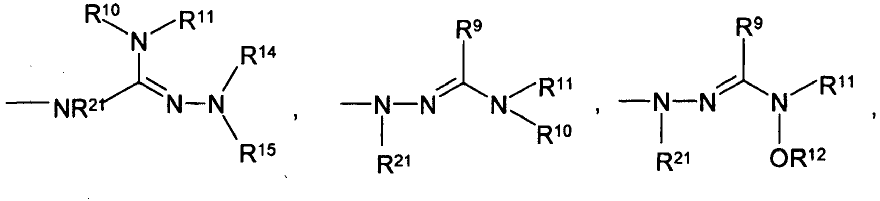 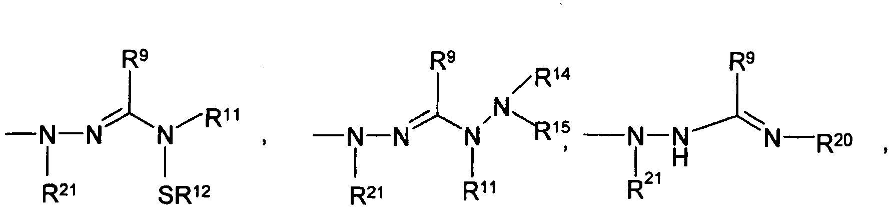 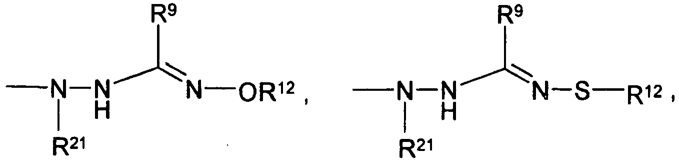 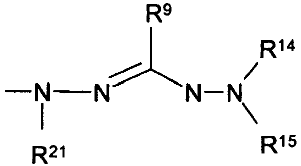
wobei die Reste R⁹ bis R²¹ weiter unten definiert sind.

Von besonderem Interesse sind auch Verbindungen, bei denen in den vorstehend genannten Resten jeweils zwei bestimmte Reste mit den sie verbindenden Atomen einen Ring bilden können, d. h. worin
R¹⁰ und R¹¹ gemeinsam mit dem Stickstoffatom der Gruppe NR¹⁰R¹¹ oder
R¹¹ und OR¹² gemeinsam mit dem Stickstoffatom der Gruppe NOR¹²R¹¹ oder
R¹¹ und SR¹² gemeinsam mit dem Stickstoffatom der Gruppe NSR¹²R¹¹ oder
R¹⁴ und R¹⁵ gemeinsam mit dem Stickstoffatom der Gruppe NR¹⁴R¹⁵ oder
OR¹⁶ und OR¹⁷ bzw. SR¹⁶ und SR¹⁷ bzw. OR¹³ und R¹⁸ bzw. SR¹³ und R¹⁸ bzw. R¹⁸ und R¹⁹ gemeinsam mit dem Kohlenstoffatom der jeweiligen Atomgruppierung der Formel =C(OR¹⁶)(OR¹⁷), =C(SR¹⁶)(SR¹⁷), =C(OR¹³)(R¹⁸), =C(SR¹³)(R¹⁸) oder =C(R¹⁸)(R¹⁹) in dem entsprechenden Rest der Formeln 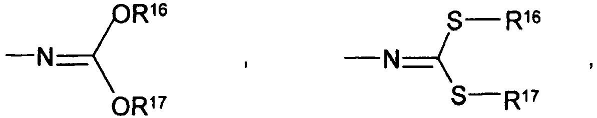 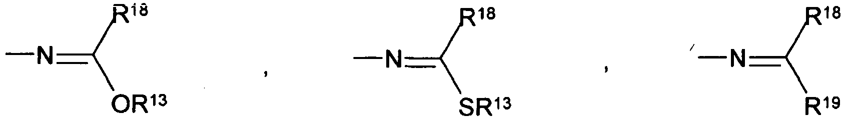 oder
R⁹ und R¹¹ gemeinsam mit der Atomgruppierung 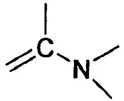 in den Resten der Formeln 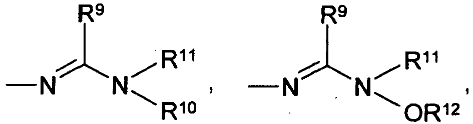 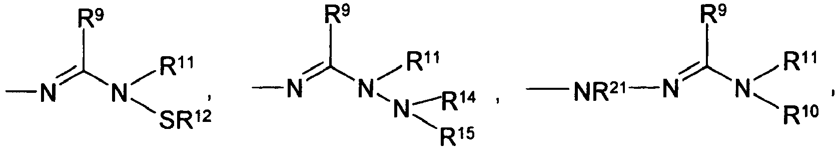 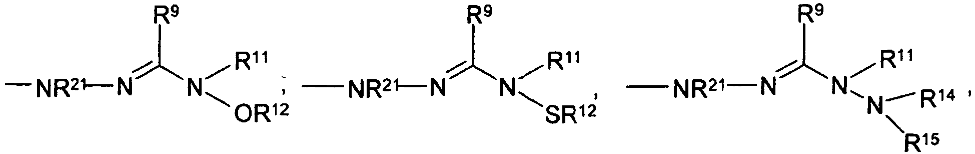 oder
R⁹ und NR²¹ gemeinsam mit dem Kohlenstoffatom der Gruppe der Formel 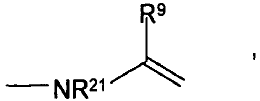 in den jeweiligen Resten oder,
R⁹ und R²⁰ gemeinsam mit dem gesamten Rest 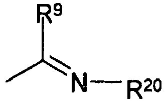 der Gruppen 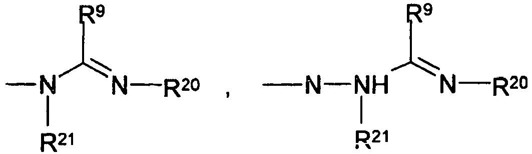 R¹¹ und R¹⁴ gemeinsam mit der Atomgruppierung 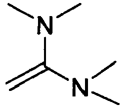 der Gruppen 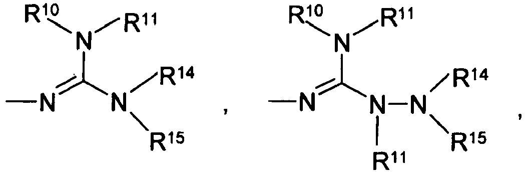 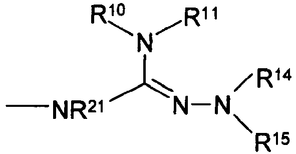 jeweils unabhängig voneinander einen carbocyclischen Ring mit 3 bis 9 Ringatomen bzw. einen heterocyclischen Ring mit 3 bis 7 Ringatomen und 1 bis 6 Heteroatomen, der das genannte Heteroatom bzw. die genannte Atomgruppierung enthält und wobei die gegebenenfalls weiteren Heteroringatome aus der Gruppe N, O und S ausgewählt sind und der carbocyclische oder heterocyclische Ring jeweils unsubstituiert oder substituiert ist,
bilden, wobei die Reste R⁹ bis R²¹ wie weiter unten definiert sind.

In vorstehend genannten Atomgruppierungen deutet speziell die einseitig gebundene Doppelbindung "=" die Bindungsstelle einer Doppelbindung oder die freie Doppelbindung an (gleichbedeutend mit der Bindungsstelle eines Ylidenrestes) und nicht die Kurzschreibweise für Vinyl.

R⁹, R¹⁰, R¹¹, R¹², R¹³, R¹⁴, R¹⁵, R¹⁸, R¹⁹, R²⁰, R²¹ in den vorstehenden Formeln bedeuten unabhängig voneinander
Wasserstoff, Aryl, das unsubstituiert oder substituiert ist und inklusive Substituenten vorzugsweise 6 bis 30 C-Atome aufweist, oder (C₄-C₉)Cycloalkyl, das unsubstituiert oder substituiert ist und inklusive Substituenten vorzugsweise 3 bis 30 C-Atome aufweist, (C₄-C₉)Cycloalkenyl, das unsubstituiert oder substituiert ist und inklusive Substituenten vorzugsweise 4 bis 30 C-Atome aufweist, oder Heterocyclyl, das substituiert oder unsubstituiert ist und inklusive Substituenten vorzugsweise 2 bis 30 C-Atome aufweist, oder
(C₁-C₆)Alkyl, (C₂-C₆)Alkenyl oder (C₂-C₆)Alkinyl,
wobei jeder der letztgenannten 3 Reste unsubstituiert oder durch einen oder mehrere Reste aus der Gruppe Halogen, Hydroxy, Cyano, Nitro, Thiocyanato, (C₁-C₄)Alkoxy, (C₁-C₄)Haloalkoxy, (C₂-C₄)Alkenyloxy, (C₂-C₄)Haloalkenyloxy, (C₁-C₄)Alkylthio, (C₁-C₄)Alkylsulfinyl, (C₁-C₄)Alkylsulfonyl, (C₁-C₄)Haloalkylsulfinyl, (C₁-C₄)Haloalkyl-sulfonyl,
(C₄-C₉)Cycloalkyl, das unsubstituiert oder substituiert ist, Phenyl, das unsubstituiert oder substituiert ist, Heterocyclyl, das unsubstituiert oder substituiert ist, und Reste der Formeln R'-C(=Z')-, R'-C(=Z')-Z-, R'-Z-C(=Z')-, R'R"N-C(=Z')-, R'-Z-C(=Z')-O-, R'R"N-C(=Z')-Z-, R'-Z-C(=Z')-NR"- und R'R"N-C(=Z')-NR"'-,
worin R', R" und R"' jeweils unabhängig voneinander (C₁-C₆)Alkyl, Aryl, Aryl-(C₁-C₆)alkyl, (C₄-C₉)Cycloalkyl oder (C₄-C₉)Cycloalkyl-(C₁-C₆)alkyl, wobei jeder der 5 letztgenannten Reste unsubstituiert oder substituiert ist, bedeuten und worin Z und Z' unabhängig voneinander jeweils ein Sauerstoff- oder Schwefelatom sind,
substituiert ist und inklusive Substituenten vorzugsweise 1 bis 30 C-Atome aufweist.

R¹⁶, R¹⁷ bedeuten jeweils unabhängig voneinander
Aryl, das unsubstituiert oder substituiert ist und inklusive Substituenten vorzugsweise 6 bis 30 C-Atome aufweist, oder (C₄-C₉)Cycloalkyl, das unsubstituiert oder substituiert ist und inklusive Substituenten vorzugsweise 3 bis 30 C-Atome aufweist, oder Heterocyclyl, das substituiert oder unsubstituiert ist und inklusive Substituenten vorzugsweise 2 bis 30 C-Atome aufweist, oder
(C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, Hydroxy, Cyano, Nitro, Thiocyanato, (C₁-C₄)Alkoxy, (C₁-C₄)Haloalkoxy, (C₂-C₄)Alkenyloxy, (C₂-C₄)Haloalkenyloxy, (C₁-C₄)Alkylthio, (C₁-C₄)Alkylsulfinyl, (C₁-C₄)Alkylsulfonyl, (C₁-C₄)Haloalkylsulfinyl, (C₁-C₄)Haloalkylsulfonyl, (C₄-C₉)Cycloalkyl, das unsubstituiert oder substituiert ist, Phenyl, das unsubstituiert oder substituiert ist, Heterocyclyl, das unsubstituiert oder substituiert ist, und Reste der Formeln R'-C(=Z')-, R'-C(=Z')-Z-, R'-Z-C(=Z')-, R'R"N-C(=Z')-, R'-Z-C(=Z')-O-,
R'R"N-C(=Z')-Z-, R'-Z-C(=Z')-NR"- und R'R"N-C(=Z')-NR"'-,
worin R', R" und R"' jeweils unabhängig voneinander (C₁-C₆)Alkyl, Aryl, Aryl-(C₁-C₆)alkyl, (C₄-C₉)Cycloalkyl oder (C₄-C₉)Cycloalkyl-(C₁-C₆)alkyl, wobei jeder der 5 letztgenannten Reste unsubstituiert oder substituiert ist, bedeuten und worin Z und Z' unabhängig voneinander jeweils ein Sauerstoff- oder Schwefelatom sind,
substituiert ist und inklusive Substituenten vorzugsweise 1 bis 30 C-Atome aufweist.

Vorzugsweise bedeuten die Reste R⁹, R¹⁰, R¹¹, R¹², R¹³, R¹⁴, R¹⁵, R¹⁸, R¹⁹, R²⁰, R²¹ jeweils unabhängig voneinander Wasserstoff.
Vorzugsweise bedeuten die Reste R⁹, R¹⁰, R¹¹, R¹², R¹³, R¹⁴, R¹⁵, R¹⁸, R¹⁹, R²⁰, R²¹ jeweils unabhängig voneinander auch
Phenyl, das unsubstituiert oder durch einen oder mehrere Reste aus der Gruppe Halogen, Hydroxy, Amino, Nitro, Formyl, Carboxy, Sulfo, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₄-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist und inklusive Substituenten 6 bis 30 C-Atome, vorzugsweise 6 bis 20 C-Atome, insbesondere 6 bis 15 C-Atome aufweist.

Vorzugsweise bedeuten die Reste R⁹, R¹⁰, R¹¹, R¹², R¹³, R¹⁴, R¹⁵, R¹⁸, R¹⁹, R²⁰, R²¹ jeweils unabhängig voneinander auch
(C₄-C₉)Cycloalkyl, das unsubstituiert oder durch einen oder mehrere Reste aus der Gruppe Halogen, Hydroxy, Amino, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino und Di(C₁-C₄)alkylamino substituiert ist und inklusive Substituenten 3 bis 30 C-Atome, vorzugsweise 3 bis 20 C-Atome, insbesondere 3 bis 15 C-Atome aufweist.

Vorzugsweise bedeuten die Reste R⁹, R¹⁰, R¹¹, R¹², R¹³, R¹⁴, R¹⁵, R¹⁸, R¹⁹, R²⁰, R²¹ jeweils unabhängig voneinander auch
Heterocyclyl, das unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₄-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist und inklusive Substituenten 2 bis 30 C-Atome, vorzugsweise 2 bis 20 C-Atome, insbesondere 2 bis 15 C-Atome aufweist.

Dabei und auch in anderen Resten ist Heterocyclyl wie weiter oben allgemein definiert bzw. vorzugsweise definiert.

R⁹, R¹⁰, R¹¹, R¹², R¹³, R¹⁴, R¹⁵, R¹⁸, R¹⁹, R²⁰, R²¹ sind jeweils unabhängig voneinander bevorzugt (C₁-C₄)Alkyl, das unsubstituiert oder durch einen oder mehrere Reste aus der Gruppe Halogen, (C₁-C₄)Alkoxy, (C₁-C₄)Alkylthio, (C₁-C₄)Alkylsulfonyl, (C₄-C₉)Cycloalkyl, das unsubstituiert oder substituiert ist, und Phenyl, das unsubstituiert oder durch einen oder mehrere Reste aus der Gruppe Halogen, (C₁-C₄)Alkyl und (C₁-C₄)Haloalkyl, (C₁-C₄)Alkoxy, (C₁-C₄)Haloalkoxy, (C₁-C₄)Alkylthio, Amino, Mono- und Di[(C₁-C₄)alkyl]amino, (C₁-C₄)Alkanoylamino, Benzoylamino, Nitro, Cyano, [(C₁-C₄)Alkyl]carbonyl, Formyl, Carbamoyl, Mono- und Di-[(C₁-C₄)alkyl]aminocarbonyl und (C₁-C₄)Alkylsulfonyl substituiert ist, und Heterocyclyl mit 3 bis 6 Ringatomen und 1 bis 3 Heteroringatomen aus der Gruppe N, O und S, wobei der Ring unsubstituiert oder durch einen oder mehrere Reste aus der Gruppe Halogen, (C₁-C₄)Alkyl und Oxo substituiert ist, substituiert ist, oder Phenyl, das unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₄-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist und inklusive Substituenten 2 bis 30 C-Atome, vorzugsweise 2 bis 20 C-Atome, insbesondere 2 bis 15 C-atome aufweist.

R¹⁶, R¹⁷ sind jeweils unabhängig voneinander vorzugsweise auch Phenyl, das unsubstituiert oder durch einen oder mehrere Reste aus der Gruppe Halogen, Hydroxy, Amino, Nitro, Formyl, Carboxy, Sulfo, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₄-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist und inklusive Substituenten 6 bis 30 C-Atome, vorzugsweise 6 bis 20 C-Atome, insbesondere 6 bis 15 C-Atome aufweist.

R¹⁶, R¹⁷ sind jeweils unabhängig voneinander vorzugsweise auch (C₄-C₉)Cycloalkyl, das unsubstituiert oder durch einen oder mehrere Reste aus der Gruppe Halogen, Hydroxy, Amino, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, und Di(C₁-C₄)alkylamino substituiert ist und inklusive Substituenten 3 bis 30 C-Atome, vorzugsweise 3 bis 20 C-Atome, insbesondere 3 bis 15 C-Atome aufweist.

R¹⁶, R¹⁷ sind jeweils unabhängig voneinander vorzugsweise auch Heterocyclyl, das unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₄-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist und inklusive Substituenten 2 bis 30 C-Atome, vorzugsweise 2 bis 20 C-Atome, insbesondere 2 bis 15 C-Atome aufweist.

Dabei ist Heterocyclyl wie weiter oben allgemein bzw. vorzugsweise definiert.

R¹⁶, R¹⁷ sind jeweils unabhängig voneinander bevorzugt (C₁-C₄)Alkyl, das unsubstituiert oder durch einen oder mehrere Reste aus der Gruppe Halogen, (C₁-C₄)Alkoxy, (C₁-C₄)Alkylthio, (C₁-C₄)Alkylsulfonyl, (C₄-C₉)Cycloalkyl, das unsubstituiert oder substituiert ist, und Phenyl, das unsubstituiert oder durch einen oder mehrere Reste aus der Gruppe Halogen, (C₁-C₄)Alkyl und (C₁-C₄)Haloalkyl, (C₁-C₄)Alkoxy, (C₁-C₄)Haloalkoxy, (C₁-C₄)Alkylthio, Amino, Mono- und Di[(C₁-C₄)alkyl]amino, (C₁-C₄)Alkanoylamino, Benzoylamino, Nitro, Cyano, [(C₁-C₄)Alkyl]carbonyl, Formyl, Carbamoyl, Mono- und Di-[(C₁-C₄)alkyl]aminocarbonyl und (C₁-C₄)Alkylsulfonyl substituiert ist, und Heterocyclyl mit 3 bis 6 Ringatomen und 1 bis 3 Heteroringatomen aus der Gruppe N, O und S, wobei der Ring unsubstituiert oder durch einen oder mehrere Reste aus der Gruppe Halogen, (C₁-C₄)Alkyl und Oxo substituiert ist, substituiert ist, oder Phenyl, das unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₄-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist und inklusive Substituenten 2 bis 30 C-Atome, vorzugsweise 2 bis 20 C-Atome, insbesondere 2 bis 15 C-atome aufweist.

Bevorzugt bedeuten B¹ und B² unabhängig voneinander jeweils eine divalente Gruppe der Formeln -C(=Z*)-, -C(=Z*)-Z**-, -C(=Z*)-NH- oder -C(=Z*)-NR*-, wobei Z* = O oder S, Z** = O oder S und R* = (C₁-C₄)Alkyl, Phenyl, Phenyl-(C₁-C₄)alkyl, (C₄-C₆)Cycloalkyl oder (C₄-C₆)Cycloalkyl-(C₁-C₄)alkyl, wobei jeder der 5 letztgenannten Reste unsubstituiert oder durch einen oder mehrere Reste aus der Gruppe Halogen, Hydroxy, Amino, Formyl, (C₁-C₄)Alkoxy, (C₁-C₄)Haloalkoxy, (C₁-C₄)Alkylthio, Mono(C₁-C₄)alkylamino, Di(C₁-C₄)alkylamino, (C₄-C₉)Cycloalkyl, [(C₁-C₄)Alkyllcarbonyl, [(C₁-C₄)Alkoxy]carbonyl, Aminocarbonyl, Mono(C₁-C₄)alkylamino-carbonyl, Di(C₁-C₄)alkylamino-carbonyl und im Fall cyclischer Reste auch (C₁-C₄)Alkyl und (C₁-C₄)Haloalkyl substituiert ist, sind, insbesondere Z* ein Sauerstoffatom und insbesondere R* (C₁-C₄)Alkyl, (C₄-C₆)Cycloalkyl, Phenyl oder Phenyl-(C₁-C₄)alkyl, wobei jeder der beiden letztgenannten Reste im Phenylteil unsubstituiert oder durch einen oder mehrere Reste aus der Gruppe Halogen, (C₁-C₄)Alkyl, (C₁-C₄)Haloalkyl, (C₁-C₄)Alkoxy oder (C₁-C₄)Haloalkoxy substituiert ist, bedeuten.

Bevorzugt bedeuten D¹ und D² unabhängig voneinander jeweils Wasserstoff, (C₁-C₄)Alkyl, Phenyl, Phenyl-(C₁-C₄)alkyl, (C₄-C₆)Cycloalkyl oder (C₄-C₆)Cycloalkyl-(C₁-C₄)alkyl, wobei jeder der 5 letztgenannten Reste unsubstituiert oder durch einen oder mehrere Reste aus der Gruppe Halogen, Hydroxy, Amino, Formyl, (C₁-C₄)Alkoxy, (C₁-C₄)Haloalkoxy, (C₁-C₄)Alkylthio, Mono(C₁-C₄)alkylamino, Di(C₁-C₄)alkylamino, (C₄-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl und im Fall cyclischer Reste auch (C₁-C₄)Alkyl und (C₁-C₄)Haloalkyl substituiert ist,
und bedeuten insbesondere
Wasserstoff, (C₁-C₄)Alkyl oder (C₄-C₆)Cycloalkyl oder Phenyl oder Phenyl-(C₁-C₄)alkyl, wobei jeder der beiden letztgenannten Reste im Phenylteil unsubstituiert oder durch einen oder mehrere Reste aus der Gruppe Halogen, (C₁-C₄)Alkyl, (C₁-C₄)Haloalkyl, (C₁-C₄)Alkoxy oder (C₁-C₄)Haloalkoxy substituiert ist.

Besonders bevorzugt bedeutet R¹ eine Aminogruppen, die unsubstituiert oder mit einer oder zwei unter physiologischen Bedingungen in Pflanzen leicht abspaltbaren Gruppen substituiert ist.
- R¹: ist vorzugsweise Amino, Acylamino mit 1 bis 6 C-Atomen, Di(C₁-C₄)alkylamino-(C₁-C₄)alkylidenamino oder N-Heterocyclylamino-(C₁-C₄)alkylidenamino, wobei der N-Heterocyclus ein gesättigter heterocyclischer Ring mit 1 bis 3 Heteroringatomen aus der Gruppe N, O und S und mindestens einem N-Atom als Heteroringatom, das an der Alkylidengruppe gebunden ist, darstellt.
- R¹: ist besonders bevorzugt Amino, (C₁-C₆)Alkanoylamino, das im Alkylteil unsubstituiert oder durch Halogen substituiert ist, Di-[(C₁-C₄)alkyl]-amino-methyliden-amino oder N-Morpholin-4-ylamino-methylidenamino, inbesondere Amino.
- R²: ist vorzugsweise Wasserstoff, (C₁-C₆)Alkyl, (C₂-C₆)Alkenyl oder (C₂-C₆)Alkinyl, wobei jede der drei letztgenannten Gruppen unsubstituiert oder durch einen oder mehrere Reste aus der Gruppe, welche aus Halogen, (C₁-C₄)Alkoxy, (C₁-C₄)Alkylthio, (C₁-C₄)Haloalkoxy, (C₁-C₂)Alkoxy-(C₁-C₂)alkoxy und gegebenenfalls durch halogen- oder (C₁-C₄)alkylsubstituiertes (C₄-C₆)Cycloalkyl besteht, substituiert ist, oder
(C₄-C₆)Cycloalkyl, das unsubstituiert oder durch einen oder mehrere Reste aus der Gruppe bestehend aus Halogen und (C₁-C₄)Alkyl substituiert ist.
- R²: ist weiter bevorzugt (C₁-C₄)Alkyl, das unsubstituiert oder durch einen oder mehrere Reste aus der Gruppe Halogen, wie Fluor und Chlor, substituiert ist, oder
Cyclopropyl, das unsubstituiert oder durch einen oder mehrere Reste aus der Gruppe bestehend aus Halogen und (C₁-C₄)Alkyl substituiert ist.
- R²: ist ganz bevorzugt (C₁-C₄)Alkyl, das unsubstituiert oder durch einen oder mehrere Reste aus der Gruppe Fluor und Chlor in 1-Position des Alkylrestes substituiert ist.

Beispiele für bevorzugte Bedeutungen von R² sind die Reste:
Methyl, Ethyl, n-Propyl, i-Propyl, Cyclopropyl, 1-Fluorcyclopropyl, Difluormethyl, Trifluormethyl, 1-Fluorethyl [(R)-, (S)- oder (RS)-1-Fluorethyl], 1-Fluor-n-propyl [(R)-, (S)- oder (RS)-1-Fluor-n-propyl], 1-Fluorisopropyl, 1-Fluorbutyl [(R)-, (S)- oder (RS)-1-Fluor-n-butyl].
   - R³: ist vorzugsweise Cyciopropyl oder Cyclobutyl, wobei jeder der letztgenannten beiden Reste unsubstituiert oder durch einen oder mehrere Reste aus der Gruppe bestehend aus Halogen, (C₁-C₄)Alkyl, (C₂-C₄)Alkenyl und (C₂-C₄)Alkinyl substituiert ist.
   - R³: ist weiter bevorzugt Cyclopropyl oder Cyclobutyl, wobei jeder der letztgenannten beiden Reste unsubstituiert oder durch einen oder mehrere Reste aus der Gruppe bestehend aus Halogen und (C₁-C₄)Alkyl, substituiert ist.
   - R³: ist insbesondere Cyclopropyl, das unsubstituiert oder durch einen oder mehrere Reste aus der Gruppe bestehend aus Halogen und (C₁-C₄)Alkyl, substituiert ist.

Beispiele für bevorzugte Bedeutungen von R³ sind die Reste:
Cyclopropyl, 1-Methyl-cyclopropyl, 1-Fluor-cyclopropyl, 1-Chlor-cyclopropyl, 2,2-Dimethylcyclopropyl, 2,2-Dichlorcyclopropyl, Cyclobutyl, wobei unsubstituiertes Cyclopropyl ganz besonders bevorzugt ist.

- R⁴ und R⁵: bedeuten vorzugsweise unabhängig voneinander jeweils (C₁-C₄)Alkyl, (C₂-C₄)Alkenyl oder (C₂-C₄)Alkinyl, wobei jeder der letztgenannten drei Reste unsubstituiert oder durch einen oder mehrere Reste aus der Gruppe bestehend aus Halogen, (C₁-C₄)Alkoxy, (C₁-C₄)Haloalkoxy und (C₁-C₄)Alkylthio substituiert ist, oder
Cyclopropyl, das unsubstituiert oder durch einen oder mehrere Reste aus der Gruppe bestehend aus Halogen und (C₁-C₄)Alkyl substituiert ist.
- R⁴ und R⁵: bedeuten weiter bevorzugt unabhängig voneinander jeweils (C₁-C₄)Alkyl, welches unsubstituiert oder durch einen oder mehrere Reste aus der Gruppe bestehend aus Halogen und (C₁-C₄)Alkoxy substituiert ist, oder
Cyclopropyl, das unsubstituiert oder durch einen oder mehrere Reste aus der Gruppe bestehend aus Halogen und (C₁-C₄)Alkyl substituiert ist.

Bevorzugt bedeutet R⁴ Methyl oder Cyclopropyl, inbesondere Methyl.
Bevorzugt bedeutet R⁵ Methyl oder Cyclopropyl, inbesondere Methyl.
R⁴ und R⁵ bedeuten weiter bevorzugt gemeinsam mit dem an sie gebundenen C-Atom einen 4- bis 6-gliedrigen carbocyclischen Ring, vorzugsweise Cyclopropyl, der unsubstituiert oder durch einen oder mehrere Reste aus der Gruppe bestehend aus Halogen und (C₁-C₄)Alkyl substituiert ist.
- R⁴ und R⁵: bedeuten insbesondere bevorzugt gemeinsam mit dem an sie gebundenen C-Atom einen 4- bis 6-gliedrigen carbocyclischen Ring, vorzugsweise Cyclopropyl, der unsubstituiert oder durch einen oder mehrere Reste aus der Gruppe bestehend aus Halogen und (C₁-C₄)Alkyl substituiert ist, wobei unsubstituiertes Cyclopropyl dabei besonderes bevorzugt ist.
- R⁴ und R⁵: bedeuten auch bevorzugt gemeinsam mit dem an sie gebundenen C-Atom einen 4- bis 6-gliedrigen carbocyclischen Ring, vorzugsweise Cyclopropyl, der unsubstituiert oder durch einen oder mehrere Reste aus der Gruppe bestehend aus Halogen und (C₁-C₄)Alkyl substituiert ist.
- R⁴: ist vorzugweise auch eine divalente Gruppe der Formel -CH₂-.
- R⁶: ist vorzugsweise Wasserstoff oder (C₁-C₄)Alkyl, das unsubstituiert oder durch einen oder mehrere Reste aus der Gruppe bestehend aus Halogen, (C₁-C₄)Alkoxy, (C₁-C₄)Haloalkoxy und (C₁-C₄)Alkylthio substituiert ist.
- R⁶: ist insbesondere Wasserstoff oder Methyl, ganz besonders Wasserstoff.
- R⁷: ist vorzugsweise Wasserstoff, Methyl, Ethyl oder Cyclopropyl, das unsubstituiert oder durch einen oder mehrere Reste aus der Gruppe bestehend aus Halogen, (C₁-C₄)Alkyl und (C₁-C₄)Haloalkyl substituiert ist,
- R⁷: ist insbesondere Wasserstoff, Methyl, Ethyl, Cyclopropyl, 1-Methyl-cyclopropyl, 1-Fluor-cyclopropyl, 1-Chlor-cyclopropyl, 2,2-Dimethylcyclopropyl, 2,2-Dichlorcyclopropyl, ganz besonders Wasserstoff.

Bevorzugt sind Verbindungen der Formel (I) oder deren Salze, worin
- R¹: Amino, Acylamino mit 1 bis 6 C-atomen, Di(C₁-C₄)alkylamino-(C₁-C₄)alkylidenamino oder N-Heterocyclylamino-(C₁-C₄)alkylidenamino (wie oben bei R¹ bereits definiert), inbesondere Amino,
- R²: Wasserstoff, (C₁-C₆)Alkyl, (C₂-C₆)Alkenyl oder (C₂-C₆)Alkinyl, wobei jede der drei letztgenannten Gruppen unsubstituiert oder durch einen oder mehrere Reste aus der Gruppe, welche aus Halogen, (C₁-C₄)Alkoxy, (C₁-C₄)Alkylthio, (C₁-C₄)Haloalkoxy, (C₁-C₂)Alkoxy-(C₁-C₂)alkoxy und gegebenenfalls durch halogen- oder (C₁-C₄)alkylsubstituiertes (C₄-C₆)Cycloalkyl besteht, substituiert ist, oder
(C₄-C₆)Cycloalkyl, das unsubstituiert oder durch einen oder mehrere Reste aus der Gruppe bestehend aus Halogen und (C₁-C₄)Alkyl substituiert ist.
- R³: Cyclopropyl oder Cyclobutyl, wobei jeder der letztgenannten beiden Reste unsubstituiert oder durch einen oder mehrere Reste aus der Gruppe bestehend aus Halogen, (C₁-C₄)Alkyl, (C₂-C₄)Alkenyl und (C₂-C₄)Alkinyl substituiert ist,
- R⁴ und R⁵: unabhängig voneinander jeweils (C₁-C₄)Alkyl, (C₂-C₄)Alkenyl oder (C₂-C₄)Alkinyl, wobei jeder der letztgenannten drei Reste unsubstituiert oder durch einen oder mehrere Reste aus der Gruppe bestehend aus Halogen, (C₁-C₄)Alkoxy, (C₁-C₄)Haloalkoxy und (C₁-C₄)Alkylthio substituiert ist, oder
Cyclopropyl, das unsubstituiert oder durch einen oder mehrere Reste aus der Gruppe bestehend aus Halogen und (C₁-C₄)Alkyl substituiert ist, oder
- R⁴ und R⁵: gemeinsam mit dem an sie gebundenen C-Atom einen 4- bis 6-gliedrigen carbocyclischen Ring, der unsubstituiert oder durch einen oder mehrere Reste aus der Gruppe bestehend aus Halogen und (C₁-C₄)Alkyl substituiert ist,
- R⁶: Wasserstoff oder (C₁-C₄)Alkyl, das unsubstituiert oder durch einen oder mehrere Reste aus der Gruppe bestehend aus Halogen, (C₁-C₄)Alkoxy, (C₁-C₄)Haloalkoxy und (C₁-C₄)Alkylthio substituiert ist.
- R⁷: Wasserstoff, Methyl, Ethyl oder Cyclopropyl, das unsubstituiert oder durch einen oder mehrere Reste aus der Gruppe bestehend aus Halogen, (C₁-C₄)Alkyl und (C₁-C₄)Haloalkyl substituiert ist,
bedeuten.

Weiter bevorzugt sind Verbindungen der Formel (I) oder deren Salze, worin
- R¹: vorzugsweise Amino, Acylamino mit 1 bis 6 C-Atomen, Di(C₁-C₄)alkylamino-(C₁-C₄)alkylidenamino oder N-Heterocyclylamino-(C₁-C₄)alkylidenamino (wie oben bei R¹ bereits näher definiert), inbesondere Amino.
- R²: (C₁-C₄)Alkyl, das unsubstituiert oder durch einen oder mehrere Reste aus der Gruppe Halogen, wie Fluor und Chlor, substituiert ist, oder Cyclopropyl, das unsubstituiert oder durch einen oder mehrere Reste aus der Gruppe bestehend aus Halogen und (C₁-C₄)Alkyl substituiert ist, insbesondere
(C₁-C₄)Alkyl, das unsubstituiert oder durch einen oder mehrere Reste aus der Gruppe Fluor und Chlor in 1-Position des Alkylrestes substituiert ist,
- R³: Cyclopropyl, das unsubstituiert oder durch einen oder mehrere Reste aus der Gruppe bestehend aus Halogen und (C₁-C₄)Alkyl, substituiert ist,
- R⁴ und R⁵: unabhängig voneinander jeweils (C₁-C₄)Alkyl, welches unsubstituiert oder durch einen oder mehrere Reste aus der Gruppe bestehend aus Halogen und (C₁-C₄)Alkoxy substituiert ist, oder
Cyclopropyl, das unsubstituiert oder durch einen oder mehrere Reste aus der Gruppe bestehend aus Halogen und (C₁-C₄)Alkyl substituiert ist, oder
- R⁴ und R⁵: gemeinsam mit dem an sie gebundenen C-Atom einen 4- bis 6-gliedrigen carbocyclischen Ring, vorzugsweise Cyclopropyl, der unsubstituiert oder durch einen oder mehrere Reste aus der Gruppe bestehend aus Halogen und (C₁-C₄)Alkyl substituiert ist,
- R⁶: Wasserstoff oder Methyl, ganz besonders Wasserstoff,
- R⁷: Wasserstoff, Methyl, Ethyl oder Cyclopropyl, das unsubstituiert oder durch einen oder mehrere Reste aus der Gruppe bestehend aus Halogen, (C₁-C₄)Alkyl und (C₁-C₄)Haloalkyl substituiert ist, insbesondere Wasserstoff, Methyl, Ethyl, Cyclopropyl, 1-Methyl-cyclopropyl, 1-Fluor-cyclopropyl, 1-Chlor-cyclopropyl, 2,2-Dimethylcyclopropyl, 2,2-Dichlorcyclopropyl, ganz besonders Wasserstoff,
bedeuten.

Bevorzugt sind Verbindungen der Formel (I-A) und deren Salze, 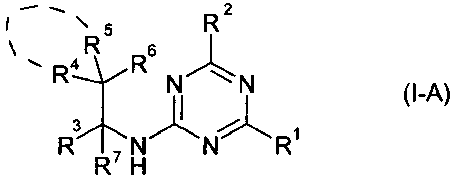 worin
R¹ bis R⁷ die genannte bzw. bevorzugt genannte Bedeutung haben und die Gruppen
R⁴ und R⁵ cyclisch verbunden sein können.

Von den Verbindungen (I-A) sind die Verbindungen der Formeln (Ia) (Ib), (Ic), (Id), (Ie) und (If) und deren Salze besonders bevorzugt. Dabei bedeuten:
Verbindungen der Formel (Ia) = Verbindungen der Formel (I-A), worin R¹ = Amino,
Verbindungen der Formel (Ib) = Verbindungen der Formel (I-A), worin R¹ = Acetylamino,
Verbindungen der Formel (Ic) = Verbindungen der Formel (I-A), worin R¹ = Propionylamino,
Verbindungen der Formel (Id) = Verbindungen der Formel (I-A), worin R¹ den Rest der Formel NH-CO-CHCl-CH₃ (2-Chlorpropionylamino) bedeutet,
Verbindungen der Formel (Ie) = Verbindungen der Formel (I-A), worin R¹ den Rest Dimethylamino-methylen-amino bedeutet,
Verbindungen der Formel (If) = Verbindungen der Formel (I-A), worin R¹ den Rest Morpholin-4-yl-methylen-amino bedeutet.

Dabei sind besonders die Verbindungen der allgemeinen Formeln (la) (Ib), (Ic), (Id), (Ie) und (If) bevorzugt, in welchen die Reste R¹ bis R⁷ die in den Beispieltabellen verwendeten Restebedeutungen aufweisen.

Die erfindungsgemäßen Verbindungen der Formel (I) und (I-A) umfassen alle Stereoisomeren, welche aufgrund der Asymmetriezentren oder Doppelbindungen im Molekül, deren Konfiguration in der Formel nicht speziell bezeichnet oder die nicht speziell angegeben sind, auftreten können, und deren Mischung, inklusive der racemischen Verbindungen und der teilweise mit bestimmten Stereoisomeren angereicherten Mischungen.

Die Erfindung umfasst auch alle Tautomeren, wie Keto- und Enol-Tautomeren, und deren Mischungen und Salze, wenn entsprechende funktionelle Gruppen vorhanden sind.

Gegenstand der Erfindung sind auch Verfahren zur Herstellung der Verbindungen der Formel (I) und deren Salze, dadurch gekennzeichnet, dass
a) eine Verbindung der Formel (II),

   R² - Fu (II)

   worin Fu eine funktionelle Gruppe aus der Gruppe Carbonsäureester, Carbonsäureorthoester, Carbonsäurechlorid, Carbonsäureamid, Carbonsäureanhydrid und Trichlormethyl bedeutet, mit einer Verbindung der Formel (III) oder einem Säureadditionssalz hiervon 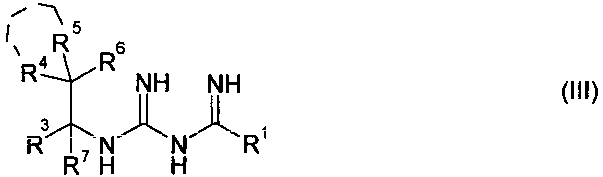 umsetzt oder
b) eine Verbindung der Formel (IV), 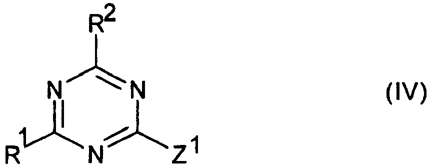 worin Z¹ einen austauschfähigen Rest oder eine Abgangsgruppe, z.B. Chlor, Trichlormethyl, (C₁-C₄)Alkylsulfonyl, unsubstituiertes oder substituiertes Phenyl-(C₁-C₄)alkylsulfonyl oder (C₁-C₄)Alkyl-phenylsulfonyl, bedeutet, mit einem geeigneten Amin der Formel (V) oder einem Säureadditionssalz hiervon 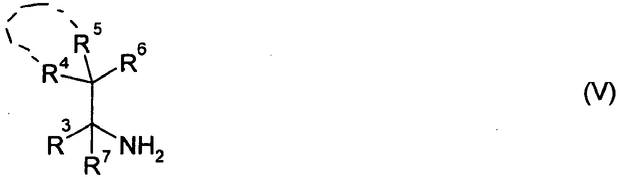 umsetzt oder
c) eine Verbindung der Formel (I') oder deren Salz, 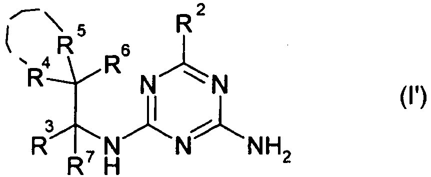 an der Aminogruppe zur Verbindung der Formel (I) derivatisiert,
wobei in den Formeln (II), (III), (IV), (V) und (I') die Reste R¹, R², R³, R⁴, R⁵, R⁶ und R⁷ wie in Formel (I) definiert sind.

Die Umsetzung der Verbindungen der Formel (II)und (III) erfolgt vorzugsweise basenkatalysiert in einem inerten organischen Lösungsmittel, wie z.B. Tetrahydrofuran (THF), Dioxan, Acetonitril, Dimethylformamid (DMF), Methanol und Ethanol, bei Temperaturen zwischen -10 °C und dem Siedepunkt des Lösungsmittel, vorzugsweise bei 20 °C bis 60 °C; falls Säureadditionssalze der Formel (III) verwendet werden, setzt man diese in der Regel mit Hilfe einer Base in situ frei. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei beispielsweise im Bereich von 0,1 bis 3 Moläquivalenten bezogen auf die Verbindung der Formel (III) eingesetzt. Die Verbindung der Formel (II)kann im Verhältnis zur Verbindung der Formel (III) beispielsweise äquimolar oder im Überschuß, in der Regel im Molverhältnis von (III):(II) bis zu 1:4, meist bis zu 1:3 eingesetzt werden. Analoge Verfahren sind aus der Literatur bekannt (vergleiche: Comprehensive Heterocyclic Chemistry, A.R. Katritzky, C.W. Rees, Pergamon Press, Oxford, New York, 1984, Vol.3; Part 2B; ISBN 0-08-030704-5, S.290).
Die Umsetzung der Verbindungen der Formel (IV) und (V) erfolgt vorzugsweise basenkatalysiert in einem inerten organischen Lösungsmittel, wie z.B. THF, Dioxan, Acetonitril, DMF, Methanol und Ethanol, bei Temperaturen zwischen -10 °C und dem Siedepunkt des jeweiligen Lösungsmittels oder Lösungsmittelgemisches, vorzugsweise bei 20 °C bis 60 °C, wobei die Verbindung (V), falls als Säureadditionssalz eingesetzt, gegebenenfalls in situ mit einer Base freigesetzt wird. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei in der Regel im Bereich von 1 bis 3 Moläquivalenten bezogen auf die Verbindung der Formel (IV) eingesetzt. Die Verbindung der Formel (IV) kann beispielsweise äquimolar zur Verbindung der Formel (V) oder mit bis zu 2 Moläquivalenten Überschuß eingesetzt werden. Analoge Verfahren sind aus der Literatur bekannt (vgl. Comprehensive Heterocyclic Chemistry, A.R. Katritzky, C.W. Rees, Pergamon Press, Oxford, New York, 1984, Vol.3; Part 2B; ISBN 0-08-030704-5, S. 482).

Die Edukte der Formeln (II), (III), (IV) und (V) sind entweder kommerziell erhältlich oder können nach oder analog literaturbekannten Verfahren hergestellt werden. Die Verbindungen der Formel (III) sind neu und ebenfalls Gegenstand der Erfindung. Die Verbindungen können beispielsweise auch nach einem der nachfolgend beschriebenen Verfahren hergestellt werden.
Die Verbindung der Formel (IV), oder eine direkte Vorstufe davon, läßt sich beispielweise wie folgt herstellen:
1. Durch Reaktion einer Verbindung der Formel (II) mit einem Amidino-thioharnstoff-Derivat der Formel (VI), 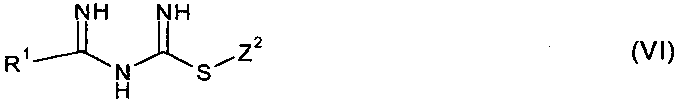 worin Z² (C₁-C₄)-Alkyl oder Phenyl-(C₁-C₄)-alkyl bedeutet und R¹ wie in Formel (I) definiert sind, werden Verbindungen der Formel (IV) erhalten, in denen Z¹ = -SZ² bedeutet.
2. Durch Umsetzung eines Amidins der Formel (VII) oder eines Säureadditionssalzes davon,

   H₂N-CR²=NH (VII)

   worin R² wie in Formel (I) definiert ist,
   mit einem N-Cyanodithioiminocarbonat der Formel (VIII),

   NC-N=C(S-Z³)₂ (VIII)

   worin Z³ (C₁-C₄)Alkyl oder Phenyl-(C₁-C₄)alkyl bedeutet, werden Verbindungen der Formel (IV) erhalten, worin Z¹ = -S-Z³ bedeutet.
3. Durch Umsetzung eines Alkali-dicyanamids mit einem Carbonsäurederivat der genannten Formel (II) werden Verbindungen der Formel (IV) erhalten, worin Z¹ = NH₂ bedeutet,
4. Durch Umsetzung von Trichloracetonitril mit einem Nitril der Formel (IX),

   R² - CN (IX)

   worin R² wie in Formel (I) definiert ist, werden zunächst Verbindungen der Formel (X), 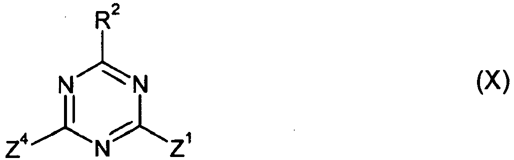 worin Z¹ und Z⁴ jeweils CCl₃ bedeuten, erhalten, welche durch nachfolgende Umsetzung mit Verbindungen der Formel H-R² (R² wie in Formel (I)), zu Verbindungen der Formel (IV), worin Z¹ = CCl₃ bedeutet, führen.

Die Umsetzung der Carbonsäurederivate der Formel (II) mit den Amidinothioharnstoff-Derivaten der Formel (VI) erfolgt vorzugsweise basenkatalysiert in einem organischen Lösungsmittel, wie z.B. Aceton, THF, Dioxan, Acetonitril, DMF, Methanol, Ethanol, bei Temperaturen von -10 °C bis zum Siedepunkt des Lösungsmittel, vorzugsweise bei 0 °C bis 20 °C. Die Umsetzung kann aber auch in Wasser oder in wässrigen Lösungsmitttelgemischen mit einem oder mehreren der oben genannten organischen Lösungsmitteln erfolgen. Falls die Verbindung der Formel (VI) als Säureadditionssalz eingesetzt wird, kann sie gegebenenfalls in situ mit einer Base freigesetzt werden. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei z.B. im Bereich von 1 bis 3 Moläquivalenten bezogen auf die Verbindung der Formel (VI) eingesetzt. Verbindungen der Formel (II) und (VI) können beispielsweise äquimolar oder im Überschuß, in der Regel im Molverhältnis von (VI):(II) bis zu 1:4, meist bis zu 1:3 eingesetzt werden. Analoge Verfahren sind literaturbekannt (vergl.: H. Eilingsfeld, H. Scheuermann, Chem. Ber.; 1967, 100, 1874).

Die Umsetzung der Amidine der Formel (VII) mit den N-Cyanodithioiminocarbonaten der Formel (VIII) erfolgt vorzugsweise basenkatalysiert in einem inerten organischen Lösungsmittel, wie z.B. Acetonitril, DMF, Dimethylacetamid (DMA), N-Methylpyrrolidon (NMP), Methanol und Ethanol, bei Temperaturen von -10 °C bis zum Siedepunkt des Lösungsmittels, vorzugsweise bei 20 °C bis 80 °C. Falls Verbindung (VII) als Säureadditionssalz eingesetzt wird, kann sie gegebenenfalls in situ mit einer Base freigesetzt werden. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei z. B. in Bereich von 1 bis 3 Moläquivalenten bezogen auf die Verbindung der Formel (VIII) eingesetzt, Verbindungen der Formel (VII) und (VIII) können in der Regel äquimolar oder mit 2 Moläquivalenten Überschuß an Verbindung der Formel (VII) eingesetzt werden. Analoge Verfahren sind literaturbekannt (vergl.: T.A. Riley, W.J. Henney, N.K. Dalley, B.E. Wilson, R.K. Robins; J. Heterocyclic Chem.; 1986, 23 (6), 1706-1714).

Die Herstellung von Zwischenprodukten der Formel (X) mit Z¹ = Chlor kann durch Reaktion von Alkali-dicyanamid mit einem Carbonsäurederivat der Formel (II), wobei dann Fu bevorzugt die funktionelle Gruppe Carbonsäurechlorid (-COCI, Chlorcarbonyl) oder Carbonsäureamid (-CONH₂, Aminocarbonyl, Carbamoyl) bedeutet, erfolgen. Die Umsetzung der Reaktionskomponenten erfolgt beispielsweise säurekatalysiert in einem inerten organischen Lösungsmittel wie z.B. Toluol, Chlorbenzol, chlorierten Kohlenwasserstoffen bei Temperaturen zwischen -10 °C und dem Siedepunkt des Lösungsmittels, vorzugsweise bei 20 °C bis 80 °C, wobei die entstehenden Intermediate in situ mit einem geeigneten Chlorierungsreagenz wie beispielsweise Phosphoroxychlorid chloriert werden können. Geeignete Säuren sind z.B. Halogenwasserstoffsäuren, wie HCl, oder auch Lewis-Säuren, wie z.B. AlCl₃ oder BF₃ (vergl. US-A-5095113, DuPont).

Die Herstellung von Zwischenprodukten der Formel (X) mit Z¹, Z⁴ = Trihalogenmethyl kann durch Reaktion der entsprechenden Trihalogenessigsäurenitrile mit einem Carbonsäurenitril der Formel (IX) erfolgen. Die Umsetzung der Reaktionskomponenten erfolgt beispielsweise säurekatalysiert in einem inerten organischen Lösungsmittel wie z.B. Toluol, Chlorbenzol, chlorierten Kohlenwasserstoffen bei Temperaturen zwischen -40 °C und dem Siedepunkt des Lösungsmittels, vorzugsweise bei -10 °C bis 30 °C. Geeignete Säuren sind z.B. Halogenwasserstoffsäuren wie HCl oder auch Lewis-Säuren wie z.B. AlCl₃ oder BF₃ (vgl. EP-A-130939, Ciba Geigy).

Zwischenprodukte der Formel (IV), worin Z¹ = (C₁-C₄)Alkylmercapto oder unsubstituiertes Phenyl-(C₁-C₄)-alkylmercapto ist, können in einem inerten organischen Lösungsmittel wie z.B. Toluol, Chlorbenzol, chlorierten Kohlenwasserstoffen oder anderen bei Temperaturen zwischen -40 °C und dem Siedepunkt des Lösungsmittel, vorzugsweise bei 20 °C bis 80 °C, mit einem geeigneten Chlorierungsreagenz wie z.B. elementarem Chlor oder Phosphoroxychlorid zu reaktionsfähigeren Chlortriazinen der Formel (IV), worin Z¹ = Cl ist, überführt werden (vgl. J.K. Chakrabarti, D.E. Tupper; Tetrahedron 1975, 31(16), 1879-1882).

Zwischenprodukte der Formel (IV), wobei Z¹ = (C₁-C₄)Alkylmercapto oder unsubstituiertes oder substituiertes Phenyl-(C₁-C₄)-alkylmercapto oder (C₁-C₄)Alkyl-phenylthio ist, können in einem geeigneten Lösungsmittel wie z.B. chlorierten Kohlenwasserstoffen, Essigsäure, Wasser, Alkoholen, Aceton oder Mischungen hiervon bei Temperaturen zwischen 0 °C und dem Siedepunkt des Lösungsmittels, vorzugsweise von 20 °C bis 80 °C, mit einem geeigneten Oxidationssreagenz wie z.B. m-Chlorperbenzoesäure, Wasserstoffperoxid, Kaliumperoxomonosulfat oxidiert werden (vergl.: T.A. Riley, W.J. Henney, N.K. Dalley, B.E. Wilson, R.K. Robins; J. Heterocyclic Chem.; 1986, 23 (6), 1706-1714).

Die Verbindungen der Formel (III) können aus Verbindungen der Formel (V) und/oder deren Säureaddukten durch Umsetzung mit Cyanoguaniden ("Dicyandiamid") der Formel (XI), 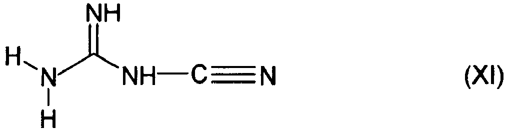 gegebenenfalls in Gegenwart eines Reaktionshilfsmittels, wie z.B. Hydrochlorid, und gegebenenfalls eines Verdünnungsmittels, wie z.B. n-Decan oder 1,2-Dichlorbenzol, bei Temperaturen von beispielsweise zwischen 100 °C und 200 °C (vgl. EP-A-492615, Herstellungsbeispiele) hergestellt werden.

Die Amine der Formel (V) können aus einfachen Strukturbausteinen als Vorstufen analog bekannten Methoden aufgebaut werden. Die Aminogruppe kann beispielsweise aus entsprechenden Ketonen durch reduktive Aminierung erhalten werden (vgl. oben genannnte Literatur, z. B. auf Seite 1 zu Aminotriazin-Herbiziden).

Die Herstellung der Verbindungen (I) aus Verbindungen (I') und deren Salzen nach Verfahrensvariante (c) können auf unterschiedliche Weise erfolgen, wobei im Prinzip Derivatisierungsreaktionen von Aminen in Frage kommen, beispielsweise Reaktionen bei denen Amine acyliert, in Imine und deren Derivate überführt, zu Amidinen, Harnstoffen oder Aminalen umgesetzt werden.

Die Verbindungen (I) können beispielsweise hergestellt werden, in dem analog den in WO 00/32580 beschriebenen Verfahren hergestellte Aminoverbindungen der Formel (I') mit reaktiven Carbonsäurederivaten, wie Anhydriden, Säurehalogeniden und aktivierten Estern, oder auch entsprechenden Säurederivaten von Sulfonsäuren oder Sulfinsäuren gemäß Standardbedingungen umgesetzt werden. Beispielsweise können die Aminoverbindungen (I') mit Carbonsäureanhydriden in äquimolarer Menge oder in bis zu zwanzigfachem Überschuss ohne Lösungsmittel oder in einem inerten Lösemittel bei 40 bis 150 °C zur Reaktion gebracht und zu acylierten Derivaten der Formel (I) umgesetzt werden. Analog lassen sich Umsetzungen mit Alkyl- oder Aryl-sulfonylhalogeniden oder -sulfinylhalogeniden zu acylierten Derivaten (I) durchführen, wobei die Acylgruppe im erstgenannten Fall dann eine Alkylsulfonyl oder Alkylsulfinylgruppe darstellt.

Andere Derivatisierungsreaktionen können beispielsweise mit Dialkyl- bevorzugt Dimethyl- oder Diethylacetalen in einem polaren Lösemittel wie beispielsweise Alkoholen, bevorzugt Methanol oder Ethanol bei 10° C bis zur Rückflusstemperatur des Lösemittel, durchgeführt werden, wobei die Reaktion des Amins (I') mit den Acetalen durch H⁺-erzeugende Reagenzien wie p-Toluolsulfonsäure katalysiert werden kann. Auf diese Weise können Alkylimidamide der Amine erhalten werden, wobei der Stickstoff des Amins die Doppelbindung der Imidgruppe trägt.

Ein weitere Derivatisierungsreaktion besteht in der Herstellung von Harnstoffen oder Thioharnstoffen mit Isocyanaten oder Isothiocyanaten, gegebenenfalls mit vorgeschalteter Reaktion mit einer Base wie beispielsweise Natriumhydrid, in einem geeigneten inerten Lösemittel wie Dimethylformamid bei -20 °C bis +60 °C bevorzugt bei 0 °C bis +30 °C.

Eine weitere Derivatisierungsreaktion führt zu Additionsprodukten im Sinne einer Michael-Addition mit einem Reaktanten mit einer Doppelbindung, beispielsweise durch Reaktion von Acrylnitril oder in einem inerten Lösemittel wie beispielsweise Acetonitril unter basischer Katalyse, zum Beispiel mit Kaliumhydroxid oder Triton B, bei Temperaturen von 20 bis 100 °C zu N-(2-Cyanoethyl)-Verbindungen, die unter physiologischen Bedingungen wieder abspaltbar sind.

Zur Herstellung der Säureadditionssalze der Verbindungen der Formel (I) kommen beispielsweise folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphthalindisulfonsäure. Die Säureadditionsverbindungen der Formel (I) können in einfacher Weise nach den üblichen Salzbildungsmethoden, z.B. durch Lösen einer Verbindung der Formel (I) in einem geeigneten organischen Lösungsmittel wie z.B. Methanol, Aceton, Methylenchlorid oder Benzin und Hinzufügen der Säure bei Temperaturen von 0 °C bis 100 °C erhalten werden und in bekannter Weise, z.B. durch Abfiltrieren, isoliert und gegebenfalls durch Waschen mit einem inerten organischen Lösemittel gereinigt werden.

Die Basenadditionssalze der Verbindungen der Formel (I) werden vorzugsweise in inerten polaren Lösungsmitteln wie z.B. Wasser, Methanol oder Aceton bei Temperaturen von 0 °C bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, Alkali- und Erdalkalihydride, z.B. NaH, Alkali- und Erdalalkoholate, z.B. Natriummethanolat, Kalium-tert.Butylat, oder Ammoniak oder Ethanolamin. Quartäre Ammoniumsalze können z.B. durch Umsalzung oder Kondensation mit quartären Ammoniumsalzen der Formel [NRR'R"R"']⁺X⁻, worin R, R', R" und R'" unabhängig voneinander (C₁-C₄)Alkyl,

Phenyl oder Benzyl bedeuten und X⁻ ein Anion, z.B. Cl⁻ bzw. OH⁻ ist, hergestellt werden.

Mit den in den vorstehenden Verfahrensvarianten bezeichneten "inerten Lösungsmitteln" (teilweise auch als Lösemittel bezeichnet) sind jeweils Lösungsmittel gemeint, die unter den jeweiligen Reaktionsbedingungen inert sind, jedoch nicht unter beliebigen Reaktionsbedingungen inert sein müssen.

Eine Kollektion aus Verbindungen der Formel (I), die nach den obengenannten Verfahren synthetisiert werden können, können zusätzlich in parallelisierter Weise hergestellt werden , wobei dies in manueller, teilweise automatisierter oder vollständig automatisierter Weise geschehen kann. Dabei ist es möglich, sowohl die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch S. H. DeWitt in "Annual Reports in Combinatorial Chemistry and Molecular Diversity: Automated Synthesis", Band 1, Verlag Escom, 1997, Seite 69 bis 77 beschrieben wird.

Zur parallelisierten Reaktionsdurchführung und Aufarbeitung können eine Reihe von im Handel erhältlichen Geräten verwendet werden wie sie beispielsweise von den Firmen Stem Corporation, Woodrolfe Road, Tollesbury, Essex, CM9 8SE, England oder H + P Labortechnik GmbH, Bruckmannring 28, 85764 Oberschleißheim, Deutschland angeboten werden. Für die parallelisierte Aufreinigung von Verbindungen (I) oder von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA. Die aufgeführten Apparaturen ermöglichen eine modulare Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise von Roboter bedient werden. Derartige Automationssysteme können zum Beispiel von der Firma Zymark Corporation, Zymark Center, Hopkinton, MA 01748, USA bezogen werden.

Neben den beschriebenen Methoden kann die Herstellung von Verbindungen (I) vollständig oder partiell durch festphasen-unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen unterstützte Synthesemethoden sind in der Fachliteratur hinreichend beschrieben, z. B.: Barry A. Bunin in "The Combinatorial Index", Verlag Academic Press, 1998.
Die Verwendung von Festphasen unterstützten Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci., 1985, 82, 5131 - 5135) mit Produkten der Firma IRORI, 11149 North Torrey Pines Road, La Jolla, CA 92037, USA teilweise automatisiert werden. Die Automatisierung von Festphasen unterstützter Parallelsynthese gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß den hier beschriebenen Verfahren liefert Verbindungen (I) in Form von Substanzkollektionen oder -bibliotheken. Gegenstand der vorliegenden Erfindung sind daher auch Bibliotheken der Verbindungen (I), die mindestens zwei Verbindungen (I) enthalten und deren Vorprodukten.

Die erfindungsgemäßen Verbindungen der Formel (I) und deren Salze, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im Einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.
Auf der Seite der monokotylen Unkrautarten werden z.B. Agrostis, Alopecurus, Apera, Avena, Brachicaria, Bromus, Dactyloctenium, Digitaria, Echinochloa, Eleocharis, Eleusine, Festuca, Fimbristylis, Ischaemum, Lolium, Monochoria, Panicum, Paspalum, Phalaris, Phleum, Poa, Sagittaria, Scirpus, Setaria, Sphenoclea sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.
Herbizide Wirkung wird auch erreicht bei dikotylen Schadpflanzen wie Ambrosia, Anthemis, Carduus, Centaurea, Chenopodium, Cirsium, Convolvulus, Datura, Emex, Galeopsis, Galinsoga, Kochia, Lepidium, Lindernia, Papaver, Portlaca, Polygonum, Ranunculus, Rorippa, Rotala, Seneceio, Sesbania, Solanum, Sonchus, Taraxacum, Trifolium, Urtica und Xanthium.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.
Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Soja, insbesondere Plantagenkulturen wie Ölpalme, Olive, Kokosnuss, Gummibaum, Zitrus, Ananas, Apfel, Birne, Kirsche, Baumwolle, Kaffee, Kakau, Wein und andere vergleichbare Obst und Plantagenkulturen nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen inklusive Zierpflanzungen. Auch eignen sich die Wirkstoffe, gegebenenfalls in Kombination mit anderen Wirkstoffen für die Anwendung im Nichtkulturland, wie auf Wegen, Plätzen, Beeten, Rasenflächen, Bahndämmen, Industrieanlagen zur Bekämpfung von unerwünschtem Pflanzenwuchs.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Emteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärfcegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel (I) oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.
Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.
Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 424-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Herbizide aus der Gruppe der Imidazolinone, Sulfonytharnstoffe, Glufosinate-ammonium oder Glyphosateisopropylammonium und analoge Wirkstoffe resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen (I) als Herbizide zur Bekämpfung von Schadpflanzen in transgenen Kulturpflanzen.

Die erfindungsgemäße Verwendung zur Bekämpfung von Schadpflanzen oder zur Wachstumsregulierung von Pflanzen schließt auch den Fall ein, bei dem der Wirkstoff der Formel (I) oder dessen Salz erst nach der Ausbringung auf der Pflanze, in der Pflanze oder im Boden aus einer Vorläufersubstanz ("Prodrug") gebildet wird. Beispielsweise wird angenommen, dass in den meisten Fällen die Verbindungen der Formel (I), worin R¹ von Amino verschieden ist, unter den physiologischen Bedingungen der Pflanzen zu Verbindungen der Formel (I'), d. h. Verbindungen der Formel (I), worin R¹ Amino bedeutet, metabolisiert werden. Diese Metabolite sind dann ebenfalls oder sogar primär herbizid wirksam. Erstere können dann als "Prodrugs" im beschriebenen Sinne verstanden werden.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.
Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und

Luftstrahimühien feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.
Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die Verbindungen der Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.
Als Kombinationspartner für die erfindungsgemäßen Wirkstoffe in Mischungsformulierungen oder im Tank-Mix sind beispielsweise bekannte Wirkstoffe, die auf einer Inhibition von beispielsweise Acetolactat-Synthase, Acetyl-Coenzyl-A-Carboxylase, PS I, PS II, HPPDO, Phytoene-Desaturase, Protoporphyrinogen-Oxidase, Glutamine-Synthetase, Cellulosebiosynthese, 5-Enolpyruvylshikimat-4-phosphat-Synthetase beruhen, einsetzbar. Solche Verbindungen und auch andere einsetzbare Verbindungen mit teilweise unbekanntem oder anderem Wirkungsmechanismus sind z.B. in Weed Research 26, 441-445 (1986), oder "The Pesticide Manual", 14. Auflage 2006, herausgegeben vom British Crop Protection Council, (im Folgenden auch kurz "PM"), und dort zitierter Literatur beschrieben. Als literaturbekannte Herbizide, Pflanzenwachstumsregulatoren und Herbizid-Safenern, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet): acetochlor; acibenzolar-S-methyl, acifluorfen(-sodium); aclonifen; AD-67, AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim(-sodium); ametryn; amicarbazone, amidochlor, amidosulfuron; aminopyralid, amitrole; AMS, d.h. Ammoniumsulfamat; ancymidol, anilofos; asulam; atrazine; aviglycine, azafenidin, azimsulfuron (DPX-A8947); aziprotryn; barban; BAS 516 H, d.h. 5-Fluor-2-phenyl-4H-3,1-benzoxazin-4-on; beflubutamid, benazolin(-ethyl); bencarbzone, benfluralin; benfuresate; benoxacor, bensulfuron(-methyl); bensulide; bentazone; benzfendizone, benzobicyclon, benzofenap; benzofluor; benzoylprop(-ethyl); benzthiazuron; bialaphos; bifenox; bilanafos (bialaphos), bispyribac(-sodium), borax, bromacil; bromobutide; bromofenoxim; bromoxynil; bromuron; buminafos; busoxinone; butachlor; butafenacil, butamifos; butenachlor; buthidazole; butralin; butroxydim, butylate; cafenstrole (CH-900); carbetamide; carfentrazone(-ethyl) (ICI-A0051); caloxydim, CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, 2-Chlor-5-[2,6-dioxo-4-(trifluor-methyl)-3,6-dihydropyrimidin-1(2H)-yl]-4-fluor-N-[methyl(1-methylethyl)-sulfamoyl]-benzamid (WO 2001/083459), [[4-[2-Chlor-5-[3,6-dihydro-4-methyl-2,6-dioxo-4-(trifluormethyl)-1 (2H)-pyrimidinyl]-4-fluorphenoxy]-2-pyridinyl]oxy]-essigsäureethylester (SYN-523) (WO 2006/061562, EP 1122244), chlorflurenol(-methyl), chlormequat (-chloride), chlormesulon (ICI-A0051); chlorbromuron; chlorbufam; chlorfenac; chloridazon; chlorimuron(-ethyl); chlornitrofen; chlorotoluron; chloroxuron; chlorpropham; chlorsulfuron; chlorthal-dimethyl; chlorthiamid; chlortoluron, cinidon(-methyl und -ethyl), cinmethylin; cinosulfuron, clefoxydim, clethodim; clodinafop und dessen Esterderivate (z.B. clodinafop-propargyl); clofencet, clomazone; clomeprop; cloprop, cloproxydim; clopyralid; clopyrasulfuron(-methyl), cloquintocet(-mexyl), cloransulam(-methyl), cumyluron (JC 940); cyanamide, cyanazine; cyclanilide, cycloate; cyclosulfamuron (AC 104); cycloxydim; cycluron; cyhalofop und dessen Esterderivate (z.B. Butylester, DEH-112); cyperquat; cyprazine; cyprazole; cyprosulfamide, daimuron; 2,4-D, 2,4-DB; 2,4-DB, dalapon; daminozide, dazomet, n-decanol, desmedipham; desmetryn; di-allate; dicamba; dichlobenil; dichlormid, dichlorprop(-P) (-salze); diclofop und dessen Ester wie diciofop-methyl; diclosulam, diethatyl(-ethyl); difenoxuron; difenzoquat(-metilsulfate); diflufenican; diflufenzopyr, dimefuron; dimepiperate, dimethachlor; dimethametryn; dimethazone, dimethenamid (SAN-582H); dimethenamid-P, dimethylarsinic acid, dimexyflam, dimethipin; dimetrasulfuron, dinitramine; dinoseb; dinoterb; diphenamid; dipropetryn; diquat (-salze); dithiopyr; diuron; DNOC; eglinazine-ethyl; EL 77, d.h. 5-Cyano-1-(1,1-dimethylethyl)-N-methyl-1H-pyrazole-4-carboxamid; endothal; epoprodan, EPTC; esprocarb; ethalfluralin; ethametsulfuron-methyl; ethephon, ethidimuron; ethiozin; ethofumesate; ethoxyfen und dessen Ester (z.B. Ethylester, HN-252); ethoxysulfuron, etobenzanid (HW 52); F5231, d.h. N-[2-Chlor-4-fluor-5-[4-(4-fluorpropyl)-4,5-dihydro-5-oxo-1H-tetrazol-1-yl]-phenyl]-ethansulfonamid; fenchlorazole(-ethyl), fenclorim, fenoprop; fenoxan, fenoxaprop und fenoxaprop-P sowie deren Ester, z.B. fenoxaprop-P-ethyl und fenoxaprop-ethyl; fenoxydim; fentrazamide, fenuron; ferrous sulfate, flamprop(-methyl oder -isopropyl oder -isopropyl-L); flazasulfuron; floazulate, florasulam, fluazifop und fluazifop-P und deren Ester, z.B. fluazifop-butyl und fluazifop-P-butyl; fluazolate, flucarbazone(-sodium), flucetosulfuron, fluchloralin; flufenacet, flufenpyr(-ethyl), flumetralin, flumetsulam; flumeturon; flumiclorac(-pentyl), flumioxazin (S-482); flumipropyn; fluometuron, fluorochloridone, fluorodifen; fluoroglycofen(-ethyl); flupoxam (KNW-739); flupropacil (UBIC-4243); flupropanoate, flupyrsulfuron(-methyl)(-sodium), flurazole, flurenol(-butyl), fluridone; flurochloridone; fluroxypyr(-meptyl); flurprimidol, flurtamone; fluthiacet(-methyl), fluthiamide, fluxofenim, fomesafen; foramsulfuron, forchlorfenuron, fosamine; furilazole, furyloxyfen; gibberellic acid, glufosinate(-ammonium); glyphosate(-isopropylammonium); halosafen; halosulfuron(-methyl) und dessen Ester (z.B. Methylester, NC-319); haloxyfop und dessen Ester; haloxyfop-P (= R-haloxyfop) und dessen Ester; HC-252, hexazinone; imazamethabenz(-methyl); imazamox, imazapic, imazapyr; imazaquin und Salze wie das Ammoniumsalz; imazamethapyr, imazapic, imazethamethapyr; imazethapyr; imazosulfuron; inabenfide, indanofan, indol-4-ylacetic acid, 4-indol-4-ylbutyric acid, iodosulfuron-methyl(-sodium), ioxynil; isocarbamid; isopropalin; isoproturon; isouron; isoxaben; isoxachlortole, isoxadifen(-ethyl), isoxaflutole, isoxapyrifop; karbutilate; lactofen; lenacil; linuron; maleic hydrazide, MCPA; MCPB; mecoprop(-P), mefenacet; mefenpyr-diethyl, mefluidid; mepiquat (chloride), mesotrione, mesosulfuron(-methyl), mesotrione, metam, metamifop, metamitron; metazachlor; methabenzthiazuron; methazole; methoxyphenone; methylarsonic acid, 1-methyl-cyclopropene, methyldymron; methyl isothiocyanate, metobenzuron, metobromuron; (alpha-)metolachlor; metosulam (XRD 511); metoxuron; metribuzin; metsulfuron-methyl; MH; MK-616, molinate; monalide; monocarbamide dihydrogensulfate; monolinuron; monuron; MT 128, d.h. 6-Chlor-N-(4-chlor-2-propenyl)-5-methyl-N-phenyl-4-pyridazinamin; MT 5950, d.h. N-[4-Chlor-4-(1-methylethyl)-phenyl]-2-methylpentanamid; 2-(1-naphthyl)acetamide, 1-naphthylacetic acid, 2-naphthyloxyacetic acid, naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrophenolate mixture, nitrofluorfen; nonanoic acid, norflurazon; orbencarb; orthasulfamuron, oryzalin; oxabetrinil, oxadiargyl (RP-020630); oxadiazon; oxasulfuron, oxaziclomefone, oxyfluorfen; paclobutrazol, paraquat(dichloride); pebulate; pelargonic acid, pendimethalin; penoxulam, pentachlorophenol, pentanochlor, pentoxazone, perfluidone; pethoxamid, phenisopham; phenmedipham; picloram; picolinafen, pinoxaden, piperophos; piributicarb; pirifenop-butyl; pretilachlor; primisulfuron(-methyl); probenazole, procarbazone-(sodium), procyazine; prodiamine; profluralin; profoxydim, prohexadione(-calcium), prohydrojasmon, proglinazine(-ethyl); prometon; prometryn; propachlor; propanil; propaquizafop und dessen Ester; propazine; propham; propisochlor; propoxycarbazone(-sodium), n-propyl dihydrojasmonate, propyzamide; prosulfalin; prosulfocarb; prosulfuron (CGA-152005); prynachlor; pyraclonil, pyraflufen(-ethyl), pyrasulfotole, pyrazolynate; pyrazon; pyrazosulfuron(-ethyl); pyrazoxyfen; pyribenzoxim, pyributicarb, pyridafol, pyridate; pyriftalid, pyriminobac(-methyl), pyrimisulfan, pyrithiobac(-sodium) (KIH-2031); pyroxasulfone, pyroxofop und dessen Ester (z.B. Propargylester); pyroxulam, quinclorac; quinmerac; quinoclamine, quinofop und dessen Esterderivate, quizalofop und quizalofop-P und deren Esterderivate z.B. quizalofop(-ethyl); quizalofop(-P-tefuryl und -ethyl); renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; sintofen, SN 106279, d.h. 2-[[7-[2-Chlor-4-(trifluormethyl)-phenoxy]-2-naphthalenyl]-oxy]-propansäure und -methylester; sulcotrione, sulfentrazone (FMC-97285, F-6285); sulfazuron; sulfometuron(-methyl); sulfosate (ICI-A0224); sulfosulfuron, 2,3,6-TBA, TCA; tebutam (GCP-5544); tebuthiuron; tecnazene, tefurlyltrione, tembotrione, tepraloxydim, terbacil; terbucarb; terbuchlor; terbumeton; terbuthylazine; terbutryn; TFH 450, d.h. N,N-Diethyl-4-[(2-ethyl-6-methylphenyl)-sulfonyl]-1H-1,2,4-triazol-1-carboxamid; thenylchlor (NSK-850); thiafluamide, thiazafluron; thiazopyr (Mon-13200); thidiazimin (SN-24085); thidiazuron, thiencarbazone, thifensulfuron(-methyl); thiobencarb; TI-35, tiocarbazil; topramezone, tralkoxydim; tri-allate; triasulfuron; triaziflam, triazofenamide; tribenuron(-methyl); triclopyr; tridiphane; trietazine; trifloxysulfuron(-sodium), trifluralin; triflusulfuron und Ester (z.B. Methylester, DPX-66037); trimeturon; trinexapac(-ethyl), tritosulfuron, tsitodef; uniconazole, vemolate; WL 110547, d.h. 5-Phenoxy-1-[4-(trifluormethyl)-phenyl]-1H-tetrazol; BAY MKH 6561, UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Von besonderem Interesse ist die selektive Bekämpfung von Schadpflanzen in Kulturen von Nutz- und Zierpflanzen. Obgleich die erfindungsgemäßen Verbindungen (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen (I) von besonderem Interesse, welche die Verbindungen (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide. Folgende Gruppen von Verbindungen kommen beispielsweise als Safener für die Verbindungen (I) und deren Kombinationen mit weiteren Pestiziden in Frage:
a) Verbindungen vom Typ der Dichlorphenylpyrazolin-4-carbonsäure, vorzugsweise Verbindungen wie
   1-(2,4-Dichlorphenyl)-5-(ethoxycarbonyl)-5-methyl-2-pyrazolin-4-carbonsäureethylester (S1-1) ("Mefenpyr-diethyl", PM), und verwandte Verbindungen, wie sie in der WO 91/07874 beschrieben sind,
b) Derivate der Dichlorphenylpyrazolcarbonsäure, vorzugsweise Verbindungen wie 1-(2,4-Dichlorphenyl)-5-methyl-pyrazol-4-carbonsäureethylester (S1-2), 1-(2,4-Dichlorphenyl)-5-isopropyl-pyrazol-4-carbonsäureethylester (S1-3), 1-(2,4-Dichlorphenyl)-5-(1,1-dimethyl-ethyl)pyrazol-4-carbonsäureethyl-ester (S1-4), 1-(2,4-Dichlorphenyl)-5-phenyl-pyrazol-4-carbonsäureethylester (S1-5) und verwandte Verbindungen, wie sie in EP-A-333 131 und EP-A-269 806 beschrieben sind.
c) Verbindungen vom Typ der Triazolcarbonsäuren, vorzugsweise Verbindungen wie Fenchlorazol(-ethylester), d.h.
   1-(2,4-Dichlorphenyl)-5-trichlormethyl-(1H)-1,2,4-triazol-3 carbonsäureethylester (S1-6), und verwandte Verbindungen EP-A-174 562 und EP-A-346 620);
d) Verbindungen vom Typ der 5-Benzyl- oder 5-Phenyl-2-isoxazolin-4-carbonsäure, oder der 5,5-Diphenyl-2-isoxazolin-4-carbonsäure vorzugsweise Verbindungen, wie 5-(2,4-Dichlorbenzyl)-2-isoxazolin-4-carbonsäureethylester (S1-7) oder 5-Phenyl-2-isoxazolin-4-carbonsäureethylester (S1-8) und verwandte Verbindungen, wie sie in WO 91/08202 beschrieben sind, bzw. der 5,5-Diphenyl-2-isoxazolin-carbonsäureethylester (S1-9) ("Isoxadifen-ethyl") oder -n-propylester (S1-10) oder der 5-(4-Fluorphenyl)-5-phenyl-2-isoxazolin-4-carbonsäureethylester (S1-11), wie sie in der deutschen Patentanmeldung (WO-A-95/07897) beschrieben sind.
e) Verbindungen vom Typ der 8-Chinolinoxyessigsäure (S2), vorzugsweise
   (5-Chlor-8-chinolinoxy)-essigsäure-(1-methyl-hex-1-yl)-ester (Common name "Cloquintocet-mexyl" (S2-1) (siehe PM)
   (5-Chlor-8-chinolinoxy)-essigsäure-(1,4-dimethyl-but-1-yl)-ester (S2-2),
   (5-Chlor-8-chinolinoxy)-essigsäure-4-allyl-oxy-butylester (S2-3),
   (5-Chlor-8-chinolinoxy)-essigsäure-1-allyloxy-prop-2-ylester (S2-4),
   (5-Chlor-8-chinolinoxy)-essigsäureethylester (S2-5),
   (5-Chlor-8-chinolinoxy)-essigsäuremethylester (S2-6),
   (5-Chlor-8-chinolinoxy)-essigsäureallylester (S2-7),
   (5-Chlor-8-chinolinoxy)-essigsäure-2-(2-propyliden-iminoxy)-1-ethylester (S2-8),
   (5-Chlor-8-chinolinoxy)-essigsäure-2-oxo-prop-1-ylester (S2-9)
   und verwandte Verbindungen, wie sie in EP-A-86 750, EP-A-94 349 und EP-A-191 736 oder EP-A-0 492 366 beschrieben sind.
f) Verbindungen vom Typ der (5-Chlor-8-chinolinoxy)-malonsäure, vorzugsweise Verbindungen wie (5-Chlor-8-chinolinoxy)-malonsäurediethylester, (5-Chlor-8-chinolinoxy)-malonsäurediallylester, (5-Chlor-8-chinolinoxy)-malonsäure-methyl-ethylester und verwandte Verbindungen, wie sie in EP-A-0 582 198 beschrieben sind.
g) Wirkstoffe vom Typ der Phenoxyessig- bzw. -propionsäurederivate bzw. der aromatischen Carbonsäuren, wie z.B. 2,4-Dichlorphenoxyessigsäure(ester) (2,4-D), 4-Chlor-2-methyl-phenoxy-propionester (Mecoprop), MCPA oder 3,6-Dichlor-2-methoxy-benzoesäure(ester) (Dicamba).
h) Wirkstoffe vom Typ der Pyrimidine, die als bodenwirksame Safener in Reis angewendet werden, wie z. B.
   "Fenclorim" (PM) (= 4,6-Dichlor-2-phenylpyrimidin), das als Safener für Pretilachlor in gesätem Reis bekannt ist,
i) Wirkstoffe vom Typ der Dichloracetamide, die häufig als Vorauflaufsafener (bodenwirksame Safener) angewendet werden, wie z. B.
   "Dichlormid" (PM) (= N,N-Diallyl-2,2-dichloracetamid),
   "R-29148" (= 4-Dichloracetyl-2,2,5-trimethyl-1,4-oxazolidin von der Firma Stauffer),
   "Benoxacor" (PM) (= 4-Dichloracetyl-3,4-dihydro-4-methyl-2H-1,4-benzoxazin).
   "PPG-1292" (= N-Allyl-N-[(1,4-dioxolan-2-yl)-methyl]-dichloracetamid von der Firma PPG Industries),
   "DK-24" (= N-Allyl-N-[(allylaminocarbonyl)-methyl]-dichloracetamid von der Firma Sagro-Chem),
   "AD-67" oder "MON 4660" (= 4-Dichloracetyl-1-oxa-4-aza-spiro[4,5]decan von der Firma Nitrokemia bzw. Monsanto),
   "Diclonon" oder "BAS145138" oder "LAB145138" (= (= 4-Dichloracetyl-2,5,5-trimethyl-1,4-diazabicyclo[4.3.0]nonan von der Firma BASF) und
   "Furilazol" oder "MON 13900" (siehe PM) (= (RS)-4-Dichloracetyl-5-(2-furyl)-2,2-dimethyloxazolidin)
j) Wirkstoffe vom Typ der Dichloracetonderivate, wie z. B.
   "MG 191" (CAS-Reg. Nr. 96420-72-3) (= 2-Dichlormethyl-2-methyl-1,4-dioxolan von der Firma Nitrokemia), das als Safener für Mais bekannt ist,
k) Wirkstoffe vom Typ der Oxyimino-Verbindungen, die als Saatbeizmittel bekannt sind, wie z. B.
   "Oxabetrinil" (PM) (= (Z)-1,4-Dioxolan-2-ylmethoxyimino(phenyl)acetonitril), das als Saatbeiz-Safener für Hirse gegen Schäden von Metolachlor bekannt ist,
   "Fluxofenim" (PM) (= 1-(4-Chlorphenyl)-2,2,2-trifluor-1-ethanon-O-(1,4-dioxolan-2-ylmethyl)-oxim, das als Saatbeiz-Safener für Hirse gegen Schäden von Metolachlor bekannt ist, und
   "Cyometrinil" oder "-CGA-43089" (PM) (= (Z)-Cyanomethoxyimino(phenyl)acetonitril), das als Saatbeiz-Safener für Hirse gegen Schäden von Metolachlor bekannt ist,
l) Wirkstoffe vom Typ der Thiazolcarbonsäureester, die als Saatbeizmittel bekannt sind, wie z. B.
   "Flurazol" (PM) (= 2-Chlor-4-trifluormethyl-1,4-thiazol-5-carbonsäurebenzylester), das als Saatbeiz-Safener für Hirse gegen Schäden von Alachlor und Metolachlor bekannt ist,
m) Wirkstoffe vom Typ der Naphthalindicarbonsäurederivate, die als Saatbeizmittel bekannt sind, wie z. B.
   "Naphthalic anhydrid" (PM) (= 1,8-Naphthalindicarbonsäureanhydrid), das als Saatbeiz-Safener für Mais gegen Schäden von Thiocarbamatherbiziden bekannt ist,
n) Wirkstoffe vom Typ Chromanessigsäurederivate, wie z. B.
   "CL 304415" (CAS-Reg. Nr. 31541-57-8) (= 2-(4-Carboxy-chroman-4-yl)-essigsäure von der Firma American Cyanamid), das als Safener für Mais gegen Schäden von Imidazolinonen bekannt ist,
o) Wirkstoffe, die neben einer herbiziden Wirkung gegen Schadpflanzen auch Safenerwirkung an Kulturpflanzen wie Reis aufweisen, wie z. B.
   "Dimepiperate" oder "MY-93" (PM) (= Piperidin-1-thiocarbonsäure-S-1-methyl-1-phenylethylester), das als Safener für Reis gegen Schäden des Herbizids Molinate bekannt ist,
   "Daimuron" oder "SK 23" (PM) (= 1-(1-Methyl-1-phenylethyl)-4-p-tolyl-harnstoff), das als Safener für Reis gegen Schäden des Herbizids Imazosulfuron bekannt ist,
   "Cumyluron" = "JC-940" (= 4-(2-Chlorphenylmethyl)-1-(1-methyl-1-phenylethyl)-harnstoff, siehe JP-A-60087254), das als Safener für Reis gegen Schäden einiger Herbizide bekannt ist,
   "Methoxyphenon" oder "NK 049" (= 3,3'-Dimethyl-4-methoxy-benzophenon), das als Safener für Reis gegen Schäden einiger Herbizide bekannt ist, "CSB" (= 1-Brom-4-(chlormethylsulfonyl)-benzol) (CAS-Reg. Nr. 54091-06-4 von Kumiai), das als Safener gegen Schäden einiger Herbizide in Reis bekannt ist,
p) N-Acylsulfonamide der Formel (S3) und ihre Salze, 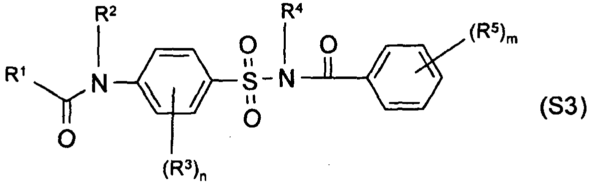 wie sie in WO-A-97/45016 beschrieben sind,
q) Acylsulfamoylbenzoesäureamide der allgemeinen Formel (S4), gegebenenfalls auch in Salzform, 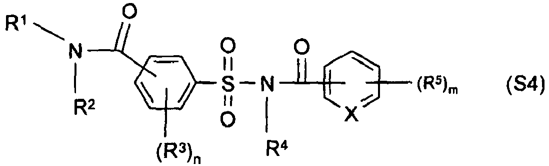 wie sie in der Internationalen Anmeldung Nr. PCT/EP98/06097 beschrieben sind, beispielsweise "cyprosulfamide" (S4-1) und
r) Verbindungen der Formel (S5), 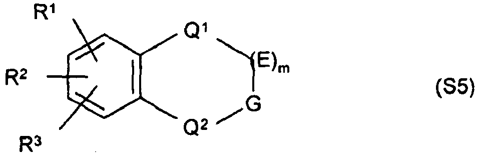
wie sie in der WO-A 98/13 361 beschrieben sind,
einschließlich der Stereoisomeren und den in der Landwirtschaft gebräuchlichen Salzen.

Von besonderem Interesse sind unter den genannten Safenern sind (S1-1), (S1-9), (S2-1) und (S4-1).
Einige der Safener sind bereits als Herbizide bekannt und entfalten somit neben der Herbizidwirkung bei Schadpflanzen zugleich auch Schutzwirkung bei den Kulturpflanzen.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

In den folgenden Beispielen beziehen sich Mengenangaben (auch Prozentangaben) auf das Gewicht, sofern nichts anderes speziell angegeben ist. Die im Rahmen der Beschreibung und der Beispiele verwendeten Bezeichnungen "R" und "S" für die absolute Konfiguration am jeweiligen Chiralitätszentrum der Stereoisomeren der Formel (I) folgt der RS-Nomenklatur nach der Cahn-Ingold- Prelog-Regel.

### (A) Chemische Beispiele

In den Formeln der Schemata zu den Beispielen 1 bis 6 wird die Gruppe Methyl als "Me" abgekürzt.

### Beispiel 1: Keton-Synthese (Vorprodukt) über eine Addition einer Grignard-Verbindung an ein Nitril

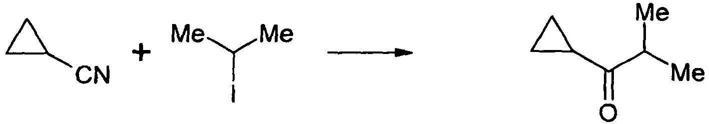

4,43 g (0,18 mol) Magnesium wurden in 10 ml trockenem Diethylether unter Schutzgas vorgelegt. Von insgesamt 28,15 g (0,17 mol) 2-lodpropan wurde eine kleine Portion zugegeben, die Reaktion sprang daraufhin an. Der Rest des 2-lodpropans wurde mit 90 ml trockenem Diethylether verdünnt und dann langsam zugetropft, so dass das Reaktionsgemisch die Siedetemperatur erreichte. Der Inhalt wurde eine Stunde unter Rückfluss erhitzt und anschließend auf 0-4 °C abgekühlt. Bei dieser Temperatur wurde eine Lösung von 10,0 g (0,15 mol) Cyclopropylcyanid in 100 ml trockenem Dietylether zugetropft, und anschließend wurde das Reaktionsgemisch 2 h unter Rückfluss erhitzt.
Das Reaktionsgemisch wurde im Eisbad abgekühlt, und bei 5°C wurden langsam 80 ml 10%-ige Salzsäure zugetropft. Die zwei Phasen wurden getrennt, die wässrige Phase wurde zweimal mit Diethylether extrahiert, die drei organischen Phasen wurden vereinigt, mit einer gesättigten Natriumchlorid-Lösung extrahiert, und anschließend wurden die organischen Phasen über Natriumsulfat getrocknet und das Filtrat unter reduziertem Druck vom Lösungsmittel befreit.

Es wurden 7,07 g (Ausbeute: 42 %) des Cyclopropyl-isopropyl-ketons isoliert, die Struktur wird von einem ¹H-NMR-Spektrum bestätigt.

### Beispiel 2: Cyclopropanierung von Cyclopentenon zu einem Keton-Vorprodukt

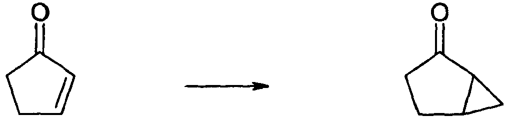

12,28 g (80 % Reinheit, 0,41 mol) Natriumhydrid werden in 100 ml Dimethylsulfoxid unter Schutzgas vorgelegt. Anschließend werden 28,0 g (0,34 mol) Cyclopent-2-en-1-on und 90.06 g (0,41 mol) Trimethyloxosulfoniumiodid zugegeben. Das Reaktionsgemisch wurde über Nacht bei Raumtemperatur gerührt und schließlich mit Wasser versetzt. Das Gemisch wurde zweimal mit Methyl-tert-butylether extrahiert, und die vereinigten organischen Phasen wurden mit gesättigter wässriger Natriumchlorid- Lösung extrahiert. Die organische Phase wurde über Magnesiumsulfat getrocknet, und das Filtrat wurde am Rotationsverdampfer eingeengt. Der Rückstand wurde im Vakuum rektifiziert, wobei 10,1 g des gewünschten Ketons als farblose Flüssigkeit erhalten wurden.

Das Keton konnte analog den nachfolgenden Beispielen zunächst über eine reduktive Aminierung zum entsprechenden Amin und das Amin anschließend zum gewünschten, entsprechend substituierten 2,4-Diamino-1,3,5-triazin erfolgreich umgesetzt werden.

### Beispiel 3 Herstellung eines Amins durch reduktive Aminierung (Vorprodukt)

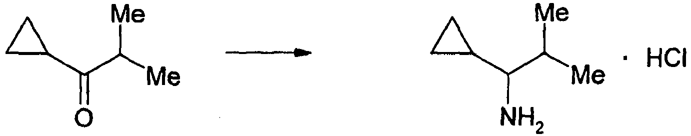

Eine Lösung von 1,0 g (8,9 mmol) Cyclopropylisopropylketon in 70 ml Methanol wurde mit 17,2 g (0,22 mol) Ammoniumacetat versetzt und 5 min. bei Raumtemperatur gerührt. Danach wurden 1,7 g (26,7 mmol) Natriumcyanoborhydrid zugegeben. Das Gemisch wurde mehrere Tage bei Raumtemperatur gerührt und anschließend mit 1 M Kalilauge versetzt. Der Kolbeninhalt wurde 10 min. gerührt, anschließend wurden 40 ml 20%-ige Natronlauge zugegeben. Das Gemisch wurde mit Essigsäureethylester extrahiert. Die organische Phase wurde mit Salzsäure angesäuert, die wässrige Phase wurde abgetrennt und nach Zugabe von Essigsäureethylester unter Kühlung mit Natronlauge alkalisiert. Nach der Extraktion mit Essigsäureethylester wurden die organischen Phasen über Magnesiumsulfat getrocknet, und das Filtrat wurde unter Kühlung mit 15 ml 1,25 M ethanolischer HCl-Lösung versetzt. Das Gemisch wurde 30 min. gerührt und anschließend unter reduziertem Druck vom Lösungsmittel befreit. Es wurden 0,6 g des 1-Cyclopropyl-2-methyl-propylamins in Form seines Hydrochlorids isoliert (Ausbeute: 45 %). Ein ¹H-NMR-Spektrum bestätigt die Struktur.

### Beispiel 4 Herstellung von 4-Amino-2-(1-cyclopropyl-2-methyl-propylamino)-6-(R,S-1-fluor-ethyl)-1,3,5-triazin

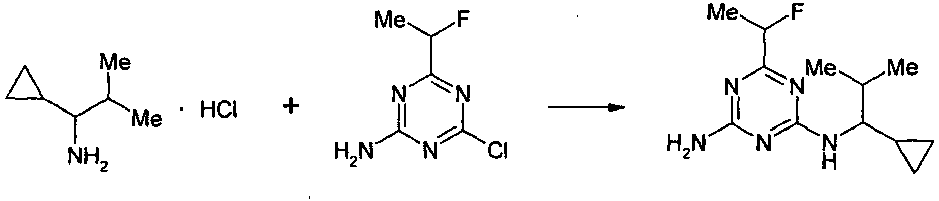

300 mg (2,0 mmol) 1-Cyclopropyl-2-methylpropylamin-Hydrochlorid, 354 mg (2,0 mmol) 2-Amino-4-chlor-6-(1-fluorethyl)-1,3,5-triazin und 609 mg (4,4 mmol) Kaliumcarbonat wurden in 15 ml Acetonitril vorgelegt und 16 h auf 80 °C erhitzt. Das Reaktionsgemisch wurde über Celite abgesaugt, und der Rückstand wurde mit Essigsäureethylester gewaschen. Das Filtrat wurde unter reduziertem Druck vom Lösungsmittel befreit. Der Rückstand wurde chromatographisch gereinigt, wobei 83 mg (Ausbeute: 16,4 %) des gewünschten Produkts erhalten wurden. Ein ¹H-NMR-sowie ein Massenspektrum bestätigen die Struktur.

### Beispiel 5 Herstellung von Dicyclopropyl-methyl-biguanid

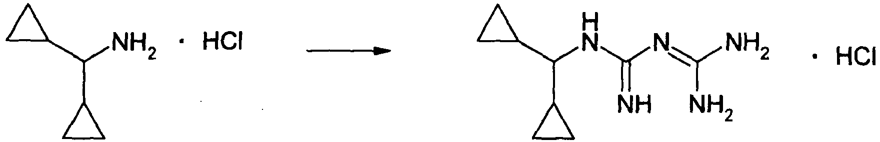

1,6 g (10,8 mmol) 1,1-Dicyclopropylmethylamin-Hydrochlorid wurden in einem Gemisch aus 6 ml Decan und 20 ml Toluol mit 0,91 g (10,8 mmol) Cyanoguanidin versetzt. Das Gemisch wurde 5 h auf eine Temperatur von 140 °C erhitzt, wobei Toluol abdestillierte. Der Kolbeninhalt wurde auf Raumtemperatur abgekühlt, anschließend wurde Aceton zugegeben. Der Niederschlag wurde abdekantiert und unter reduziertem Druck von restlichem Lösungsmittel befreit. Es wurden 1,7 g des Rohprodukts (Reinheit ca. 80 Gew.-%) erhalten, die ohne weitere Aufreinigung für die nächste Synthese-Stufe eingesetzt wurden.

### Beispiel 6 4-Amino-2-(dicyclopropyl-methylamino)-6-methyl-1,3,5-triazin

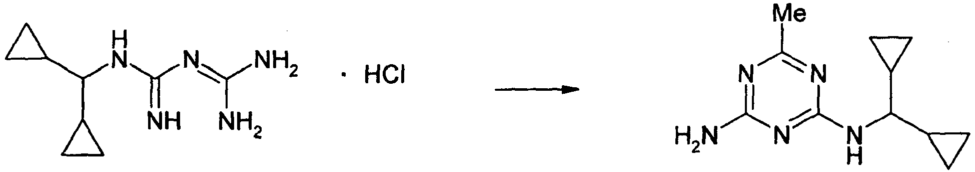

272 mg (80 Gew.-%, 0,94 mmol) 1,1-Dicyclopropylmethylbiguanidid-Hydrochlorid wurden in 4 ml Methanol aufgenommen, mit 85 mg (30 Gew.-%, 0,47 mmol) methanolische Natriummethylat-Lösung versetzt und danach 10 min. bei Raumtemperatur gerührt. Anschließend wurden nacheinander 165 mg (1,88 mmol) Essigsäureethylester und 84 mg (30 Gew.-%, 0,47 mmol) methanolische Natriummethylat-Lösung zugetropft. Das Gemisch rührte über Nacht bei Raumtemperatur. Zur Aufarbeitung wurde Wasser zugegeben, anschließend wurde die wässrige Phase zweimal mit Essigsäureethylester extrahiert. Die vereinigten organischen Phasen wurden getrocknet und unter reduziertem Druck vom Lösungsmittel befreit. Der Rückstand wurde chromatographisch gereinigt, wobei 23 mg (Ausbeute: 11,2 %) des gewünschten Produkts erhalten wurden. Ein ¹H-NMR-sowie ein Massenspektrum bestätigen die Struktur.

Die weiteren Beispiele in den nachfolgenden Tabellen 1 bis 7 werden analog den genannten Verfahren erhalten.

### Beispieltabellen 1 bis 6

**Tabelle mit Abkürzungen zu Tabellen 1-6**

| Abkürzung | chemischer Name | chemische Formel*) |
|---|---|---|
| Me | Methyl | CH₃ |
| Et | Ethyl | C₂H₅ |
| c-Pr | Cyclopropyl | 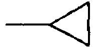 |
| c-Bu | Cyclobutyl | 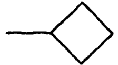 |
| 1-Me-c-Pr | 1-Methyl-cyclopropyl | 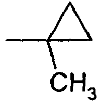 |
| 1-Cl-c-Pr | 1-Chlor-cyclopropyl | 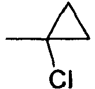 |
| 1-F-c-Pr | 1-Fluor-cyclopropyl | 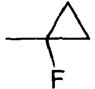 |
| 2,2-Me₂-c-Pr | 2,2-Dimethyl-cyclopropyl | 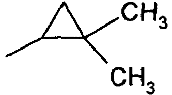 |
| 2,2-Cl₂-c-Pr | 2,2-Dichlor-cyclopropyl | 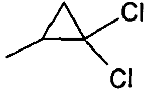 |
| Ac | Acetyl | -CO-CH₃ |
| NHAc | Acetylamino | -NH-CO-CH₃ |
| NHCOEt | Propionylamino | -NH-CO-CH₂CH₃ |
| NHCOCHFMe | 2-Fluor-propionylamino | -NH-CO-CHF-CH₃ |
| N=CH-NMe₂ | Dimethylaminomethyliden-amino | 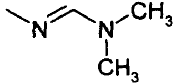 |
| N=CH-morph | Morpholin-4-ylmethyliden-amino | 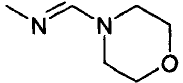 |

| | | |
|---|---|---|
| *) Anmerkung zur Vortabelle: In den chemischen Formeln bezeichnet der Strich an einem Rest dessen freie Bindung (nicht zu verwechseln mit der Kurzschreibweise für eine Methylgruppe) | | |

In der Spalte zu physikalische Daten ("phys. Daten) der Tabellen 1-7 bedeuten:
- "NMR" =: Daten gemäß ¹H-NMR-Spektum (¹H-Kernresonanz-Daten) liegen vor und sind am Ende der jeweiligen Tabelle angegeben.
- "Harz" =: Die Verbindung wurde als harzartige Substanz ("zähflüssiges Öl") erhalten

**Tabelle 1: Verbindungen der Formel (la)**

| | | | | | | | |
|---|---|---|---|---|---|---|---|
| 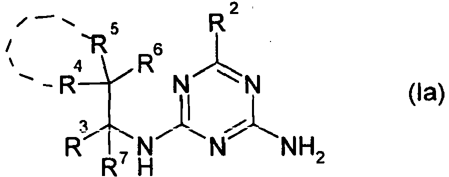 | | | | | | | |

| Nr. | R² | R³ | R⁴ | R⁵ | R⁶ | R⁷ | phys. Daten |
|---|---|---|---|---|---|---|---|
| 1-1 | H | c-Pr | Me | Me | H | H | |
| 1-2 | H | c-Pr | Me | Me | H | Me | |
| 1-3 | H | c-Pr | Me | Me | H | c-Pr | |
| 1-4 | H | c-Pr | Me | Me | Me | H | |
| 1-5 | H | c-Pr | Me | Me | Me | Me | |
| 1-6 | H | c-Pr | Me | Me | Me | c-Pr | |
| 1-7 | H | c-Pr | c-Pr | Me | H | H | |
| 1-8 | H | c-Pr | c-Pr | Me | H | Me | |
| 1-9 | H | c-Pr | c-Pr | Me | H | c-Pr | |
| 1-10 | H | c-Pr | c-Pr | Me | Me | H | |
| 1-11 | H | c-Pr | c-Pr | Me | Me | Me | |
| 1-12 | H | c-Pr | c-Pr | Me | Me | c-Pr | |
| 1-13 | H | c-Pr | -CH₂-OMe | Me | H | H | |
| 1-14 | H | c-Pr | -CH₂-OMe | Me | H | Me | |
| 1-15 | H | c-Pr | -CH₂-OMe | Me | H | c-Pr | |
| 1-16 | H | c-Pr | -CH₂-OMe | Me | Me | H | |
| 1-17 | H | c-Pr | -CH₂-OMe | Me | Me | Me | |
| 1-18 | H | c-Pr | -CH₂-OMe | Me | Me | c-Pr | |
| 1-19 | H | c-Pr | -(CH₂)₂- | | H | H | NMR |
| 1-20 | H | c-Pr | -(CH₂)₃- | | H | H | |
| 1-21 | H | c-Pr | -(CH₂)₂- | | H | Me | |
| 1-22 | H | c-Pr | -(CH₂)₃- | | H | Me | |
| 1-23 | H | c-Pr | -(CH₂)₂- | | H | c-Pr | |
| 1-24 | H | c-Pr | -(CH₂)₃- | | H | c-Pr | |
| 1-25 | H | c-Bu | -(CH₂)₃- | | H | H | |
| 1-26 | H | c-Bu | -(CH₂)₃- | | H | Me | |
| 1-27 | H | c-Bu | -(CH₂)₃- | | H | c-Pr | |
| 1-28 | H | 1-Me-c-Pr | Me | Me | H | H | |
| 1-29 | H | 1-Me-c-Pr | Me | Me | Me | H | |
| 1-30 | H | 1-Me-c-Pr | Me | Me | Me | 1-Me-c-Pr | |
| 1-31 | H | 1-Me-c-Pr | c-Pr | Me | H | H | |
| 1-32 | H | 1-Me-c-Pr | c-Pr | Me | Me | H | |
| 1-33 | H | 1-Me-c-Pr | c-Pr | Me | Me | 1-Me-c-Pr | |
| 1-34 | H | 1-Me-c-Pr | -CH₂-OMe | Me | H | H | |
| 1-35 | H | 1-Me-c-Pr | -CH₂-OMe | Me | Me | H | |
| 1-36 | H | 1-Me-c-Pr | -CH₂-OMe | Me | Me | 1-Me-c-Pr | |
| 1-37 | H | 1-Me-c-Pr | -(CH₂)₂- | | H | H | |
| 1-38 | H | 1-Me-c-Pr | -(CH₂)₃- | | H | H | |
| 1-39 | H | 1-Me-c-Pr | -(CH₂)₂- | | Me | H | |
| 1-40 | H | 1-Me-c-Pr | -(CH₂)₂- | | Me | 1-Me-c-Pr | |
| 1-41 | H | 1-Cl-c-Pr | Me | Me | H | H | |
| 1-42 | H | 1-Cl-c-Pr | Me | Me | Me | H | |
| 1-43 | H | 1-Cl-c-Pr | Me | Me | Me | 1-Cl-c-Pr | |
| 1-44 | H | 1-Cl-c-Pr | c-Pr | Me | H | H | |
| 1-45 | H | 1-Cl-c-Pr | c-Pr | Me | Me | H | |
| 1-46 | H | 1-Cl-c-Pr | c-Pr | Me | Me | 1-Cl-c-Pr | |
| 1-47 | H | 1-Cl-c-Pr | -CH₂-OMe | Me | H | H | |
| 1-48 | H | 1-Cl-c-Pr | -CH₂-OMe | Me | Me | H | |
| 1-49 | H | 1-Cl-c-Pr | -CH₂-OMe | Me | Me | 1-Cl-c-Pr | |
| 1-50 | H | 1-Cl-c-Pr | -(CH₂)₂- | | H | H | |
| 1-51 | H | 1-Cl-c-Pr | -(CH₂)₃- | | H | H | |
| 1-52 | H | 1-Cl-c-Pr | -(CH₂)₂- | | Cl | H | |
| 1-53 | H | 1-Cl-c-Pr | -(CH₂)₂- | | Cl | 1-Cl-c-Pr | |
| 1-54 | H | 2,2-Me₂-c-Pr | Me | Me | H | H | |
| 1-55 | H | 2,2-Me₂-c-Pr | Me | Me | Me | H | |
| 1-56 | H | 2,2-Me₂-c-Pr | Me | Me | Me | 2,2-Me₂-c-Pr | |
| 1-57 | H | 2.2-Me₂-c-Pr | c-Pr | Me | H | H | |
| 1-58 | H | 2,2-Me₂-c-Pr | c-Pr | Me | Me | H | |
| 1-59 | H | 2,2-Me₂-c-Pr | c-Pr | Me | Me | *2,2*-*Me₂-c-Pr* | |
| 1-60 | H | 2,2-Me₂-c-Pr | -CH₂-OMe | Me | H | H | |
| 1-61 | H | 2,2-Me₂-c-Pr | -CH₂-OMe | Me | Me | H | |
| 1-62 | H | 2,2-Me₂-c-Pr | -CH₂-OMe | Me | Me | 2,2-Me₂-c-Pr | |
| 1-63 | H | 2,2-Me₂-c-Pr | -(CH₂)₂- | | H | H | |
| 1-64 | H | 2,2-Me₂-c-Pr | -(CH₂)₃- | | H | H | |
| 1-65 | H | 2,2-Me₂-c-Pr | -CH₂-CMe₂- | | H | H | |
| 1-66 | H | 2,2-Me₂-c-Pr | -CH₂-CMe₂- | | H | 2,2-Me₂-c-Pr | |
| 1-67 | H | 2,2-Cl₂-c-Pr | Me | Me | H | H | |
| 1-68 | H | 2,2-Cl₂-c-Pr | Me | Me | Me | H | |
| 1-69 | H | 2,2-Cl₂-c-Pr | Me | Me | Me | 2,2-Cl₂-c-Pr | |
| 1-70 | H | 2,2-Cl₂-c-Pr | c-Pr | Me | H | H | |
| 1-71 | H | 2,2-Cl₂-c-Pr | c-Pr | Me | Me | H | |
| 1-72 | H | 2,2-Cl₂-c-Pr | c-Pr | Me | Me | 2,2-Cl₂-c-Pr | |
| 1-73 | H | 2,2-Cl₂-c-Pr | -CH₂-OMe | Me | H | H | |
| 1-74 | H | 2,2-Cl₂-c-Pr | -CH₂-OMe | Me | Me | H | |
| 1-75 | H | 2,2-Cl₂-c-Pr | -CH₂-OMe | Me | Me | 2,2-Cl₂-c-Pr | |
| 1-76 | H | 2,2-Cl₂-c-Pr | -(CH₂)₂- | | H | H | |
| 1-77 | H | 2,2-Cl₂-c-Pr | -(CH₂)₃- | | H | H | |
| 1-78 | H | 2,2-Cl₂-c-Pr | -CH₂-CCl₂- | | H | H | |
| 1-79 | H | 2,2-Cl₂-c-Pr | -CH₂-CCl₂ | | H | 2,2-Cl₂-c-Pr | |
| 1-80 | Me | c-Pr | Me | Me | H | H | |
| 1-81 | Me | c-Pr | Me | Me | H | Me | |
| 1-82 | Me | c-Pr | Me | Me | H | c-Pr | |
| 1-83 | Me | c-Pr | Me | Me | Me | H | |
| 1-84 | Me | c-Pr | Me | Me | Me | Me | |
| 1-85 | Me | c-Pr | Me | Me | Me | c-Pr | |
| 1-86 | Me | c-Pr | c-Pr | Me | H | H | |
| 1-87 | Me | c-Pr | c-Pr | Me | H | Me | |
| 1-88 | Me | c-Pr | c-Pr | Me | H | c-Pr | |
| 1-89 | Me | c-Pr | c-Pr | Me | Me | H | |
| 1-90 | Me | c-Pr | c-Pr | Me | Me | Me | |
| 1-91 | Me | c-Pr | c-Pr | Me | Me | c-Pr | |
| 1-92 | Me | c-Pr | -CH₂-OMe | Me | H | H | |
| 1-93 | Me | c-Pr | -CH₂-OMe | Me | H | Me | |
| 1-94 | Me | c-Pr | -CH₂-OMe | Me | H | c-Pr | |
| 1-95 | Me | c-Pr | -CH₂-OMe | Me | Me | H | |
| 1-96 | Me | c-Pr | -CH₂-OMe | Me | Me | Me | |
| 1-97 | Me | c-Pr | -CH₂-OMe | Me | Me | c-Pr | |
| 1-98 | Me | c-Pr | -(CH₂)₂- | | H | H | NMR |
| 1-99 | Me | c-Pr | -(CH₂)₃- | | H | H | |
| 1-100 | Me | c-Pr | -(CH₂)₂- | | H | Me | |
| 1-101 | Me | c-Pr | -(CH₂)₃- | | H | Me | |
| 1-102 | Me | c-Pr | -(CH₂)₂- | | H | c-Pr | |
| 1-103 | Me | c-Pr | -(CH₂)₃- | | H | c-Pr | |
| 1-104 | Me | c-Bu | -(CH₂)₃- | | H | H | |
| 1-105 | Me | c-Bu | -(CH₂)₃- | | H | Me | |
| 1-106 | Me | c-Bu | -(CH₂)₃- | | H | c-Pr | |
| 1-107 | Me | 1-Me-c-Pr | Me | Me | H | H | |
| 1-108 | Me | 1-Me-c-Pr | Me | Me | Me | H | |
| 1-109 | Me | 1-Me-c-Pr | Me | Me | Me | 1-Me-c-Pr | |
| 1-110 | Me | 1-Me-c-Pr | c-Pr | Me | H | H | |
| 1-111 | Me | 1-Me-c-Pr | c-Pr | Me | Me | H | |
| 1-112 | Me | 1-Me-c-Pr | c-Pr | Me | Me | 1-Me-c-Pr | |
| 1-113 | Me | 1-Me-c-Pr | -CH₂-OMe | Me | H | H | |
| 1-114 | Me | 1-Me-c-Pr | -CH₂-OMe | Me | Me | H | |
| 1-115 | Me | 1-Me-c-Pr | -CH₂-OMe | Me | Me | 1-Me-c-Pr | |
| 1-116 | Me | 1-Me-c-Pr | -(CH₂)₂- | | H | H | |
| 1-117 | Me | 1-Me-c-Pr | -(CH₂)₃- | | H | H | |
| 1-118 | Me | 1-Me-c-Pr | -(CH₂)₂- | | Me | H | |
| 1-119 | Me | 1-Me-c-Pr | -(CH₂)₂- | | Me | 1-Me-c-Pr | |
| 1-120 | Me | 1-Cl-c-Pr | Me | Me | H | H | |
| 1-121 | Me | 1-Cl-c-Pr | Me | Me | Me | H | |
| 1-122 | Me | 1-Cl-c-Pr | Me | Me | Me | 1-Cl-c-Pr | |
| 1-123 | Me | 1-Cl-c-Pr | c-Pr | Me | H | H | |
| 1-124 | Me | 1-Cl-c-Pr | c-Pr | Me | Me | H | |
| 1-125 | Me | 1-Cl-c-Pr | c-Pr | Me | Me | 1-Cl-c-Pr | |
| 1-126 | Me | 1-Cl-c-Pr | -CH₂-OMe | Me | H | H | |
| 1-127 | Me | 1-Cl-c-Pr | -CH₂-OMe | Me | Me | H | |
| 1-128 | Me | 1-Cl-c-Pr | -CH₂-OMe | Me | Me | 1-Cl-c-Pr | |
| 1-129 | Me | 1-Cl-c-Pr | -(CH₂)₂- | | H | H | |
| 1-130 | Me | 1-Cl-c-Pr | -(CH₂)₃- | | H | H | |
| 1-131 | Me | 1-Cl-c-Pr | -(CH₂)₂- | | Cl | H | |
| 1-132 | Me | 1-Cl-c-Pr | -(CH₂)₂- | | Cl | 1-Cl-c-Pr | |
| 1-133 | Me | 2,2-Me₂-c-Pr | Me | Me | H | H | |
| 1-134 | Me | 2,2-Me₂-c-Pr | Me | Me | Me | H | |
| 1-135 | Me | 2,2-Me₂-c-Pr | Me | Me | Me | 2,2-Me₂-c-Pr | |
| 1-136 | Me | 2,2-Me₂-c-Pr | c-Pr | Me | H | H | |
| 1-137 | Me | 2,2-Me₂-c-Pr | c-Pr | Me | Me | H | |
| 1-138 | Me | 2,2-Me₂-c-Pr | c-Pr | Me | Me | 2,2-Me₂-c-Pr | |
| 1-139 | Me | 2,2-Me₂-c-Pr | -CH₂-OMe | Me | H | H | |
| 1-140 | Me | 2,2-Me₂-c-Pr | -CH₂-OMe | Me | Me | H | |
| 1-141 | Me | 2,2-Me₂-c-Pr | -CH₂-OMe | Me | Me | 2,2-Me₂-c-Pr | |
| 1-142 | Me | 2,2-Me₂-c-Pr | -(CH₂)₂- | | H | H | |
| 1-143 | Me | 2,2-Me₂-c-Pr | -(CH₂)₃- | | H | H | |
| 1-144 | Me | 2,2-Me₂-c-Pr | -CH₂-CMe₂- | | H | H | |
| 1-145 | Me | 2,2-Me₂-c-Pr | -CH₂-CMe₂- | | H | 2,2-Me₂-c-Pr | |
| 1-146 | Me | 2,2-Cl₂-c-Pr | Me | Me | H | H | |
| 1-147 | Me | 2,2-Cl₂-c-Pr | Me | Me | Me | H | |
| 1-148 | Me | 2,2-Cl₂-c-Pr | Me | Me | Me | 2,2-Cl₂-c-Pr | |
| 1-149 | Me | 2,2-Cl₂-c-Pr | c-Pr | Me | H | H | |
| 1-150 | Me | 2,2-Cl₂-c-Pr | c-Pr | Me | Me | H | |
| 1-151 | Me | 2,2-Cl₂-c-Pr | c-Pr | Me | Me | 2,2-Cl₂-c-Pr | |
| 1-152 | Me | 2,2-Cl₂-c-Pr | -CH₂-OMe | Me | H | H | |
| 1-153 | Me | 2,2-Cl₂-c-Pr | -CH₂-OMe | Me | Me | H | |
| 1-154 | Me | 2,2-Cl₂-c-Pr | -CH₂-OMe | Me | Me | 2,2-Cl₂-c-Pr | |
| 1-155 | Me | 2,2-Cl₂-c-Pr | -(CH₂)₂- | | H | H | |
| 1-156 | Me | 2,2-Cl₂-c-Pr | -(CH₂)₃- | | H | H | |
| 1-157 | Me | 2,2-Cl₂-c-Pr | -CH₂-CCl₂- | | H | H | |
| 1-158 | Me | 2,2-Cl₂-c-Pr | -CH₂-CCl₂- | | H | 2,2-Cl₂-c-Pr | |
| 1-159 | Et | c-Pr | Me | Me | H | H | |
| 1-160 | Et | c-Pr | Me | Me | H | Me | |
| 1-161 | Et | c-Pr | Me | Me | H | c-Pr | |
| 1-162 | Et | c-Pr | Me | Me | Me | H | |
| 1-163 | Et | c-Pr | Me | Me | Me | Me | |
| 1-164 | Et | c-Pr | Me | Me | Me | c-Pr | |
| 1-165 | Et | c-Pr | c-Pr | Me | H | H | |
| 1-166 | Et | c-Pr | c-Pr | Me | H | Me | |
| 1-167 | Et | c-Pr | c-Pr | Me | H | c-Pr | |
| 1-168 | Et | c-Pr | c-Pr | Me | Me | H | |
| 1-169 | Et | c-Pr | c-Pr | Me | Me | Me | |
| 1-170 | Et | c-Pr | c-Pr | Me | Me | c-Pr | |
| 1-171 | Et | c-Pr | -CH₂-OMe | Me | H | H | |
| 1-172 | Et | c-Pr | -CH₂-OMe | Me | H | Me | |
| 1-173 | Et | c-Pr | -CH₂-OMe | Me | H | c-Pr | |
| 1-174 | Et | c-Pr | -CH₂-OMe | Me | Me | H | |
| 1-175 | Et | c-Pr | -CH₂-OMe | Me | Me | Me | |
| 1-176 | Et | c-Pr | -CH₂-OMe | Me | Me | c-Pr | |
| 1-177 | Et | c-Pr | -(CH₂)₂- | | H | H | NMR |
| 1-178 | Et | c-Pr | -(CH₂)₃- | | H | H | |
| 1-179 | Et | c-Pr | -(CH₂)₂- | | H | Me | |
| 1-180 | Et | c-Pr | -(CH₂)₃- | | H | Me | |
| 1-181 | Et | c-Pr | -(CH₂)₂- | | H | c-Pr | |
| 1-182 | Et | c-Pr | -(CH₂)₃- | | H | c-Pr | |
| 1-183 | Et | c-Bu | -(CH₂)₃- | | H | H | NMR |
| 1-184 | Et | c-Bu | -(CH₂)₃- | | H | Me | |
| 1-185 | Et | c-Bu | -(CH₂)₃- | | H | c-Pr | |
| 1-186 | Et | 1-Me-c-Pr | Me | Me | H | H | |
| 1-187 | Et | 1-Me-c-Pr | Me | Me | Me | H | |
| 1-188 | Et | 1-Me-c-Pr | Me | Me | Me | 1-Me-c-Pr | |
| 1-189 | Et | 1-Me-c-Pr | c-Pr | Me | H | H | |
| 1-190 | Et | 1-Me-c-Pr | c-Pr | Me | Me | H | |
| 1-191 | Et | 1-Me-c-Pr | c-Pr | Me | Me | 1-Me-c-Pr | |
| 1-192 | Et | 1-Me-c-Pr | -CH₂-OMe | Me | H | H | |
| 1-193 | Et | 1-Me-c-Pr | -CH₂-OMe | Me | Me | H | |
| 1-194 | Et | 1-Me-c-Pr | -CH₂-OMe | Me | Me | 1-Me-c-Pr | |
| 1-195 | Et | 1-Me-c-Pr | -(CH₂)₂- | | H | H | |
| 1-196 | Et | 1-Me-c-Pr | -(CH₂)₃- | | H | H | |
| 1-197 | Et | 1-Me-c-Pr | -(CH₂)₂- | | Me | H | |
| 1-198 | Et | 1-Me-c-Pr | -(CH₂)₂- | | Me | 1-Me-c-Pr | |
| 1-199 | Et | 1-Cl-c-Pr | Me | Me | H | H | |
| 1-200 | Et | 1-Cl-c-Pr | Me | Me | Me | H | |
| 1-201 | Et | 1-Cl-c-Pr | Me | Me | Me | 1-Cl-c-Pr | |
| 1-202 | Et | 1-Cl-c-Pr | c-Pr | Me | H | H | |
| 1-203 | Et | 1-Cl-c-Pr | c-Pr | Me | Me | H | |
| 1-204 | Et | 1-Cl-c-Pr | c-Pr | Me | Me | 1-Cl-c-Pr | |
| 1-205 | Et | 1-Cl-c-Pr | -CH₂-OMe | Me | H | H | |
| 1-206 | Et | 1-Cl-c-Pr | -CH₂-OMe | Me | Me | H | |
| 1-207 | Et | 1-Cl-c-Pr | -CH₂-OMe | Me | Me | 1-Cl-c-Pr | |
| 1-208 | Et | 1-Cl-c-Pr | -(CH₂)₂- | | H | H | |
| 1-209 | Et | 1-Cl-c-Pr | -(CH₂)₃- | | H | H | |
| 1-210 | Et | 1-Cl-c-Pr | -(CH₂)₂- | | Cl | H | |
| 1-211 | Et | 1-Cl-c-Pr | -(CH₂)₂- | | Cl | 1-Cl-c-Pr | |
| 1-212 | Et | 2,2-Me₂-c-Pr | Me | Me | H | H | |
| 1-213 | Et | 2,2-Me₂-c-Pr | Me | Me | Me | H | |
| 1-214 | Et | 2,2-Me₂-c-Pr | Me | Me | Me | 2,2-Me₂-c-Pr | |
| 1-215 | Et | 2,2-Me₂-c-Pr | c-Pr | Me | H | H | |
| 1-216 | Et | 2,2-Me₂-c-Pr | c-Pr | Me | Me | H | |
| 1-217 | Et | 2,2-Me₂-c-Pr | c-Pr | Me | Me | 2,2-Me₂-c-Pr | |
| 1-218 | Et | 2,2-Me₂-c-Pr | -CH₂-OMe | Me | H | H | |
| 1-219 | Et | 2,2-Me₂-c-Pr | -CH₂-OMe | Me | Me | H | |
| 1-220 | Et | 2,2-Me₂-c-Pr | -CH₂-OMe | Me | Me | 2,2-Me₂-c-Pr | |
| 1-221 | Et | 2,2-Me₂-c-Pr | -(CH₂)₂- | | H | H | |
| 1-222 | Et | 2,2-Me₂-c-Pr | -(CH₂)₃- | | H | H | |
| 1-223 | Et | 2,2-Me₂-c-Pr | -CH₂-CMe₂- | | H | H | |
| 1-224 | Et | 2,2-Me₂-c-Pr | -CH₂-CMe₂- | | H | 2,2-Me₂-c-Pr | |
| 1-225 | Et | 2,2-Me₂-c-Pr | Me | Me | H | H | |
| 1-226 | Et | 2,2-Cl₂-c-Pr | Me | Me | Me | H | |
| 1-227 | Et | 2,2-Cl₂-c-Pr | Me | Me | Me | 2,2-Cl₂-c-Pr | |
| 1-228 | Et | 2,2-Cl₂-c-Pr | c-Pr | Me | H | H | |
| 1-229 | Et | 2,2-Cl₂-c-Pr | c-Pr | Me | Me | H | |
| 1-230 | Et | 2,2-Cl₂-c-Pr | c-Pr | Me | Me | 2,2-Cl₂-c-Pr | |
| 1-231 | Et | 2,2-Cl₂-c-Pr | -CH₂-OMe | Me | H | H | |
| 1-232 | Et | 2,2-Cl₂-c-Pr | -CH₂-OMe | Me | Me | H | |
| 1-233 | Et | 2,2-Cl₂-c-Pr | -CH₂-OMe | Me | Me | 2,2-Cl₂-c-Pr | |
| 1-234 | Et | 2,2-Cl₂-c-Pr | -(CH₂)₂- | | H | H | |
| 1-235 | Et | 2,2-Cl₂-c-Pr | -(CH₂)₃- | | H | H | |
| 1-236 | Et | 2,2-Cl₂-c-Pr | -CH₂-CCl₂- | | H | H | |
| 1-237 | Et | 2,2-Cl₂-c-Pr | -CH₂-CCl₂- | | H | 2,2-Cl₂-c-Pr | |
| 1-238 | c-Pr | c-Pr | Me | Me | H | H | |
| 1-239 | c-Pr | c-Pr | Me | Me | H | Me | |
| 1-240 | c-Pr | c-Pr | Me | Me | H | c-Pr | |
| 1-241 | c-Pr | c-Pr | Me | Me | Me | H | |
| 1-242 | c-Pr | c-Pr | Me | Me | Me | Me | |
| 1-243 | c-Pr | c-Pr | Me | Me | Me | c-Pr | |
| 1-244 | c-Pr | c-Pr | c-Pr | Me | H | H | |
| 1-245 | c-Pr | c-Pr | c-Pr | Me | H | Me | |
| 1-246 | c-Pr | c-Pr | c-Pr | Me | H | c-Pr | |
| 1-247 | c-Pr | c-Pr | c-Pr | Me | Me | H | |
| 1-248 | c-Pr | c-Pr | c-Pr | Me | Me | Me | |
| 1-249 | c-Pr | c-Pr | c-Pr | Me | Me | c-Pr | |
| 1-250 | c-Pr | c-Pr | -CH₂-OMe | Me | H | H | |
| 1-251 | c-Pr | c-Pr | -CH₂-OMe | Me | H | Me | |
| 1-252 | c-Pr | c-Pr | -CH₂-OMe | Me | H | c-Pr | |
| 1-253 | c-Pr | c-Pr | -CH₂-OMe | Me | Me | H | |
| 1-254 | c-Pr | c-Pr | -CH₂-OMe | Me | Me | Me | |
| 1-255 | c-Pr | c-Pr | -CH₂-OMe | Me | Me | c-Pr | |
| 1-256 | c-Pr | c-Pr | -(CH₂)₂- | | H | H | NMR |
| 1-257 | c-Pr | c-Pr | -(CH₂)₃- | | H | H | |
| 1-258 | c-Pr | c-Pr | -(CH₂)₂- | | H | Me | |
| 1-259 | c-Pr | c-Pr | -(CH₂)₃- | | H | Me | |
| 1-260 | c-Pr | c-Pr | -(CH₂)₂- | | H | c-Pr | |
| 1-261 | c-Pr | c-Pr | -(CH₂)₃- | | H | c-Pr | |
| 1-262 | c-Pr | c-Bu | -(CH₂)₃- | | H | H | |
| 1-263 | c-Pr | c-Bu | -(CH₂)₃- | | H | Me | |
| 1-264 | c-Pr | c-Bu | -(CH₂)₃- | | H | c-Pr | |
| 1-265 | c-Pr | 1-Me-c-Pr | Me | Me | H | H | |
| 1-266 | c-Pr | 1-Me-c-Pr | Me | Me | Me | H | |
| 1-267 | c-Pr | 1-Me-c-Pr | Me | Me | Me | 1-Me-c-Pr | |
| 1-268 | c-Pr | 1-Me-c-Pr | c-Pr | Me | H | H | |
| 1-269 | c-Pr | 1-Me-c-Pr | c-Pr | Me | Me | H | |
| 1-270 | c-Pr | 1-Me-c-Pr | c-Pr | Me | Me | 1-Me-c-Pr | |
| 1-271 | c-Pr | 1-Me-c-Pr | -CH₂-OMe | Me | H | H | |
| 1-272 | c-Pr | 1-Me-c-Pr | -CH₂-OMe | Me | Me | H | |
| 1-273 | c-Pr | 1-Me-c-Pr | -CH₂-OMe | Me | Me | 1-Me-c-Pr | |
| 1-274 | c-Pr | 1-Me-c-Pr | -(CH₂)₂- | | H | H | |
| 1-275 | c-Pr | 1-Me-c-Pr | -(CH₂)₃- | | H | H | |
| 1-276 | c-Pr | 1-Me-c-Pr | -(CH₂)₂- | | Me | H | |
| 1-277 | c-Pr | 1-Me-c-Pr | -(CH₂)₂- | | Me | 1-Me-c-Pr | |
| 1-278 | c-Pr | 1-Cl-c-Pr | Me | Me | H | H | |
| 1-279 | c-Pr | 1-Cl-c-Pr | Me | Me | Me | H | |
| 1-280 | c-Pr | 1-Cl-c-Pr | Me | Me | Me | 1-Cl-c-Pr | |
| 1-281 | c-Pr | 1-Cl-c-Pr | c-Pr | Me | H | H | |
| 1-282 | c-Pr | 1-Cl-c-Pr | c-Pr | Me | Me | H | |
| 1-283 | c-Pr | 1-Cl-c-Pr | c-Pr | Me | Me | 1-Cl-c-Pr | |
| 1-284 | c-Pr | 1-Cl-c-Pr | -CH₂-OMe | Me | H | H | |
| 1-285 | c-Pr | 1-Cl-c-Pr | -CH₂-OMe | Me | Me | H | |
| 1-286 | c-Pr | 1-Cl-c-Pr | -CH₂-OMe | Me | Me | 1-Cl-c-Pr | |
| 1-287 | c-Pr | 1-Cl-c-Pr | -(CH₂)₂- | | H | H | |
| 1-288 | c-Pr | 1-Cl-c-Pr | -(CH₂)₃- | | H | H | |
| 1-289 | c-Pr | 1-Cl-c-Pr | -(CH₂)₂- | | Cl | H | |
| 1-290 | c-Pr | 1-Cl-c-Pr | -(CH₂)₂- | | Cl | 1-Cl-c-Pr | |
| 1-291 | c-Pr | 2,2-Me₂-c-Pr | Me | Me | H | H | |
| 1-292 | c-Pr | 2,2-Me₂-c-Pr | Me | Me | Me | H | |
| 1-293 | c-Pr | 2,2-Me₂-c-Pr | Me | Me | Me | 2,2-Me₂-c-Pr | |
| 1-294 | c-Pr | 2,2-Me₂-c-Pr | c-Pr | Me | H | H | |
| 1-295 | c-Pr | 2,2-Me₂-c-Pr | c-Pr | Me | Me | H | |
| 1-296 | c-Pr | 2,2-Me₂-c-Pr | c-Pr | Me | Me | 2,2-Me₂-c-Pr | |
| 1-297 | c-Pr | 2,2-Me₂-c-Pr | -CH₂-OMe | Me | H | H | |
| 1-298 | c-Pr | 2,2-Me₂-c-Pr | -CH₂-OMe | Me | Me | H | |
| 1-299 | c-Pr | 2,2-Me₂-c-Pr | -CH₂-OMe | Me | Me | 2,2-Me₂-c-Pr | |
| 1-300 | c-Pr | 2,2-Me₂-c-Pr | -(CH₂)₂- | | H | H | |
| 1-301 | c-Pr | 2,2-Me₂-c-Pr | -(CH₂)₃- | | H | H | |
| 1-302 | c-Pr | 2,2-Me₂-c-Pr | -CH₂-CMe₂- | | H | H | |
| 1-303 | c-Pr | 2,2-Me₂-c-Pr | -CH₂-CMe₂- | | H | 2,2-Me₂-c-Pr | |
| 1-304 | c-Pr | 2,2-Cl₂-c-Pr | Me | Me | H | H | |
| 1-305 | c-Pr | 2,2-Cl₂-c-Pr | Me | Me | Me | H | |
| 1-306 | c-Pr | 2,2-Cl₂-c-Pr | Me | Me | Me | 2,2-Cl₂-c-Pr | |
| 1-307 | c-Pr | 2,2-Cl₂-c-Pr | c-Pr | Me | H | H | |
| 1-308 | c-Pr | 2,2-Cl₂-c-Pr | c-Pr | Me | Me | H | |
| 1-309 | c-Pr | 2,2-Cl₂-c-Pr | c-Pr | Me | Me | 2,2-Cl₂-c-Pr | |
| 1-310 | c-Pr | 2,2-Cl₂-c-Pr | -CH₂-OMe | Me | H | H | |
| 1-311 | c-Pr | 2,2-Cl₂-c-Pr | -CH₂-OMe | Me | Me | H | |
| 1-312 | c-Pr | 2,2-Cl₂-c-Pr | -CH₂-OMe | Me | Me | 2,2-Cl₂-c-Pr | |
| 1-313 | c-Pr | 2,2-Cl₂-c-Pr | -(CH₂)₂- | | H | H | |
| 1-314 | c-Pr | 2,2-Cl₂-c-Pr | -(CH₂)₃- | | H | H | |
| 1-315 | c-Pr | 2,2-Cl₂-c-Pr | -CH₂-CCl₂- | | H | H | |
| 1-316 | c-Pr | 2,2-Cl₂-c-Pr | -CH₂-CCl₂- | | H | 2,2-Cl₂-c-Pr | |
| 1-317 | CHFCH₃ | c-Pr | Me | Me | H | H | NMR |
| 1-318 | CHFCH₃ | c-Pr | Me | Me | H | Me | |
| 1-319 | CHFCH₃ | c-Pr | Me | Me | H | c-Pr | |
| 1-320 | CHFCH₃ | c-Pr | Me | Me | Me | H | |
| 1-321 | CHFCH₃ | c-Pr | Me | Me | Me | Me | |
| 1-322 | CHFCH₃ | c-Pr | Me | Me | Me | c-Pr | |
| 1-323 | CHFCH₃ | c-Pr | c-Pr | Me | H | H | |
| 1-324 | CHFCH₃ | c-Pr | c-Pr | Me | H | Me | |
| 1-325 | CHFCH₃ | c-Pr | c-Pr | Me | H | c-Pr | |
| 1-326 | CHFCH₃ | c-Pr | c-Pr | Me | Me | H | |
| 1-327 | CHFCH₃ | c-Pr | c-Pr | Me | Me | Me | |
| 1-328 | CHFCH₃ | c-Pr | c-Pr | Me | Me | c-Pr | |
| 1-329 | CHFCH₃ | c-Pr | -CH₂-OMe | Me | H | H | |
| 1-330 | CHFCH₃ | c-Pr | -CH₂-OMe | Me | H | Me | |
| 1-331 | CHFCH₃ | c-Pr | -CH₂-OMe | Me | H | c-Pr | |
| 1-332 | CHFCH₃ | c-Pr | -CH₂-OMe | Me | Me | H | |
| 1-333 | CHFCH₃ | c-Pr | -CH₂-OMe | Me | Me | Me | |
| 1-334 | CHFCH₃ | c-Pr | -CH₂-OMe | Me | Me | c-Pr | |
| 1-335 | CHFCH₃ | c-Pr | -(CH₂)₂- | | H | H | NMR |
| 1-336 | CHFCH₃ | c-Pr | -(CH₂)₃- | | H | H | |
| 1-337 | CHFCH₃ | c-Pr | -(CH₂)₂- | | H | Me | |
| 1-338 | CHFCH₃ | c-Pr | -(CH₂)₃- | | H | Me | |
| 1-339 | CHFCH₃ | c-Pr | -(CH₂)₂- | | H | c-Pr | |
| 1-340 | CHFCH₃ | c-Pr | -(CH₂)₃- | | H | c-Pr | |
| 1-341 | CHFCH₃ | c-Bu | -(CH₂)₃- | | H | H | NMR |
| 1-342 | CHFCH₃ | c-Bu | -(CH₂)₃- | | H | Me | |
| 1-343 | CHFCH₃ | c-Bu | -(CH₂)₃- | | H | c-Pr | |
| 1-344 | CHFCH₃ | 1-Me-c-Pr | Me | Me | H | H | |
| 1-345 | CHFCH₃ | 1-Me-c-Pr | Me | Me | Me | H | |
| 1-346 | CHFCH₃ | 1-Me-c-Pr | Me | Me | Me | 1-Me-c-Pr | |
| 1-347 | CHFCH₃ | 1-Me-c-Pr | c-Pr | Me | H | H | |
| 1-348 | CHFCH₃ | 1-Me-c-Pr | c-Pr | Me | Me | H | |
| 1-349 | CHFCH₃ | 1-Me-c-Pr | c-Pr | Me | Me | 1-Me-c-Pr | |
| 1-350 | CHFCH₃ | 1-Me-c-Pr | -CH₂-OMe | Me | H | H | |
| 1-351 | CHFCH₃ | 1-Me-c-Pr | -CH₂-OMe | Me | Me | H | |
| 1-352 | CHFCH₃ | 1-Me-c-Pr | -CH₂-OMe | Me | Me | -Me-c-Pr | |
| 1-353 | CHFCH₃ | 1-Me-c-Pr | -(CH₂)₂- | | H | H | |
| 1-354 | CHFCH₃ | 1-Me-c-Pr | -(CH₂)₃- | | H | H | |
| 1-355 | CHFCH₃ | 1-Me-c-Pr | -(CH₂)₂- | | Me | H | |
| 1-356 | CHFCH₃ | 1-Me-c-Pr | -(CH₂)₂- | | Me | 1-Me-c-Pr | |
| 1-357 | CHFCH₃ | 1-Cl-c-Pr | Me | Me | H | H | |
| 1-358 | CHFCH₃ | 1-Cl-c-Pr | Me | Me | Me | H | |
| 1-359 | CHFCH₃ | 1-Cl-c-Pr | Me | Me | Me | 1-Cl-c-Pr | |
| 1-360 | CHFCH₃ | 1-Cl-c-Pr | c-Pr | Me | H | H | |
| 1-361 | CHFCH₃ | 1-Cl-c-Pr | c-Pr | Me | Me | H | |
| 1-362 | CHFCH₃ | 1-Cl-c-Pr | c-Pr | Me | Me | 1-Cl-c-Pr | |
| 1-363 | CHFCH₃ | 1-Cl-c-Pr | -CH₂-OMe | Me | H | H | |
| 1-364 | CHFCH₃ | 1-Cl-c-Pr | -CH₂-OMe | Me | Me | H | |
| 1-365 | CHFCH₃ | 1-Cl-c-Pr | -CH₂-OMe | Me | Me | 1-Cl-c-Pr | |
| 1-366 | CHFCH₃ | 1-Cl-c-Pr | -(CH₂)₂- | | H | H | |
| 1-367 | CHFCH₃ | 1-Cl-c-Pr | -(CH₂)₃- | | H | H | |
| 1-368 | CHFCH₃ | 1-Cl-c-Pr | -(CH₂)₂- | | Cl | H | |
| 1-369 | CHFCH₃ | 1-Cl-c-Pr | -(CH₂)₂- | | Cl | 1-Cl-c-Pr | |
| 1-370 | CHFCH₃ | 2,2-Me₂-c-Pr | Me | Me | H | H | |
| 1-371 | CHFCH₃ | 2,2-Me₂-c-Pr | Me | Me | Me | H | |
| 1-372 | CHFCH₃ | 2,2-Me₂-c-Pr | Me | Me | Me | 2,2-Me₂-c-Pr | |
| 1-373 | CHFCH₃ | 2,2-Me₂-c-Pr | c-Pr | Me | H | H | |
| 1-374 | CHFCH₃ | 2,2-Me₂-c-Pr | c-Pr | Me | Me | H | |
| 1-375 | CHFCH₃ | 2,2-Me₂-c-Pr | c-Pr | Me | Me | 2,2-Me₂-c-Pr | |
| 1-376 | CHFCH₃ | 2,2-Me₂-c-Pr | -CH₂-OMe | Me | H | H | |
| 1-377 | CHFCH₃ | 2,2-Me₂-c-Pr | -CH₂-OMe | Me | Me | H | |
| 1-378 | CHFCH₃ | 2,2-Me₂-c-Pr | -CH₂-OMe | Me | Me | 2,2-Me₂-c-Pr | |
| 1-379 | CHFCH₃ | 2,2-Me₂-c-Pr | -(CH₂)₂- | | H | H | |
| 1-380 | CHFCH₃ | 2,2-Me₂-c-Pr | -(CH₂)₃- | | H | H | |
| 1-381 | CHFCH₃ | 2,2-Me₂-c-Pr | -CH₂-CMe₂- | | H | H | |
| 1-382 | CHFCH₃ | 2,2-Me₂-c-Pr | -CH₂-CMe₂- | | H | 2,2-Me₂-c-Pr | |
| 1-383 | CHFCH₃ | 2,2-Cl₂-c-Pr | Me | Me | H | H | |
| 1-384 | CHFCH₃ | 2,2-Cl₂-c-Pr | Me | Me | Me | H | |
| 1-385 | CHFCH₃ | 2,2-Cl₂-c-Pr | Me | Me | Me | 2,2-Cl₂-c-Pr | |
| 1-386 | CHFCH₃ | 2,2-Cl₂-c-Pr | c-Pr | Me | H | H | |
| 1-387 | CHFCH₃ | 2,2-Cl₂-c-Pr | c-Pr | Me | Me | H | |
| 1-388 | CHFCH₃ | 2,2-Cl₂-c-Pr | c-Pr | Me | Me | 2,2-Cl₂-c-Pr | |
| 1-389 | CHFCH₃ | 2,2-Cl₂-c-Pr | -CH₂-OMe | Me | H | H | |
| 1-390 | CHFCH₃ | 2,2-Cl₂-c-Pr | -CH₂-OMe | Me | Me | H | |
| 1-391 | CHFCH₃ | 2,2-Cl₂-c-Pr | -CH₂-OMe | Me | Me | 2,2-Cl₂-c-Pr | |
| 1-392 | CHFCH₃ | 2,2-Cl₂-c-Pr | -(CH₂)₂- | | H | H | |
| 1-393 | CHFCH₃ | 2,2-Cl₂-c-Pr | -(CH₂)₃- | | H | H | |
| 1-394 | CHFCH₃ | 2,2-Cl₂-c-Pr | -CH₂-CCl₂- | | H | H | |
| 1-395 | CHFCH₃ | 2,2-Cl₂-c-Pr | -CH₂-CCl₂- | | H | 2,2-Cl₂-c-Pr | |
| 1-396 | CHFC₂H₅ | c-Pr | Me | Me | H | H | |
| 1-397 | CHFC₂H₅ | c-Pr | Me | Me | H | Me | |
| 1-398 | CHFC₂H₅ | c-Pr | Me | Me | H | c-Pr | |
| 1-399 | CHFC₂H₅ | c-Pr | Me | Me | Me | H | |
| 1-400 | CHFC₂H₅ | c-Pr | Me | Me | Me | Me | |
| 1-401 | CHFC₂H₅ | c-Pr | Me | Me | Me | c-Pr | |
| 1-402 | CHFC₂H₅ | c-Pr | c-Pr | Me | H | H | |
| 1-403 | CHFC₂H₅ | c-Pr | c-Pr | Me | H | Me | |
| 1-404 | CHFC₂H₅ | c-Pr | c-Pr | Me | H | c-Pr | |
| 1-405 | CHFC₂H₅ | c-Pr | c-Pr | Me | Me | H | |
| 1-406 | CHFC₂H₅ | c-Pr | c-Pr | Me | Me | Me | |
| 1-407 | CHFC₂H₅ | c-Pr | c-Pr | Me | Me | c-Pr | |
| 1-408 | CHFC₂H₅ | c-Pr | -CH₂-OMe | Me | H | H | |
| 1-409 | CHFC₂H₅ | c-Pr | -CH₂-OMe | Me | H | Me | |
| 1-410 | CHFC₂H₅ | c-Pr | -CH₂-OMe | Me | H | c-Pr | |
| 1-411 | CHFC₂H₅ | c-Pr | -CH₂-OMe | Me | Me | H | |
| 1-412 | CHFC₂H₅ | c-Pr | -CH₂-OMe | Me | Me | Me | |
| 1-413 | CHFC₂H₅ | c-Pr | -CH₂-OMe | Me | Me | c-Pr | |
| 1-414 | CHFC₂H₅ | c-Pr | -(CH₂)₂- | | H | H | NMR |
| 1-415 | CHFC₂H₅ | c-Pr | -(CH₂)₃- | | H | H | |
| 1-416 | CHFC₂H₅ | c-Pr | -(CH₂)₂- | | H | Me | |
| 1-417 | CHFC₂H₅ | c-Pr | -(CH₂)₃- | | H | Me | |
| 1-418 | CHFC₂H₅ | c-Pr | -(CH₂)₂- | | H | c-Pr | |
| 1-419 | CHFC₂H₅ | c-Pr | -(CH₂)₃- | | H | c-Pr | |
| 1-420 | CHFC₂H₅ | c-Bu | -(CH₂)₃- | | H | H | NMR |
| 1-421 | CHFC₂H₅ | c-Bu | -(CH₂)₃- | | H | Me | |
| 1-422 | CHFC₂H₅ | c-Bu | -(CH₂)₃- | | H | c-Pr | |
| 1-423 | CHFC₂H₅ | 1-Me-c-Pr | Me | Me | H | H | |
| 1-424 | CHFC₂H₅ | 1-Me-c-Pr | Me | Me | Me | H | |
| 1-425 | CHFC₂H₅ | 1-Me-c-Pr | Me | Me | Me | 1-Me-c-Pr | |
| 1-426 | CHFC₂H₅ | 1-Me-c-Pr | c-Pr | Me | H | H | |
| 1-427 | CHFC₂H₅ | 1-Me-c-Pr | c-Pr | Me | Me | H | |
| 1-428 | CHFC₂H₅ | 1-Me-c-Pr | c-Pr | Me | Me | 1-Me-c-Pr | |
| 1-429 | CHFC₂H₅ | 1-Me-c-Pr | -CH₂-OMe | Me | H | H | |
| 1-430 | CHFC₂H₅ | 1-Me-c-Pr | -CH₂-OMe | Me | Me | H | |
| 1-431 | CHFC₂H₅ | 1-Me-c-Pr | -CH₂-OMe | Me | Me | 1-Me-c-Pr | |
| 1-432 | CHFC₂H₅ | 1-Me-c-Pr | -(CH₂)₂- | | H | H | |
| 1-433 | CHFC₂H₅ | 1-Me-c-Pr | -(CH₂)₃- | | H | H | |
| 1-434 | CHFC₂H₅ | 1-Me-c-Pr | -(CH₂)₂- | | Me | H | |
| 1-435 | CHFC₂H₅ | 1-Me-c-Pr | -(CH₂)₂- | | Me | 1-Me-c-Pr | |
| 1-436 | CHFC₂H₅ | 1-Cl-c-Pr | Me | Me | H | H | |
| 1-437 | CHFC₂H₅ | 1-Cl-c-Pr | Me | Me | Me | H | |
| 1-438 | CHFC₂H₅ | 1-Cl-c-Pr | Me | Me | Me | 1-Cl-c-Pr | |
| 1-439 | CHFC₂H₅ | 1-Cl-c-Pr | c-Pr | Me | H | H | |
| 1-440 | CHFC₂H₅ | 1-Cl-c-Pr | c-Pr | Me | Me | H | |
| 1-441 | CHFC₂H₅ | 1-Cl-c-Pr | c-Pr | Me | Me | 1-Cl-c-Pr | |
| 1-442 | CHFC₂H₅ | 1-Cl-c-Pr | -CH₂-OMe | Me | H | H | |
| 1-443 | CHFC₂H₅ | 1-Cl-c-Pr | -CH₂-OMe | Me | Me | H | |
| 1-444 | CHFC₂H₅ | 1-Cl-c-Pr | -CH₂-OMe | Me | Me | 1-Cl-c-Pr | |
| 1-445 | CHFC₂H₅ | 1-Cl-c-Pr | -(CH₂)₂- | | H | H | |
| 1-446 | CHFC₂H₅ | 1-Cl-c-Pr | -(CH₂)₃- | | H | H | |
| 1-447 | CHFC₂H₅ | 1-Cl-c-Pr | -(CH₂)₂- | | Cl | H | |
| 1-448 | CHFC₂H₅ | 1-Cl-c-Pr | -(CH₂)₂- | | Cl | 1-Cl-c-Pr | |
| 1-449 | CHFC₂H₅ | 2,2-Me₂-c-Pr | Me | Me | H | H | |
| 1-450 | CHFC₂H₅ | 2,2-Me₂-c-Pr | Me | Me | Me | H | |
| 1-451 | CHFC₂H₅ | 2,2-Me₂-c-Pr | Me | Me | Me | 2,2-Me₂-c-Pr | |
| 1-452 | CHFC₂H₅ | 2,2-Me₂-c-Pr | c-Pr | Me | H | H | |
| 1-453 | CHFC₂H₅ | 2,2-Me₂-c-Pr | c-Pr | Me | Me | H | |
| 1-454 | CHFC₂H₅ | 2,2-Me₂-c-Pr | c-Pr | Me | Me | 2,2-Me₂-c-Pr | |
| 1-455 | CHFC₂H₅ | 2,2-Me₂-c-Pr | -CH₂-OMe | Me | H | H | |
| 1-456 | CHFC₂H₅ | 2,2-Me₂-c-Pr | -CH₂-OMe | Me | Me | H | |
| 1-457 | CHFC₂H₅ | 2,2-Me₂-c-Pr | -CH₂-OMe | Me | Me | 2,2-Me₂-c-Pr | |
| 1-458 | CHFC₂H₅ | 2,2-Me₂-c-Pr | -(CH₂)₂- | | H | H | |
| 1-459 | CHFC₂H₅ | 2,2-Me₂-c-Pr | -(CH₂)₃- | | H | H | |
| 1-460 | CHFC₂H₅ | 2,2-Me₂-c-Pr | -CH₂-CMe₂- | | H | H | |
| 1-461 | CHFC₂H₅ | 2,2-Me₂-c-Pr | -CH₂-CMe₂- | | H | 2,2-Me₂-c-Pr | |
| 1-462 | CHFC₂H₅ | 2,2-Cl₂-c-Pr | Me | Me | H | H | |
| 1-463 | CHFC₂H₅ | 2,2-Cl₂-c-Pr | Me | Me | Me | H | |
| 1-464 | CHFC₂H₅ | 2,2-Cl₂-c-Pr | Me | Me | Me | 2,2-Cl₂-c-Pr | |
| 1-465 | CHFC₂H₅ | 2,2-Cl₂-c-Pr | c-Pr | Me | H | H | |
| 1-466 | CHFC₂H₅ | 2,2-Cl₂-c-Pr | c-Pr | Me | Me | H | |
| 1-467 | CHFC₂H₅ | 2,2-Cl₂-c-Pr | c-Pr | Me | Me | 2,2-Cl₂-c-Pr | |
| 1-468 | CHFC₂H₅ | 2,2-Cl₂-c-Pr | -CH₂-OMe | Me | H | H | |
| 1-469 | CHFC₂H₅ | 2,2-Cl₂-c-Pr | -CH₂-OMe | Me | Me | H | |
| 1-470 | CHFC₂H₅ | 2,2-Cl₂-c-Pr | -CH₂-OMe | Me | Me | 2,2-Cl₂-c-Pr | |
| 1-471 | CHFC₂H₅ | 2,2-Cl₂-c-Pr | -(CH₂)₂- | | H | H | |
| 1-472 | CHFC₂H₅ | 2,2-Cl₂-c-Pr | -(CH₂)₃- | | H | H | |
| 1-473 | CHFC₂H₅ | 2,2-Cl₂-c-Pr | -CH₂-CCl₂- | | H | H | |
| 1-474 | CHFC₂H₅ | 2,2-Cl₂-c-Pr | -CH₂-CCl₂- | | H | 2,2-Cl₂-c-Pr | |
| 1-475 | CF(CH₃)₂ | c-Pr | Me | Me | H | H | NMR |
| 1-476 | CF(CH₃)₂ | c-Pr | Me | Me | H | Me | |
| 1-477 | CF(CH₃)₂ | c-Pr | Me | Me | H | c-Pr | |
| 1-478 | CF(CH₃)₂ | c-Pr | Me | Me | Me | H | |
| 1-479 | CF(CH₃)₂ | c-Pr | Me | Me | Me | Me | |
| 1-480 | CF(CH₃)₂ | c-Pr | Me | Me | Me | c-Pr | |
| 1-481 | CF(CH₃)₂ | c-Pr | c-Pr | Me | H | H | |
| 1-482 | CF(CH₃)₂ | c-Pr | c-Pr | Me | H | Me | |
| 1-483 | CF(CH₃)₂ | c-Pr | c-Pr | Me | H | c-Pr | |
| 1-484 | CF(CH₃)₂ | c-Pr | c-Pr | Me | Me | H | |
| 1-485 | CF(CH₃)₂ | c-Pr | c-Pr | Me | Me | Me | |
| 1-486 | CF(CH₃)₂ | c-Pr | c-Pr | Me | Me | c-Pr | |
| 1-487 | CF(CH₃)₂ | c-Pr | -CH₂-OMe | Me | H | H | |
| 1-488 | CF(CH₃)₂ | c-Pr | -CH₂-OMe | Me | H | Me | |
| 1-489 | CF(CH₃)₂ | c-Pr | -CH₂-OMe | Me | H | c-Pr | |
| 1-490 | CF(CH₃)₂ | c-Pr | -CH₂-OMe | Me | Me | H | |
| 1-491 | CF(CH₃)₂ | c-Pr | -CH₂-OMe | Me | Me | Me | |
| 1-492 | CF(CH₃)₂ | c-Pr | -CH₂-OMe | Me | Me | c-Pr | |
| 1-493 | CF(CH₃)₂ | c-Pr | -(CH₂)₂- | | H | H | NMR |
| 1-494 | CF(CH₃)₂ | c-Pr | -(CH₂)₃- | | H | H | |
| 1-495 | CF(CH₃)₂ | c-Pr | -(CH₂)₂- | | H | Me | |
| 1-496 | CF(CH₃)₂ | c-Pr | -(CH₂)₃- | | H | Me | |
| 1-497 | CF(CH₃)₂ | c-Pr | -(CH₂)₂- | | H | c-Pr | |
| 1-498 | CF(CH₃)₂ | c-Pr | -(CH₂)₃- | | H | c-Pr | |
| 1-499 | CF(CH₃)₂ | c-Bu | -(CH₂)₃- | | H | H | NMR |
| 1-500 | CF(CH₃)₂ | c-Bu | -(CH₂)₃- | | H | Me | |
| 1-501 | CF(CH₃)₂ | c-Bu | -(CH₂)₃- | | H | c-Pr | |
| 1-502 | CF(CH₃)₂ | 1-Me-c-Pr | Me | Me | H | H | |
| 1-503 | CF(CH₃)₂ | 1-Me-c-Pr | Me | Me | Me | H | |
| 1-504 | CF(CH₃)₂ | 1-Me-c-Pr | Me | Me | Me | 1-Me-c-Pr | |
| 1-505 | CF(CH₃)₂ | 1-Me-c-Pr | c-Pr | Me | H | H | |
| 1-506 | CF(CH₃)₂ | 1-Me-c-Pr | c-Pr | Me | Me | H | |
| 1-507 | CF(CH₃)₂ | 1-Me-c-Pr | c-Pr | Me | Me | 1-Me-c-Pr | |
| 1-508 | CF(CH₃)₂ | 1-Me-c-Pr | -CH₂-OMe | Me | H | H | |
| 1-509 | CF(CH₃)₂ | 1-Me-c-Pr | -CH₂-OMe | Me | Me | H | |
| 1-510 | CF(CH₃)₂ | 1-Me-c-Pr | -CH₂-OMe | Me | Me | 1-Me-c-Pr | |
| 1-511 | CF(CH₃)₂ | 1-Me-c-Pr | -(CH₂)₂- | | H | H | |
| 1-512 | CF(CH₃)₂ | 1-Me-c-Pr | -(CH₂)₃- | | H | H | |
| 1-513 | CF(CH₃)₂ | 1-Me-c-Pr | -(CH₂)₂- | | Me | H | |
| 1-514 | CF(CH₃)₂ | 1-Me-c-Pr | -(CH₂)₂- | | Me | 1-Me-c-Pr | |
| 1-515 | CF(CH₃)₂ | 1-Cl-c-Pr | Me | Me | H | H | |
| 1-516 | CF(CH₃)₂ | 1-Cl-c-Pr | Me | Me | Me | H | |
| 1-517 | CF(CH₃)₂ | 1-Cl-c-Pr | Me | Me | Me | 1-Cl-c-Pr | |
| 1-518 | CF(CH₃)₂ | 1-Cl-c-Pr | c-Pr | Me | H | H | |
| 1-519 | CF(CH₃)₂ | 1-Cl-c-Pr | c-Pr | Me | Me | H | |
| 1-520 | CF(CH₃)₂ | 1-Cl-c-Pr | c-Pr | Me | Me | 1-Cl-c-Pr | |
| 1-521 | CF(CH₃)₂ | 1-Cl-c-Pr | -CH₂-OMe | Me | H | H | |
| 1-522 | CF(CH₃)₂ | 1-Cl-c-Pr | -CH₂-OMe | Me | Me | H | |
| 1-523 | CF(CH₃)₂ | 1-Cl-c-Pr | -CH₂-OMe | Me | Me | 1-Cl-c-Pr | |
| 1-524 | CF(CH₃)₂ | 1-Cl-c-Pr | -(CH₂)₂- | | H | H | |
| 1-525 | CF(CH₃)₂ | 1-Cl-c-Pr | -(CH₂)₃- | | H | H | |
| 1-526 | CF(CH₃)₂ | 1-Cl-c-Pr | -(CH₂)₂- | | Cl | H | |
| 1-527 | CF(CH₃)₂ | 1-Cl-c-Pr | -(CH₂)₂- | | Cl | 1-Cl-c-Pr | |
| 1-528 | CF(CH₃)₂ | 2,2-Me₂-c-Pr | Me | Me | H | H | |
| 1-529 | CF(CH₃)₂ | 2,2-Me₂-c-Pr | Me | Me | Me | H | |
| 1-530 | CF(CH₃)₂ | 2,2-Me₂-c-Pr | Me | Me | Me | 2,2-Me₂-c-Pr | |
| 1-531 | CF(CH₃)₂ | 2,2-Me₂-c-Pr | c-Pr | Me | H | H | |
| 1-532 | CF(CH₃)₂ | 2,2-Me₂-c-Pr | c-Pr | Me | Me | H | |
| 1-533 | CF(CH₃)₂ | 2,2-Me₂-c-Pr | c-Pr | Me | Me | 2,2-Me₂-c-Pr | |
| 1-534 | CF(CH₃)₂ | 2,2-Me₂-c-Pr | -CH₂-OMe | Me | H | H | |
| 1-535 | CF(CH₃)₂ | 2,2-Me₂-c-Pr | -CH₂-OMe | Me | Me | H | |
| 1-536 | CF(CH₃)₂ | 2,2-Me₂-c-Pr | -CH₂-OMe | Me | Me | 2,2-Me₂-c-Pr | |
| 1-537 | CF(CH₃)₂ | 2,2-Me₂-c-Pr | -(CH₂)₂- | | H | H | |
| 1-538 | CF(CH₃)₂ | 2,2-Me₂-c-Pr | -(CH₂)₃- | | H | H | |
| 1-539 | CF(CH₃)₂ | 2,2-Me₂-c-Pr | -CH₂-CMe₂- | | H | H | |
| 1-540 | CF(CH₃)₂ | 2,2-Me₂-c-Pr | -CH₂-CMe₂- | | H | 2,2-Me₂-c-Pr | |
| 1-541 | CF(CH₃)₂ | 2,2-Cl₂-c-Pr | Me | Me | H | H | |
| 1-542 | CF(CH₃)₂ | 2,2-Cl₂-c-Pr | Me | Me | Me | H | |
| 1-543 | CF(CH₃)₂ | 2,2-Cl₂-c-Pr | Me | Me | Me | 2,2-Cl₂-c-Pr | |
| 1-544 | CF(CH₃)₂ | 2,2-Cl₂-c-Pr | c-Pr | Me | H | H | |
| 1-545 | CF(CH₃)₂ | 2,2-Cl₂-c-Pr | c-Pr | Me | Me | H | |
| 1-546 | CF(CH₃)₂ | 2,2-Cl₂-c-Pr | c-Pr | Me | Me | 2,2-Cl₂-c-Pr | |
| 1-547 | CF(CH₃)₂ | 2,2-Cl₂-c-Pr | -CH₂-OMe | Me | H | H | |
| 1-548 | CF(CH₃)₂ | 2,2-Cl₂-c-Pr | -CH₂-OMe | Me | Me | H | |
| 1-549 | CF(CH₃)₂ | 2,2-Cl₂-c-Pr | -CH₂-OMe | Me | Me | 2,2-Cl₂-c-Pr | |
| 1-550 | CF(CH₃)₂ | 2,2-Cl₂-c-Pr | -(CH₂)₂- | | H | H | |
| 1-551 | CF(CH₃)₂ | 2,2-Cl₂-c-Pr | -(CH₂)₃- | | H | H | |
| 1-552 | CF(CH₃)₂ | 2,2-Cl₂-c-Pr | -CH₂-CCl₂- | | H | H | |
| 1-553 | CF(CH₃)₂ | 2,2-Cl₂-c-Pr | -CH₂-CCl₂- | | H | 2,2-Cl₂-c-Pr | |
| 1-554 | 1-F-c-Pr | c-Pr | Me | Me | H | H | |
| 1-555 | 1-F-c-Pr | c-Pr | Me | Me | H | Me | |
| 1-556 | 1-F-c-Pr | c-Pr | Me | Me | H | c-Pr | |
| 1-557 | 1-F-c-Pr | c-Pr | Me | Me | Me | H | |
| 1-558 | 1-F-c-Pr | c-Pr | Me | Me | Me | Me | |
| 1-559 | 1-F-c-Pr | c-Pr | Me | Me | Me | c-Pr | |
| 1-560 | 1-F-c-Pr | c-Pr | c-Pr | Me | H | H | |
| 1-561 | 1-F-c-Pr | c-Pr | c-Pr | Me | H | Me | |
| 1-562 | 1-F-c-Pr | c-Pr | c-Pr | Me | H | c-Pr | |
| 1-563 | 1-F-c-Pr | c-Pr | c-Pr | Me | Me | H | |
| 1-564 | 1-F-c-Pr | c-Pr | c-Pr | Me | Me | Me | |
| 1-565 | 1-F-c-Pr | c-Pr | c-Pr | Me | Me | c-Pr | |
| 1-566 | 1-F-c-Pr | c-Pr | -CH -OMe | Me | H | H | |
| 1-567 | 1-F-c-Pr | c-Pr | -CH₂-OMe | Me | H | Me | |
| 1-568 | 1-F-c-Pr | c-Pr | -CH₂-OMe | Me | H | c-Pr | |
| 1-569 | 1-F-c-Pr | c-Pr | -CH₂-OMe | Me | Me | H | |
| 1-570 | 1-F-c-Pr | c-Pr | -CH₂-OMe | Me | Me | Me | |
| 1-571 | 1-F-c-Pr | c-Pr | -CH₂-OMe | Me | Me | c-Pr | |
| 1-572 | 1-F-c-Pr | c-Pr | -(CH₂)₂- | | H | H | NMR |
| 1-573 | 1-F-c-Pr | c-Pr | -(CH₂)₃- | | H | H | |
| 1-574 | 1-F-c-Pr | c-Pr | -(CH₂)₂- | | H | Me | |
| 1-575 | 1-F-c-Pr | c-Pr | -(CH₂)₃- | | H | Me | |
| 1-576 | 1-F-c-Pr | c-Pr | -(CH₂)₂- | | H | c-Pr | |
| 1-577 | 1-F-c-Pr | c-Pr | -(CH₂)₃- | | H | c-Pr | |
| 1-578 | 1-F-c-Pr | c-Bu | -(CH₂)₃- | | H | H | |
| 1-579 | 1-F-c-Pr | c-Bu | -(CH₂)₃- | | H | Me | |
| 1-580 | 1-F-c-Pr | c-Bu | -(CH₂)₃- | | H | c-Pr | |
| 1-581 | 1-F-c-Pr | 1-Me-c-Pr | Me | Me | H | H | |
| 1-582 | 1-F-c-Pr | 1-Me-c-Pr | Me | Me | Me | H | |
| 1-583 | 1-F-c-Pr | 1-Me-c-Pr | Me | Me | Me | 1-Me-c-Pr | |
| 1-584 | 1-F-c-Pr | 1-Me-c-Pr | c-Pr | Me | H | H | |
| 1-585 | 1-F-c-Pr | 1-Me-c-Pr | c-Pr | Me | Me | H | |
| 1-586 | 1-F-c-Pr | 1-Me-c-Pr | c-Pr | Me | Me | 1-Me-c-Pr | |
| 1-587 | 1-F-c-Pr | 1-Me-c-Pr | -CH₂-OMe | Me | H | H | |
| 1-588 | 1-F-c-Pr | 1-Me-c-Pr | -CH₂-OMe | Me | Me | H | |
| 1-589 | 1-F-c-Pr | 1-Me-c-Pr | -CH₂-OMe | Me | Me | 1-Me-c-Pr | |
| 1-590 | 1-F-c-Pr | 1-Me-c-Pr | -(CH₂)₂- | | H | H | |
| 1-591 | 1-F-c-Pr | 1-Me-c-Pr | -(CH₂)₃- | | H | H | |
| 1-592 | 1-F-c-Pr | 1-Me-c-Pr | -(CH₂)₂- | | Me | H | |
| 1-593 | 1-F-c-Pr | 1-Me-c-Pr | -(CH₂)₂- | | Me | 1-Me-c-Pr | |
| 1-594 | 1-F-c-Pr | 1-Cl-c-Pr | Me | Me | H | H | |
| 1-595 | 1-F-c-Pr | 1-Cl-c-Pr | Me | Me | Me | H | |
| 1-596 | 1-F-c-Pr | 1-Cl-c-Pr | Me | Me | Me | 1-Cl-c-Pr | |
| 1-597 | 1-F-c-Pr | 1-Cl-c-Pr | c-Pr | Me | H | H | |
| 1-598 | 1-F-c-Pr | 1-Cl-c-Pr | c-Pr | Me | Me | H | |
| 1-599 | 1-F-c-Pr | 1-Cl-c-Pr | c-Pr | Me | Me | 1-Cl-c-Pr | |
| 1-600 | 1-F-c-Pr | 1-Cl-c-Pr | -CH₂-OMe | Me | H | H | |
| 1-601 | 1-F-c-Pr | 1-Cl-c-Pr | -CH₂-OMe | Me | Me | H | |
| 1-602 | 1-F-c-Pr | 1-Cl-c-Pr | -CH₂-OMe | Me | Me | 1-Cl-c-Pr | |
| 1-603 | 1-F-c-Pr | 1-Cl-c-Pr | -(CH₂)₂- | | H | H | |
| 1-604 | 1-F-c-Pr | 1-Cl-c-Pr | -(CH₂)₃- | | H | H | |
| 1-605 | 1-F-c-Pr | 1-Cl-c-Pr | -(CH₂)₂- | | Cl | H | |
| 1-606 | 1-F-c-Pr | 1-Cl-c-Pr | -(CH₂)₂- | | Cl | 1-Cl-c-Pr | |
| 1-607 | 1-F-c-Pr | 2,2-Me₂-c-Pr | Me | Me | H | H | |
| 1-608 | 1-F-c-Pr | 2,2-Me₂-c-Pr | Me | Me | Me | H | |
| 1-609 | 1-F-c-Pr | 2,2-Me₂-c-Pr | Me | Me | Me | 2,2-Me₂-c-Pr | |
| 1-610 | 1-F-c-Pr | 2,2-Me₂-c-Pr | c-Pr | Me | H | H | |
| 1-611 | 1-F-c-Pr | 2,2-Me₂-c-Pr | c-Pr | Me | Me | H | |
| 1-612 | 1-F-c-Pr | 2,2-Me₂-c-Pr | c-Pr | Me | Me | 2,2-Me₂-c-Pr | |
| 1-613 | 1-F-c-Pr | 2,2-Me₂-c-Pr | -CH₂-OMe | Me | H | H | |
| 1-614 | 1-F-c-Pr | 2,2-Me₂-c-Pr | -CH₂-OMe | Me | Me | H | |
| 1-615 | 1-F-c-Pr | 2,2-Me₂-c-Pr | -CH₂-OMe | Me | Me | 2,2-Me₂-c-Pr | |
| 1-616 | 1-F-c-Pr | 2,2-Me₂-c-Pr | -(CH₂)₂- | | H | H | |
| 1-617 | 1-F-c-Pr | 2,2-Me₂-c-Pr | -(CH₂)₃- | | H | H | |
| 1-618 | 1-F-c-Pr | 2,2-Me₂-c-Pr | -CH₂-CMe₂- | | H | H | |
| 1-619 | 1-F-c-Pr | 2,2-Me₂-c-Pr | -CH₂-CMe₂- | | H | 2,2-Me₂-c-Pr | |
| 1-620 | 1-F-c-Pr | 2,2-Cl₂-c-Pr | Me | Me | H | H | |
| 1-621 | 1-F-c-Pr | 2,2-Cl₂-c-Pr | Me | Me | Me | H | |
| 1-622 | 1-F-c-Pr | 2,2-Cl₂-c-Pr | Me | Me | Me | 2,2-Cl₂-c-Pr | |
| 1-623 | 1-F-c-Pr | 2,2-Me₂-c-Pr | c-Pr | Me | H | H | |
| 1-624 | 1-F-c-Pr | 2,2-Cl₂-c-Pr | c-Pr | Me | Me | H | |
| 1-625 | 1-F-c-Pr | 2,2-Cl₂-c-Pr | c-Pr | Me | Me | 2,2-Cl₂-c-Pr | |
| 1-626 | 1-F-c-Pr | 2,2-Cl₂-c-Pr | -CH₂-OMe | Me | H | H | |
| 1-627 | 1-F-c-Pr | 2,2-Cl₂-c-Pr | -CH₂-OMe | Me | Me | H | |
| 1-628 | 1-F-c-Pr | 2,2-Cl₂-c-Pr | -CH₂-OMe | Me | Me | 2,2-Cl₂-c-Pr | |
| 1-629 | 1-F-c-Pr | 2,2-Cl₂-c-Pr | -(CH₂)₂- | | H | H | |
| 1-630 | 1-F-c-Pr | 2,2-Cl₂-c-Pr | -(CH₂)₃- | | H | H | |
| 1-631 | 1-F-c-Pr | 2,2-Cl₂-c-Pr | -CH₂-CCl₂- | | H | H | |
| 1-632 | 1-F-c-Pr | 2,2-Cl₂-c-Pr | -CH₂-CCl₂- | | H | 2,2-Cl₂-c-Pr | |
| 1-633 | CHF2 | c-Pr | Me | Me | H | H | |
| 1-634 | CHF2 | c-Pr | Me | Me | H | Me | |
| 1-635 | CHF2 | c-Pr | Me | Me | H | c-Pr | |
| 1-636 | CHF2 | c-Pr | Me | Me | Me | H | |
| 1-637 | CHF2 | c-Pr | Me | Me | Me | Me | |
| 1-638 | CHF2 | c-Pr | Me | Me | Me | c-Pr | |
| 1-639 | CHF2 | c-Pr | c-Pr | Me | H | H | |
| 1-640 | CHF2 | c-Pr | c-Pr | Me | H | Me | |
| 1-641 | CHF2 | c-Pr | c-Pr | Me | H | c-Pr | |
| 1-642 | CHF2 | c-Pr | c-Pr | Me | Me | H | |
| 1-643 | CHF2 | c-Pr | c-Pr | Me | Me | Me | |
| 1-644 | CHF2 | c-Pr | c-Pr | Me | Me | c-Pr | |
| 1-645 | CHF2 | c-Pr | -CH₂-OMe | Me | H | H | |
| 1-646 | CHF2 | c-Pr | -CH₂-OMe | Me | H | Me | |
| 1-647 | CHF2 | c-Pr | -CH₂-OMe | Me | H | c-Pr | |
| 1-648 | CHF2 | c-Pr | -CH₂-OMe | Me | Me | H | |
| 1-649 | CHF2 | c-Pr | -CH₂-OMe | Me | Me | Me | |
| 1-650 | CHF2 | c-Pr | -CH₂-OMe | Me | Me | c-Pr | |
| 1-651 | CHF2 | c-Pr | -(CH₂)₂- | | H | H | NMR |
| 1-652 | CHF2 | c-Pr | -(CH₂)₃- | | H | H | |
| 1-653 | CHF2 | c-Pr | -(CH₂)₂- | | H | Me | |
| 1-654 | CHF2 | c-Pr | -(CH₂)₃- | | H | Me | |
| 1-655 | CHF2 | c-Pr | -(CH₂)₂- | | H | c-Pr | |
| 1-656 | CHF2 | c-Pr | -(CH₂)₃- | | H | c-Pr | |
| 1-657 | CHF2 | c-Bu | -(CH₂)₃- | | H | H | |
| 1-658 | CHF2 | c-Bu | -(CH₂)₃- | | H | Me | |
| 1-659 | CHF2 | c-Bu | -(CH₂)₃- | | H | c-Pr | |
| 1-660 | CHF2 | 1-Me-c-Pr | Me | Me | H | H | |
| 1-661 | CHF2 | 1-Me-c-Pr | Me | Me | Me | H | |
| 1-662 | CHF2 | 1-Me-c-Pr | Me | Me | Me | 1-Me-c-Pr | |
| 1-663 | CHF2 | 1-Me-c-Pr | c-Pr | Me | H | H | |
| 1-664 | CHF2 | 1-Me-c-Pr | c-Pr | Me | Me | H | |
| 1-665 | CHF2 | 1-Me-c-Pr | c-Pr | Me | Me | 1-Me-c-Pr | |
| 1-666 | CHF2 | 1-Me-c-Pr | -CH₂-OMe | Me | H | H | |
| 1-667 | CHF2 | 1-Me-c-Pr | -CH₂-OMe | Me | Me | H | |
| 1-668 | CHF2 | 1-Me-c-Pr | -CH₂-OMe | Me | Me | 1-Me-c-Pr | |
| 1-669 | CHF2 | 1-Me-c-Pr | -(CH₂)₂- | | H | H | |
| 1-670 | CHF2 | 1-Me-c-Pr | -(CH₂)₃- | | H | H | |
| 1-671 | CHF2 | 1-Me-c-Pr | -(CH₂)₂- | | Me | H | |
| 1-672 | CHF2 | 1-Me-c-Pr | -(CH₂)₂- | | Me | 1-Me-c-Pr | |
| 1-673 | CHF2 | 1-Cl-c-Pr | Me | Me | H | H | |
| 1-674 | CHF2 | 1-Cl-c-Pr | Me | Me | Me | H | |
| 1-675 | CHF2 | 1-Cl-c-Pr | Me | Me | Me | 1-Cl-c-Pr | |
| 1-676 | CHF2 | 1-Cl-c-Pr | c-Pr | Me | H | H | |
| 1-677 | CHF2 | 1-Cl-c-Pr | c-Pr | Me | Me | H | |
| 1-678 | CHF2 | 1-Cl-c-Pr | c-Pr | Me | Me | 1-Cl-c-Pr | |
| 1-679 | CHF2 | 1-Cl-c-Pr | -CH₂-OMe | Me | H | H | |
| 1-680 | CHF2 | 1-Cl-c-Pr | -CH₂-OMe | Me | Me | H | |
| 1-681 | CHF2 | 1-Cl-c-Pr | -CH₂-OMe | Me | Me | 1-Cl-c-Pr | |
| 1-682 | CHF2 | 1-Cl-c-Pr | -(CH₂)₂- | | H | H | |
| 1-683 | CHF2 | 1-Cl-c-Pr | -(CH₂)₃- | | H | H | |
| 1-684 | CHF2 | 1-Cl-c-Pr | -(CH₂)₂- | | Cl | H | |
| 1-685 | CHF2 | 1-Cl-c-Pr | -(CH₂)₂- | | Cl | 1-Cl-c-Pr | |
| 1-686 | CHF2 | 2,2-Me₂-c-Pr | Me | Me | H | H | |
| 1-687 | CHF2 | 2,2-Me₂-c-Pr | Me | Me | Me | H | |
| 1-688 | CHF2 | 2,2-Me₂-c-Pr | Me | Me | Me | 2,2-Me₂-c-Pr | |
| 1-689 | CHF2 | 2,2-Me₂-c-Pr | c-Pr | Me | H | H | |
| 1-690 | CHF2 | 2,2-Me₂-c-Pr | c-Pr | Me | Me | H | |
| 1-691 | CHF2 | 2,2-Me₂-c-Pr | c-Pr | Me | Me | 2,2-Me₂-c-Pr | |
| 1-692 | CHF2 | 2,2-Me₂-c-Pr | -CH₂-OMe | Me | H | H | |
| 1-693 | CHF2 | 2,2-Me₂-c-Pr | -CH₂-OMe | Me | Me | H | |
| 1-694 | CHF2 | 2,2-Me₂-c-Pr | -CH₂-OMe | Me | Me | 2,2-Me₂-c-Pr | |
| 1-695 | CHF2 | 2,2-Me₂-c-Pr | -(CH₂)₂- | | H | H | |
| 1-696 | CHF2 | 2,2-Me₂-c-Pr | -(CH₂)₃- | | H | H | |
| 1-697 | CHF2 | 2,2-Me₂-c-Pr | -CH₂-CMe₂- | | H | H | |
| 1-698 | CHF2 | 2,2-Me₂-c-Pr | - CH₂-CMe₂- | | H | 2,2-Me₂-c-Pr | |
| 1-699 | CHF2 | 2,2-Cl₂-c-Pr | Me | Me | H | H | |
| 1-700 | CHF2 | 2,2-Cl₂-c-Pr | Me | Me | Me | H | |
| 1-701 | CHF2 | 2,2-Cl₂-c-Pr | Me | Me | Me | 2,2-Cl₂-c-Pr | |
| 1-702 | CHF2 | 2,2-Me₂-c-Pr | c-Pr | Me | H | H | |
| 1-703 | CHF2 | 2,2-Cl₂-c-Pr | c-Pr | Me | Me | H | |
| 1-704 | CHF2 | 2,2-Cl₂-c-Pr | c-Pr | Me | Me | 2,2-Cl₂-c-Pr | |
| 1-705 | CHF2 | 2,2-Cl₂-c-Pr | -CH₂-OMe | Me | H | H | |
| 1-706 | CHF2 | 2,2-Cl₂-c-Pr | -CH₂-OMe | Me | Me | H | |
| 1-707 | CHF2 | 2,2-Cl₂-c-Pr | -CH₂-OMe | Me | Me | 2,2-Cl₂-c-Pr | |
| 1-708 | CHF2 | 2,2-Cl₂-c-Pr | -(CH₂)₂- | | H | H | |
| 1-709 | CHF2 | 2,2-Cl₂-c-Pr | -(CH₂)₃- | | H | H | |
| 1-710 | CHF2 | 2,2-Cl₂-c-Pr | -CH₂-CCl₂- | | H | H | |
| 1-711 | CHF2 | 2,2-Cl₂-c-Pr | -CH₂-CCl₂- | | H | 2,2-Cl₂-c-Pr | |

### Erläuterungen zu Tabelle 1:

"NMR" der Beispielverbindungen wurden, wenn nicht anders angegeben, jeweils als ¹H-NMR-Spektum bei 300 MHz (CDCl₃) (¹H-Kernresonanz-Daten) gemessen.
Nachfolgend sind charakterische chemische Verschiebungen δ (ppm) für einige Beispielverbindungen aufgeführt:

| Beisp. Nr. | δ (ppm) = |
|---|---|
| 1-19: | 8.10 (s, 1H), 3.30 (m, 1H), 0.95 (m, 2H), 0.55-0.25 (m, 8H); |
| 1-98: | 3.35 (m, 1H), 2.25 (s, 3H), 0.95 (m, 2H), 0.55-0.25 (m, 8H); |
| 1-177: | 3.35 (q, 1H), 2.50 (q, 2H), 1.25 (t, 3H), 0.95 (m, 2H), 0.55-0.25 (m, 8H); |
| 1-183: | 4.00 (q, 1H), 2.45 (m, 2H), 2.30 (m, 2H), 1.90-1.65 (m, 12H), 1.20 (t, 3H); |
| 1-256: | 3.20 (q, 1H), 1.65 (m, 1H), 1.00-0.95 (m, 2H), 0.90-0.80 (m, 4H), 0.45-0.20 (m, 8H); |
| 1-317: | 5.40-5.10 (m, 1H), 3.35 (m, 1H), 2.00-1.80 (m, 1H), 1.60 (dd, 3H), 0.95 (m, 7H), 0.55-0.25 (m, 4H); |
| 1-335: | 5.25 (dq, 1H), 3.30 (q, 1H), 1.60 (dd, 3H), 1.00-0.85 (m, 2H), 0.55-0.25 (m, 8H); |
| 1-341: | 5.30-5.00 (m, 1H), 4.10-3.90(m, 1H), 2.30 (m, 2H), 1.90-1.50 (m, 15H); |
| 1-414: | 5.15-4.90 (ddd, 1H), 3.35 (m, 1H), 2.05-1.85 (m, 2H), 1.05 (dd, 3H), 0.95 (m, 2H), 0.55-0.25 (m, 8H); |
| 1-420: | 5.10-4.80 (m, 1H), 3.95 (m, 1H), 2.25 (m, 2H), 2.00-1.30 (m, 14H), 0.95 (dd, 3H); |
| 1-475: | 3.30 (m, 1H), 1.95 (m, 1H), 1.60 (d, 6H), 0.95 (d, 6H), 0.85 (m, 1H), 0.60-0.25 (m, 4H); |
| 1-493: | 3.30 (m, 1H), 1.65 (d, 6H), 0.90 (m, 2H), 0.55-0.25 (m, 8H); |
| 1-499: | 4.00 (m, 1H), 2.25 (m, 2H), 1.90-1.50 (m, 18H); |
| 1-572: | 3.30-2.80 (m, 1H), 1.40-1.25 (d, 4H), 0.80 (m, 2H), 0.45-0.15 (m, 8H) |
| 1-651: | 6.12 (t, 1H), 3.30 (m, 1H), 0.95 (m, 2H), 0.60-0.30 (m, 8H) 1-651 wurde als ¹H-NMR-Spektum bei 400 MHz (CDCl₃) gemessen. |

**Tabelle 2: Verbindungen der Formel (Ib)**

| | | | | | | | |
|---|---|---|---|---|---|---|---|
| 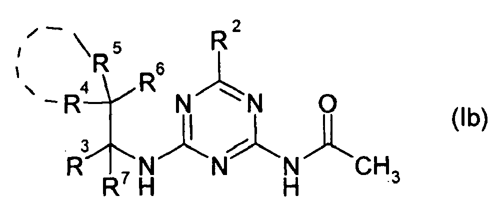 | | | | | | | |

| Nr. | R² | R³ | R⁴ | R⁵ | R⁶ | R⁷ | phys. Daten |
|---|---|---|---|---|---|---|---|
| 2-1 | H | c-Pr | Me | Me | H | H | |
| 2-2 | H | c-Pr | Me | Me | Me | H | |
| 2-3 | H | c-Pr | c-Pr | Me | H | H | |
| 2-4 | H | c-Pr | c-Pr | Me | Me | H | |
| 2-5 | H | c-Pr | -CH₂-OMe | Me | H | H | |
| 2-6 | H | c-Pr | -CH₂-OMe | Me | Me | H | |
| 2-7 | H | c-Pr | -(CH₂)₂- | | H | H | |
| 2-8 | H | c-Pr | -(CH₂)₂- | | H | Me | |
| 2-9 | H | c-Pr | -(CH₂)₂- | | H | c-Pr | |
| 2-10 | H | c-Bu | -(CH₂)₃- | | H | H | |
| 2-11 | H | c-Bu | -(CH₂)₃- | | H | Me | |
| 2-12 | H | c-Bu | -(CH₂)₃- | | H | c-Pr | |
| 2-13 | Me | c-Pr | Me | Me | H | H | |
| 2-14 | Me | c-Pr | Me | Me | Me | H | |
| 2-15 | Me | c-Pr | c-Pr | Me | H | H | |
| 2-16 | Me | c-Pr | c-Pr | Me | Me | H | |
| 2-17 | Me | c-Pr | -CH₂-OMe | Me | H | H | |
| 2-18 | Me | c-Pr | -CH₂-OMe | Me | Me | H | |
| 2-19 | Me | c-Pr | -(CH₂)₂- | | H | H | |
| 2-20 | Me | c-Pr | -(CH₂)₂- | | H | Me | |
| 2-21 | Me | c-Pr | -(CH₂)₂- | | H | c-Pr | |
| 2-22 | Me | c-Bu | -(CH₂)₃- | | H | H | |
| 2-23 | Me | c-Bu | -(CH₂)₃- | | H | Me | |
| 2-24 | Me | c-Bu | -(CH₂)₃- | | H | c-Pr | |
| 2-25 | Me | 1-Me-c-Pr | Me | Me | H | H | |
| 2-26 | Me | 1-Me-c-Pr | Me | Me | Me | H | |
| 2-27 | Me | 1-Me-c-Pr | c-Pr | Me | H | H | |
| 2-28 | Me | 1-Me-c-Pr | c-Pr | Me | Me | H | |
| 2-29 | Me | 1-Me-c-Pr | -(CH₂)₂- | | H | H | |
| 2-30 | Me | 1-Me-c-Pr | -(CH₂)₂- | | Me | H | |
| 2-31 | Me | 1-Cl-c-Pr | Me | Me | H | H | |
| 2-32 | Me | 1-Cl-c-Pr | Me | Me | Me | H | |
| 2-33 | Me | 1-Cl-c-Pr | c-Pr | Me | H | H | |
| 2-34 | Me | 1-Cl-c-Pr | c-Pr | Me | Me | H | |
| 2-35 | Me | 1-Cl-c-Pr | -(CH₂)₂- | | H | H | |
| 2-36 | Me | 1-Cl-c-Pr | -(CH₂)₂- | | Me | H | |
| 2-37 | Me | 2,2-Me₂-c-Pr | Me | Me | H | H | |
| 2-38 | Me | 2,2-Me₂-c-Pr | Me | Me | Me | H | |
| 2-39 | Me | 2,2-Me₂-c-Pr | c-Pr | Me | H | H | |
| 2-40 | Me | 2,2-Me₂-c-Pr | c-Pr | Me | Me | H | |
| 2-41 | Me | 2,2-Me₂-c-Pr | -(CH₂)₂- | | H | H | |
| 2-42 | Me | 2,2-Me₂-c-Pr | -CH₂-CMe₂- | | H | H | |
| 2-43 | Me | 2,2-Cl₂-c-Pr | Me | Me | H | H | |
| 2-44 | Me | 2,2-Cl₂-c-Pr | Me | Me | Me | H | |
| 2-45 | Me | 2,2-Cl₂-c-Pr | c-Pr | Me | H | H | |
| 2-46 | Me | 2,2-Cl₂-c-Pr | c-Pr | Me | Me | H | |
| 2-47 | Me | 2,2-Cl₂-c-Pr | -(CH₂)₂- | | H | H | |
| 2-48 | Me | 2,2-Cl₂-c-Pr | -CH₂-CCl₂ | | H | H | |
| 2-49 | Et | c-Pr | Me | Me | H | H | |
| 2-50 | Et | c-Pr | Me | Me | Me | H | |
| 2-51 | Et | c-Pr | c-Pr | Me | H | H | |
| 2-52 | Et | c-Pr | c-Pr | Me | Me | H | |
| 2-53 | Et | c-Pr | -CH₂-OMe | Me | H | H | |
| 2-54 | Et | c-Pr | -CH₂-OMe | Me | Me | H | |
| 2-55 | Et | c-Pr | -(CH₂)₂- | | H | H | |
| 2-56 | Et | c-Pr | -(CH₂)₂- | | H | Me | |
| 2-57 | Et | c-Pr | -(CH₂)₂- | | H | c-Pr | |
| 2-58 | Et | c-Bu | -(CH₂)₃- | | H | H | |
| 2-59 | Et | c-Bu | -(CH₂)₃- | | H | Me | |
| 2-60 | Et | c-Bu | -(CH₂)₃- | | H | c-Pr | |
| 2-61 | Et | 1-Me-c-Pr | Me | Me | H | H | |
| 2-62 | Et | 1-Me-c-Pr | Me | Me | Me | H | |
| 2-63 | Et | 1-Me-c-Pr | c-Pr | Me | H | H | |
| 2-64 | Et | 1-Me-c-Pr | c-Pr | Me | Me | H | |
| 2-65 | Et | 1-Me-c-Pr | -(CH₂)₂- | | H | H | |
| 2-66 | Et | 1-Me-c-Pr | -(CH₂)₂- | | Me | H | |
| 2-67 | Et | 1-Cl-c-Pr | Me | Me | H | H | |
| 2-68 | Et | 1-Cl-c-Pr | Me | Me | Me | H | |
| 2-69 | Et | 1-Cl-c-Pr | c-Pr | Me | H | H | |
| 2-70 | Et | 1-Cl-c-Pr | c-Pr | Me | Me | H | |
| 2-71 | Et | 1-Cl-c-Pr | -(CH₂)₂- | | H | H | |
| 2-72 | Et | 1-Cl-c-Pr | -(CH₂)₂- | | Me | H | |
| 2-73 | Et | 2,2-Me₂-c-Pr | Me | Me | H | H | |
| 2-74 | Et | 2,2-Me₂-c-Pr | Me | Me | Me | H | |
| 2-75 | Et | 2,2-Me₂-c-Pr | c-Pr | Me | H | H | |
| 2-76 | Et | 2,2-Me₂-c-Pr | c-Pr | Me | Me | H | |
| 2-77 | Et | 2,2-Me₂-c-Pr | -(CH₂)₂- | | H | H | |
| 2-78 | Et | 2,2-Me₂-c-Pr | -CH₂-CMe₂- | | H | H | |
| 2-79 | Et | 2,2-Cl₂-c-Pr | Me | Me | H | H | |
| 2-80 | Et | 2,2-Cl₂-c-Pr | Me | Me | Me | H | |
| 2-81 | Et | 2,2-Cl₂-c-Pr | c-Pr | Me | H | H | |
| 2-82 | Et | 2,2-Cl₂-c-Pr | c-Pr | Me | Me | H | |
| 2-83 | Et | 2,2-Cl₂-c-Pr | -(CH₂)₂- | | H | H | |
| 2-84 | Et | 2,2-Cl₂-c-Pr | -CH₂-CCl₂- | | H | H | |
| 2-85 | c-Pr | c-Pr | Me | Me | H | H | |
| 2-86 | c-Pr | c-Pr | Me | Me | Me | H | |
| 2-87 | c-Pr | c-Pr | c-Pr | Me | H | H | |
| 2-88 | c-Pr | c-Pr | c-Pr | Me | Me | H | |
| 2-89 | c-Pr | c-Pr | -CH₂-OMe | Me | H | H | |
| 2-90 | c-Pr | c-Pr | -CH₂-OMe | Me | Me | H | |
| 2-91 | c-Pr | c-Pr | -(CH₂)₂- | | H | H | |
| 2-92 | c-Pr | c-Pr | -(CH₂)₂- | | H | Me | |
| 2-93 | c-Pr | c-Pr | -(CH₂)₂- | | H | c-Pr | |
| 2-94 | c-Pr | c-Bu | -(CH₂)₃- | | H | H | |
| 2-95 | c-Pr | c-Bu | -(CH₂)₃- | | H | Me | |
| 2-96 | c-Pr | c-Bu | -(CH₂)₃- | | H | c-Pr | |
| 2-97 | c-Pr | 1-Me-c-Pr | Me | Me | H | H | |
| 2-98 | c-Pr | 1-Me-c-Pr | Me | Me | Me | H | |
| 2-99 | c-Pr | 1-Me-c-Pr | c-Pr | Me | H | H | |
| 2-100 | c-Pr | 1-Me-c-Pr | c-Pr | Me | Me | H | |
| 2-101 | c-Pr | 1-Me-c-Pr | -(CH₂)₂- | | H | H | |
| 2-102 | c-Pr | 1-Me-c-Pr | -(CH₂)₂- | | Me | H | |
| 2-103 | c-Pr | 1-Cl-c-Pr | Me | Me | H | H | |
| 2-104 | c-Pr | 1-Cl-c-Pr | Me | Me | Me | H | |
| 2-105 | c-Pr | 1-Cl-c-Pr | c-Pr | Me | H | H | |
| 2-106 | c-Pr | 1-Cl-c-Pr | c-Pr | Me | Me | H | |
| 2-107 | c-Pr | 1-Cl-c-Pr | -(CH₂)₂- | | H | H | |
| 2-108 | c-Pr | 1-Cl-c-Pr | -(CH₂)₂- | | Me | H | |
| 2-109 | c-Pr | 2,2-Me₂-c-Pr | Me | Me | H | H | |
| 2-110 | c-Pr | 2,2-Me₂-c-Pr | Me | Me | Me | H | |
| 2-111 | c-Pr | 2,2-Me₂-c-Pr | c-Pr | Me | H | H | |
| 2-112 | c-Pr | 2,2-Me₂-c-Pr | c-Pr | Me | Me | H | |
| 2-113 | c-Pr | 2,2-Me₂-c-Pr | -(CH₂)₂- | | H | H | |
| 2-114 | c-Pr | 2,2-Me₂-c-Pr | -CH₂-CMe₂- | | H | H | |
| 2-115 | c-Pr | 2,2-Cl₂-c-Pr | Me | Me | H | H | |
| 2-116 | c-Pr | 2,2-Cl₂-c-Pr | Me | Me | Me | H | |
| 2-117 | c-Pr | 2,2-Cl₂-c-Pr | c-Pr | Me | H | H | |
| 2-118 | c-Pr | 2,2-Cl₂-c-Pr | c-Pr | Me | Me | H | |
| 2-119 | c-Pr | 2,2-Cl₂-c-Pr | -(CH₂)₂- | | H | H | |
| 2-120 | c-Pr | 2,2-Cl₂-c-Pr | -CH₂-CCl₂- | | H | H | |
| 2-121 | CHFCH₃ | c-Pr | Me | Me | H | H | |
| 2-122 | CHFCH₃ | c-Pr | Me | Me | Me | H | |
| 2-123 | CHFCH₃ | c-Pr | c-Pr | Me | H | H | |
| 2-124 | CHFCH₃ | c-Pr | c-Pr | Me | Me | H | |
| 2-125 | CHFCH₃ | c-Pr | -CH₂-OMe | Me | H | H | |
| 2-126 | CHFCH₃ | c-Pr | -CH₂-OMe | Me | Me | H | |
| 2-127 | CHFCH₃ | c-Pr | -(CH₂)₂- | | H | H | |
| 2-128 | CHFCH₃ | c-Pr | -(CH₂)₂- | | H | Me | |
| 2-129 | CHFCH₃ | c-Pr | -(CH₂)₂- | | H | c-Pr | |
| 2-130 | CHFCH₃ | c-Bu | -(CH₂)₃- | | H | H | |
| 2-131 | CHFCH₃ | c-Bu | -(CH₂)₃- | | H | Me | |
| 2-132 | CHFCH₃ | c-Bu | -(CH₂)₃- | | H | c-Pr | |
| 2-133 | CHFCH₃ | 1-Me-c-Pr | Me | Me | H | H | |
| 2-134 | CHFCH₃ | 1-Me-c-Pr | Me | Me | Me | H | |
| 2-135 | CHFCH₃ | 1-Me-c-Pr | c-Pr | Me | H | H | |
| 2-136 | CHFCH₃ | 1-Me-c-Pr | c-Pr | Me | Me | H | |
| 2-137 | CHFCH₃ | 1-Me-c-Pr | -(CH₂)₂- | | H | H | |
| 2-138 | CHFCH₃ | 1-Me-c-Pr | -(CH₂)₂- | | Me | H | |
| 2-139 | CHFCH₃ | 1-Cl-c-Pr | Me | Me | H | H | |
| 2-140 | CHFCH₃ | 1-Cl-c-Pr | Me | Me | Me | H | |
| 2-141 | CHFCH₃ | 1-Cl-c-Pr | c-Pr | Me | H | H | |
| 2-142 | CHFCH₃ | 1-Cl-c-Pr | c-Pr | Me | Me | H | |
| 2-143 | CHFCH₃ | 1-Cl-c-Pr | -(CH₂)₂- | | H | H | |
| 2-144 | CHFCH₃ | 1-Cl-c-Pr | - (CH₂)₂ - | | Me | H | |
| 2-145 | CHFCH₃ | 2,2-Me₂-c-Pr | Me | Me | H | H | |
| 2-146 | CHFCH₃ | 2,2-Me₂-c-Pr | Me | Me | Me | H | |
| 2-147 | CHFCH₃ | 2,2-Me₂-c-Pr | c-Pr | Me | H | H | |
| 2-148 | CHFCH₃ | 2,2-Me₂-c-Pr | c-Pr | Me | Me | H | |
| 2-149 | CHFCH₃ | 2,2-Me₂-c-Pr | -(CH₂)₂- | | H | H | |
| 2-150 | CHFCH₃ | 2,2-Me₂-c-Pr | -CH₂-CMe₂- | | H | H | |
| 2-151 | CHFCH₃ | 2,2-Cl₂-c-Pr | Me | Me | H | H | |
| 2-152 | CHFCH₃ | 2,2-Cl₂-c-Pr | Me | Me | Me | H | |
| 2-153 | CHFCH₃ | 2,2-Cl₂-c-Pr | c-Pr | Me | H | H | |
| 2-154 | CHFCH₃ | 2,2-Cl₂-c-Pr | c-Pr | Me | Me | H | |
| 2-155 | CHFCH₃ | 2,2-Cl₂-c-Pr | -(CH₂)₂- | | H | H | |
| 2-156 | CHFCH₃ | 2,2-Cl₂-c-Pr | -CH₂-CCl₂- | | H | H | |
| 2-157 | CHFC₂H₅ | c-Pr | Me | Me | H | H | |
| 2-158 | CHFC₂H₅ | c-Pr | Me | Me | Me | H | |
| 2-159 | CHFC₂H₅ | c-Pr | c-Pr | Me | H | H | |
| 2-160 | CHFC₂H₅ | c-Pr | c-Pr | Me | Me | H | |
| 2-161 | CHFC₂H₅ | c-Pr | -CH₂-OMe | Me | H | H | |
| 2-162 | CHFC₂H₅ | c-Pr | -CH₂-OMe | Me | Me | H | |
| 2-163 | CHFC₂H₅ | c-Pr | -(CH₂)₂- | | H | H | |
| 2-164 | CHFC₂H₅ | c-Pr | -(CH₂)₂- | | H | Me | |
| 2-165 | CHFC₂H₅ | c-Pr | -(CH₂)₂- | | H | c-Pr | |
| 2-166 | CHFC₂H₅ | c-Bu | -(CH₂)₃- | | H | H | |
| 2-167 | CHFC₂H₅ | c-Bu | -(CH₂)₃- | | H | Me | |
| 2-168 | CHFC₂H₅ | c-Bu | -(CH₂)₃- | | H | c-Pr | |
| 2-169 | CHFC₂H₅ | 1-Me-c-Pr | Me | Me | H | H | |
| 2-170 | CHFC₂H₅ | 1-Me-c-Pr | Me | Me | Me | H | |
| 2-171 | CHFC₂H₅ | 1-Me-c-Pr | c-Pr | Me | H | H | |
| 2-172 | CHFC₂H₅ | 1-Me-c-Pr | c-Pr | Me | Me | H | |
| 2-173 | CHFC₂H₅ | 1-Me-c-Pr | -(CH₂)₂- | | H | H | |
| 2-174 | CHFC₂H₅ | 1-Me-c-Pr | -(CH₂)₂- | | Me | H | |
| 2-175 | CHFC₂H₅ | 1-Cl-c-Pr | Me | Me | H | H | |
| 2-176 | CHFC₂H₅ | 1-Cl-c-Pr | Me | Me | Me | H | |
| 2-177 | CHFC₂H₅ | 1-Cl-c-Pr | c-Pr | Me | H | H | |
| 2-178 | CHFC₂H₅ | 1-Cl-c-Pr | c-Pr | Me | Me | H | |
| 2-179 | CHFC₂H₅ | 1-Cl-c-Pr | -(CH₂)₂- | | H | H | |
| 2-180 | CHFC₂H₅ | 1-Cl-c-Pr | -(CH₂)₂- | | Me | H | |
| 2-181 | CHFC₂H₅ | 2,2-Me₂-c-Pr | Me | Me | H | H | |
| 2-182 | CHFC₂H₅ | 2,2-Me₂-c-Pr | Me | Me | Me | H | |
| 2-183 | CHFC₂H₅ | 2,2-Me₂-c-Pr | c-Pr | Me | H | H | |
| 2-184 | CHFC₂H₅ | 2,2-Me₂-c-Pr | c-Pr | Me | Me | H | |
| 2-185 | CHFC₂H₅ | 2,2-Me₂-c-Pr | -(CH₂)₂- | | H | H | |
| 2-186 | CHFC₂H₅ | 2,2-Me₂-c-Pr | -CH₂-CMe₂- | | H | H | |
| 2-187 | CHFC₂H₅ | 2,2-Cl₂-c-Pr | Me | Me | H | H | |
| 2-188 | CHFC₂H₅ | 2,2-Cl₂-c-Pr | Me | Me | Me | H | |
| 2-189 | CHFC₂H₅ | 2,2-Cl₂-c-Pr | c-Pr | Me | H | H | |
| 2-190 | CHFC₂H₅ | 2,2-Cl₂-c-Pr | c-Pr | Me | Me | H | |
| 2-191 | CHFC₂H₅ | 2,2-Cl₂-c-Pr | -(CH₂)₂- | | H | H | |
| 2-192 | CHFC₂H₅ | 2,2-Cl₂-c-Pr | -CH₂-CCl₂- | | H | H | |
| 2-193 | CF(CH₃)₂ | c-Pr | Me | Me | H | H | Harz |
| 2-194 | CF(CH₃)₂ | c-Pr | Me | Me | Me | H | |
| 2-195 | CF(CH₃)₂ | c-Pr | c-Pr | Me | H | H | |
| 2-196 | CF(CH₃)₂ | c-Pr | c-Pr | Me | Me | H | |
| 2-197 | CF(CH₃)₂ | c-Pr | -CH₂-OMe | Me | H | H | |
| 2-198 | CF(CH₃)₂ | c-Pr | -CH₂-OMe | Me | Me | H | |
| 2-199 | CF(CH₃)₂ | c-Pr | -(CH₂)₂- | | H | H | NMR |
| 2-200 | CF(CH₃)₂ | c-Pr | -(CH₂)₂- | | H | Me | |
| 2-201 | CF(CH₃)₂ | c-Pr | -(CH₂)₂- | | H | c-Pr | |
| 2-202 | CF(CH₃)₂ | c-Bu | -(CH₂)₃- | | H | H | Harz |
| 2-203 | CF(CH₃)₂ | c-Bu | -(CH₂)₃- | | H | Me | |
| 2-204 | CF(CH₃)₂ | c-Bu | -(CH₂)₃- | | H | c-Pr | |
| 2-205 | CF(CH₃)₂ | 1-Me-c-Pr | Me | Me | H | H | |
| 2-206 | CF(CH₃)₂ | 1-Me-c-Pr | Me | Me | Me | H | |
| 2-207 | CF(CH₃)₂ | 1-Me-c-Pr | c-Pr | Me | H | H | |
| 2-208 | CF(CH₃)₂ | 1-Me-c-Pr | c-Pr | Me | Me | H | |
| 2-209 | CF(CH₃)₂ | 1-Me-c-Pr | -(CH₂)₂- | | H | H | |
| 2-210 | CF(CH₃)₂ | 1-Me-c-Pr | -(CH₂)₂- | | Me | H | |
| 2-211 | CF(CH₃)₂ | 1-Cl-c-Pr | Me | Me | H | H | |
| 2-212 | CF(CH₃)₂ | 1-Cl-c-Pr | Me | Me | Me | H | |
| 2-213 | CF(CH₃)₂ | 1-Cl-c-Pr | c-Pr | Me | H | H | |
| 2-214 | CF(CH₃)₂ | 1-Cl-c-Pr | c-Pr | Me | Me | H | |
| 2-215 | CF(CH₃)₂ | 1-Cl-c-Pr | -(CH₂)₂- | | H | H | |
| 2-216 | CF(CH₃)₂ | 1-Cl-c-Pr | -(CH₂)₂- | | Me | H | |
| 2-217 | CF(CH₃)₂ | 2,2-Me₂-c-Pr | Me | Me | H | H | |
| 2-218 | CF(CH₃)₂ | 2,2-Me₂-c-Pr | Me | Me | Me | H | |
| 2-219 | CF(CH₃)₂ | 2,2-Me₂-c-Pr | c-Pr | Me | H | H | |
| 2-220 | CF(CH₃)₂ | 2,2-Me₂-c-Pr | c-Pr | Me | Me | H | |
| 2-221 | CF(CH₃)₂ | 2,2-Me₂-c-Pr | -(CH₂)₂- | | H | H | |
| 2-222 | CF(CH₃)₂ | 2,2-Me₂-c-Pr | -CH₂-CMe₂- | | H | H | |
| 2-223 | CF(CH₃)₂ | 2,2-Cl₂-c-Pr | Me | Me | H | H | |
| 2-224 | CF(CH₃)₂ | 2,2-Cl₂-c-Pr | Me | Me | Me | H | |
| 2-225 | CF(CH₃)₂ | 2,2-Cl₂-c-Pr | c-Pr | Me | H | H | |
| 2-226 | CF(CH₃)₂ | 2,2-Cl₂-c-Pr | c-Pr | Me | Me | H | |
| 2-227 | CF(CH₃)₂ | 2,2-Cl₂-c-Pr | -(CH₂)₂- | | H | H | |
| 2-228 | CF(CH₃)₂ | 2,2-Cl₂-c-Pr | -CH₂-CCl₂- | | H | H | |
| 2-229 | 1-F-c-Pr | c-Pr | Me | Me | H | H | |
| 2-230 | 1-F-c-Pr | c-Pr | Me | Me | Me | H | |
| 2-231 | 1-F-c-Pr | c-Pr | c-Pr | Me | H | H | |
| 2-232 | 1-F-c-Pr | c-Pr | c-Pr | Me | Me | H | |
| 2-233 | 1-F-c-Pr | c-Pr | -CH₂-OMe | Me | H | H | |
| 2-234 | 1-F-c-Pr | c-Pr | -CH₂-OMe | Me | Me | H | |
| 2-235 | 1-F-c-Pr | c-Pr | -(CH₂)₂- | | H | H | |
| 2-236 | 1-F-c-Pr | c-Pr | -(CH₂)₂- | | H | Me | |
| 2-237 | 1-F-c-Pr | c-Pr | -(CH₂)₂- | | H | c-Pr | |
| 2-238 | 1-F-c-Pr | c-Bu | -(CH₂)₃- | | H | H | |
| 2-239 | 1-F-c-Pr | c-Bu | -(CH₂)₃- | | H | Me | |
| 2-240 | 1-F-c-Pr | c-Bu | -(CH₂)₃- | | H | c-Pr | |
| 2-241 | 1-F-c-Pr | 1-Me-c-Pr | Me | Me | H | H | |
| 2-242 | 1-F-c-Pr | 1-Me-c-Pr | Me | Me | Me | H | |
| 2-243 | 1-F-c-Pr | 1-Me-c-Pr | c-Pr | Me | H | H | |
| 2-244 | 1-F-c-Pr | 1-Me-c-Pr | c-Pr | Me | Me | H | |
| 2-245 | 1-F-c-Pr | 1-Me-c-Pr | -(CH₂)₂- | | H | H | |
| 2-246 | 1-F-c-Pr | 1-Me-c-Pr | -(CH₂)₂- | | Me | H | |
| 2-247 | 1-F-c-Pr | 1-Cl-c-Pr | Me | Me | H | H | |
| 2-248 | 1-F-c-Pr | 1-Cl-c-Pr | Me | Me | Me | H | |
| 2-249 | 1-F-c-Pr | 1-Cl-c-Pr | c-Pr | Me | H | H | |
| 2-250 | 1-F-c-Pr | 1-Cl-c-Pr | c-Pr | Me | Me | H | |
| 2-251 | 1-F-c-Pr | 1-Cl-c-Pr | -(CH₂)₂- | | H | H | |
| 2-252 | 1-F-c-Pr | 1-Cl-c-Pr | -(CH₂)₂- | | Me | H | |
| 2-253 | 1-F-c-Pr | 2,2-Me₂-c-Pr | Me | Me | H | H | |
| 2-254 | 1-F-c-Pr | 2,2-Me₂-c-Pr | Me | Me | Me | H | |
| 2-255 | 1-F-c-Pr | 2,2-Me₂-c-Pr | c-Pr | Me | H | H | |
| 2-256 | 1-F-c-Pr | 2,2-Me₂-c-Pr | c-Pr | Me | Me | H | |
| 2-257 | 1-F-c-Pr | 2,2-Me₂-c-Pr | -(CH₂)₂- | | H | H | |
| 2-258 | 1-F-c-Pr | 2,2-Me₂-c-Pr | -CH₂-CMe₂- | | H | H | |
| 2-259 | 1-F-c-Pr | 2,2-Cl₂-c-Pr | Me | Me | H | H | |
| 2-260 | 1-F-c-Pr | 2,2-Cl₂-c-Pr | Me | Me | Me | H | |
| 2-261 | 1-F-c-Pr | 2,2-Cl₂-c-Pr | c-Pr | Me | H | H | |
| 2-262 | 1-F-c-Pr | 2,2-Cl₂-c-Pr | c-Pr | Me | Me | H | |
| 2-263 | 1-F-c-Pr | 2,2-Cl₂-c-Pr | -(CH₂)₂- | | H | H | |
| 2-264 | 1-F-c-Pr | 2,2-Cl₂-c-Pr | -CH₂-CCl₂- | | H | H | |
| 2-265 | CHF2 | c-Pr | Me | Me | H | H | |
| 2-266 | CHF2 | c-Pr | Me | Me | Me | H | |
| 2-267 | CHF2 | c-Pr | c-Pr | Me | H | H | |
| 2-268 | CHF2 | c-Pr | c-Pr | Me | Me | H | |
| 2-269 | CHF2 | c-Pr | -CH₂-OMe | Me | H | H | |
| 2-270 | CHF2 | c-Pr | -CH₂-OMe | Me | Me | H | |
| 2-271 | CHF2 | c-Pr | -(CH₂)₂- | | H | H | |
| 2-272 | CHF2 | c-Pr | -(CH₂)₂- | | H | Me | |
| 2-273 | CHF2 | c-Pr | -(CH₂)₂- | | H | c-Pr | |
| 2-274 | CHF2 | c-Bu | -(CH₂)₃- | | H | H | |
| 2-275 | CHF2 | c-Bu | -(CH₂)₃- | | H | Me | |
| 2-276 | CHF2 | c-Bu | -(CH₂)₃- | | H | c-Pr | |

### Erläuterungen zu Tabelle 2:

"NMR" der Beispielverbindungen wurden jeweils als ¹H-NMR-Spektum bei 400 MHz (CDCl₃) (¹H-Kernresonanz-Daten) gemessen. Nachfolgend sind charakterische chemische Verschiebungen δ (ppm) für Beispielverbindungen aufgeführt:

| Beisp. Nr. | δ (ppm) = |
|---|---|
| 2-199: | 3.30 (m, 1H), 2.60 und 2.55 (jeweils s, zusammen 3H), 1.70 - 1.60 (zweimal d, zusammen 6H), 1.10-0.90 (m, 2H), 0.55-0.25 (m, 8H) |

**Tabelle 3: Verbindungen der Formel (Ic)**

| | | | | | | | |
|---|---|---|---|---|---|---|---|
| 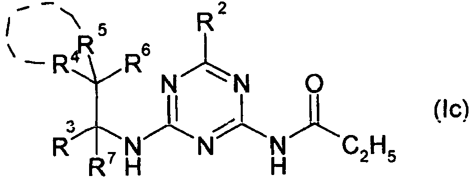 | | | | | | | |

| Nr. | R² | R³ | R⁴ | R⁵ | R⁶ | R⁷ | phys. Daten |
|---|---|---|---|---|---|---|---|
| 3-1 | H | c-Pr | Me | Me | H | H | |
| 3-2 | H | c-Pr | Me | Me | Me | H | |
| 3-3 | H | c-Pr | c-Pr | Me | H | H | |
| 3-4 | H | c-Pr | c-Pr | Me | Me | H | |
| 3-5 | H | c-Pr | -CH₂-OMe | Me | H | H | |
| 3-6 | H | c-Pr | -CH₂-OMe | Me | Me | H | |
| 3-7 | H | c-Pr | -(CH₂)₂- | | H | H | |
| 3-8 | H | c-Pr | -(CH₂)₂- | | H | Me | |
| 3-9 | H | c-Pr | -(CH₂)₂- | | H | c-Pr | |
| 3-10 | H | c-Bu | -(CH₂)₃- | | H | H | |
| 3-11 | H | c-Bu | -(CH₂)₃- | | H | Me | |
| 3-12 | H | c-Bu | -(CH₂)₃- | | H | c-Pr | |
| 3-13 | H | 1-Me-c-Pr | Me | Me | H | H | |
| 3-14 | H | 1-Me-c-Pr | Me | Me | Me | H | |
| 3-15 | H | 1-Me-c-Pr | c-Pr | Me | H | H | |
| 3-16 | H | 1-Me-c-Pr | c-Pr | Me | Me | H | |
| 3-17 | Me | c-Pr | Me | Me | H | H | |
| 3-18 | Me | c-Pr | Me | Me | Me | H | |
| 3-19 | Me | c-Pr | c-Pr | Me | H | H | |
| 3-20 | Me | c-Pr | c-Pr | Me | Me | H | |
| 3-21 | Me | c-Pr | -CH₂-OMe | Me | H | H | |
| 3-22 | Me | c-Pr | -CH₂-OMe | Me | Me | H | |
| 3-23 | Me | c-Pr | -(CH₂)₂- | | H | H | |
| 3-24 | Me | c-Pr | -(CH₂)₂- | | H | Me | |
| 3-25 | Me | c-Pr | -(CH₂)₂- | | H | c-Pr | |
| 3-26 | Me | c-Bu | -(CH₂)₃- | | H | H | |
| 3-27 | Me | c-Bu | -(CH₂)₃- | | H | Me | |
| 3-28 | Me | c-Bu | -(CH₂)₃- | | H | c-Pr | |
| 3-29 | Me | 1-Me-c-Pr | Me | Me | H | H | |
| 3-30 | Me | 1-Me-c-Pr | Me | Me | Me | H | |
| 3-31 | Me | 1-Me-c-Pr | c-Pr | Me | H | H | |
| 3-32 | Me | 1-Me-c-Pr | c-Pr | Me | Me | H | |
| 3-33 | Me | 1-Me-c-Pr | -(CH₂)₂- | | H | H | |
| 3-34 | Me | 1-Me-c-Pr | -(CH₂)₂- | | Me | H | |
| 3-35 | Me | 1-Cl-c-Pr | Me | Me | H | H | |
| 3-36 | Me | 1-Cl-c-Pr | Me | Me | Me | H | |
| 3-37 | Me | 1-Cl-c-Pr | c-Pr | Me | H | H | |
| 3-38 | Me | 1-Cl-c-Pr | c-Pr | Me | Me | H | |
| 3-39 | Me | 1-Cl-c-Pr | -(CH₂)₂- | | H | H | |
| 3-40 | Me | 1-Cl-c-Pr | -(CH₂)₂- | | Me | H | |
| 3-41 | Me | 2,2-Me₂-c-Pr | Me | Me | H | H | |
| 3-42 | Me | 2,2-Me₂-c-Pr | Me | Me | Me | H | |
| 3-43 | Me | 2,2-Me₂-c-Pr | c-Pr | Me | H | H | |
| 3-44 | Me | 2,2-Me₂-c-Pr | c-Pr | Me | Me | H | |
| 3-45 | Me | 2,2-Me₂-c-Pr | -(CH₂)₂- | | H | H | |
| 3-46 | Me | 2,2-Me₂-c-Pr | -CH₂-CMe₂- | | H | H | |
| 3-47 | Me | 2,2-Cl₂-c-Pr | Me | Me | H | H | |
| 3-48 | Me | 2,2-Cl₂-c-Pr | Me | Me | Me | H | |
| 3-49 | Me | 2,2-Cl₂-c-Pr | c-Pr | Me | H | H | |
| 3-50 | Me | 2,2-Cl₂-c-Pr | c-Pr | Me | Me | H | |
| 3-51 | Me | 2,2-Cl₂-c-Pr | -(CH₂)₂- | | H | H | |
| 3-52 | Me | 2,2-Cl₂-c-Pr | -CH₂-CCl₂- | | H | H | |
| 3-53 | Et | c-Pr | Me | Me | H | H | |
| 3-54 | Et | c-Pr | Me | Me | Me | H | |
| 3-55 | Et | c-Pr | c-Pr | Me | H | H | |
| 3-56 | Et | c-Pr | c-Pr | Me | Me | H | |
| 3-57 | Et | c-Pr | -CH₂-OMe | Me | H | H | |
| 3-58 | Et | c-Pr | -CH₂-OMe | Me | Me | H | |
| 3-59 | Et | c-Pr | -(CH₂)₂- | | H | H | |
| 3-60 | Et | c-Pr | -(CH₂)₂- | | H | Me | |
| 3-61 | Et | c-Pr | -(CH₂)₂- | | H | c-Pr | |
| 3-62 | Et | c-Bu | -(CH₂)₃- | | H | H | |
| 3-63 | Et | c-Bu | -(CH₂)₃- | | H | Me | |
| 3-64 | Et | c-Bu | -(CH₂)₃- | | H | c-Pr | |
| 3-65 | Et | 1-Me-c-Pr | Me | Me | H | H | |
| 3-66 | Et | 1-Me-c-Pr | Me | Me | Me | H | |
| 3-67 | Et | 1-Me-c-Pr | c-Pr | Me | H | H | |
| 3-68 | Et | 1-Me-c-Pr | c-Pr | Me | Me | H | |
| 3-69 | Et | 1-Me-c-Pr | -(CH₂)₂- | | H | H | |
| 3-70 | Et | 1-Me-c-Pr | -(CH₂)₂- | | Me | H | |
| 3-71 | Et | 1-Cl-c-Pr | Me | Me | H | H | |
| 3-72 | Et | 1-Cl-c-Pr | Me | Me | Me | H | |
| 3-73 | Et | 1-Cl-c-Pr | c-Pr | Me | H | H | |
| 3-74 | Et | 1-Cl-c-Pr | c-Pr | Me | Me | H | |
| 3-75 | Et | 1-Cl-c-Pr | -(CH₂)₂- | | H | H | |
| 3-76 | Et | 1-Cl-c-Pr | -(CH₂)₂- | | Me | H | |
| 3-77 | Et | 2,2-Me₂-c-Pr | Me | Me | H | H | |
| 3-78 | Et | 2,2-Me₂-c-Pr | Me | Me | Me | H | |
| 3-79 | Et | 2,2-Me₂-c-Pr | c-Pr | Me | H | H | |
| 3-80 | Et | 2,2-Me₂-c-Pr | c-Pr | Me | Me | H | |
| 3-81 | Et | 2,2-Me₂-c-Pr | -(CH₂)₂- | | H | H | |
| 3-82 | Et | 2,2-Me₂-c-Pr | -CH₂-CMe₂- | | H | H | |
| 3-83 | Et | 2,2-Cl₂-c-Pr | Me | Me | H | H | |
| 3-84 | Et | 2,2-Cl₂-c-Pr | Me | Me | Me | H | |
| 3-85 | Et | 2,2-Cl₂-c-Pr | c-Pr | Me | H | H | |
| 3-86 | Et | 2,2-Cl₂-c-Pr | c-Pr | Me | Me | H | |
| 3-87 | Et | 2,2-Cl₂-c-Pr | -(CH₂)₂- | | H | H | |
| 3-88 | Et | 2,2-Cl₂-c-Pr | -CH₂-CCl₂- | | H | H | |
| 3-89 | c-Pr | c-Pr | Me | Me | H | H | |
| 3-90 | c-Pr | c-Pr | Me | Me | Me | H | |
| 3-91 | c-Pr | c-Pr | c-Pr | Me | H | H | |
| 3-92 | c-Pr | c-Pr | c-Pr | Me | Me | H | |
| 3-93 | c-Pr | c-Pr | -CH₂-OMe | Me | H | H | |
| 3-94 | c-Pr | c-Pr | -CH₂-OMe | Me | Me | H | |
| 3-95 | c-Pr | c-Pr | -(CH₂)₂- | | H | H | |
| 3-96 | c-Pr | c-Pr | -(CH₂)₂- | | H | Me | |
| 3-97 | c-Pr | c-Pr | -(CH₂)₂- | | H | c-Pr | |
| 3-98 | c-Pr | c-Bu | -(CH₂)₃- | | H | H | |
| 3-99 | c-Pr | c-Bu | -(CH₂)₃- | | H | Me | |
| 3-100 | c-Pr | c-Bu | -(CH₂)₃- | | H | c-Pr | |
| 3-101 | c-Pr | 1-Me-c-Pr | Me | Me | H | H | |
| 3-102 | c-Pr | 1-Me-c-Pr | Me | Me | Me | H | |
| 3-103 | c-Pr | 1-Me-c-Pr | c-Pr | Me | H | H | |
| 3-104 | c-Pr | 1-Me-c-Pr | c-Pr | Me | Me | H | |
| 3-105 | c-Pr | 1-Me-c-Pr | -(CH₂)₂- | | H | H | |
| 3-106 | c-Pr | 1-Me-c-Pr | -(CH₂)₂- | | Me | H | |
| 3-107 | c-Pr | 1-Cl-c-Pr | Me | Me | H | H | |
| 3-108 | c-Pr | 1-Cl-c-Pr | Me | Me | Me | H | |
| 3-109 | c-Pr | 1-Cl-c-Pr | c-Pr | Me | H | H | |
| 3-110 | c-Pr | 1-Cl-c-Pr | c-Pr | Me | Me | H | |
| 3-111 | c-Pr | 1-Cl-c-Pr | -(CH₂)₂- | | H | H | |
| 3-112 | c-Pr | 1-Cl-c-Pr | -(CH₂)₂- | | Me | H | |
| 3-113 | c-Pr | 2,2-Me₂-c-Pr | Me | Me | H | H | |
| 3-114 | c-Pr | 2,2-Me₂-c-Pr | Me | Me | Me | H | |
| 3-115 | c-Pr | 2,2-Me₂-c-Pr | c-Pr | Me | H | H | |
| 3-116 | c-Pr | 2,2-Me₂-c-Pr | c-Pr | Me | Me | H | |
| 3-117 | c-Pr | 2,2-Me₂-c-Pr | -(CH₂)₂- | | H | H | |
| 3-118 | c-Pr | 2,2-Me₂-c-Pr | -CH₂-CMe₂- | | H | H | |
| 3-119 | c-Pr | 2,2-Cl₂-c-Pr | Me | Me | H | H | |
| 3-120 | c-Pr | 2,2-Cl₂-c-Pr | Me | Me | Me | H | |
| 3-121 | c-Pr | 2,2-Cl₂-c-Pr | c-Pr | Me | H | H | |
| 3-122 | c-Pr | 2,2-Cl₂-c-Pr | c-Pr | Me | Me | H | |
| 3-123 | c-Pr | 2,2-Cl₂-c-Pr | -(CH₂)₂- | | H | H | |
| 3-124 | c-Pr | 2,2-Cl₂-c-Pr | -CH₂-CCl₂- | | H | H | |
| 3-125 | CHFCH₃ | c-Pr | Me | Me | H | H | |
| 3-126 | CHFCH₃ | c-Pr | Me | Me | Me | H | |
| 3-127 | CHFCH₃ | c-Pr | c-Pr | Me | H | H | |
| 3-128 | CHFCH₃ | c-Pr | c-Pr | Me | Me | H | |
| 3-129 | CHFCH₃ | c-Pr | -CH₂-OMe | Me | H | H | |
| 3-130 | CHFCH₃ | c-Pr | -CH₂-OMe | Me | Me | H | |
| 3-131 | CHFCH₃ | c-Pr | -(CH₂)₂- | | H | H | |
| 3-132 | CHFCH₃ | c-Pr | -(CH₂)₂- | | H | Me | |
| 3-133 | CHFCH₃ | c-Pr | -(CH₂)₂- | | H | c-Pr | |
| 3-134 | CHFCH₃ | c-Bu | -(CH₂)₃- | | H | H | |
| 3-135 | CHFCH₃ | c-Bu | -(CH₂)₃- | | H | Me | |
| 3-136 | CHFCH₃ | c-Bu | -(CH₂)₃- | | H | c-Pr | |
| 3-137 | CHFCH₃ | 1-Me-c-Pr | Me | Me | H | H | |
| 3-138 | CHFCH₃ | 1-Me-c-Pr | Me | Me | Me | H | |
| 3-139 | CHFCH₃ | 1-Me-c-Pr | c-Pr | Me | H | H | |
| 3-140 | CHFCH₃ | 1-Me-c-Pr | c-Pr | Me | Me | H | |
| 3-141 | CHFCH₃ | 1-Me-c-Pr | -(CH₂)₂- | | H | H | |
| 3-142 | CHFCH₃ | 1-Me-c-Pr | -(CH₂)₂- | | Me | H | |
| 3-143 | CHFCH₃ | 1-Cl-c-Pr | Me | Me | H | H | |
| 3-144 | CHFCH₃ | 1-Cl-c-Pr | Me | Me | Me | H | |
| 3-145 | CHFCH₃ | 1-Cl-c-Pr | c-Pr | Me | H | H | |
| 3-146 | CHFCH₃ | 1-Cl-c-Pr | c-Pr | Me | Me | H | |
| 3-147 | CHFCH₃ | 1-Cl-c-Pr | -(CH₂)₂- | | H | H | |
| 3-148 | CHFCH₃ | 1-Cl-c-Pr | -(CH₂)₂- | | Me | H | |
| 3-149 | CHFCH₃ | 2,2-Me₂-c-Pr | Me | Me | H | H | |
| 3-150 | CHFCH₃ | 2,2-Me₂-c-Pr | Me | Me | Me | H | |
| 3-151 | CHFCH₃ | 2,2-Me₂-c-Pr | c-Pr | Me | H | H | |
| 3-152 | CHFCH₃ | 2,2-Me₂-c-Pr | c-Pr | Me | Me . | H | |
| 3-153 | CHFCH₃ | 2,2-Me₂-c-Pr | -(CH₂)₂- | | H | H | |
| 3-154 | CHFCH₃ | 2,2-Me₂-c-Pr | -CH₂-CMe₂- | | H | H | |
| 3-155 | CHFCH₃ | 2,2-Cl₂-c-Pr | Me | Me | H | H | |
| 3-156 | CHFCH₃ | 2,2-Cl₂-c-Pr | Me | Me | Me | H | |
| 3-157 | CHFCH₃ | 2,2-Cl₂-c-Pr | c-Pr | Me | H | H | |
| 3-158 | CHFCH₃ | 2,2-Cl₂-c-Pr | c-Pr | Me | Me | H | |
| 3-159 | CHFCH₃ | 2,2-Cl₂-c-Pr | -(CH₂)₂- | | H | H | |
| 3-160 | CHFCH₃ | 2,2-Cl₂-c-Pr | -CH₂-CCl₂- | | H | H | |
| 3-161 | CHFC₂H₅ | c-Pr | Me | Me | H | H | |
| 3-162 | CHFC₂H₅ | c-Pr | Me | Me | Me | H | |
| 3-163 | CHFC₂H₅ | c-Pr | c-Pr | Me | H | H | |
| 3-164 | CHFC₂H₅ | c-Pr | c-Pr | Me | Me | H | |
| 3-165 | CHFC₂H₅ | c-Pr | -CH₂-OMe | Me | H | H | |
| 3-166 | CHFC₂H₅ | c-Pr | -CH₂-OMe | Me | Me | H | |
| 3-167 | CHFC₂H₅ | c-Pr | -(CH₂)₂- | | H | H | |
| 3-168 | CHFC₂H₅ | c-Pr | -(CH₂)₂- | | H | Me | |
| 3-169 | CHFC₂H₅ | c-Pr | -(CH₂)₂- | | H | c-Pr | |
| 3-170 | CHFC₂H₅ | c-Bu | -(CH₂)₃- | | H | H | |
| 3-171 | CHFC₂H₅ | c-Bu | -(CH₂)₃- | | H | Me | |
| 3-172 | CHFC₂H₅ | c-Bu | -(CH₂)₃- | | H | c-Pr | |
| 3-173 | CHFC₂H₅ | 1-Me-c-Pr | Me | Me | H | H | |
| 3-174 | CHFC₂H₅ | 1-Me-c-Pr | Me | Me | Me | H | |
| 3-175 | CHFC₂H₅ | 1-Me-c-Pr | c-Pr | Me | H | H | |
| 3-176 | CHFC₂H₅ | 1-Me-c-Pr | c-Pr | Me | Me | H | |
| 3-177 | CHFC₂H₅ | 1-Me-c-Pr | -(CH₂)₂- | | H | H | |
| 3-178 | CHFC₂H₅ | 1-Me-c-Pr | -(CH₂)₂- | | Me | H | |
| 3-179 | CHFC₂H₅ | 1-Cl-c-Pr | Me | Me | H | H | |
| 3-180 | CHFC₂H₅ | 1-Cl-c-Pr | Me | Me | Me | H | |
| 3-181 | CHFC₂H₅ | 1-Cl-c-Pr | c-Pr | Me | H | H | |
| 3-182 | CHFC₂H₅ | 1-Cl-c-Pr | c-Pr | Me | Me | H | |
| 3-183 | CHFC₂H₅ | 1-Cl-c-Pr | -(CH₂)₂- | | H | H | |
| 3-184 | CHFC₂H₅ | 1-Cl-c-Pr | -(CH₂)₂- | | Me | H | |
| 3-185 | CHFC₂H₅ | 2,2-Me₂-c-Pr | Me | Me | H | H | |
| 3-186 | CHFC₂H₅ | 2,2-Me₂-c-Pr | Me | Me | Me | H | |
| 3-187 | CHFC₂H₅ | 2,2-Me₂-c-Pr | c-Pr | Me | H | H | |
| 3-188 | CHFC₂H₅ | 2,2-Me₂-c-Pr | c-Pr | Me | Me | H | |
| 3-189 | CHFC₂H₅ | 2,2-Me₂-c-Pr | -(CH₂)₂- | | H | H | |
| 3-190 | CHFC₂H₅ | 2,2-Me₂-c-Pr | -CH₂-CMe₂- | | H | H | |
| 3-191 | CHFC₂H₅ | 2,2-Cl₂-c-Pr | Me | Me | H | H | |
| 3-192 | CHFC₂H₅ | 2,2-Cl₂-c-Pr | Me | Me | Me | H | |
| 3-193 | CHFC₂H₅ | 2,2-Cl₂-c-Pr | c-Pr | Me | H | H | |
| 3-194 | CHFC₂H₅ | 2,2-Cl₂-c-Pr | c-Pr | Me | Me | H | |
| 3-195 | CHFC₂H₅ | 2,2-Cl₂-c-Pr | -(CH₂)₂- | | H | H | |
| 3-196 | CHFC₂H₅ | 2,2-Cl₂-c-Pr | -CH₂-CCl₂- | | H | H | |
| 3-197 | CF(CH₃)₂ | c-Pr | Me | Me | H | H | Harz |
| 3-198 | CF(CH₃)₂ | c-Pr | Me | Me | Me | H | |
| 3-199 | CF(CH₃)₂ | c-Pr | c-Pr | Me | H | H | |
| 3-200 | CF(CH₃)₂ | c-Pr | c-Pr | Me | Me | H | |
| 3-201 | CF(CH₃)₂ | c-Pr | -CH₂-OMe | Me | H | H | |
| 3-202 | CF(CH₃)₂ | c-Pr | -CH₂-OMe | Me | Me | H | |
| 3-203 | CF(CH₃)₂ | c-Pr | -(CH₂)₂- | | H | H | Harz |
| 3-204 | CF(CH₃)₂ | c-Pr | -(CH₂)₂- | | H | Me | |
| 3-205 | CF(CH₃)₂ | c-Pr | -(CH₂)₂- | | H | c-Pr | |
| 3-206 | CF(CH₃)₂ | c-Bu | -(CH₂)₃- | | H | H | Harz |
| 3-207 | CF(CH₃)₂ | c-Bu | -(CH₂)₃- | | H | Me | |
| 3-208 | CF(CH₃)₂ | c-Bu | -(CH₂)₃- | | H | c-Pr | |
| 3-209 | CF(CH₃)₂ | 1-Me-c-Pr | Me | Me | H | H | |
| 3-210 | CF(CH₃)₂ | 1-Me-c-Pr | Me | Me | Me | H | |
| 3-211 | CF(CH₃)₂ | 1-Me-c-Pr | c-Pr | Me | H | H | |
| 3-212 | CF(CH₃)₂ | 1-Me-c-Pr | c-Pr | Me | Me | H | |
| 3-213 | CF(CH₃)₂ | 1-Me-c-Pr | -(CH₂)₂- | | H | H | |
| 3-214 | CF(CH₃)₂ | 1-Me-c-Pr | -(CH₂)₂- | | Me | H | |
| 3-215 | CF(CH₃)₂ | 1-Cl-c-Pr | Me | Me | H | H | |
| 3-216 | CF(CH₃)₂ | 1-Cl-c-Pr | Me | Me | Me | H | |
| 3-217 | CF(CH₃)₂ | 1-Cl-c-Pr | c-Pr | Me | H | H | |
| 3-218 | CF(CH₃)₂ | 1-Cl-c-Pr | c-Pr | Me | Me | H | |
| 3-219 | CF(CH₃)₂ | 1-Cl-c-Pr | -(CH₂)₂- | | H | H | |
| 3-220 | CF(CH₃)₂ | 1-Cl-c-Pr | -(CH₂)₂- | | Me | H | |
| 3-221 | CF(CH₃)₂ | 2,2-Me₂-c-Pr | Me | Me | H | H | |
| 3-222 | CF(CH₃)₂ | 2,2-Me₂-c-Pr | Me | Me | Me | H | |
| 3-223 | CF(CH₃)₂ | 2,2-Me₂-c-Pr | c-Pr | Me | H | H | |
| 3-224 | CF(CH₃)₂ | 2,2-Me₂-c-Pr | c-Pr | Me | Me | H | |
| 3-225 | CF(CH₃)₂ | 2,2-Me₂-c-Pr | -(CH₂)₂- | | H | H | |
| 3-226 | CF(CH₃)₂ | 2,2-Me₂-c-Pr | -CH₂-CMe₂- | | H | H | |
| 3-227 | CF(CH₃)₂ | 2,2-Cl₂-c-Pr | Me | Me | H | H | |
| 3-228 | CF(CH₃)₂ | 2,2-Cl₂-c-Pr | Me | Me | Me | H | |
| 3-229 | CF(CH₃)₂ | 2,2-Cl₂-c-Pr | c-Pr | Me | H | H | |
| 3-230 | CF(CH₃)₂ | 2,2-Cl₂-c-Pr | c-Pr | Me | Me | H | |
| 3-231 | CF(CH₃)₂ | 2,2-Cl₂-c-Pr | -(CH₂)₂- | | H | H | |
| 3-232 | CF(CH₃)₂ | 2,2-Cl₂-c-Pr | -CH₂-CCl₂- | | H | H | |
| 3-233 | 1-F-c-Pr | c-Pr | Me | Me | H | H | |
| 3-234 | 1-F-c-Pr | c-Pr | Me | Me | Me | H | |
| 3-235 | 1-F-c-Pr | c-Pr | c-Pr | Me | H | H | |
| 3-236 | 1-F-c-Pr | c-Pr | c-Pr | Me | Me | H | |
| 3-237 | 1-F-c-Pr | c-Pr | -CH₂-OMe | Me | H | H | |
| 3-238 | 1-F-c-Pr | c-Pr | -CH₂-OMe | Me | Me | H | |
| 3-239 | 1-F-c-Pr | c-Pr | -(CH₂)₂- | | H | H | |
| 3-240 | 1-F-c-Pr | c-Pr | -(CH₂)₂- | | H | Me | |
| 3-241 | 1-F-c-Pr | c-Pr | -(CH₂)₂- | | H | c-Pr | |
| 3-242 | 1-F-c-Pr | c-Bu | -(CH₂)₃- | | H | H | |
| 3-243 | 1-F-c-Pr | c-Bu | -(CH₂)₃- | | H | Me | |
| 3-244 | 1-F-c-Pr | c-Bu | -(CH₂)₃- | | H | c-Pr | |
| 3-245 | 1-F-c-Pr | 1-Me-c-Pr | Me | Me | H | H | |
| 3-246 | 1-F-c-Pr | 1-Me-c-Pr | Me | Me | Me | H | |
| 3-247 | 1-F-c-Pr | 1-Me-c-Pr | c-Pr | Me | H | H | |
| 3-248 | 1-F-c-Pr | 1-Me-c-Pr | c-Pr | Me | Me | H | |
| 3-249 | 1-F-c-Pr | 1-Me-c-Pr | -(CH₂)₂- | | H | H | |
| 3-250 | 1-F-c-Pr | 1-Me-c-Pr | -(CH₂)₂- | | Me | H | |
| 3-251 | 1-F-c-Pr | 1-Cl-c-Pr | Me | Me | H | H | |
| 3-252 | 1-F-c-Pr | 1-Cl-c-Pr | Me | Me | Me | H | |
| 3-253 | 1-F-c-Pr | 1-Cl-c-Pr | c-Pr | Me | H | H | |
| 3-254 | 1-F-c-Pr | 1-Cl-c-Pr | c-Pr | Me | Me | H | |
| 3-255 | 1-F-c-Pr | 1-Cl-c-Pr | -(CH₂)₂- | | H | H | |
| 3-256 | 1-F-c-Pr | 1-Cl-c-Pr | -(CH₂)₂- | | Me | H | |
| 3-257 | 1-F-c-Pr | 2,2-Me₂-c-Pr | Me | Me | H | H | |
| 3-258 | 1-F-c-Pr | 2,2-Me₂-c-Pr | Me | Me | Me | H | |
| 3-259 | 1-F-c-Pr | 2,2-Me₂-c-Pr | c-Pr | Me | H | H | |
| 3-260 | 1-F-c-Pr | 2,2-Me₂-c-Pr | c-Pr | Me | Me | H | |
| 3-261 | 1-F-c-Pr | 2,2-Me₂-c-Pr | -(CH₂)₂- | | H | H | |
| 3-262 | 1-F-c-Pr | 2,2-Me₂-c-Pr | -CH₂-CMe₂- | | H | H | |
| 3-263 | 1-F-c-Pr | 2,2-Cl₂-c-Pr | Me | Me | H | H | |
| 3-264 | 1-F-c-Pr | 2,2-Cl₂-c-Pr | Me | Me | Me | H | |
| 3-265 | 1-F-c-Pr | 2,2-Cl₂-c-Pr | c-Pr | Me | H | H | |
| 3-266 | 1-F-c-Pr | 2,2-Cl₂-c-Pr | c-Pr | Me | Me | H | |
| 3-267 | 1-F-c-Pr | 2,2-Cl₂-c-Pr | -(CH₂)₂- | | H | H | |
| 3-268 | 1-F-c-Pr | 2,2-Cl₂-c-Pr | -CH₂-CCl₂- | | H | H | |
| 3-269 | CHF2 | c-Pr | Me | Me | H | H | |
| 3-270 | CHF2 | c-Pr | Me | Me | Me | H | |
| 3-271 | CHF2 | c-Pr | c-Pr | Me | H | H | |
| 3-272 | CHF2 | c-Pr | c-Pr | Me | Me | H | |
| 3-273 | CHF2 | c-Pr | -CH₂-OMe | Me | H | H | |
| 3-274 | CHF2 | c-Pr | -CH₂-OMe | Me | Me | H | |
| 3-275 | CHF2 | c-Pr | -(CH₂)₂- | | H | H | |
| 3-276 | CHF2 | c-Pr | -(CH₂)₂- | | H | Me | |
| 3-277 | CHF2 | c-Pr | -(CH₂)₂- | | H | c-Pr | |
| 3-278 | CHF2 | c-Bu | -(CH₂)₃- | | H | H | |
| 3-279 | CHF2 | c-Bu | -(CH₂)₃- | | H | Me | |
| 3-280 | CHF2 | c-Bu | -(CH₂)₃- | | H | c-Pr | |

**Tabelle 4: Verbindungen der Formel (Id)**

| | | | | | | | |
|---|---|---|---|---|---|---|---|
| 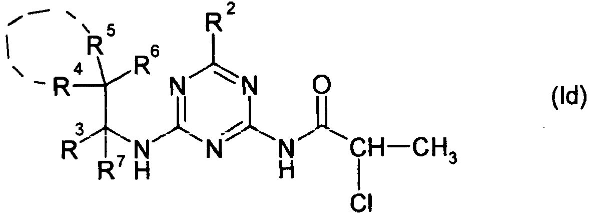 | | | | | | | |

| Nr. | R² | R³ | R⁴ | R⁵ | R⁶ | R⁷ | phys. Daten |
|---|---|---|---|---|---|---|---|
| 4-1 | H | c-Pr | Me | Me | H | H | |
| 4-2 | H | c-Pr | Me | Me | Me | H | |
| 4-3 | H | c-Pr | c-Pr | Me | H | H | |
| 4-4 | H | c-Pr | c-Pr | Me | Me | H | |
| 4-5 | H | c-Pr | -CH₂-OMe | Me | H | H | |
| 4-6 | H | c-Pr | -CH₂-OMe | Me | Me | H | |
| 4-7 | H | c-Pr | -(CH₂)₂- | | H | H | |
| 4-8 | H | c-Pr | -(CH₂)₂- | | H | Me | |
| 4-9 | H | c-Pr | -(CH₂)₂- | | H | c-Pr | |
| 4-10 | H | c-Bu | -(CH₂)₃- | | H | H | |
| 4-11 | H | c-Bu | -(CH₂)₃- | | H | Me | |
| 4-12 | H | c-Bu | -(CH₂)₃- | | H | c-Pr | |
| 4-13 | H | 1-Me-c-Pr | Me | Me | H | H | |
| 4-14 | H | 1-Me-c-Pr | Me | Me | Me | H | |
| 4-15 | Me | c-Pr | Me | Me | H | H | |
| 4-16 | Me | c-Pr | Me | Me | Me | H | |
| 4-17 | Me | c-Pr | c-Pr | Me | H | H | |
| 4-18 | Me | c-Pr | c-Pr | Me | Me | H | |
| 4-19 | Me | c-Pr | -CH₂-OMe | Me | H | H | |
| 4-20 | Me | c-Pr | -CH₂-OMe | Me | Me | H | |
| 4-21 | Me | c-Pr | -(CH₂)₂- | | H | H | |
| 4-22 | Me | c-Pr | -(CH₂)₂- | | H | Me | |
| 4-23 | Me | c-Pr | -(CH₂)₂- | | H | c-Pr | |
| 4-24 | Me | c-Bu | -(CH₂)₃- | | H | H | |
| 4-25 | Me | c-Bu | -(CH₂)₃- | | H | Me | |
| 4-26 | Me | c-Bu | -(CH₂)₃- | | H | c-Pr | |
| 4-27 | Me | 1-Me-c-Pr | Me | Me | H | H | |
| 4-28 | Me | 1-Me-c-Pr | Me | Me | Me | H | |
| 4-29 | Me | 1-Me-c-Pr | c-Pr | Me | H | H | |
| 4-30 | Me | 1-Me-c-Pr | c-Pr | Me | Me | H | |
| 4-31 | Me | 1-Me-c-Pr | -(CH₂)₂- | | H | H | |
| 4-32 | Me | 1-Me-c-Pr | -(CH₂)₂- | | Me | H | |
| 4-33 | Me | 1-Cl-c-Pr | Me | Me | H | H | |
| 4-34 | Me | 1-Cl-c-Pr | Me | Me | Me | H | |
| 4-35 | Me | 1-Cl-c-Pr | c-Pr | Me | H | H | |
| 4-36 | Me | 1-Cl-c-Pr | c-Pr | Me | Me | H | |
| 4-37 | Me | 1-Cl-c-Pr | -(CH₂)₂- | | H | H | |
| 4-38 | Me | 1-Cl-c-Pr | -(CH₂)₂- | | Me | H | |
| 4-39 | Me | 2,2-Me₂-c-Pr | Me | Me | H | H | |
| 4-40 | Me | 2,2-Me₂-c-Pr | Me | Me | Me | H | |
| 4-41 | Me | 2,2-Me₂-c-Pr | c-Pr | Me | H | H | |
| 4-42 | Me | 2,2-Me₂-c-Pr | c-Pr | Me | Me | H | |
| 4-43 | Me | 2,2-Me₂-c-Pr | -(CH₂)₂- | | H | H | |
| 4-44 | Me | 2,2-Me₂-c-Pr | -CH₂-CMe₂- | | H | H | |
| 4-45 | Me | 2,2-Cl₂-c-Pr | Me | Me | H | H | |
| 4-46 | Me | 2,2-Cl₂-c-Pr | Me | Me | Me | H | |
| 4-47 | Me | 2,2-Cl₂-c-Pr | c-Pr | Me | H | H | |
| 4-48 | Me | 2,2-Cl₂-c-Pr | c-Pr | Me | Me | H | |
| 4-49 | Me | 2,2-Cl₂-c-Pr | -(CH₂)₂- | | H | H | |
| 4-50 | Me | 2,2-Cl₂-c-Pr | -CH₂-CCl₂- | | H | H | |
| 4-51 | Et | c-Pr | Me | Me | H | H | |
| 4-52 | Et | c-Pr | Me | Me | Me | H | |
| 4-53 | Et | c-Pr | c-Pr | Me | H | H | |
| 4-54 | Et | c-Pr | c-Pr | Me | Me | H | |
| 4-55 | Et | c-Pr | -CH₂-OMe | Me | H | H | |
| 4-56 | Et | c-Pr | -CH₂-OMe | Me | Me | H | |
| 4-57 | Et | c-Pr | -(CH₂)₂- | | H | H | |
| 4-58 | Et | c-Pr | -(CH₂)₂- | | H | Me | |
| 4-59 | Et | c-Pr | -(CH₂)₂- | | H | c-Pr | |
| 4-60 | Et | c-Bu | -(CH₂)₃- | | H | H | |
| 4-61 | Et | c-Bu | -(CH₂)₃- | | H | Me | |
| 4-62 | Et | c-Bu | -(CH₂)₃- | | H | c-Pr | |
| 4-63 | Et | 1-Me-c-Pr | Me | Me | H | H | |
| 4-64 | Et | 1-Me-c-Pr | Me | Me | Me | H | |
| 4-65 | Et | 1-Me-c-Pr | c-Pr | Me | H | H | |
| 4-66 | Et | 1-Me-c-Pr | c-Pr | Me | Me | H | |
| 4-67 | Et | 1-Me-c-Pr | -(CH₂)₂- | | H | H | |
| 4-68 | Et | 1-Me-c-Pr | -(CH₂)₂- | | Me | H | |
| 4-69 | Et | 1-Cl-c-Pr | Me | Me | H | H | |
| 4-70 | Et | 1-Cl-c-Pr | Me | Me | Me | H | |
| 4-71 | Et | 1-Cl-c-Pr | c-Pr | Me | H | H | |
| 4-72 | Et | 1-Cl-c-Pr | c-Pr | Me | Me | H | |
| 4-73 | Et | 1-Cl-c-Pr | -(CH₂)₂- | | H | H | |
| 4-74 | Et | 1-Cl-c-Pr | -(CH₂)₂- | | Me | H | |
| 4-75 | Et | 2,2-Me₂-c-Pr | Me | Me | H | H | |
| 4-76 | Et | 2,2-Me₂-c-Pr | Me | Me | Me | H | |
| 4-77 | Et | 2,2-Me₂-c-Pr | c-Pr | Me | H | H | |
| 4-78 | Et | 2,2-Me₂-c-Pr | c-Pr | Me | Me | H | |
| 4-79 | Et | 2,2-Me₂-c-Pr | -(CH₂)₂- | | H | H | |
| 4-80 | Et | 2,2-Me₂-c-Pr | -CH₂-CMe₂- | | H | H | |
| 4-81 | Et | 2,2-Cl₂-c-Pr | Me | Me | H | H | |
| 4-82 | Et | 2,2-Cl₂-c-Pr | Me | Me | Me | H | |
| 4-83 | Et | 2,2-Cl₂-c-Pr | c-Pr | Me | H | H | |
| 4-84 | Et | 2,2-Cl₂-c-Pr | c-Pr | Me | Me | H | |
| 4-85 | Et | 2,2-Cl₂-c-Pr | -(CH₂)₂- | | H | H | |
| 4-86 | Et | 2,2-Cl₂-c-Pr | -CH₂-CCl₂- | | H | H | |
| 4-87 | c-Pr | c-Pr | Me | Me | H | H | |
| 4-88 | c-Pr | c-Pr | Me | Me | Me | H | |
| 4-89 | c-Pr | c-Pr | c-Pr | Me | H | H | |
| 4-90 | c-Pr | c-Pr | c-Pr | Me | Me | H | |
| 4-91 | c-Pr | c-Pr | -CH₂-OMe | Me | H | H | |
| 4-92 | c-Pr | c-Pr | -CH₂-OMe | Me | Me | H | |
| 4-93 | c-Pr | c-Pr | -(CH₂)₂- | | H | H | |
| 4-94 | c-Pr | c-Pr | -(CH₂)₂- | | H | Me | |
| 4-95 | c-Pr | c-Pr | -(CH₂)₂- | | H | c-Pr | |
| 4-96 | c-Pr | c-Bu | -(CH₂)₃- | | H | H | |
| 4-97 | c-Pr | c-Bu | -(CH₂)₃- | | H | Me | |
| 4-98 | c-Pr | c-Bu | -(CH₂)₃- | | H | c-Pr | |
| 4-99 | c-Pr | 1-Me-c-Pr | Me | Me | H | H | |
| 4-100 | c-Pr | 1-Me-c-Pr | Me | Me | Me | H | |
| 4-101 | c-Pr | 1-Me-c-Pr | c-Pr | Me | H | H | |
| 4-102 | c-Pr | 1-Me-c-Pr | c-Pr | Me | Me | H | |
| 4-103 | c-Pr | 1-Me-c-Pr | -(CH₂)₂- | | H | H | |
| 4-104 | c-Pr | 1-Me-c-Pr | -(CH₂)₂- | | Me | H | |
| 4-105 | c-Pr | 1-Cl-c-Pr | Me | Me | H | H | |
| 4-106 | c-Pr | 1-Cl-c-Pr | Me | Me | Me | H | |
| 4-107 | c-Pr | 1-Cl-c-Pr | c-Pr | Me | H | H | |
| 4-108 | c-Pr | 1-Cl-c-Pr | c-Pr | Me | Me | H | |
| 4-109 | c-Pr | 1-Cl-c-Pr | -(CH₂)₂- | | H | H | |
| 4-110 | c-Pr | 1-Cl-c-Pr | -(CH₂)₂- | | Me | H | |
| 4-111 | c-Pr | 2,2-Me₂-c-Pr | Me | Me | H | H | |
| 4-112 | c-Pr | 2,2-Me₂-c-Pr | Me | Me | Me | H | |
| 4-113 | c-Pr | 2,2-Me₂-c-Pr | c-Pr | Me | H | H | |
| 4-114 | c-Pr | 2,2-Me₂-c-Pr | c-Pr | Me | Me | H | |
| 4-115 | c-Pr | 2,2-Me₂-c-Pr | -(CH₂)₂- | | H | H | |
| 4-116 | c-Pr | 2,2-Me₂-c-Pr | -CH₂-CMe₂- | | H | H | |
| 4-117 | c-Pr | 2,2-Cl₂-c-Pr | Me | Me | H | H | |
| 4-118 | c-Pr | 2,2-Cl₂-c-Pr | Me | Me | Me | H | |
| 4-119 | c-Pr | 2,2-Cl₂-c-Pr | c-Pr | Me | H | H | |
| 4-120 | c-Pr | 2,2-Cl₂-c-Pr | c-Pr | Me | Me | H | |
| 4-121 | c-Pr | 2,2-Cl₂-c-Pr | -(CH₂)₂- | | H | H | |
| 4-122 | c-Pr | 2,2-Cl₂-c-Pr | -CH₂-CCl₂- | | H | H | |
| 4-123 | CHFCH₃ | c-Pr | Me | Me | H | H | |
| 4-124 | CHFCH₃ | c-Pr | Me | Me | Me | H | |
| 4-125 | CHFCH₃ | c-Pr | c-Pr | Me | H | H | |
| 4-126 | CHFCH₃ | c-Pr | c-Pr | Me | Me | H | |
| 4-127 | CHFCH₃ | c-Pr | -CH₂-OMe | Me | H | H | |
| 4-128 | CHFCH₃ | c-Pr | -CH₂-OMe | Me | Me | H | |
| 4-129 | CHFCH₃ | c-Pr | -(CH₂)₂- | | H | H | |
| 4-130 | CHFCH₃ | c-Pr | -(CH₂)₂- | | H | Me | |
| 4-131 | CHFCH₃ | c-Pr | -(CH₂)₂- | | H | c-Pr | |
| 4-132 | CHFCH₃ | c-Bu | -(CH₂)₃- | | H | H | |
| 4-133 | CHFCH₃ | c-Bu | -(CH₂)₃- | | H | Me | |
| 4-134 | CHFCH₃ | c-Bu | -(CH₂)₃- | | H | c-Pr | |
| 4-135 | CHFCH₃ | 1-Me-c-Pr | Me | Me | H | H | |
| 4-136 | CHFCH₃ | 1-Me-c-Pr | Me | Me | Me | H | |
| 4-137 | CHFCH₃ | 1-Me-c-Pr | c-Pr | Me | H | H | |
| 4-138 | CHFCH₃ | 1-Me-c-Pr | c-Pr | Me | Me | H | |
| 4-139 | CHFCH₃ | 1-Me-c-Pr | -(CH₂)₂- | | H | H | |
| 4-140 | CHFCH₃ | 1-Me-c-Pr | -(CH₂)₂- | | Me | H | |
| 4-141 | CHFCH₃ | 1-Cl-c-Pr | Me | Me | H | H | |
| 4-142 | CHFCH₃ | 1-Cl-c-Pr | Me | Me | Me | H | |
| 4-143 | CHFCH₃ | 1-Cl-c-Pr | c-Pr | Me | H | H | |
| 4-144 | CHFCH₃ | 1-Cl-c-Pr | c-Pr | Me | Me | H | |
| 4-145 | CHFCH₃ | 1-Cl-c-Pr | -(CH₂)₂- | | H | H | |
| 4-146 | CHFCH₃ | 1-Cl-c-Pr | -(CH₂)₂- | | Me | H | |
| 4-147 | CHFCH₃ | 2,2-Me₂-c-Pr | Me | Me | H | H | |
| 4-148 | CHFCH₃ | 2,2-Me₂-c-Pr | Me | Me | Me | H | |
| 4-149 | CHFCH₃ | 2,2-Me₂-c-Pr | c-Pr | Me | H | H | |
| 4-150 | CHFCH₃ | 2,2-Me₂-c-Pr | c-Pr | Me | Me | H | |
| 4-151 | CHFCH₃ | 2,2-Me₂-c-Pr | -(CH₂)₂- | | H | H | |
| 4-152 | CHFCH₃ | 2,2-Me₂-c-Pr | -CH₂-CMe₂- | | H | H | |
| 4-153 | CHFCH₃ | 2,2-Cl₂-c-Pr | Me | Me | H | H | |
| 4-154 | CHFCH₃ | 2,2-Cl₂-c-Pr | Me | Me | Me | H | |
| 4-155 | CHFCH₃ | 2,2-Cl₂-c-Pr | c-Pr | Me | H | H | |
| 4-156 | CHFCH₃ | 2,2-Cl₂-c-Pr | c-Pr | Me | Me | H | |
| 4-157 | CHFCH₃ | 2,2-Cl₂-c-Pr | -(CH₂)₂- | | H | H | |
| 4-158 | CHFCH₃ | 2,2-Cl₂-c-Pr | -CH₂-CCl₂- | | H | H | |
| 4-159 | CHFC₂H ₅ | c-Pr | Me | Me | H | H | |
| 4-160 | CHFC₂H ₅ | c-Pr | Me | Me | Me | H | |
| 4-161 | CHFC₂H ₅ | c-Pr | c-Pr | Me | H | H | |
| 4-162 | CHFC₂H ₅ | c-Pr | c-Pr | Me | Me | H | |
| 4-163 | CHFC₂H ₅ | c-Pr | -CH₂-OMe | Me | H | H | |
| 4-164 | CHFC₂H ₅ | c-Pr | -CH₂-OMe | Me | Me | H | |
| 4-165 | CHFC₂H ₅ | c-Pr | -(CH₂)₂- | | H | H | |
| 4-166 | CHFC₂H ₅ | c-Pr | -(CH₂)₂- | | H | Me | |
| 4-167 | CHFC₂H ₅ | c-Pr | -(CH₂)₂- | | H | c-Pr | |
| 4-168 | CHFC₂H ₅ | c-Bu | -(CH₂)₃- | | H | H | |
| 4-169 | CHFC₂H ₅ | c-Bu | -(CH₂)₃- | | H | Me | |
| 4-170 | CHFC₂H ₅ | c-Bu | -(CH₂)₃- | | H | c-Pr | |
| 4-171 | CHFC₂H ₅ | 1-Me-c-Pr | Me | Me | H | H | |
| 4-172 | CHFC₂H ₅ | 1-Me-c-Pr | Me | Me | Me | H | |
| 4-173 | CHFC₂H ₅ | 1-Me-c-Pr | c-Pr | Me | H | H | |
| 4-174 | CHFC₂H ₅ | 1-Me-c-Pr | c-Pr | Me | Me | H | |
| 4-175 | CHFC₂H ₅ | 1-Me-c-Pr | -(CH₂)₂- | | H | H | |
| 4-176 | CHFC₂H ₅ | 1-Me-c-Pr | -(CH₂)₂- | | Me | H | |
| 4-177 | CHFC₂H₅ | 1-Cl-c-Pr | Me | Me | H | H | |
| 4-178 | CHFC₂H ₅ | 1-Cl-c-Pr | Me | Me | Me | H | |
| 4-179 | CHFC₂H ₅ | 1-Cl-c-Pr | c-Pr | Me | H | H | |
| 4-180 | CHFC₂H ₅ | 1-Cl-c-Pr | c-Pr | Me | Me | H | |
| 4-181 | CHFC₂H ₅ | 1-Cl-c-Pr | -(CH₂)₂- | | H | H | |
| 4-182 | CHFC₂H ₅ | 1-Cl-c-Pr | -(CH₂)₂- | | Me | H | |
| 4-183 | CHFC₂H ₅ | 2,2-Me₂-c-Pr | Me | Me | H | H | |
| 4-184 | CHFC₂H ₅ | 2,2-Me₂-c-Pr | Me | Me | Me | H | |
| 4-185 | CHFC₂H ₅ | 2,2-Me₂-c-Pr | c-Pr | Me | H | H | |
| 4-186 | CHFC₂H ₅ | 2,2-Me₂-c-Pr | c-Pr | Me | Me | H | |
| 4-187 | CHFC₂H ₅ | 2,2-Me₂-c-Pr | -(CH₂)₂- | | H | H | |
| 4-188 | CHFC₂H ₅ | 2,2-Me₂-c-Pr | -CH₂-CMe₂- | | H | H | |
| 4-189 | CHFC₂H ₅ | 2,2-Cl₂-c-Pr | Me | Me | H | H | |
| 4-190 | CHFC₂H ₅ | 2,2-Cl₂-c-Pr | Me | Me | Me | H | |
| 4-191 | CHFC₂H ₅ | 2,2-Cl₂-c-Pr | c-Pr | Me | H | H | |
| 4-192 | CHFC₂H ₅ | 2,2-Cl₂-c-Pr | c-Pr | Me | Me | H | |
| 4-193 | CHFC₂H ₅ | 2,2-Cl₂-c-Pr | -(CH₂)₂- | | H | H | |
| 4-194 | CHFC₂H ₅ | 2,2-Cl₂-c-Pr | -CH₂-CCl₂- | | H | H | |
| 4-195 | CF(CH₃)₂ | c-Pr | Me | Me | H | H | Harz |
| 4-196 | CF(CH₃)₂ | c-Pr | Me | Me | Me | H | |
| 4-197 | CF(CH₃)₂ | c-Pr | c-Pr | Me | H | H | |
| 4-198 | CF(CH₃)₂ | c-Pr | c-Pr | Me | Me | H | |
| 4-199 | CF(CH₃)₂ | c-Pr | -CH₂-OMe | Me | H | H | |
| 4-200 | CF(CH₃)₂ | c-Pr | -CH₂-OMe | Me | Me | H | |
| 4-201 | CF(CH₃)₂ | c-Pr | -(CH₂)₂- | | H | H | Harz |
| 4-202 | CF(CH₃)₂ | c-Pr | -(CH₂)₂- | | H | Me | |
| 4-203 | CF(CH₃)₂ | c-Pr | -(CH₂)₂- | | H | c-Pr | |
| 4-204 | CF(CH₃)₂ | c-Bu | -(CH₂)₃- | | H | H | Harz |
| 4-205 | CF(CH₃)₂ | c-Bu | -(CH₂)₃- | | H | Me | |
| 4-206 | CF(CH₃)₂ | c-Bu | -(CH₂)₃- | | H | c-Pr | |
| 4-207 | CF(CH₃)₂ | 1-Me-c-Pr | Me | Me | H | H | |
| 4-208 | CF(CH₃)₂ | 1-Me-c-Pr | Me | Me | Me | H | |
| 4-209 | CF(CH₃)₂ | 1-Me-c-Pr | c-Pr | Me | H | H | |
| 4-210 | CF(CH₃)₂ | 1-Me-c-Pr | c-Pr | Me | Me | H | |
| 4-211 | CF(CH₃)₂ | 1-Me-c-Pr | -(CH₂)₂- | | H | H | |
| 4-212 | CF(CH₃)₂ | 1-Me-c-Pr | -(CH₂)₂- | | Me | H | |
| 4-213 | CF(CH₃)₂ | 1-Cl-c-Pr | Me | Me | H | H | |
| 4-214 | CF(CH₃)₂ | 1-Cl-c-Pr | Me | Me | Me | H | |
| 4-215 | CF(CH₃)₂ | 1-Cl-c-Pr | c-Pr | Me | H | H | |
| 4-216 | CF(CH₃)₂ | 1-Cl-c-Pr | c-Pr | Me | Me | H | |
| 4-217 | CF(CH₃)₂ | 1-Cl-c-Pr | -(CH₂)₂- | | H | H | |
| 4-218 | CF(CH₃)₂ | 1-Cl-c-Pr | -(CH₂)₂- | | Me | H | |
| 4-219 | CF(CH₃)₂ | 2,2-Me₂-c-Pr | Me | Me | H | H | |
| 4-220 | CF(CH₃)₂ | 2,2-Me₂-c-Pr | Me | Me | Me | H | |
| 4-221 | CF(CH₃)₂ | 2,2-Me₂-c-Pr | c-Pr | Me | H | H | |
| 4-222 | CF(CH₃)₂ | 2,2-Me₂-c-Pr | c-Pr | Me | Me | H | |
| 4-223 | CF(CH₃)₂ | 2,2-Me₂-c-Pr | -(CH₂)₂- | | H | H | |
| 4-224 | CF(CH₃)₂ | 2,2-Me₂-c-Pr | -CH₂-CMe₂- | | H | H | |
| 4-225 | CF(CH₃)₂ | 2,2-Cl₂-c-Pr | Me | Me | H | H | |
| 4-226 | CF(CH₃)₂ | 2,2-Cl₂-c-Pr | Me | Me | Me | H | |
| 4-227 | CF(CH₃)₂ | 2,2-Cl₂-c-Pr | c-Pr | Me | H | H | |
| 4-228 | CF(CH₃)₂ | 2,2-Cl₂-c-Pr | c-Pr | Me | Me | H | |
| 4-229 | CF(CH₃)₂ | 2,2-Cl₂-c-Pr | -(CH₂)₂- | | H | H | |
| 4-230 | CF(CH₃)₂ | 2,2-Cl₂-c-Pr | -CH₂-CCl₂- | | H | H | |
| 4-231 | 1-F-c-Pr | c-Pr | Me | Me | H | H | |
| 4-232 | 1-F-c-Pr | c-Pr | Me | Me | Me | H | |
| 4-233 | 1-F-c-Pr | c-Pr | c-Pr | Me | H | H | |
| 4-234 | 1-F-c-Pr | c-Pr | c-Pr | Me | Me | H | |
| 4-235 | 1-F-c-Pr | c-Pr | -CH₂-OMe | Me | H | H | |
| 4-236 | 1-F-c-Pr | c-Pr | -CH₂-OMe | Me | Me | H | |
| 4-237 | 1-F-c-Pr | c-Pr | -(CH₂)₂- | | H | H | |
| 4-238 | 1-F-c-Pr | c-Pr | -(CH₂)₂- | | H | Me | |
| 4-239 | 1-F-c-Pr | c-Pr | -(CH₂)₂- | | H | c-Pr | |
| 4-240 | 1-F-c-Pr | c-Bu | -(CH₂)₃- | | H | H | |
| 4-241 | 1-F-c-Pr | c-Bu | -(CH₂)₃- | | H | Me | |
| 4-242 | 1-F-c-Pr | c-Bu | -(CH₂)₃- | | H | c-Pr | |
| 4-243 | 1-F-c-Pr | 1-Me-c-Pr | Me | Me | H | H | |
| 4-244 | 1-F-c-Pr | 1-Me-c-Pr | Me | Me | Me | H | |
| 4-245 | 1-F-c-Pr | 1-Me-c-Pr | c-Pr | Me | H | H | |
| 4-246 | 1-F-c-Pr | 1-Me-c-Pr | c-Pr | Me | Me | H | |
| 4-247 | 1-F-c-Pr | 1-Me-c-Pr | -(CH₂)₂- | | H | H | |
| 4-248 | 1-F-c-Pr | 1-Me-c-Pr | -(CH₂)₂- | | Me | H | |
| 4-249 | 1-F-c-Pr | 1-Cl-c-Pr | Me | Me | H | H | |
| 4-250 | 1-F-c-Pr | 1-Cl-c-Pr | Me | Me | Me | H | |
| 4-251 | 1-F-c-Pr | 1-Cl-c-Pr | c-Pr | Me | H | H | |
| 4-252 | 1-F-c-Pr | 1-Cl-c-Pr | c-Pr | Me | Me | H | |
| 4-253 | 1-F-c-Pr | 1-Cl-c-Pr | -(CH₂)₂- | | H | H | |
| 4-254 | 1-F-c-Pr | 1-Cl-c-Pr | -(CH₂)₂- | | Me | H | |
| 4-255 | 1-F-c-Pr | 2,2-Me₂-c-Pr | Me | Me | H | H | |
| 4-256 | 1-F-c-Pr | 2,2-Me₂-c-Pr | Me | Me | Me | H | |
| 4-257 | 1-F-c-Pr | 2,2-Me₂-c-Pr | c-Pr | Me | H | H | |
| 4-258 | 1-F-c-Pr | 2,2-Me₂-c-Pr | c-Pr | Me | Me | H | |
| 4-259 | 1-F-c-Pr | 2,2-Me₂-c-Pr | -(CH₂)₂- | | H | H | |
| 4-260 | 1-F-c-Pr | 2,2-Me₂-c-Pr | -CH₂-CMe₂- | | H | H | |
| 4-261 | 1-F-c-Pr | 2,2-Cl₂-c-Pr | Me | Me | H | H | |
| 4-262 | 1-F-c-Pr | 2,2-Cl₂-c-Pr | Me | Me | Me | H | |
| 4-263 | 1-F-c-Pr | 2,2-Cl₂-c-Pr | c-Pr | Me | H | H | |
| 4-264 | 1-F-c-Pr | 2,2-Cl₂-c-Pr | c-Pr | Me | Me | H | |
| 4-265 | 1-F-c-Pr | 2,2-Cl₂-c-Pr | -(CH₂)₂- | | H | H | |
| 4-266 | 1-F-c-Pr | 2,2-Cl₂-c-Pr | -CH₂-CCl₂- | | H | H | |
| 4-267 | CHF2 | c-Pr | Me | Me | H | H | |
| 4-268 | CHF2 | c-Pr | Me | Me | Me | H | |
| 4-269 | CHF2 | c-Pr | c-Pr | Me | H | H | |
| 4-270 | CHF2 | c-Pr | c-Pr | Me | Me | H | |
| 4-271 | CHF2 | c-Pr | -CH₂-OMe | Me | H | H | |
| 4-272 | CHF2 | c-Pr | -CH₂-OMe | Me | Me | H | |
| 4-273 | CHF2 | c-Pr | -(CH₂)₂- | | H | H | |
| 4-274 | CHF2 | c-Pr | -(CH₂)₂- | | H | Me | |
| 4-275 | CHF2 | c-Pr | -(CH₂)₂- | | H | c-Pr | |
| 4-276 | CHF2 | c-Bu | -(CH₂)₃- | | H | H | |
| 4-277 | CHF2 | c-Bu | -(CH₂)₃- | | H | Me | |
| 4-278 | CHF2 | c-Bu | -(CH₂)₃- | | H | c-Pr | |

**Tabelle 5: Verbindungen der Formel (Ie)**

| | | | | | | | |
|---|---|---|---|---|---|---|---|
| 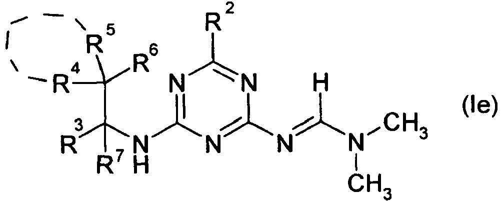 | | | | | | | |

| Nr. | R² | R³ | R⁴ | R⁵ | R⁶ | R⁷ | phys. Daten |
|---|---|---|---|---|---|---|---|
| 5-1 | H | c-Pr | Me | Me | H | H | |
| 5-2 | H | c-Pr | Me | Me | Me | H | |
| 5-3 | H | c-Pr | c-Pr | Me | H | H | |
| 5-4 | H | c-Pr | c-Pr | Me | Me | H | |
| 5-5 | H | c-Pr | -CH₂-OMe | Me | H | H | |
| 5-6 | H | c-Pr | -CH₂-OMe | Me | Me | H | |
| 5-7 | H | c-Pr | -(CH₂)₂- | | H | H | |
| 5-8 | H | c-Pr | -(CH₂)₂- | | H | Me | |
| 5-9 | H | c-Pr | -(CH₂)₂- | | H | c-Pr | |
| 5-10 | H | c-Bu | -(CH₂)₃- | | H | H | |
| 5-11 | H | c-Bu | -(CH₂)₃- | | H | Me | |
| 5-12 | H | c-Bu | -(CH₂)₃- | | H | c-Pr | |
| 5-13 | H | 1-Me-c-Pr | Me | Me | H | H | |
| 5-14 | H | 1-Me-c-Pr | Me | Me | Me | H | |
| 5-15 | Me | c-Pr | Me | Me | H | H | |
| 5-16 | Me | c-Pr | Me | Me | Me | H | |
| 5-17 | Me | c-Pr | c-Pr | Me | H | H | |
| 5-18 | Me | c-Pr | c-Pr | Me | Me | H | |
| 5-19 | Me | c-Pr | -CH₂-OMe | Me | H | H | |
| 5-20 | Me | c-Pr | -CH₂-OMe | Me | Me | H | |
| 5-21 | Me | c-Pr | -(CH₂)₂- | | H | H | |
| 5-22 | Me | c-Pr | -(CH₂)₂- | | H | Me | |
| 5-23 | Me | c-Pr | -(CH₂)₂- | | H | c-Pr | |
| 5-24 | Me | c-Bu | -(CH₂)₃- | | H | H | |
| 5-25 | Me | c-Bu | -(CH₂)₃- | | H | Me | |
| 5-26 | Me | c-Bu | -(CH₂)₃- | | H | c-Pr | |
| 5-27 | Me | 1-Me-c-Pr | Me | Me | H | H | |
| 5-28 | Me | 1-Me-c-Pr | Me | Me | Me | H | |
| 5-29 | Me | 1-Me-c-Pr | c-Pr | Me | H | H | |
| 5-30 | Me | 1-Me-c-Pr | c-Pr | Me | Me | H | |
| 5-31 | Me | 1-Me-c-Pr | -(CH₂)₂- | | H | H | |
| 5-32 | Me | 1-Me-c-Pr | -(CH₂)₂- | | Me | H | |
| 5-33 | Me | 1-Cl-c-Pr | Me | Me | H | H | |
| 5-34 | Me | 1-Cl-c-Pr | Me | Me | Me | H | |
| 5-35 | Me | 1-Cl-c-Pr | c-Pr | Me | H | H | |
| 5-36 | Me | 1-Cl-c-Pr | c-Pr | Me | Me | H | |
| 5-37 | Me | 1-Cl-c-Pr | -(CH₂)₂- | | H | H | |
| 5-38 | Me | 1-Cl-c-Pr | -(CH₂)₂- | | Me | H | |
| 5-39 | Me | 2,2-Me₂-c-Pr | Me | Me | H | H | |
| 5-40 | Me | 2,2-Me₂-c-Pr | Me | Me | Me | H | |
| 5-41 | Me | 2,2-Me₂-c-Pr | c-Pr | Me | H | H | |
| 5-42 | Me | 2,2-Me₂-c-Pr | c-Pr | Me | Me | H | |
| 5-43 | Me | 2,2-Me₂-c-Pr | -(CH₂)₂- | | H | H | |
| 5-44 | Me | 2,2-Me₂-c-Pr | -CH₂-CMe₂- | | H | H | |
| 5-45 | Me | 2,2-Cl₂-c-Pr | Me | Me | H | H | |
| 5-46 | Me | 2,2-Cl₂-c-Pr | Me | Me | Me | H | |
| 5-47 | Me | 2,2-Cl₂-c-Pr | c-Pr | Me | H | H | |
| 5-48 | Me | 2,2-Cl₂-c-Pr | c-Pr | Me | Me | H | |
| 5-49 | Me | 2,2-Cl₂-c-Pr | -(CH₂)₂- | | H | H | |
| 5-50 | Me | 2,2-Cl₂-c-Pr | -CH₂-CCl₂- | | H | H | |
| 5-51 | Et | c-Pr | Me | Me | H | H | |
| 5-52 | Et | c-Pr | Me | Me | Me | H | |
| 5-53 | Et | c-Pr | c-Pr | Me | H | H | |
| 5-54 | Et | c-Pr | c-Pr | Me | Me | H | |
| 5-55 | Et | c-Pr | -CH₂-OMe | Me | H | H | |
| 5-56 | Et | c-Pr | -CH₂-OMe | Me | Me | H | |
| 5-57 | Et | c-Pr | -(CH₂)₂- | | H | H | |
| 5-58 | Et | c-Pr | -(CH₂)₂- | | H | Me | |
| 5-59 | Et | c-Pr | -(CH₂)₂- | | H | c-Pr | |
| 5-60 | Et | c-Bu | -(CH₂)₃- | | H | H | |
| 5-61 | Et | c-Bu | -(CH₂)₃- | | H | Me | |
| 5-62 | Et | c-Bu | -(CH₂)₃- | | H | c-Pr | |
| 5-63 | Et | 1-Me-c-Pr | Me | Me | H | H | |
| 5-64 | Et | 1-Me-c-Pr | Me | Me | Me | H | |
| 5-65 | Et | 1-Me-c-Pr | c-Pr | Me | H | H | |
| 5-66 | Et | 1-Me-c-Pr | c-Pr | Me | Me | H | |
| 5-67 | Et | 1-Me-c-Pr | -(CH₂)₂- | | H | H | |
| 5-68 | Et | 1-Me-c-Pr | -(CH₂)₂- | | Me | H | |
| 5-69 | Et | 1-Cl-c-Pr | Me | Me | H | H | |
| 5-70 | Et | 1-Cl-c-Pr | Me | Me | Me | H | |
| 5-71 | Et | 1-Cl-c-Pr | c-Pr | Me | H | H | |
| 5-72 | Et | 1-Cl-c-Pr | c-Pr | Me | Me | H | |
| 5-73 | Et | 1-Cl-c-Pr | -(CH₂)₂- | | H | H | |
| 5-74 | Et | 1-Cl-c-Pr | -(CH₂)₂- | | Me | H | |
| 5-75 | Et | 2,2-Me₂-c-Pr | Me | Me | H | H | |
| 5-76 | Et | 2,2-Me₂-c-Pr | Me | Me | Me | H | |
| 5-77 | Et | 2,2-Me₂-c-Pr | c-Pr | Me | H | H | |
| 5-78 | Et | 2,2-Me₂-c-Pr | c-Pr | Me | Me | H | |
| 5-79 | Et | 2,2-Me₂-c-Pr | -(CH₂)₂- | | H | H | |
| 5-80 | Et | 2,2-Me₂-c-Pr | -CH₂-CMe₂- | | H | H | |
| 5-81 | Et | 2,2-Cl₂-c-Pr | Me | Me | H | H | |
| 5-82 | Et | 2,2-Cl₂-c-Pr | Me | Me | Me | H | |
| 5-83 | Et | 2,2-Cl₂-c-Pr | c-Pr | Me | H | H | |
| 5-84 | Et | 2,2-Cl₂-c-Pr | c-Pr | Me | Me | H | |
| 5-85 | Et | 2,2-Cl₂-c-Pr | -(CH₂)₂- | | H | H | |
| 5-86 | Et | 2,2-Cl₂-c-Pr | -CH₂-CCl₂- | | H | H | |
| 5-87 | c-Pr | c-Pr | Me | Me | H | H | |
| 5-88 | c-Pr | c-Pr | Me | Me | Me | H | |
| 5-89 | c-Pr | c-Pr | c-Pr | Me | H | H | |
| 5-90 | c-Pr | c-Pr | c-Pr | Me | Me | H | |
| 5-91 | c-Pr | c-Pr | -CH₂-OMe | Me | H | H | |
| 5-92 | c-Pr | c-Pr | -CH₂-OMe | Me | Me | H | |
| 5-93 | c-Pr | c-Pr | -(CH₂)₂- | | H | H | |
| 5-94 | c-Pr | c-Pr | -(CH₂)₂- | | H | Me | |
| 5-95 | c-Pr | c-Pr | -(CH₂)₂- | | H | c-Pr | |
| 5-96 | c-Pr | c-Bu | -(CH₂)₃- | | H | H | |
| 5-97 | c-Pr | c-Bu | -(CH₂)₃- | | H | Me | |
| 5-98 | c-Pr | c-Bu | -(CH₂)₃- | | H | c-Pr | |
| 5-99 | c-Pr | 1-Me-c-Pr | Me | Me | H | H | |
| 5-100 | c-Pr | 1-Me-c-Pr | Me | Me | Me | H | |
| 5-101 | c-Pr | 1-Me-c-Pr | c-Pr | Me | H | H | |
| 5-102 | c-Pr | 1-Me-c-Pr | c-Pr | Me | Me | H | |
| 5-103 | c-Pr | 1-Me-c-Pr | -(CH₂)₂- | | H | H | |
| 5-104 | c-Pr | 1-Me-c-Pr | -(CH₂)₂- | | Me | H | |
| 5-105 | c-Pr | 1-Cl-c-Pr | Me | Me | H | H | |
| 5-106 | c-Pr | 1-Cl-c-Pr | Me | Me | Me | H | |
| 5-107 | c-Pr | 1-Cl-c-Pr | c-Pr | Me | H | H | |
| 5-108 | c-Pr | 1-Cl-c-Pr | c-Pr | Me | Me | H | |
| 5-109 | c-Pr | 1-Cl-c-Pr | -(CH₂)₂- | | H | H | |
| 5-110 | c-Pr | 1-Cl-c-Pr | -(CH₂)₂- | | Me | H | |
| 5-111 | c-Pr | 2,2-Me₂-c-Pr | Me | Me | H | H | |
| 5-112 | c-Pr | 2,2-Me₂-c-Pr | Me | Me | Me | H | |
| 5-113 | c-Pr | 2,2-Me₂-c-Pr | c-Pr | Me | H | H | |
| 5-114 | c-Pr | 2,2-Me₂-c-Pr | c-Pr | Me | Me | H | |
| 5-115 | c-Pr | 2,2-Me₂-c-Pr | -(CH₂)₂- | | H | H | |
| 5-116 | c-Pr | 2,2-Me₂-c-Pr | -CH₂-CMe₂- | | H | H | |
| 5-117 | c-Pr | 2,2-Cl₂-c-Pr | Me | Me | H | H | |
| 5-118 | c-Pr | 2,2-Cl₂-c-Pr | Me | Me | Me | H | |
| 5-119 | c-Pr | 2,2-Cl₂-c-Pr | c-Pr | Me | H | H | |
| 5-120 | c-Pr | 2,2-Cl₂-c-Pr | c-Pr | Me | Me | H | |
| 5-121 | c-Pr | 2,2-Cl₂-c-Pr | -(CH₂)₂- | | H | H | |
| 5-122 | c-Pr | 2,2-Cl₂-c-Pr | -CH₂-CCl₂- | | H | H | |
| 5-123 | CHFCH₃ | c-Pr | Me | Me | H | H | |
| 5-124 | CHFCH₃ | c-Pr | Me | Me | Me | H | |
| 5-125 | CHFCH₃ | c-Pr | c-Pr | Me | H | H | |
| 5-126 | CHFCH₃ | c-Pr | c-Pr | Me | Me | H | |
| 5-127 | CHFCH₃ | c-Pr | -CH₂-OMe | Me | H | H | |
| 5-128 | CHFCH₃ | c-Pr | -CH₂-OMe | Me | Me | H | |
| 5-129 | CHFCH₃ | c-Pr | -(CH₂)₂- | | H | H | |
| 5-130 | CHFCH₃ | c-Pr | -(CH₂)₂- | | H | Me | |
| 5-131 | CHFCH₃ | c-Pr | -(CH₂)₂- | | H | c-Pr | |
| 5-132 | CHFCH₃ | c-Bu | -(CH₂)₃- | | H | H | |
| 5-133 | CHFCH₃ | c-Bu | -(CH₂)₃- | | H | Me | |
| 5-134 | CHFCH₃ | c-Bu | -(CH₂)₃- | | H | c-Pr | |
| 5-135 | CHFCH₃ | 1-Me-c-Pr | Me | Me | H | H | |
| 5-136 | CHFCH₃ | 1-Me-c-Pr | Me | Me | Me | H | |
| 5-137 | CHFCH₃ | 1-Me-c-Pr | c-Pr | Me | H | H | |
| 5-138 | CHFCH₃ | 1-Me-c-Pr | c-Pr | Me | Me | H | |
| 5-139 | CHFCH₃ | 1-Me-c-Pr | -(CH₂)₂- | | H | H | |
| 5-140 | CHFCH₃ | 1-Me-c-Pr | -(CH₂)₂- | | Me | H | |
| 5-141 | CHFCH₃ | 1-Cl-c-Pr | Me | Me | H | H | |
| 5-142 | CHFCH₃ | 1-Cl-c-Pr | Me | Me | Me | H | |
| 5-143 | CHFCH₃ | 1-Cl-c-Pr | c-Pr | Me | H | H | |
| 5-144 | CHFCH₃ | 1-Cl-c-Pr | c-Pr | Me | Me | H | |
| 5-145 | CHFCH₃ | 1-Cl-c-Pr | -(CH₂)₂- | | H | H | |
| 5-146 | CHFCH₃ | 1-Cl-c-Pr | -(CH₂)₂- | | Me | H | |
| 5-147 | CHFCH₃ | 2,2-Me₂-c-Pr | Me | Me | H | H | |
| 5-148 | CHFCH₃ | 2,2-Me₂-c-Pr | Me | Me | Me | H | |
| 5-149 | CHFCH₃ | 2,2-Me₂-c-Pr | c-Pr | Me | H | H | |
| 5-150 | CHFCH₃ | 2,2-Me₂-c-Pr | c-Pr | Me | Me | H | |
| 5-151 | CHFCH₃ | 2,2-Me₂-c-Pr | -(CH₂)₂- | | H | H | |
| 5-152 | CHFCH₃ | 2,2-Me₂-c-Pr | -CH₂-CMe₂- | | H | H | |
| 5-153 | CHFCH₃ | 2,2-Cl₂-c-Pr | Me | Me | H | H | |
| 5-154 | CHFCH₃ | 2,2-Cl₂-c-Pr | Me | Me | Me | H | |
| 5-155 | CHFCH₃ | 2,2-Cl₂-c-Pr | c-Pr | Me | H | H | |
| 5-156 | CHFCH₃ | 2,2-Cl₂-c-Pr | c-Pr | Me | Me | H | |
| 5-157 | CHFCH₃ | 2,2-Cl₂-c-Pr | -(CH₂)₂- | | H | H | |
| 5-158 | CHFCH₃ | 2,2-Cl₂-c-Pr | -CH₂-CCl₂- | | H | H | |
| 5-159 | CHFC₂H₅ | c-Pr | Me | Me | H | H | |
| 5-160 | CHFC₂H₆ | c-Pr | Me | Me | Me | H | |
| 5-161 | CHFC₂H₅ | c-Pr | c-Pr | Me | H | H | |
| 5-162 | CHFC₂H₅ | c-Pr | c-Pr | Me | Me | H | |
| 5-163 | CHFC₂H₅ | c-Pr | -CH₂-OMe | Me | H | H | |
| 5-164 | CHFC₂H₅ | c-Pr | -CH₂-OMe | Me | Me | H | |
| 5-165 | CHFC₂H₅ | c-Pr | -(CH₂)₂- | | H | H | |
| 5-166 | CHFC₂H₅ | c-Pr | -(CH₂)₂- | | H | Me | |
| 5-167 | CHFC₂H₅ | c-Pr | -(CH₂)₂- | | H | c-Pr | |
| 5-168 | CHFC₂H₅ | c-Bu | -(CH₂)₃- | | H | H | |
| 5-169 | CHFC₂H₅ | c-Bu | -(CH₂)₃- | | H | Me | |
| 5-170 | CHFC₂H₅ | c-Bu | -(CH₂)₃- | | H | c-Pr | |
| 5-171 | CHFC₂H₅ | 1-Me-c-Pr | Me | Me | H | H | |
| 5-172 | CHFC₂H₅ | 1-Me-c-Pr | Me | Me | Me | H | |
| 5-173 | CHFC₂H₅ | 1-Me-c-Pr | c-Pr | Me | H | H | |
| 5-174 | CHFC₂H₅ | 1-Me-c-Pr | c-Pr | Me | Me | H | |
| 5-175 | CHFC₂H₅ | 1-Me-c-Pr | -(CH₂)₂- | | H | H | |
| 5-176 | CHFC₂H₅ | 1-Me-c-Pr | -(CH₂)₂- | | Me | H | |
| 5-177 | CHFC₂H₅ | 1-Cl-c-Pr | Me | Me | H | H | |
| 5-178 | CHFC₂H₅ | 1-Cl-c-Pr | Me | Me | Me | H | |
| 5-179 | CHFC₂H₅ | 1-Cl-c-Pr | c-Pr | Me | H | H | |
| 5-180 | CHFC₂H₅ | 1-Cl-c-Pr | c-Pr | Me | Me | H | |
| 5-181 | CHFC₂H₅ | 1-Cl-c-Pr | -(CH₂)₂- | | H | H | |
| 5-182 | CHFC₂H₅ | 1-Cl-c-Pr | -(CH₂)₂- | | Me | H | |
| 5-183 | CHFC₂H₅ | 2,2-Me₂-c-Pr | Me | Me | H | H | |
| 5-184 | CHFC₂H₅ | 2,2-Me₂-c-Pr | Me | Me | Me | H | |
| 5-185 | CHFC₂H₅ | 2,2-Me₂-c-Pr | c-Pr | Me | H | H | |
| 5-186 | CHFC₂H₅ | 2,2-Me₂-c-Pr | c-Pr | Me | Me | H | |
| 5-187 | CHFC₂H₅ | 2,2-Me₂-c-Pr | -(CH₂)₂- | | H | H | |
| 5-188 | CHFC₂H₅ | 2,2-Me₂-c-Pr | -CH₂-CMe₂- | | H | H | |
| 5-189 | CHFC₂H₅ | 2,2-Cl₂-c-Pr | Me | Me | H | H | |
| 5-190 | CHFC₂H₅ | 2,2-Cl₂-c-Pr | Me | Me | Me | H | |
| 5-191 | CHFC₂H₅ | 2,2-Cl₂-c-Pr | c-Pr | Me | H | H | |
| 5-192 | CHFC₂H₅ | 2,2-Cl₂-c-Pr | c-Pr | Me | Me | H | |
| 5-193 | CHFC₂H₅ | 2,2-Cl₂-c-Pr | -(CH₂)₂- | | H | H | |
| 5-194 | CHFC₂H₅ | 2,2-Cl₂-c-Pr | -CH₂-CCl₂- | | H | H | |
| 5-195 | CF(CH₃)₂ | c-Pr | Me | Me | H | H | Harz |
| 5-196 | CF(CH₃)₂ | c-Pr | Me | Me | Me | H | |
| 5-197 | CF(CH₃)₂ | c-Pr | c-Pr | Me | H | H | |
| 5-198 | CF(CH₃)₂ | c-Pr | c-Pr | Me | Me | H | |
| 5-199 | CF(CH₃)₂ | c-Pr | -CH₂-OMe | Me | H | H | |
| 5-200 | CF(CH₃)₂ | c-Pr | -CH₂-OMe | Me | Me | H | |
| 5-201 | CF(CH₃)₂ | c-Pr | -(CH₂)₂- | | H | H | NMR |
| 5-202 | CF(CH₃)₂ | c-Pr | -(CH₂)₂- | | H | Me | |
| 5-203 | CF(CH₃)₂ | c-Pr | -(CH₂)₂- | | H | c-Pr | |
| 5-204 | CF(CH₃)₂ | c-Bu | -(CH₂)₃- | | H | H | Harz |
| 5-205 | CF(CH₃)₂ | c-Bu | -(CH₂)₃- | | H | Me | |
| 5-206 | CF(CH₃)₂ | c-Bu | -(CH₂)₃- | | H | c-Pr | |
| 5-207 | CF(CH₃)₂ | 1-Me-c-Pr | Me | Me | H | H | |
| 5-208 | CF(CH₃)₂ | 1-Me-c-Pr | Me | Me | Me | H | |
| 5-209 | CF(CH₃)₂ | 1-Me-c-Pr | c-Pr | Me | H | H | |
| 5-210 | CF(CH₃)₂ | 1-Me-c-Pr | c-Pr | Me | Me | H | |
| 5-211 | CF(CH₃)₂ | 1-Me-c-Pr | -(CH₂)₂- | | H | H | |
| 5-212 | CF(CH₃)₂ | 1-Me-c-Pr | -(CH₂)₂- | | Me | H | |
| 5-213 | CF(CH₃)₂ | 1-Cl-c-Pr | Me | Me | H | H | |
| 5-214 | CF(CH₃)₂ | 1-Cl-c-Pr | Me | Me | Me | H | |
| 5-215 | CF(CH₃)₂ | 1-Cl-c-Pr | c-Pr | Me | H | H | |
| 5-216 | CF(CH₃)₂ | 1-Cl-c-Pr | c-Pr | Me | Me | H | |
| 5-217 | CF(CH₃)₂ | 1-Cl-c-Pr | -(CH₂)₂- | | H | H | |
| 5-218 | CF(CH₃)₂ | 1-Cl-c-Pr | -(CH₂)₂- | | Me | H | |
| 5-219 | CF(CH₃)₂ | 2,2-Me₂-c-Pr | Me | Me | H | H | |
| 5-220 | CF(CH₃)₂ | 2,2-Me₂-c-Pr | Me | Me | Me | H | |
| 5-221 | CF(CH₃)₂ | 2,2-Me₂-c-Pr | c-Pr | Me | H | H | |
| 5-222 | CF(CH₃)₂ | 2,2-Me₂-c-Pr | c-Pr | Me | Me | H | |
| 5-223 | CF(CH₃)₂ | 2,2-Me₂-c-Pr | -(CH₂)₂- | | H | H | |
| 5-224 | CF(CH₃)₂ | 2,2-Me₂-c-Pr | -CH₂-CMe₂- | | H | H | |
| 5-225 | CF(CH₃)₂ | 2,2-Cl₂-c-Pr | Me | Me | H | H | |
| 5-226 | CF(CH₃)₂ | 2,2-Cl₂-c-Pr | Me | Me | Me | H | |
| 5-227 | CF(CH₃)₂ | 2,2-Cl₂-c-Pr | c-Pr | Me | H | H | |
| 5-228 | CF(CH₃)₂ | 2,2-Cl₂-c-Pr | c-Pr | Me | Me | H | |
| 5-229 | CF(CH₃)₂ | 2,2-Cl₂-c-Pr | -(CH₂)₂- | | H | H | |
| 5-230 | CF(CH₃)₂ | 2,2-Cl₂-c-Pr | -CH₂-CCl₂- | | H | H | |
| 5-231 | 1-F-c-Pr | c-Pr | Me | Me | H | H | |
| 5-232 | 1-F-c-Pr | c-Pr | Me | Me | Me | H | |
| 5-233 | 1-F-c-Pr | c-Pr | c-Pr | Me | H | H | |
| 5-234 | 1-F-c-Pr | c-Pr | c-Pr | Me | Me | H | |
| 5-235 | 1-F-c-Pr | c-Pr | -CH₂-OMe | Me | H | H | |
| 5-236 | 1-F-c-Pr | c-Pr | -CH₂-OMe | Me | Me | H | |
| 5-237 | 1-F-c-Pr | c-Pr | -(CH₂)₂- | | H | H | |
| 5-238 | 1-F-c-Pr | c-Pr | -(CH₂)₂- | | H | Me | |
| 5-239 | 1-F-c-Pr | c-Pr | -(CH₂)₂- | | H | c-Pr | |
| 5-240 | 1-F-c-Pr | c-Bu | -(CH₂)₃- | | H | H | |
| 5-241 | 1-F-c-Pr | c-Bu | -(CH₂)₃- | | H | Me | |
| 5-242 | 1-F-c-Pr | c-Bu | -(CH₂)₃- | | H | c-Pr | |
| 5-243 | 1-F-c-Pr | 1-Me-c-Pr | Me | Me | H | H | |
| 5-244 | 1-F-c-Pr | 1-Me-c-Pr | Me | Me | Me | H | |
| 5-245 | 1-F-c-Pr | 1-Me-c-Pr | c-Pr | Me | H | H | |
| 5-246 | 1-F-c-Pr | 1-Me-c-Pr | c-Pr | Me | Me | H | |
| 5-247 | 1-F-c-Pr | 1-Me-c-Pr | -(CH₂)₂- | | H | H | |
| 5-248 | 1-F-c-Pr | 1-Me-c-Pr | -(CH₂)₂- | | Me | H | |
| 5-249 | 1-F-c-Pr | 1-Cl-c-Pr | Me | Me | H | H | |
| 5-250 | 1-F-c-Pr | 1-Cl-c-Pr | Me | Me | Me | H | |
| 5-251 | 1-F-c-Pr | 1-Cl-c-Pr | c-Pr | Me | H | H | |
| 5-252 | 1-F-c-Pr | 1-Cl-c-Pr | c-Pr | Me | Me | H | |
| 5-253 | 1-F-c-Pr | 1-Cl-c-Pr | -(CH₂)₂- | | H | H | |
| 5-254 | 1-F-c-Pr | 1-Cl-c-Pr | -(CH₂)₂- | | Me | H | |
| 5-255 | 1-F-c-Pr | 2,2-Me₂-c-Pr | Me | Me | H | H | |
| 5-256 | 1-F-c-Pr | 2,2-Me₂-c-Pr | Me | Me | Me | H | |
| 5-257 | 1-F-c-Pr | 2,2-Me₂-c-Pr | c-Pr | Me | H | H | |
| 5-258 | 1-F-c-Pr | 2,2-Me₂-c-Pr | c-Pr | Me | Me | H | |
| 5-259 | 1-F-c-Pr | 2,2-Me₂-c-Pr | -(CH₂)₂- | | H | H | |
| 5-260 | 1-F-c-Pr | 2,2-Me₂-c-Pr | -CH₂-CMe₂- | | H | H | |
| 5-261 | 1-F-c-Pr | 2,2-Cl₂-c-Pr | Me | Me | H | H | |
| 5-262 | 1-F-c-Pr | 2,2-Cl₂-c-Pr | Me | Me | Me | H | |
| 5-263 | 1-F-c-Pr | 2,2-Cl₂-c-Pr | c-Pr | Me | H | H | |
| 5-264 | 1-F-c-Pr | 2,2-Cl₂-c-Pr | c-Pr | Me | Me | H | |
| 5-265 | 1-F-c-Pr | 2,2-Cl₂-c-Pr | -(CH₂)₂- | | H | H | |
| 5-266 | 1-F-c-Pr | 2,2-Cl₂-c-Pr | -CH₂-CCl₂- | | H | H | |
| 5-267 | CHF2 | c-Pr | Me | Me | H | H | |
| 5-268 | CHF2 | c-Pr | Me | Me | Me | H | |
| 5-269 | CHF2 | c-Pr | c-Pr | Me | H | H | |
| 5-270 | CHF2 | c-Pr | c-Pr | Me | Me | H | |
| 5-271 | CHF2 | c-Pr | -CH₂-OMe | Me | H | H | |
| 5-272 | CHF2 | c-Pr | -CH₂-OMe | Me | Me | H | |
| 5-273 | CHF2 | c-Pr | -(CH₂)₂- | | H | H | |
| 5-274 | CHF2 | c-Pr | -(CH₂)₂- | | H | Me | |
| 5-275 | CHF2 | c-Pr | -(CH₂)₂- | | H | c-Pr | |
| 5-276 | CHF2 | c-Bu | -(CH₂)₃- | | H | H | |
| 5-277 | CHF2 | c-Bu | -(CH₂)₃- | | H | Me | |
| 5-278 | CHF2 | c-Bu | -(CH₂)₃- | | H | c-Pr | |

### Erläuterungen zu Tabelle 5:

"NMR" der Beispielverbindungen wurden jeweils als ¹H-NMR-Spektum bei 400 MHz (CDCl₃) (¹H-Kernresonanz-Daten) gemessen. Nachfolgend sind charakterische chemische Verschiebungen δ (ppm) für Beispielverbindungen aufgeführt:

| Beisp. Nr. | δ (ppm) = |
|---|---|
| 5-201: | 8.75 (m, 1H), 3.60-3.25 (m, 1H), 3.15 (s, 6H),1.70 (m, 6H), 0.95 (m, 2H), 0.55 - 0.25 (m, 8H) |

**Tabelle 6: Verbindungen der Formel (If)**

| | | | | | | | |
|---|---|---|---|---|---|---|---|
| 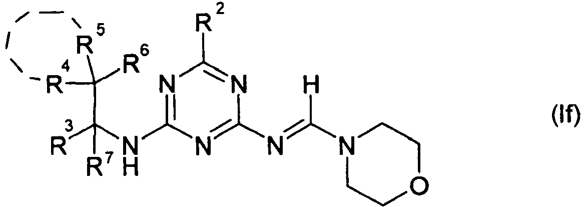 | | | | | | | |

| Nr. | R² | R³ | R⁴ | R⁵ | R⁶ | R⁷ | phys. Daten |
|---|---|---|---|---|---|---|---|
| 6-1 | H | c-Pr | Me | Me | H | H | |
| 6-2 | H | c-Pr | Me | Me | Me | H | |
| 6-3 | H | c-Pr | c-Pr | Me | H | H | |
| 6-4 | H | c-Pr | c-Pr | Me | Me | H | |
| 6-5 | H | c-Pr | -CH₂-OMe | Me | H | H | |
| 6-6 | H | c-Pr | -CH₂-OMe | Me | Me | H | |
| 6-7 | H | c-Pr | -(CH₂)₂- | | H | H | |
| 6-8 | H | c-Pr | -(CH₂)₂- | | H | Me | |
| 6-9 | H | c-Pr | -(CH₂)₂- | | H | c-Pr | |
| 6-10 | H | c-Bu | -(CH₂)₃- | | H | H | |
| 6-11 | H | c-Bu | -(CH₂)₃- | | H | Me | |
| 6-12 | H | c-Bu | -(CH₂)₃- | | H | c-Pr | |
| 6-13 | Me | c-Pr | Me | Me | H | H | |
| 6-14 | Me | c-Pr | Me | Me | Me | H | |
| 6-15 | Me | c-Pr | c-Pr | Me | H | H | |
| 6-16 | Me | c-Pr | c-Pr | Me | Me | H | |
| 6-17 | Me | c-Pr | -CH₂-OMe | Me | H | H | |
| 6-18 | Me | c-Pr | -CH₂-OMe | Me | Me | H | |
| 6-19 | Me | c-Pr | -(CH₂)₂- | | H | H | |
| 6-20 | Me | c-Pr | -(CH₂)₂- | | H | Me | |
| 6-21 | Me | c-Pr | -(CH₂)₂- | | H | c-Pr | |
| 6-22 | Me | c-Bu | -(CH₂)₃- | | H | H | |
| 6-23 | Me | c-Bu | -(CH₂)₃- | | H | Me | |
| 6-24 | Me | c-Bu | -(CH₂)₃- | | H | c-Pr | |
| 6-25 | Me | 1-Me-c-Pr | Me | Me | H | H | |
| 6-26 | Me | 1-Me-c-Pr | Me | Me | Me | H | |
| 6-27 | Me | 1-Me-c-Pr | c-Pr | Me | H | H | |
| 6-28 | Me | 1-Me-c-Pr | c-Pr | Me | Me | H | |
| 6-29 | Me | 1-Me-c-Pr | -(CH₂)₂- | | H | H | |
| 6-30 | Me | 1-Me-c-Pr | -(CH₂)₂- | | Me | H | |
| 6-31 | Me | 1-Cl-c-Pr | Me | Me | H | H | |
| 6-32 | Me | 1-Cl-c-Pr | Me | Me | Me | H | |
| 6-33 | Me | 1-Cl-c-Pr | c-Pr | Me | H | H | |
| 6-34 | Me | 1-Cl-c-Pr | c-Pr | Me | Me | H | |
| 6-35 | Me | 1-Cl-c-Pr | -(CH₂)₂- | | H | H | |
| 6-36 | Me | 1-Cl-c-Pr | -(CH₂)₂- | | Me | H | |
| 6-37 | Me | 2,2-Me₂-c-Pr | Me | Me | H | H | |
| 6-38 | Me | 2,2-Me₂-c-Pr | Me | Me | Me | H | |
| 6-39 | Me | 2,2-Me₂-c-Pr | c-Pr | Me | H | H | |
| 6-40 | Me | 2,2-Me₂-c-Pr | c-Pr | Me | Me | H | |
| 6-41 | Me | 2,2-Me₂-c-Pr | -(CH₂)₂- | | H | H | |
| 6-42 | Me | 2,2-Me₂-c-Pr | -CH₂-CMe₂- | | H | H | |
| 6-43 | Me | 2,2-Cl₂-c-Pr | Me | Me | H | H | |
| 6-44 | Me | 2,2-Cl₂-c-Pr | Me | Me | Me | H | |
| 6-45 | Me | 2,2-Cl₂-c-Pr | c-Pr | Me | H | H | |
| 6-46 | Me | 2,2-Cl₂-c-Pr | c-Pr | Me | Me | H | |
| 6-47 | Me | 2,2-Cl₂-c-Pr | -(CH₂)₂- | | H | H | |
| 6-48 | Me | 2,2-Cl₂-c-Pr | -CH₂-CCl₂- | | H | H | |
| 6-49 | Et | c-Pr | Me | Me | H | H | |
| 6-50 | Et | c-Pr | Me | Me | Me | H | |
| 6-51 | Et | c-Pr | c-Pr | Me | H | H | |
| 6-52 | Et | c-Pr | c-Pr | Me | Me | H | |
| 6-53 | Et | c-Pr | -CH₂-OMe | Me | H | H | |
| 6-54 | Et | c-Pr | -CH₂-OMe | Me | Me | H | |
| 6-55 | Et | c-Pr | -(CH₂)₂- | | H | H | |
| 6-56 | Et | c-Pr | -(CH₂)₂- | | H | Me | |
| 6-57 | Et | c-Pr | -(CH₂)₂- | | H | c-Pr | |
| 6-58 | Et | c-Bu | -(CH₂)₃- | | H | H | |
| 6-59 | Et | c-Bu | -(CH₂)₃- | | H | Me | |
| 6-60 | Et | c-Bu | -(CH₂)₃- | | H | c-Pr | |
| 6-61 | Et | 1-Me-c-Pr | Me | Me | H | H | |
| 6-62 | Et | 1-Me-c-Pr | Me | Me | Me | H | |
| 6-63 | Et | 1-Me-c-Pr | c-Pr | Me | H | H | |
| 6-64 | Et | 1-Me-c-Pr | c-Pr | Me | Me | H | |
| 6-65 | Et | 1-Me-c-Pr | -(CH₂)₂- | | H | H | |
| 6-66 | Et | 1-Me-c-Pr | -(CH₂)₂- | | Me | H | |
| 6-67 | Et | 1-Cl-c-Pr | Me | Me | H | H | |
| 6-68 | Et | 1-Cl-c-Pr | Me | Me | Me | H | |
| 6-69 | Et | 1-Cl-c-Pr | c-Pr | Me | H | H | |
| 6-70 | Et | 1-Cl-c-Pr | c-Pr | Me | Me | H | |
| 6-71 | Et | 1-Cl-c-Pr | -(CH₂)₂- | | H | H | |
| 6-72 | Et | 1-Cl-c-Pr | -(CH₂)₂- | | Me | H | |
| 6-73 | Et | 2,2-Me₂-c-Pr | Me | Me | H | H | |
| 6-74 | Et | 2,2-Me₂-c-Pr | Me | Me | Me | H | |
| 6-75 | Et | 2,2-Me₂-c-Pr | c-Pr | Me | H | H | |
| 6-76 | Et | 2,2-Me₂-c-Pr | c-Pr | Me | Me | H | |
| 6-77 | Et | 2,2-Me₂-c-Pr | -(CH₂)₂- | | H | H | |
| 6-78 | Et | 2,2-Me₂-c-Pr | -CH₂-CMe₂- | | H | H | |
| 6-79 | Et | 2,2-Cl₂-c-Pr | Me | Me | H | H | |
| 6-80 | Et | 2,2-Cl₂-c-Pr | Me | Me | Me | H | |
| 6-81 | Et | 2,2-Cl₂-c-Pr | c-Pr | Me | H | H | |
| 6-82 | Et | 2,2-Cl₂-c-Pr | c-Pr | Me | Me | H | |
| 6-83 | Et | 2,2-Cl₂-c-Pr | -(CH₂)₂- | | H | H | |
| 6-84 | Et | 2,2-Cl₂-c-Pr | -CH₂-CCl₂- | | H | H | |
| 6-85 | c-Pr | c-Pr | Me | Me | H | H | |
| 6-86 | c-Pr | c-Pr | Me | Me | Me | H | |
| 6-87 | c-Pr | c-Pr | c-Pr | Me | H | H | |
| 6-88 | c-Pr | c-Pr | c-Pr | Me | Me | H | |
| 6-89 | c-Pr | c-Pr | -CH₂-OMe | Me | H | H | |
| 6-90 | c-Pr | c-Pr | -CH₂-OMe | Me | Me | H | |
| 6-91 | c-Pr | c-Pr | -(CH₂)₂- | | H | H | |
| 6-92 | c-Pr | c-Pr | -(CH₂)₂- | | H | Me | |
| 6-93 | c-Pr | c-Pr | -(CH₂)₂- | | H | c-Pr | |
| 6-94 | c-Pr | c-Bu | - (CH₂)₃ - | | H | H | |
| 6-95 | c-Pr | c-Bu | -(CH₂)₃- | | H | Me | |
| 6-96 | c-Pr | c-Bu | -(CH₂)₃- | | H | c-Pr | |
| 6-97 | c-Pr | 1-Me-c-Pr | Me | Me | H | H | |
| 6-98 | c-Pr | 1-Me-c-Pr | Me | Me | Me | H | |
| 6-99 | c-Pr | 1-Me-c-Pr | c-Pr | Me | H | H | |
| 6-100 | c-Pr | 1-Me-c-Pr | c-Pr | Me | Me | H | |
| 6-101 | c-Pr | 1-Me-c-Pr | -(CH₂)₂- | | H | H | |
| 6-102 | c-Pr | 1-Me-c-Pr | -(CH₂)₂- | | Me | H | |
| 6-103 | c-Pr | 1-Cl-c-Pr | Me | Me | H | H | |
| 6-104 | c-Pr | 1-Cl-c-Pr | Me | Me | Me | H | |
| 6-105 | c-Pr | 1-Cl-c-Pr | c-Pr | Me | H | H | |
| 6-106 | c-Pr | 1-Cl-c-Pr | c-Pr | Me | Me | H | |
| 6-107 | c-Pr | 1-Cl-c-Pr | -(CH₂)₂- | | H | H | |
| 6-108 | c-Pr | 1-Cl-c-Pr | -(CH₂)₂- | | Me | H | |
| 6-109 | c-Pr | 2,2-Me₂-c-Pr | Me | Me | H | H | |
| 6-110 | c-Pr | 2,2-Me₂-c-Pr | Me | Me | Me | H | |
| 6-111 | c-Pr | 2,2-Me₂-c-Pr | c-Pr | Me | H | H | |
| 6-112 | c-Pr | 2,2-Me₂-c-Pr | c-Pr | Me | Me | H | |
| 6-113 | c-Pr | 2,2-Me₂-c-Pr | -(CH₂)₂- | | H | H | |
| 6-114 | c-Pr | 2,2-Me₂-c-Pr | -CH₂-CMe₂- | | H | H | |
| 6-115 | c-Pr | 2,2-Cl₂-c-Pr | Me | Me | H | H | |
| 6-116 | c-Pr | 2,2-Cl₂-c-Pr | Me | Me | Me | H | |
| 6-117 | c-Pr | 2,2-Cl₂-c-Pr | c-Pr | Me | H | H | |
| 6-118 | c-Pr | 2,2-Cl₂-c-Pr | c-Pr | Me | Me | H | |
| 6-119 | c-Pr | 2,2-Cl₂-c-Pr | -(CH₂)₂- | | H | H | |
| 6-120 | c-Pr | 2,2-Cl₂-c-Pr | -CH₂-CCl₂- | | H | H | |
| 6-121 | CHFCH₃ | c-Pr | Me | Me | H | H | |
| 6-122 | CHFCH₃ | c-Pr | Me | Me | Me | H | |
| 6-123 | CHFCH₃ | c-Pr | c-Pr | Me | H | H | |
| 6-124 | CHFCH₃ | c-Pr | c-Pr | Me | Me | H | |
| 6-125 | CHFCH₃ | c-Pr | -CH₂-OMe | Me | H | H | |
| 6-126 | CHFCH₃ | c-Pr | -CH₂-OMe | Me | Me | H | |
| 6-127 | CHFCH₃ | c-Pr | -(CH₂)₂- | | H | H | |
| 6-128 | CHFCH₃ | c-Pr | -(CH₂)₂- | | H | Me | |
| 6-129 | CHFCH₃ | c-Pr | -(CH₂)₂- | | H | c-Pr | |
| 6-130 | CHFCH₃ | c-Bu | -(CH₂)₃- | | H | H | |
| 6-131 | CHFCH₃ | c-Bu | -(CH₂)₃- | | H | Me | |
| 6-132 | CHFCH₃ | c-Bu | -(CH₂)₃- | | H | c-Pr | |
| 6-133 | CHFCH₃ | 1-Me-c-Pr | Me | Me | H | H | |
| 6-134 | CHFCH₃ | 1-Me-c-Pr | Me | Me | Me | H | |
| 6-135 | CHFCH₃ | 1-Me-c-Pr | c-Pr | Me | H | H | |
| 6-136 | CHFCH₃ | 1-Me-c-Pr | c-Pr | Me | Me | H | |
| 6-137 | CHFCH₃ | 1-Me-c-Pr | -(CH₂)₂- | | H | H | |
| 6-138 | CHFCH₃ | 1-Me-c-Pr | -(CH₂)₂- | | Me | H | |
| 6-139 | CHFCH₃ | 1-Cl-c-Pr | Me | Me | H | H | |
| 6-140 | CHFCH₃ | 1-Cl-c-Pr | Me | Me | Me | H | |
| 6-141 | CHFCH₃ | 1-Cl-c-Pr | c-Pr | Me | H | H | |
| 6-142 | CHFCH₃ | 1-Cl-c-Pr | c-Pr | Me | Me | H | |
| 6-143 | CHFCH₃ | 1-Cl-c-Pr | -(CH₂)₂- | | H | H | |
| 6-144 | CHFCH₃ | 1-Cl-c-Pr | -(CH₂)₂- | | Me | H | |
| 6-145 | CHFCH₃ | 2,2-Me₂-c-Pr | Me | Me | H | H | |
| 6-146 | CHFCH₃ | 2,2-Me₂-c-Pr | Me | Me | Me | H | |
| 6-147 | CHFCH₃ | 2,2-Me₂-c-Pr | c-Pr | Me | H | H | |
| 6-148 | CHFCH₃ | 2,2-Me₂-c-Pr | c-Pr | Me | Me | H | |
| 6-149 | CHFCH₃ | 2,2-Me₂-c-Pr | -(CH₂)₂- | | H | H | |
| 6-150 | CHFCH₃ | 2,2-Me₂-c-Pr | -CH₂-CMe₂- | | H | H | |
| 6-151 | CHFCH₃ | 2,2-Cl₂-c-Pr | Me | Me | H | H | |
| 6-152 | CHFCH₃ | 2,2-Cl₂-c-Pr | Me | Me | Me | H | |
| 6-153 | CHFCH₃ | 2,2-Cl₂-c-Pr | c-Pr | Me | H | H | |
| 6-154 | CHFCH₃ | 2,2-Cl₂-c-Pr | c-Pr | Me | Me | H | |
| 6-155 | CHFCH₃ | 2,2-Cl₂-c-Pr | -(CH₂)₂- | | H | H | |
| 6-156 | CHFCH₃ | 2,2-Cl₂-c-Pr | -CH₂-CCl₂- | | H | H | |
| 6-157 | CHFC₂H₅ | c-Pr | Me | Me | H | H | |
| 6-158 | CHFC₂H₅ | c-Pr | Me | Me | Me | H | |
| 6-159 | CHFC₂H₅ | c-Pr | c-Pr | Me | H | H | |
| 6-160 | CHFC₂H₅ | c-Pr | c-Pr | Me | Me | H | |
| 6-161 | CHFC₂H₅ | c-Pr | -CH₂-OMe | Me | H | H | |
| 6-162 | CHFC₂H₅ | c-Pr | -CH₂-OMe | Me | Me | H | |
| 6-163 | CHFC₂H₅ | c-Pr | -(CH₂)₂- | | H | H | |
| 6-164 | CHFC₂H₅ | c-Pr | -(CH₂)₂- | | H | Me | |
| 6-165 | CHFC₂H₅ | c-Pr | -(CH₂)₂- | | H | c-Pr | |
| 6-166 | CHFC₂H₅ | c-Bu | -(CH₂)₃- | | H | H | |
| 6-167 | CHFC₂H₅ | c-Bu | -(CH₂)₃- | | H | Me | |
| 6-168 | CHFC₂H₅ | c-Bu | -(CH₂)₃- | | H | c-Pr | |
| 6-169 | CHFC₂H₅ | 1-Me-c-Pr | Me | Me | H | H | |
| 6-170 | CHFC₂H₅ | 1-Me-c-Pr | Me | Me | Me | H | |
| 6-171 | CHFC₂H₅ | 1-Me-c-Pr | c-Pr | Me | H | H | |
| 6-172 | CHFC₂H₅ | 1-Me-c-Pr | c-Pr | Me | Me | H | |
| 6-173 | CHFC₂H₅ | 1-Me-c-Pr | -(CH₂)₂- | | H | H | |
| 6-174 | CHFC₂H₅ | 1-Me-c-Pr | -(CH₂)₂- | | Me | H | |
| 6-175 | CHFC₂H₅ | 1-Cl-c-Pr | Me | Me | H | H | |
| 6-176 | CHFC₂H₅ | 1-Cl-c-Pr | Me | Me | Me | H | |
| 6-177 | CHFC₂H₅ | 1-Cl-c-Pr | c-Pr | Me | H | H | |
| 6-178 | CHFC₂H₅ | 1-Cl-c-Pr | c-Pr | Me | Me | H | |
| 6-179 | CHFC₂H₅ | 1-Cl-c-Pr | -(CH₂)₂- | | H | H | |
| 6-180 | CHFC₂H₅ | 1-Cl-c-Pr | -(CH₂)₂- | | Me | H | |
| 6-181 | CHFC₂H₅ | 2,2-Me₂-c-Pr | Me | Me | H | H | |
| 6-182 | CHFC₂H₅ | 2,2-Me₂-c-Pr | Me | Me | Me | H | |
| 6-183 | CHFC₂H₅ | 2,2-Me₂-c-Pr | c-Pr | Me | H | H | |
| 6-184 | CHFC₂H₅ | 2,2-Me₂-c-Pr | c-Pr | Me | Me | H | |
| 6-185 | CHFC₂H₅ | 2,2-Me₂-c-Pr | -(CH₂)₂- | | H | H | |
| 6-186 | CHFC₂H₅ | 2,2-Me₂-c-Pr | -CH₂-CMe₂- | | H | H | |
| 6-187 | CHFC₂H₅ | 2,2.Cl₂-c-Pr | Me | Me | H | H | |
| 6-188 | CHFC₂H₅ | 2,2-Cl₂-c-Pr | Me | Me | Me | H | |
| 6-189 | CHFC₂H₅ | 2,2-Cl₂-c-Pr | c-Pr | Me | H | H | |
| 6-190 | CHFC₂H₅ | 2,2-Cl₂-c-Pr | c-Pr | Me | Me | H | |
| 6-191 | CHFC₂H₅ | 2,2-Cl₂-c-Pr | -(CH₂)₂- | | H | H | |
| 6-192 | CHFC₂H₅ | 2,2-Cl₂-c-Pr | -CH₂-CCl₂- | | H | H | |
| 6-193 | CF(CH₃)₂ | c-Pr | Me | Me | H | H | Harz |
| 6-194 | CF(CH₃)₂ | c-Pr | Me | Me | Me | H | |
| 6-195 | CF(CH₃)₂ | c-Pr | c-Pr | Me | H | H | |
| 6-196 | CF(CH₃)₂ | c-Pr | c-Pr | Me | Me | H | |
| 6-197 | CF(CH₃)₂ | c-Pr | -CH₂-OMe | Me | H | H | |
| 6-198 | CF(CH₃)₂ | c-Pr | -CH₂-OMe | Me | Me | H | |
| 6-199 | CF(CH₃)₂ | c-Pr | - (CH₂)₂ - | | H | H | NMR |
| 6-200 | CF(CH₃)₂ | c-Pr | -(CH₂)₂- | | H | Me | |
| 6-201 | CF(CH₃)₂ | c-Pr | -(CH₂)₂- | | H | c-Pr | |
| 6-202 | CF(CH₃)₂ | c-Bu | -(CH₂)₃- | | H | H | Harz |
| 6-203 | CF(CH₃)₂ | c-Bu | -(CH₂)₃- | | H | Me | |
| 6-204 | CF(CH₃)₂ | c-Bu | -(CH₂)₃- | | H | c-Pr | |
| 6-205 | CF(CH₃)₂ | 1-Me-c-Pr | Me | Me | H | H | |
| 6-206 | CF(CH₃)₂ | 1-Me-c-Pr | Me | Me | Me | H | |
| 6-207 | CF(CH₃)₂ | 1-Me-c-Pr | c-Pr | Me | H | H | |
| 6-208 | CF(CH₃)₂ | 1-Me-c-Pr | c-Pr | Me | Me | H | |
| 6-209 | CF(CH₃)₂ | 1-Me-c-Pr | -(CH₂)₂- | | H | H | |
| 6-210 | CF(CH₃)₂ | 1-Me-c-Pr | -(CH₂)₂- | | Me | H | |
| 6-211 | CF(CH₃)₂ | 1-Cl-c-Pr | Me | Me | H | H | |
| 6-212 | CF(CH₃)₂ | 1-Cl-c-Pr | Me | Me | Me | H | |
| 6-213 | CF(CH₃)₂ | 1-Cl-c-Pr | c-Pr | Me | H | H | |
| 6-214 | CF(CH₃)₂ | 1-Cl-c-Pr | c-Pr | Me | Me | H | |
| 6-215 | CF(CH₃)₂ | 1-Cl-c-Pr | -(CH₂)₂- | | H | H | |
| 6-216 | CF(CH₃)₂ | 1-Cl-c-Pr | -(CH₂)₂- | | Me | H | |
| 6-217 | CF(CH₃)₂ | 2,2-Me₂-c-Pr | Me | Me | H | H | |
| 6-218 | CF(CH₃)₂ | 2,2-Me₂-c-Pr | Me | Me | Me | H | |
| 6-219 | CF(CH₃)₂ | 2,2-Me₂-c-Pr | c-Pr | Me | H | H | |
| 6-220 | CF(CH₃)₂ | 2,2-Me₂-c-Pr | c-Pr | Me | Me | H | |
| 6-221 | CF(CH₃)₂ | 2,2-Me₂-c-Pr | -(CH₂)₂- | | H | H | |
| 6-222 | CF(CH₃)₂ | 2,2-Me₂-c-Pr | -CH₂-CMe₂- | | H | H | |
| 6-223 | CF(CH₃)₂ | 2,2-Cl₂-c-Pr | Me | Me | H | H | |
| 6-224 | CF(CH₃)₂ | 2,2-Cl₂-c-Pr | Me | Me | Me | H | |
| 6-225 | CF(CH₃)₂ | 2,2-Cl₂-c-Pr | c-Pr | Me | H | H | |
| 6-226 | CF(CH₃)₂ | 2,2-Cl₂-c-Pr | c-Pr | Me | Me | H | |
| 6-227 | CF(CH₃)₂ | 2,2-Cl₂-c-Pr | -(CH₂)₂- | | H | H | |
| 6-228 | CF(CH₃)₂ | 2,2-Cl₂-c-Pr | -CH₂-CCl₂- | | H | H | |
| 6-229 | 1-F-c-Pr | c-Pr | Me | Me | H | H | |
| 6-230 | 1-F-c-Pr | c-Pr | Me | Me | Me | H | |
| 6-231 | 1-F-c-Pr | c-Pr | c-Pr | Me | H | H | |
| 6-232 | 1-F-c-Pr | c-Pr | c-Pr | Me | Me | H | |
| 6-233 | 1-F-c-Pr | c-Pr | -CH₂-OMe | Me | H | H | |
| 6-234 | 1-F-c-Pr | c-Pr | -CH₂-OMe | Me | Me | H | |
| 6-235 | 1-F-c-Pr | c-Pr | -(CH₂)₂- | | H | H | |
| 6-236 | 1-F-c-Pr | c-Pr | -(CH₂)₂- | | H | Me | |
| 6-237 | 1-F-c-Pr | c-Pr | -(CH₂)₂- | | H | c-Pr | |
| 6-238 | 1-F-c-Pr | c-Bu | -(CH₂)₃- | | H | H | |
| 6-239 | 1-F-c-Pr | c-Bu | -(CH₂)₃- | | H | Me | |
| 6-240 | 1-F-c-Pr | c-Bu | -(CH₂)₃- | | H | c-Pr | |
| 6-241 | 1-F-c-Pr | 1-Me-c-Pr | Me | Me | H | H | |
| 6-242 | 1-F-c-Pr | 1-Me-c-Pr | Me | Me | Me | H | |
| 6-243 | 1-F-c-Pr | 1-Me-c-Pr | c-Pr | Me | H | H | |
| 6-244 | 1-F-c-Pr | 1-Me-c-Pr | c-Pr | Me | Me | H | |
| 6-245 | 1-F-c-Pr | 1-Me-c-Pr | -(CH₂)₂- | | H | H | |
| 6-246 | 1-F-c-Pr | 1-Me-c-Pr | -(CH₂)₂- | | Me | H | |
| 6-247 | 1-F-c-Pr | 1-Cl-c-Pr | Me | Me | H | H | |
| 6-248 | 1-F-c-Pr | 1-Cl-c-Pr | Me | Me | Me | H | |
| 6-249 | 1-F-c-Pr | 1-Cl-c-Pr | c-Pr | Me | H | H | |
| 6-250 | 1-F-c-Pr | 1-Cl-c-Pr | c-Pr | Me | Me | H | |
| 6-251 | 1-F-c-Pr | 1-Cl-c-Pr | -(CH₂)₂- | | H | H | |
| 6-252 | 1-F-c-Pr | 1-Cl-c-Pr | -(CH₂)₂- | | Me | H | |
| 6-253 | 1-F-c-Pr | 2,2-Me₂-c-Pr | Me | Me | H | H | |
| 6-254 | 1-F-c-Pr | 2,2-Me₂-c-Pr | Me | Me | Me | H | |
| 6-255 | 1-F-c-Pr | 2,2-Me₂-c-Pr | c-Pr | Me | H | H | |
| 6-256 | 1-F-c-Pr | 2,2-Me₂-c-Pr | c-Pr | Me | Me | H | |
| 6-257 | 1-F-c-Pr | 2,2-Me₂-c-Pr | -(CH₂)₂- | | H | H | |
| 6-258 | 1-F-c-Pr | 2,2-Me₂-c-Pr | -CH₂-CMe₂- | | H | H | |
| 6-259 | 1-F-c-Pr | 2,2-Cl₂-c-Pr | Me | Me | H | H | |
| 6-260 | 1-F-c-Pr | 2,2-Cl₂-c-Pr | Me | Me | Me | H | |
| 6-261 | 1-F-c-Pr | 2,2-Cl₂-c-Pr | c-Pr | Me | H | H | |
| 6-262 | 1-F-c-Pr | 2,2-Cl₂-c-Pr | c-Pr | Me | Me | H | |
| 6-263 | 1-F-c-Pr | 2,2-Cl₂-c-Pr | -(CH₂)₂- | | H | H | |
| 6-264 | 1-F-c-Pr | 2,2-Cl₂-c-Pr | -CH₂-CCl₂- | | H | H | |
| 6-265 | CHF2 | c-Pr | Me | Me | H | H | |
| 6-266 | CHF2 | c-Pr | Me | Me | Me | H | |
| 6-267 | CHF2 | c-Pr | c-Pr | Me | H | H | |
| 6-268 | CHF2 | c-Pr | c-Pr | Me | Me | H | |
| 6-269 | CHF2 | c-Pr | -CH₂-OMe | Me | H | H | |
| 6-270 | CHF2 | c-Pr | -CH₂-OMe | Me | Me | H | |
| 6-271 | CHF2 | c-Pr | -(CH₂)₂**-** | | H | H | |
| 6-272 | CHF2 | c-Pr | -(CH₂)₂- | | H | Me | |
| 6-273 | CHF2 | c-Pr | -(CH₂)₂- | | H | c-Pr | |
| 6-274 | CHF2 | c-Bu | -(CH₂)₃- | | H | H | |
| 6-275 | CHF2 | c-Bu | -(CH₂)₃- | | H | Me | |
| 6-276 | CHF2 | c-Bu | -(CH₂)₃- | | H | c-Pr | |

### Erläuterungen zu Tabelle 6:

"NMR" der Beispielverbindungen wurden jeweils als ¹H-NMR-Spektum bei 400 MHz (CDCl₃) (¹H-Kernresonanz-Daten) gemessen. Nachfolgend sind charakterische chemische Verschiebungen δ (ppm) für Beispielverbindungen aufgeführt:

| Beisp. Nr. | δ (ppm) = |
|---|---|
| 6-199: | 8.80 (m, 1H), 3.90-3.40 (m, 8H), 2.65 (m, 1H), 1.70 (m, 6H), 0.95 (m, 2H), 0.55-0.25 (m, 8H) |

### (B) Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man

| | | |
|---|---|---|
| 75 | Gewichtsteile | einer Verbindung der Formel (I), |
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gewichtsteile einer Verbindung der Formel (I),

| | | |
|---|---|---|
| 5 | " | 2,2'-dinaphthylmethan-6,6'-disulfonsaures Natrium |
| 2 | " | oleoylmethyltaurinsaures Natrium, |
| 1 | Gewichtsteil | Polyvinylalkohol, |
| 17 | Gewichtsteile | Calciumcarbonat und |
| 50 | " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### (C) Biologische Beispiele

### 1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. Auflaufschäden erfolgte nach dem Auflaufen der Versuchspflanzen nach einer Versuchszeit von 3 bis 4 Wochen im Vergleich zu unbehandelten Kontrollen. Wie die Testergebnisse zeigten, wiesen die erfindungsgemäßen Verbindungen eine gute herbizide Vorauflaufwirksamkeit gegen ein breites Spektrum von Ungräsern und Unkräutern auf. Beispielsweise zeigten die Beispiele Nr. 1-19, 1-98, 1-317, 1-335, 1-420, 1-475, 1-493, 1-499, 1-572, 1-651, 2-199, 5-201 und 6-199 der Tabellen 1 bis 6 im Test sehr gute herbizide Wirkung gegen Schadpflanzen wie Stellaria media, Lolium multiflorum, Amaranthus retroflexus, Sinapis alba, Avena sativa und Setaria viridis im Vorauflaufverfahren bei einer Aufwandmenge von 500 g oder weniger Aktivsubstanz pro Hektar.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt. Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht. Nach ca. 3 bis 4 Wochen Standzeit der Versuchspflanzen im Gewächshaus unter optimalen Wachstumsbedingungen wurde die Wirkung der Präparate optisch im Vergleich zu unbehandelten Kontrollen bonitiert. Die erfindungsgemäßen Mittel wiesen auch im Nachauflauf eine gute herbizide Wirksamkeit gegen ein breites Spektrum wirtschaftlich wichtiger Ungräser und Unkräuter auf. Beispielsweise zeigten die Beispiele Nr. 1-19, 1-256, 1-317, 1-335, 1-414, 1-420, 1-475, 1-493, 1-572, 1-651, 2-199, 5-201 und 6-199 der Tabellen 1 bis 6 sehr gute herbizide Wirkung gegen Schadpflanzen wie Sinapis alba, Echinochloa crus-galli, Lolium multiflorum, Stellaria media, Cyperus iria, Amaranthus retroflexus, Setaria viridis, Avena sativa, Lamium purpureum, Matricaria inodora, Papaver rhoeas, Veronica persica, Viola trocolor, Kochia spp und Chenopodium album im Nachauflaufverfahren bei einer Aufwandmenge von 500 g und weniger Aktivsubstanz pro Hektar.

### 3. Unkrautwirkung in Plantagenkultur

In einem Feldversuch wurden Plantagenpflanzen in Versuchsparzellen unter natürlichen Bedingungen herangezogen, wobei ein natürlicher Befall von Schadpflanzen auftrat. Danach erfolgte die Behandlung mit den erfindungsgemäßen Verbindungen durch Besprühen der Schadpflanzen mit einer wäßrigen Dispersion der jeweiligen Verbindung. Etwa drei Wochen nach der Applikation der so durchgeführten Behandlung wurden die Versuchsparzellen hinsichtlich des Schadpflanzenbewuchses und der Schädigung and den Plantagenpflanzen visuell im Vergleich zu Kontrolparzellen bonitiert.

Die erfindungsgemäßen Verbindungen wiesen eine sehr gute herbizide Wirkung gegen die Schadpflanzen auf, wobei Pflantagenkulturen wie Ölpalme, Kokosnusspalme, Gummibaum, Zitronenbaum, Ananas, Baumwolle, Kaffeebaum keine erkennbaren phytotoxischen Schäden aufwiesen.

## Patentansprüche

1. Verbindungen der Formel (I) oder deren Salze, worin 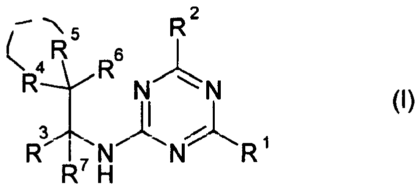 R¹ einen Rest der Formel -NH₂, -NH(B¹-D¹), -N(B¹-D¹)(B²-D²), worin jeweils B¹, B², D¹ und D² wie unten definiert sind, oder eine Gruppe der Formel 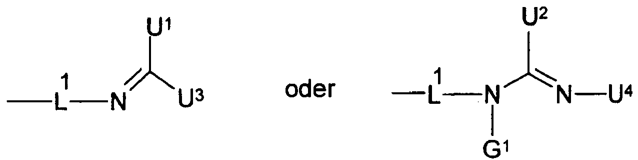 wobei
L¹ eine direkte Bindung, -O-, -S- oder eine Gruppe der Formel -NG²- bedeutet,
U¹, U² unabhängig voneinander eine Gruppe der Formel G³, OG⁴, SG⁵, NG⁶G⁷, NG⁸NG⁹G¹⁰, NG¹¹OG¹² oder NG¹¹SG¹² bedeuten,
U³ eine Gruppe der Formel G¹³, OG¹⁴, SG¹⁵, NG¹⁶G¹⁷, NG¹⁸NG¹⁹G²⁰, NG²¹OG²² oder NG²³SG²⁴ bedeutet,
U⁴ eine Gruppe der Formel G²⁵, OG²⁶, SG²⁷ oder NG²⁸G²⁹ bedeutet,
wobei die Reste G¹ bis G²⁹ unabhängig voneinander Wasserstoff, Aryl, das unsubstituiert oder substituiert ist und inklusive Substituenten 6 bis 30 C-Atome aufweist, oder (C₃-C₉)Cycloalkyl, das unsubstituiert oder substituiert ist und inklusive Substituenten 3 bis 30 C-Atome aufweist, oder Heterocyclyl, das substituiert oder unsubstituiert ist und inklusive Substituenten 2 bis 30 C-Atome aufweist, oder
(C₁-C₆)Alkyl, (C₂-C₆)Alkenyl oder (C₂-C₆)Alkinyl bedeuten,
wobei jeder der letztgenannten 3 Reste unsubstituiert oder durch einen oder mehrere Reste aus der Gruppe bestehend aus Halogen, Hydroxy, Cyano, Nitro, Thiocyanato, (C₁-C₄)Alkoxy, (C₁-C₄)Haloalkoxy, (C₂-C₄)Alkenyloxy, (C₂-C₄)Haloalkenyloxy, (C₁-C₄)Alkylthio, (C₁-C₄)Haloalkylthio, Acyl, (C₃-C₉)Cycloalkyl, das unsubstituiert oder substituiert ist, Phenyl, das unsubstituiert oder substituiert ist, und Heterocyclyl, das unsubstituiert oder substituiert ist, substituiert ist und inklusive Substituenten 1 bis 30 C-Atome aufweist,
oder die Reste U¹ und U³ oder U² und U⁴ oder U² und G¹ oder U⁴ und G¹ paarweise mit den sie verbindenden Atomen jeweils einen carbocyclischen bzw. heterocyclischen Ring mit 4 bis 7 Ringatomen bedeuten, wobei der Ring unsubstituiert oder substituiert ist und inklusive Substituenten bis zu 30 C-Atome aufweiset,
B¹ und B² jeweils unabhängig voneinander eine divalente Gruppe der Formel -C(=Z*)-, -C(=Z*)-Z**-, -C(=Z*)-NH- oder -C(=Z*)-NR*-
bedeuten, wobei Z* ein Sauerstoff- oder Schwefelatom, Z** ein Sauerstoff- oder Schwefelatom und R* (C₁-C₆)Alkyl, Aryl, Aryl-(C₁-C₆)alkyl, (C₃-C₉)Cycloalkyl oder (C₃-C₉)Cycloalkyl-(C₁-C₆)alkyl, wobei jeder der 5 letztgenannten Reste unsubstituiert oder substituiert ist und inklusive Substituenten bis zu 30 C-Atome aufweist, bedeuten,
D¹ und D² jeweils unabhängig voneinander Wasserstoff, (C₁-C₆)Alkyl, Aryl, Aryl-(C₁-C₆)alkyl, (C₃-C₉)Cycloalkyl oder (C₃-C₉)Cycloalkyl-(C₁-C₆)alkyl bedeuten, wobei jeder der 5 letztgenannten Reste unsubstituiert oder substituiert ist und inklusive Substituenten bis zu 30 C-Atome aufweist,
R² Wasserstoff, (C₁-C₆)Alkyl, (C₂-C₆)Alkenyl oder (C₂-C₆)Alkinyl, wobei jede der drei letztgenannten Gruppen unsubstituiert oder durch einen oder mehrere Reste aus der Gruppe, welche aus Halogen, Hydroxy, Cyano, (C₁-C₆)Alkoxy, (C₁-C₆)Alkylthio, (C₁-C₆)Haloalkoxy, (C₁-C₄)Alkoxy-(C₁-C₄)alkoxy und gegebenenfalls durch halogen-, cyano-, (C₁-C₄)alkyl- doer (C₁-C₄)haloalkylsubstituiertes (C₃-C₆)Cycloalkyl besteht, substituiert ist, oder
(C₃-C₆)Cycloalkyl, das unsubstituiert oder durch einen oder mehrere Reste aus der Gruppe bestehend aus Halogen, Cyano, (C₁-C₄)Alkyl und (C₁-C₄)Haloalkyl substituiert ist,
R³ Cyclopropyl oder Cyclobutyl, wobei jeder der letztgenannten beiden Reste unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist,
und
R⁴ und R⁵ unabhängig voneinander jeweils
(C₁-C₆)Alkyl, (C₂-C₆)Alkenyl oder (C₂-C₆)Alkinyl, wobei jeder der letztgenannten drei Reste unsubstituiert oder durch einen oder mehrere Reste aus der Gruppe bestehend aus Halogen, (C₁-C₄)Alkoxy, (C₁-C₄)Haloalkoxy und (C₁-C₄)Alkylthio substituiert ist, oder
Cyclopropyl, das unsubstituiert oder durch einen oder mehrere Reste aus der Gruppe bestehend aus Halogen, (C₁-C₄)Alkyl, (C₁-C₄)Haloalkyl, (C₂-C₄)Alkenyl, (C₂-C₄)Alkinyl, (C₁-C₄)Alkoxy, (C₁-C₄)Haloalkoxy und (C₁-C₄)Alkylthio substituiert ist, oder R⁴ und R⁵ gemeinsam mit dem an sie gebundenen C-Atom einen 3- bis 9-gliedrigen carbocyclischen Ring, der unsubstituiert oder durch einen oder mehrere Reste aus der Gruppe bestehend aus Halogen, (C₁-C₄)Alkyl, (C₁-C₄)Haloalkyl, (C₁-C₄)Alkoxy-(C₁-C₄)Alkyl, (C₁-C₄)Alkoxy, (C₁-C₄)Haloalkoxy und (C₁-C₄)Alkylthio substituiert ist, und
R⁶ Wasserstoff oder (C₁-C₄)Alkyl, das unsubstituiert oder durch einen oder mehrere Reste aus der Gruppe bestehend aus Halogen, (C₁-C₄)Alkoxy, (C₁-C₄)Haloalkoxy und (C₁-C₄)Alkylthio substituiert ist, und
R⁷ Wasserstoff, Methyl, Ethyl oder Cyclopropyl, das unsubstituiert oder durch einen oder mehrere Reste aus der Gruppe bestehend aus Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist,
bedeuten.

2. Verbindungen der Formel (I) oder deren Salze nach Anspruch 1, **dadurch gekennzeichnet, dass**
R¹ einen Rest der Formel -NH₂, -NH(B¹-D¹), -N(B¹-D¹)(B²-D²), worin jeweils B¹, B², D¹ und D² wie unten definiert sind, oder eine Gruppe der Formel 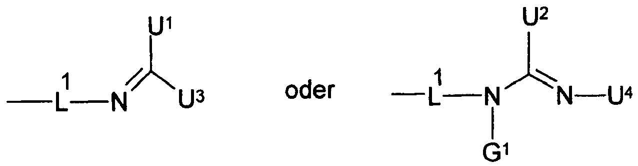 wobei
L¹ eine direkte Bindung, -O-, -S- oder eine Gruppe der Formel -NG²-, vorzugsweise eine direkte Bindung bedeutet,
U¹, U² unabhängig voneinander eine Gruppe der Formel G³, OG⁴, SG⁵, NG⁶G⁷, NG⁸NG⁹G¹⁰, NG¹¹OG¹² oder NG¹¹SG¹² bedeuten,
U³ eine Gruppe der Formel G¹³, OG¹⁴, SG¹⁵, NG¹⁶G¹⁷, NG¹⁸NG¹⁹G²⁰, NG²¹OG²² oder NG²³SG²⁴ bedeutet,
U⁴ eine Gruppe der Formel G²⁵, OG²⁶, SG²⁷ oder NG²⁸G²⁹ bedeutet,
wobei die Reste G¹ bis G²⁹ unabhängig voneinander Wasserstoff, Phenyl, das unsubstituiert oder substituiert ist und inklusive Substituenten 6 bis 30 C-Atome aufweist, oder (C₃-C₉)Cycloalkyl, das unsubstituiert oder substituiert ist und inklusive Substituenten 3 bis 30 C-Atome aufweist, oder Heterocyclyl, das substituiert oder unsubstituiert ist und inklusive Substituenten 2 bis 30 C-Atome aufweist, oder
(C₁-C₆)Alkyl, (C₂-C₆)Alkenyl oder (C₂-C₆)Alkinyl bedeuten,
wobei jeder der letztgenannten 3 Reste unsubstituiert oder durch einen oder mehrere Reste aus der Gruppe Halogen, Hydroxy, Cyano, Nitro, Thiocyanato, (C₁-C₄)Alkoxy, (C₁-C₄)Haloalkoxy, (C₂-C₄)Alkenyloxy, (C₂-C₄)Haloalkenyloxy, (C₁-C₄)Alkylthio, (C₁-C₄)Alkylsulfinyl, (C₁-C₄)Alkylsulfonyl, (C₁-C₄)Haloalkylthio, (C₁-C₄)Haloalkylsulfinyl, (C₁-C₄)Haloalkylsulfonyl, (C₃-C₉)Cycloalkyl, das unsubstituiert oder substituiert ist, Phenyl, das unsubstituiert oder substituiert ist, und Heterocyclyl, das unsubstituiert oder substituiert ist, und Reste der Formeln R'-C(=Z')-, R'-C(=Z')-Z-, R'-Z-C(=Z')-, R'R"N-C(=Z')-, R'-Z-C(=Z')-O-, R'R"N-C(=Z')-Z-, R'-Z-C(=Z')-NR"- und R'R"N-C(=Z')-NR"'-,
worin R', R" und R'" jeweils unabhängig voneinander (C₁-C₆)Alkyl, Phenyl, Phenyl-(C₁-C₆)alkyl, (C₃-C₉)Cycloalkyl oder (C₃-C₉)Cycloalkyl-(C₁-C-₆)alkyl, wobei jeder der 5 letztgenannten Reste unsubstituiert oder substituiert ist, bedeuten und worin Z und Z' unabhängig voneinander jeweils ein Sauerstoff- oder Schwefelatom sind,
substituiert ist,
oder die Reste U¹ und U³ oder U² und U⁴ oder U² und G¹ oder U⁴ und G¹ paarweise mit den sie verbindenden Atomen jeweils einen carbocyclischen bzw. heterocyclischen Ring mit 4 bis 7 Ringatomen bedeuten, wobei der Ring unsubstituiert oder substituiert ist,
B¹ und B² jeweils unabhängig voneinander eine divalente Gruppe der Formeln -C(=Z*)-, -C(=Z*)-Z**-, -C(=Z*)-NH- oder -C(=Z*)-NR*- bedeuten, wobei Z* ein Sauerstoff- oder Schwefelatom, Z** ein Sauerstoff- oder Schwefelatom und R* (C₁-C₆)Alkyl, Phenyl, Phenyl-(C₁-C₆)alkyl, (C₃-C₉)Cycloalkyl oder (C₃-C₉)Cycloalkyl-(C₁-C₆)alkyl, wobei jeder der 5 letztgenannten Reste unsubstituiert oder substituiert ist und inklusive Substituenten bis zu 20 C-Atome aufweist, bedeuten,
D¹ und D² jeweils unabhängig voneinander Wasserstoff, (C₁-C₆)Alkyl, Phenyl, Phenyl-(C₁-C₆)alkyl, (C₃-C₉)Cycloalkyl oder (C₃-C₉)Cycloalkyl-(C₁-C₆)alkyl bedeuten, wobei jeder der 5 letztgenannten Reste unsubstituiert oder substituiert ist und inklusive Substituenten bis zu 20 C-Atome aufweist,
bedeutet.

3. Verbindungen der Formel (I) oder deren Salze nach Anspruch 1 oder 2, **dadurch gekennzeichnet, dass**
R¹ Amino, Acylamino mit 1 bis 6 C-Atomen, Di(C₁-C₄)alkylamino-(C₁-C₄)alkylidenamino oder N-Heterocyclylamino-(C₁-C₄)alkylidenamino, wobei der N-Heterocyclus ein gesättigter heterocyclischer Ring mit 1 bis 3 Heteroringatomen aus der Gruppe N, O und S und mindestens einem N-Atom als Heteroringatom, das an der Alkylidengruppe gebunden ist, darstellt, bedeutet.

4. Verbindungen der Formel (I) oder deren Salze nach einem der Ansprüche 1 bis 3, **dadurch gekennzeichnet, dass**
R² Wasserstoff, (C₁-C₆)Alkyl, (C₂-C₆)Alkenyl oder (C₂-C₆)Alkinyl, wobei jede der drei letztgenannten Gruppen unsubstituiert oder durch einen oder mehrere Reste aus der Gruppe, welche aus Halogen, (C₁-C₄)Alkoxy, (C₁-C₄)Alkylthio, (C₁-C₄)Haloalkoxy, (C₁-C₂)Alkoxy-(C₁-C₂)alkoxy und gegebenenfalls durch halogen- oder (C₁-C₄)alkylsubstituiertes (C₃-C₆)Cycloalkyl besteht, substituiert ist, oder (C₃-C₆)Cycloalkyl, das unsubstituiert oder durch einen oder mehrere Reste aus der Gruppe bestehend aus Halogen und (C₁-C₄)Alkyl substituiert ist,
R³ Cyclopropyl oder Cyclobutyl, wobei jeder der letztgenannten beiden Reste unsubstituiert oder durch einen oder mehrere Reste aus der Gruppe bestehend aus Halogen, (C₁-C₄)Alkyl, (C₂-C₄)Alkenyl und (C₂-C₄)Alkinyl substituiert ist,
R⁴ und R⁵ unabhängig voneinander jeweils
(C₁-C₄)Alkyl, (C₂-C₄)Alkenyl oder (C₂-C₄)Alkinyl, wobei jeder der letztgenannten drei Reste unsubstituiert oder durch einen oder mehrere Reste aus der Gruppe bestehend aus Halogen, (C₁-C₄)Alkoxy, (C₁-C₄)Haloalkoxy und (C₁-C₄)Alkylthio substituiert ist, oder
Cyclopropyl, das unsubstituiert oder durch einen oder mehrere Reste aus der Gruppe bestehend aus Halogen und (C₁-C₄)Alkyl substituiert ist, oder
R⁴ und R⁵ gemeinsam mit dem an sie gebundenen C-Atom einen 3- bis 6-gliedrigen carbocyclischen Ring, der unsubstituiert oder durch einen oder mehrere Reste aus der Gruppe bestehend aus Halogen und (C₁-C₄)Alkyl substituiert ist,
R⁶ Wasserstoff oder (C₁-C₄)Alkyl, das unsubstituiert oder durch einen oder mehrere Reste aus der Gruppe bestehend aus Halogen, (C₁-C₄)Alkoxy, (C₁-C₄)Haloalkoxy und (C₁-C₄)Alkylthio substituiert ist,
R⁷ Wasserstoff, Methyl, Ethyl oder Cyclopropyl, das unsubstituiert oder durch einen oder mehrere Reste aus der Gruppe bestehend aus Halogen, (C₁-C₄)Alkyl und (C₁-C₄)Haloalkyl substituiert ist,
bedeuten.

5. Verbindungen der Formel (I) oder deren Salze nach einem der Ansprüche 1 bis 4, **dadurch gekennzeichnet, dass** sie Verbindungen der Formel (I-A) und deren Salze, 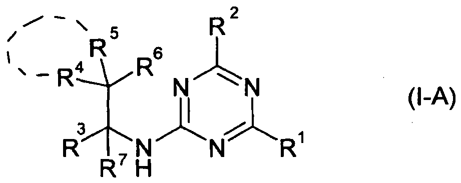 worin
R¹ bis R⁷ wie in Formel (I) definiert sind und wobei die Gruppen R⁴ und R⁵ cyclisch verbunden sein können, darstellen.

6. Verfahren zur Herstellung von Verbindungen der Formel (I) oder deren Salze nach einem der Ansprüche 1 bis 5, **dadurch gekennzeichnet, dass** man
a) eine Verbindung der Formel (II),
R² - Fu (II)
worin Fu eine funktionelle Gruppe aus der Gruppe Carbonsäureester, Carbonsäureorthoester, Carbonsäurechlorid, Carbonsäureamid, Carbonsäureanhydrid und Trichlormethyl bedeutet, mit einer Verbindung der Formel (III) oder einem Säureadditionssalz hiervon 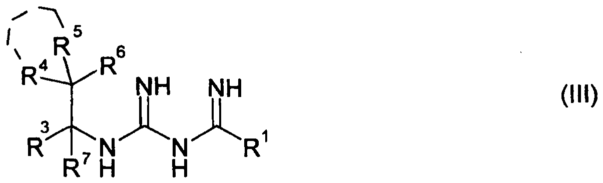 umsetzt oder
b) eine Verbindung der Formel (IV), 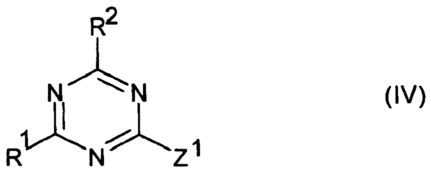 worin Z¹ einen austauschfähigen Rest oder eine Abgangsgruppe, z.B. Chlor, Trichlormethyl, (C₁-C₄)Alkylsulfonyl, unsubstituiertes oder
substituiertes Phenyl-(C₁-C₄)alkylsulfonyl oder (C₁-C₄)Alkyl-phenylsulfonyl, bedeutet, mit einem Amin der Formel (V) oder einem Säureadditionssalz hiervon 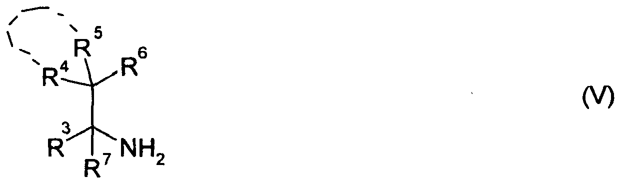 umsetzt oder
c) eine Verbindung der Formel (I') oder deren Salz, 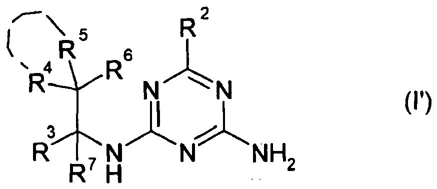 an der Aminogruppe zur Verbindung der Formel (I) derivatisiert,
wobei in den Formeln (II), (III), (IV), (V) und (I') die Reste R¹, R², R³, R⁴, R⁵, R⁶ und R⁷ wie in Formel (I) definiert sind.

7. Herbizides oder pflanzenwachstumsregulierendes Mittel, **dadurch gekennzeichnet, dass** es eine oder mehrere Verbindungen der Formel (I) oder deren Salze nach einem der Ansprüche 1 bis 5 und im Pflanzenschutz übliche Formulierungshilfsmittel enthält.

8. Verfahren zur Bekämpfung von Schadpflanzen oder zur Wachstumsregulierung von Pflanzen, **dadurch gekennzeichnet, dass** man eine wirksame Menge von einer oder mehreren Verbindungen der Formel (I) oder deren Salzen nach einem der Ansprüche 1 bis 5 auf die Pflanzen, Pflanzensamen oder die Anbaufläche appliziert.

9. Verwendung von Verbindungen der Formel (I) oder deren Salzen nach einem der Ansprüche 1 bis 5 als Herbizide oder Pflanzenwachstumsregulatoren.

10. Verwendung nach Anspruch 9, **dadurch gekennzeichnet, dass** die Verbindungen der Formel (I) oder deren Salze zur Bekämpfung von Schadpflanzen oder zur Wachstumsregulierung in Kulturen von Nutz- oder Zierpflanzen eingesetzt werden.

11. Verwendung nach Anspruch 10, **dadurch gekennzeichnet, dass** die Kulturpflanzen transgene Kulturpflanzen sind.

12. Verwendung nach Anspruch 10 oder 11, **dadurch gekennzeichnet, dass** die Kulturpflanzen aus Plantagenkulturen ausgewählt sind.

13. Verwendung nach einem der Ansprüche 9 bis 12, **dadurch gekennzeichnet, dass** Schadpflanzen selektiv in Kulturen von Nutz- oder Zierpflanzen bekämpft werden.

14. Verbindungen der Formel (III), wie sie nach Anspruch 6 definiert sind.

## Claims

1. A compound of the formula (I) or salt thereof, in which 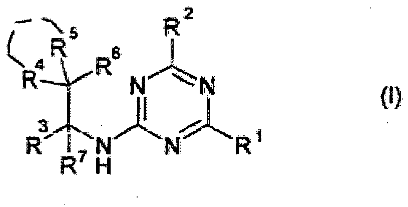 R¹ is a radical of the formula -NH₂, -NH(B¹-D¹), or -N(B¹-D¹) (B²-D²), in each of which B¹, B², D¹ and D² are each as defined below, or a group of the formula 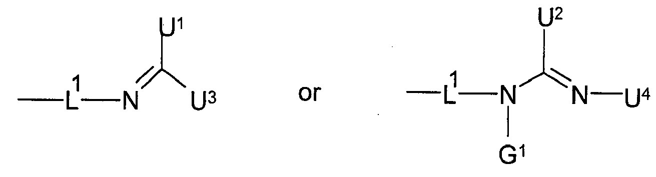 where
L¹ is a direct bond, -O-, -S- or a group of the formula -NG²-,
U¹, U² are each independently a group of the formula G³, OG⁴, SG⁵, NG⁶G⁷, NG⁸NG⁹G¹⁰, NG¹¹OG¹² or NG¹¹SG¹²,
U³ is a group of the formula G¹³, OG¹⁴, SG¹⁵, NG¹⁶G¹⁷, NG¹⁸NG¹⁹G²⁰, NG²¹OG²² or NG²³SG²⁴,
U⁴ is a group of the formula G²⁵, OG²⁶, SG²⁷ or NG²⁸G²⁹,
where the G¹ to G²⁹ radicals are each independently hydrogen, aryl
which is unsubstituted or substituted and has from 6 to 30 carbon atoms including substituents, or (C₃-C₉)cycloalkyl which is unsubstituted or substituted and has from 3 to 30 carbon atoms including substituents, or heterocyclyl which is substituted or unsubstituted and has from 2 to 30 carbon atoms including substituents, or
(C₁-C₆)alkyl, (C₂-C₆)alkenyl or (C₂-C₆)alkynyl,
where each of the 3 latter radicals is unsubstituted or substituted by one or more radicals from the group consisting of halogen, hydroxyl, cyano, nitro, thiocyanato, (C₁-C₄)alkoxy, (C₁-C₄)haloalkoxy, (C₂-C₄)alkenyloxy, (C₂-C₄)haloalkenyloxy, (C₁-C₄)alkylthio, (C₁-C₄)haloalkylthio, acyl, (C₃-C₉)cycloalkyl, which is unsubstituted or substituted, phenyl which is unsubstituted or substituted, and heterocyclyl which is unsubstituted or substituted, and has from 1 to 30 carbon atoms including substituents,
or the U¹ and U³ or U² and U⁴ or U² and G¹ or U⁴ and G¹ radicals, in pairs with the atoms connecting them, are each a carbocyclic or heterocyclic ring having from 4 to 7 ring atoms, where the ring is unsubstituted or substituted, and has up to 30 carbon atoms including substituents,
B¹ and B² are each independently a divalent group of the formula
-C(=Z*)-, -C(=Z*)-Z**-, -C(=Z*)-NH- or -C(=Z*)-NR*-, where Z* is an oxygen or sulfur atom, Z** is an oxygen or sulfur atom and R* is (C₁-C₆)alkyl, aryl, aryl(C₁-C₆)alkyl, (C₃-C₉)cycloalkyl or (C₃-C₉) cycloalkyl (C₁-C₆)alkyl, where each of the 5 latter radicals is unsubstituted or substituted and has up to 30 carbon atoms including substituents,
D¹ and D² are each independently hydrogen, (C₁-C₆)alkyl, aryl, aryl(C₁-C6)alkyl, (C₃-C₉)cycloalkyl or (C₃-C₉)cycloalkyl(C₁-C₆)alkyl, where each of the 5 latter radicals is unsubstituted or substituted and has up to 30 carbon atoms including substituents,
R² is hydrogen, (C₁-C₆)alkyl, (C₂-C₆)alkenyl or (C₂-C₆)alkynyl, where each of the three latter groups is unsubstituted or substituted by one or more of the radicals from the group which consists of halogen, hydroxyl, cyano, (C₁-C₆)alkoxy, (C₁-C₆)alkylthio, (C₁-C₆)haloalkoxy, (C₁-C₄)alkoxy-(C₁-C₄)alkoxy and optionally halogen-, cyano-, (C₁-C₄)alkyl- or (C₁-C₄)haloalkyl-substituted (C₃-C₆)cycloalkyl, or
is (C₃-C₆)cycloalkyl which is unsubstituted or substituted by one or more radicals from the group consisting of halogen, cyano, (C₁-C₄)alkyl and (C₁-C₄)haloalkyl,
R³ is cyclopropyl or cyclobutyl, where each of the latter two radicals is unsubstituted or substituted by one or more radicals from the group consisting of halogen, (C₁-C₄)alkyl, (C₁-C₄)haloalkyl, (C₂-C₄)alkenyl, (C₂-C₄)alkynyl, (C₁-C₄)alkoxy, (C₁-C₄)haloalkoxy and (C₁-C₄)alkylthio,
R⁴ and R⁵ are each independently
(C₁-C₆)alkyl, (C₂-C₆)alkenyl or (C₂-C₆)alkynyl, where each of the latter three radicals is unsubstituted or substituted by one or more radicals from the group consisting of halogen, (C₁-C₄)alkoxy, (C₁-C₄)haloalkoxy and (C₁-C₄)alkylthio, or are each independently cyclopropyl which is unsubstituted or substituted by one or more radicals from the group consisting of halogen, (C₁-C₄)alkyl, (C₁-C₄)haloalkyl, (C₂-C₄)alkenyl, (C₂-C₄)alkynyl, (C₁-C₄)alkoxy, (C₁-C₄)haloalkoxy and (C₁-C₄)alkylthio, or
R⁴ and R⁵, together with the carbon atom bonded to them, are a 3- to 9-membered carbocyclic ring which is unsubstituted or substituted by one or more radicals from the group consisting of halogen, (C₁-C₄)alkyl, (C₁-C₄)haloalkyl, (C₁-C₄)alkoxy-(C₁-C₄)alkyl, (C₁-C₄)alkoxy, (C₁-C₄)haloalkoxy and (C₁-C₄)alkylthio, and
R⁶ is hydrogen or (C₁-C₄)alkyl which is unsubstituted or substituted by one or more radicals from the group consisting of halogen, (C₁-C₄)alkoxy, (C₁-C₄)haloalkoxy and (C₁-C₄)alkylthio, and
R⁷ is hydrogen, methyl, ethyl or cyclopropyl which is unsubstituted or substituted by one or more radicals from the group consisting of halogen, (C₁-C₄)alkyl, (C₁-C₄)haloalkyl, (C₁-C₄)alkoxy, (C₁-C₄)haloalkoxy and (C₁-C₄)alkylthio.

2. A compound of the formula (I) or salts thereof as claimed in claim 1, wherein
R¹ is a radical of the formula -NH₂, -NH(B¹-D¹), or -N(B¹-D¹)(B²-D²), in each of which B¹, B², D¹ and D² are as defined below, or a group of the formula 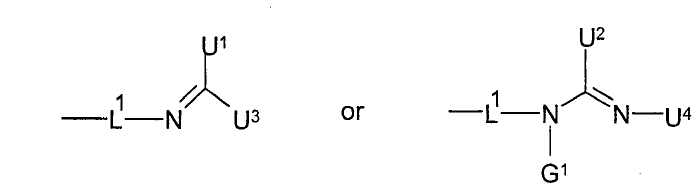 where
L¹ is a direct bond, -O-, -S- or a group of the formula -NG²-, preferably a direct bond, U¹, U² are each independently a group of the formula G³, OG⁴, SG⁵, NG⁶G⁷, NG⁸NG⁹G¹⁰, NG¹¹OG¹² or NG¹¹SG¹²,
U³ is a group of the formula G¹³, OG¹⁴, SG¹⁵, NG¹⁶G¹⁷, NG¹⁸NG¹⁹G²⁰, NG²¹OG²² or NG²³SG²⁴,
U⁴ is a group of the formula G²⁵, OG²⁶, SG²⁷ or NG²⁸G²⁹,
where the G¹ to G²⁹ radicals are each independently hydrogen or phenyl which is unsubstituted or substituted and has from 6 to 30 carbon atoms including substituents, or (C₃-C₉)cycloalkyl which is unsubstituted or substituted and has from 3 to 30 carbon atoms including substituents, or heterocyclyl which is substituted or unsubstituted and has from 2 to 30 carbon atoms including substituents, or
(C₁-C₆)alkyl, (C₂-C₆)alkenyl or (C₂-C₆)alkynyl,
where each of the 3 latter radicals is unsubstituted or substituted by one or more radicals from the group of halogen, hydroxyl, cyano, nitro, thiocyanato, (C₁-C₄)alkoxy, (C₁-C₄)haloalkoxy, (C₂-C₄)alkenyloxy, (C₂-C₄)haloalkenyloxy, (C₁-C₄)alkylthio, (C₁-C₄)alkylsulfinyl, (C₁-C₄)alkylsulfonyl, (C₁-C₄)haloalkylthio, (C₁-C₄)haloalkylsulfinyl, (C₁-C₄)haloalkylsulfonyl, (C₃-C₉)cycloalkyl which is unsubstituted or substituted, phenyl which is unsubstituted or substituted, and heterocyclyl which is unsubstituted or substituted, and radicals of the formulae R'-C(=Z')-, R'-C(=Z')-Z-, R'-Z-C(=Z')-, R'R''N-C(=Z')-, R'-Z-C(=Z')-O-, R'R''N-C(=Z')-Z-, R'-Z-C(=Z')-NR''- and R'R''N-C(=Z')-NR'''-,
in which R', R'' and R''' are each independently (C₁-C₆)alkyl, phenyl, phenyl (C₁-C₆)alkyl, (C₃-C₉)cycloalkyl or (C₃-C₉)cycloalkyl (C₁-C₆)alkyl, where each of the 5 latter radicals is unsubstituted or substituted, and in which Z and Z' are each independently an oxygen or sulfur atom,
or the U¹ and U³ or U² and U⁴ or U² and G¹ or U⁴ and G¹ radicals, in pairs with the atoms connecting them, are each a carbocyclic or heterocyclic ring having from 4 to 7 ring atoms, where the ring is unsubstituted or substituted,
B¹ and B² are each independently a divalent group of the formula -C(=Z*)-,
-C(=Z*)-Z**-, -C(=Z*)-NH- or -C(=Z*)-NR*-, where Z* is an oxygen or sulfur atom, Z** is an oxygen or sulfur atom and R* is (C₁-C₆)alkyl, phenyl, phenyl (C₁-C₆)alkyl, (C₃-C₉)cycloalkyl or (C₃-C₉)cycloalkyl(C₁-C₆)alkyl, where each of the 5 latter radicals is unsubstituted or substituted and has up to 20 carbon atoms, including substituents,
D¹ and D² are each independently hydrogen, (C₁-C₆)alkyl, phenyl, phenyl (C₁-C₆)alkyl, (C₃-C₉)cycloalkyl or (C₃-C₉)cycloalkyl(C₁-C₆)alkyl, where each of the 5 latter radicals is unsubstituted or substituted and has up to 20 carbon atoms, including substituents.

3. A compound of the formula (I) or salts thereof as claimed in claim 1 or 2, wherein
R¹ is amino, acylamino having from 1 to 6 carbon atoms, di(C₁-C₄)alkylamino(C₁-C₄)alkylideneamino or N-heterocyclylamino-(C₁-C₄)alkylideneamino, where the N-heterocycle is a saturated heterocyclic ring having from 1 to 3 ring heteroatoms from the group of N, O and S and at least one nitrogen atom as a ring heteroatom which is bonded to the alkylidene group.

4. A compound of the formula (I) or salts thereof as claimed in one of claims 1 to 3, wherein
R² is hydrogen, (C₁-C₆)alkyl, (C₂-C₆)alkenyl or (C₂-C₆)alkynyl, where each of the three latter groups is unsubstituted or substituted by one or more radicals from the group which consists of halogen, (C₁-C₄)alkoxy, (C₁-C₄)alkylthio, (C₁-C₄)haloalkoxy, (C₁-C₂)alkoxy (C₁-C₂)alkoxy and optionally halogen- or (C₁-C₄)alkyl-substituted (C₃-C₆)cycloalkyl, or is
(C₃-C₆)cycloalkyl which is unsubstituted or substituted by one or more radicals from the group consisting of halogen and (C₁-C₄)alkyl,
R³ is cyclopropyl or cyclobutyl, where each of the two latter radicals is unsubstituted or substituted by one or more radicals from the group consisting of halogen, (C₁-C₄)alkyl, (C₂-C₄)alkenyl and (C₂-C₄)alkynyl, R⁴ and R⁵ are each independently
(C₁-C₄)alkyl, (C₂-C₄)alkenyl or (C₂-C₄)alkynyl, where each of the latter three radicals is unsubstituted or substituted by one or more radicals from the group consisting of halogen, (C₁-C₄)alkoxy, (C₁-C₄)haloalkoxy and (C₁-C₄)alkylthio, or
cyclopropyl which is unsubstituted or substituted by one or more radicals from the group consisting of halogen and (C₁-C₄)alkyl, or
R⁴ and R⁵, together with the carbon atom bonded to them, is a 3- to 6-membered carbocyclic ring which is unsubstituted or substituted by one or more radicals from the group consisting of halogen and (C₁-C₄)alkyl,
R⁶ is hydrogen or (C₁-C₄)alkyl which is unsubstituted or substituted by one or more radicals from the group consisting of halogen, (C₁-C₄)alkoxy, (C₁-C₄)haloalkoxy and (C₁-C₄)alkylthio,
R⁷ is hydrogen, methyl, ethyl or cyclopropyl which is unsubstituted or substituted by one or more radicals from the group consisting of halogen, (C₁-C₄)alkyl and (C₁-C₄)haloalkyl.

5. A compound of the formula (I) or salts thereof as claimed in one of claims 1 to 4, which are compounds of the formula (I-A) and salts thereof 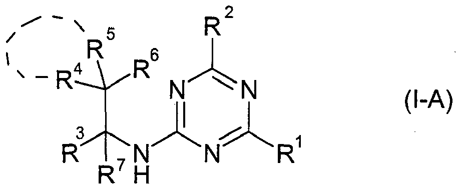 in which
R¹ to R⁷ are each as defined in formula (I) and where the R⁴ and R⁵ groups may be connected in a cyclic system.

6. A process for preparing compounds of the formula (I) or salts thereof as claimed in one of claims 1 to 5, which comprises
a) reacting a compound of the formula (II)
R² - Fu (II)
in which Fu is a functional group from the group of carboxylic ester, carboxylic orthoester, carbonyl chloride, carboxamide, carboxylic anhydride and trichloromethyl with a compound of the formula (III) or an acid addition salt thereof 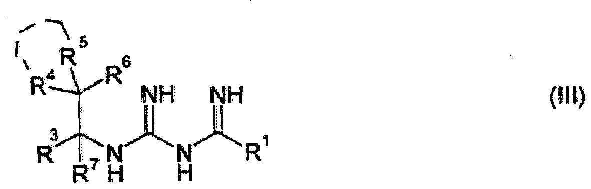 or
b) reacting a compound of the formula (IV) 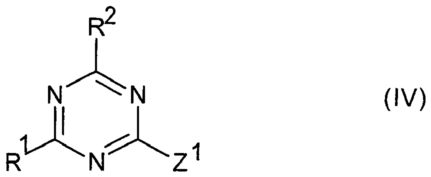 in which Z¹ is an exchangeable radical or a leaving group, for example chlorine, trichloromethyl, (C₁-C₄)alkylsulfonyl, unsubstituted or substituted phenyl-(C₁-C₄)alkylsulfonyl or (C₁-C₄)alkylphenylsulfonyl with an amine of the formula (V) or an acid addition salt thereof 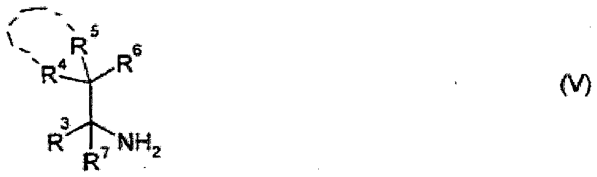 or
c) derivatizing a compound of the formula (I') or salt thereof 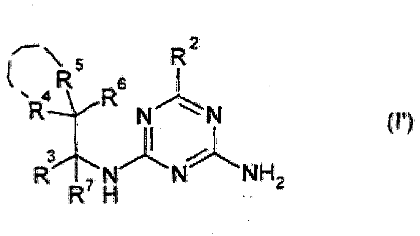 on the amino group to give the compound of the formula (I),
where, in the formulae (II), (III), (IV), (V) and (I'), the R¹, R², R³, R⁴, R⁵, R⁶ and R⁷ radicals are each as defined in formula (I).

7. A herbicidal or crop growth-regulating composition, which comprises one or more compounds of the formula (I) or salts thereof as claimed in one of claims 1 to 5 and formulation assistants customary in crop protection.

8. A method for controlling harmful plants or for regulating the growth of plants, which comprises applying an effective amount of one or more compounds of the formula (I) or salts thereof as claimed in one of claims 1 to 5 to the plants, plant seeds or the area under cultivation.

9. The use of compounds of the formula (I) or salts thereof as claimed in one of claims 1 to 5 as herbicides or crop growth regulators.

10. The use as claimed in claim 9, wherein the compounds of the formula (I) or salts thereof are used to control harmful plants or to regulate the growth of crops of useful or ornamental plants.

11. The use as claimed in claim 10, wherein the crop plants are transgenic crop plants.

12. The use as claimed in claim 10 or 11, wherein the crop plants are selected from plantation crops.

13. The use as claimed in one of claims 9 to 12, wherein harmful plants are selectively controlled in crops of useful or ornamental plants.

14. A compound of the formula (III) as defined in claim 6.

## Revendications

1. Composés de formule (I) ou leurs sels, dans lesquels 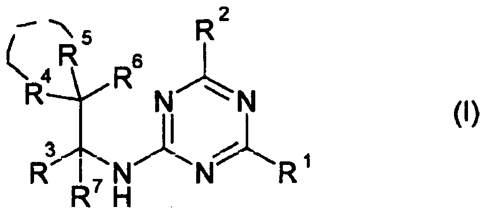 R¹ représente un radical de formule -NH₂, -NH(B¹-D¹), -N(B¹-D¹)(B²-D²), dans laquelle B¹, B², D¹ et D² sont chacun tels que définis ci-dessous, ou un groupe de formule 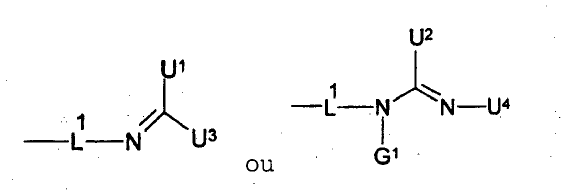 où
L¹ représente une liaison directe, -O-, -S- ou un groupe de formule -NG²-,
U¹, U² représentent indépendamment l'un de l'autre un groupe de formule G³, OG⁴, SG⁵, NG⁶G⁷, NG⁸NG⁹G¹⁰, NG¹¹OG¹² ou NG¹¹SG¹²,
U³ représente un groupe de formule G¹³, OG¹⁴, SG¹⁵, NG¹⁶G¹⁷, NG¹⁸NG¹⁹G²⁰, NG²¹OG²² ou NG²³SG²⁴,
U⁴ représente un groupe de formule G²⁵, OG²⁶, SG²⁷ ou NG²⁸G²⁹,
les radicaux G¹ à G²⁹ représentant indépendamment les uns des autres un atome d'hydrogène, un groupe aryle qui est non substitué ou substitué et comporte substituants inclus 6 à 30 atomes de carbone, ou un groupe cycloalkyle en C₃-C₉ qui est non substitué ou substitué et comporte substituants inclus 3 à 30 atomes de carbone, ou un groupe hétérocyclyle qui est substitué ou non substitué et comporte, substituants inclus, 2 à 30 atomes de carbone, ou
un radical alkyle en C₁-C₆, alcényle en C₂-C₆ ou alcynyle en C₂-C₆,
chacun des 3 derniers radicaux nommés étant non substitué ou substitué par un ou plusieurs radicaux choisis dans le groupe constitué par halogéno, hydroxy, cyano, nitro, thiocyanato, alcoxy en C₁-C₄, halogénoalcoxy(C₁-C₄), alcényloxy(C₂-C₄), halogénoalcényloxy(C₂-C₄), alkyl(C₁-C₄)thio, halogénoalkyl(C₁-C₄)thio, acyle, cycloalkyle en C₃-C₉ qui est substitué ou non substitué, phényle qui est substitué ou non substitué et hétérocyclyle qui est substitué ou non substitué et comporte, substituants inclus, 1 à 30 atomes de carbone,
ou les radicaux U¹ et U³ ou U² et U⁴ ou U² et G¹ ou U⁴ et G¹ représentent par paires avec les atomes qui les lient chaque fois un cycle carbocyclique ou hétérocyclique ayant de 4 à 7 atomes formant le cycle, le cycle étant substitué ou non substitué et comportant, substituants inclus, jusqu'à 30 atomes de carbone,
B¹ et B² représentent chacun, indépendamment l'un de l'autre, un groupe divalent de formule -C(=Z*)-, -C(=Z*)-Z**, -C(=Z*)-NH- ou -C(=Z*)-NR*-,
Z* représentant un atome d'oxygène ou de soufre, Z** représentant un atome d'oxygène ou de soufre et R* représentant un groupe alkyle en C₁-C₆, aryle, aryl-alkyle(C₁-C₆), cycloalkyle en C₃-C₉ ou cycloalkyl(C₃-C₉)-alkyle(C₁-C₆), chacun des 5 derniers radicaux nommés étant substitué ou non substitué et comportant, substituants inclus, jusqu'à 30 atomes de carbone,
D¹ et D² représentent chacun, indépendamment l'un de l'autre, un atome d'hydrogène, un groupe alkyle en C₁-C₆, aryle, aryl-alkyle(C₁-C₆), cycloalkyle en C₃-C₉ ou cycloalkyl(C₃-C₉)-alkyle(C₁-C₆), chacun des 5 derniers radicaux nommés étant substitué ou non substitué et comportant, substituants inclus, jusqu'à 30 atomes de carbone,
R² représente un atome d'hydrogène, un groupe alkyle en C₁-C₆, alcényle en C₂-C₆ ou alcynyle en C₂-C₆, chacun des trois derniers groupes nommés étant non substitué ou substitué par un ou plusieurs radicaux choisis dans le groupe qui est constitué par halogéno, hydroxy, cyano, alcoxy en C₁-C₆, alkyl(C₁-C₆)thio, halogénoalcoxy(C₁-C₆), alcoxy(C₁-C₄)-alcoxy(C₁-C₄) et cycloalkyle en C₃-C₆ éventuellement substitué par halogéno, cyano, alkyle en C₁-C₄ ou halogénoalkyle(C₁-C₄), ou
un groupe cycloalkyle en C₃-C₆ qui est non substitué ou substitué par un ou plusieurs radicaux choisis dans le groupe constitué par halogéno, cyano, alkyle en C₁-C₄ et halogénoalkyle en C₁-C₄,
R³ représente un groupe cyclopropyle ou cyclobutyle, chacun des deux derniers groupes nommés étant non substitué ou substitué par un ou plusieurs radicaux choisis dans le groupe constitué par halogéno, alkyle en C₁-C₄, halogénoalkyle(C₁-C₄), alcényle en C₂-C₄, alcynyle en C₂-C₄, alcoxy en C₁-C₄, halogénoalcoxy(C₁-C₄) et alkyl(C₁-C₄)thio,
et
R⁴ et R⁵ représentent chacun, indépendamment l'un de l'autre,
un groupe alkyle en C₁-C₆, alcényle en C₂-C₆ ou alcynyle en C₂-C₆, chacun des trois derniers groupes nommés étant non substitué ou substitué par un ou plusieurs radicaux choisis dans le groupe constitué par halogéno, alcoxy en C₁-C₄, halogénoalcoxy(C₁-C₄), et alkyl(C₁-C₄)thio, ou un groupe cyclopropyle, qui est non substitué ou substitué par un ou plusieurs radicaux choisis dans le groupe constitué par halogéno, alkyle en C₁-C₄, halogénoalkyle(C₁-C₄), alcényle en C₂-C₄, alcynyle en C₂-C₄, alcoxy en C₁-C₄, halogénoalcoxy(C₁-C₄) et alkyl(C₁-C₄)thio, ou
R⁴ et R⁵ forment ensemble avec l'atome de carbone lié à ces groupes un cycle carbocyclique à 3-9 chaînons, qui est non substitué ou substitué par un ou plusieurs radicaux choisis dans le groupe constitué par halogéno, alkyle en C₁-C₄, halogénoalkyle(C₁-C₄), alcoxy(C₁-C₄)-alkyle(C₁-C₄), alcoxy en C₁-C₄, halogénoalcoxy(C₁-C₄) et alkyl(C₁-C₄)thio,
et
R⁶ représente un atome d'hydrogène ou un groupe alkyle en C₁-C₄, qui est non substitué ou substitué par un ou plusieurs radicaux choisis dans le groupe constitué par halogéno, alcoxy en C₁-C₄, halogénoalcoxy(C₁-C₄) et alkyl(C₁-C₄)thio, et
R⁷ représente un atome d'hydrogène, un groupe méthyle, éthyle ou cyclopropyle, qui est non substitué. ou substitué par un ou plusieurs radicaux choisis dans le groupe constitué par halogéno, alkyle en C₁-C₄, halogénoalkyle(C₁-C₄), alcoxy en C₁-C₄, halogénoalcoxy(C₁-C₄) et alkyl(C₁-C₄)thio.

2. Composés de formule (I) ou leurs sels selon la revendication 1, **caractérisés en ce que**
R¹ représente un radical de formule -NH₂, -NH(B¹-D¹), -N(B¹-D¹)(B²-D²), dans laquelle B¹, B², D¹ et D² sont chacun tels que définis ci-dessous, ou un groupe de formule 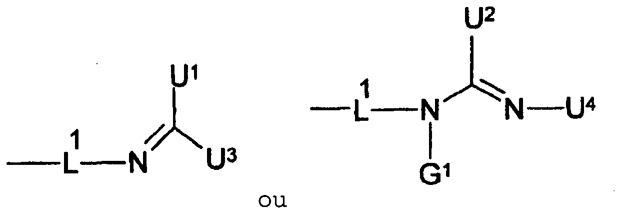 où
L¹ représente une liaison directe, -O-, -S- ou un groupe de formule -NG²-, de préférence une liaison directe,
U¹, U² représentent indépendamment l'un de l'autre un groupe de formule G³, OG⁴, SG⁵, NG⁶G⁷, NG⁸NG⁹G¹⁰, NG¹¹OG¹² ou NG¹¹SG¹²,
U³ représente un groupe de formule G¹³, OG¹⁴, SG¹⁵, NG¹⁶G¹⁷, NG¹⁸NG¹⁹G²⁰, NG²¹OG²² ou NG²³SG²⁴,
U⁴ représente un groupe de formule G²⁵, OG²⁶, SG²⁷ ou NG²⁸G²⁹,
les radicaux G¹ à G²⁹ représentant indépendamment les uns des autres un atome d'hydrogène, un groupe phényle qui est non substitué ou substitué et comporte, substituants inclus, 6 à 30 atomes de carbone, ou un groupe cycloalkyle en C₃-C₉ qui est non substitué ou substitué et comporte, substituants inclus, 3 à 30 atomes de carbone, ou un groupe hétérocyclyle qui est substitué ou non substitué et comporte, substituants inclus, 2 à 30 atomes de carbone, ou
un radical alkyle en C₁-C₆, alcényle en C₂-C₆ ou alcynyle en C₂-C₆,
chacun des 3 derniers radicaux nommés étant non substitué ou substitué par un ou plusieurs radicaux choisis dans le groupe constitué par halogéno, hydroxy, cyano, nitro, thiocyanato, alcoxy en C₁-C₄, halogénoalcoxy(C₁-C₄), alcényloxy(C₂-C₄), halogénoalcényloxy(C₂-C₄), alkyl (C₁-C₄)thio, alkyl(C₁-C₄)-sulfinyle, alkyl(C₁-C₄)sulfonyle, halogénoalkyl(C₁-C₄)thio, halogénoalkyl(C₁-C₄)sulfinyle, halogénoalkyl(C₁-C₄)sulfonyle, cycloalkyle en C₃-C₉ qui est substitué ou non substitué, phényle qui est substitué ou non substitué et hétérocyclyle qui est substitué ou non substitué, et des radicaux de formules R'-C(=Z')-, R'-C(=Z')-Z-, R'-Z-C(=Z')-, R'R''N-C(=Z')-, R'-Z-C(=Z')-O-, R'R''N-C(=Z')-Z-, R'-Z-C(=Z')-NR''- et R'R''N-C(=Z')-NR''',
dans lesquelles R', R'' et R''' représentent chacun indépendamment des autres un groupe alkyle en C₁-C₆, phényle, phényl-alkyle(C₁-C₆), cycloalkyle en C₃-C₉ ou cycloakyl(C₃-C₉)-alkyle(C₁-C₆), chacun des cinq derniers groupes nommés étant substitué ou non substitué, et dans lesquelles Z et Z' représentent chacun indépendamment l'un de l'autre un atome d'oxygène ou de soufre,
ou les radicaux U¹ et U³ ou U² et U⁴ ou U² et G¹ ou U⁴ et G¹ représentent par paires avec les atomes qui les lient chaque fois un cycle carbocyclique ou hétérocyclique ayant de 4 à 7 atomes formant le cycle, le cycle étant substitué ou non substitué,
B¹ et B² représentent chacun, indépendamment l'un de l'autre, un groupe divalent de formule -C(=Z*)-, -C(=Z*)-Z**, -C(=Z*)-NH- ou -C(=Z*)-NR*-,
Z* représentant un atome d'oxygène ou de soufre, Z** représentant un atome d'oxygène ou de soufre et R* représentant un groupe alkyle en C₁-C₆, phényle, phénylalkyle(C₁-C₆), cycloalkyle en C₃-C₉ ou cycloalkyl(C₃-C₉)-alkyle(C₁-C₆), chacun des 5 derniers radicaux nommés étant substitué ou non substitué et comportant, substituants, inclus jusqu'à 20 atomes de carbone,
D¹ et D² représentent chacun, indépendamment l'un de l'autre, un atome d'hydrogène, un groupe alkyle en C₁-C₆, phényle, phényl-alkyle(C₁-C₆), cycloalkyle en C₃-C₉ ou cycloalkyl(C₃-C₉)-alkyle(C₁-C₆), chacun des 5 derniers radicaux nommés étant substitué ou non substitué et comportant, substituants inclus, jusqu'à 20 atomes de carbone,

3. Composés de formule (I) ou leurs sels selon la revendication 1 ou 2, **caractérisés en ce que**
R¹ représente un groupe amino, acylamino ayant de 1 à 6 atomes de carbone, dialkyl(C₁-C₄)amino-alkylidène(C₁-C₄)amino ou N-hétérocyclylamino-alkylidène(C₁-C₄)amino, le N-hétérocycle représentant un cycle hétérocyclique saturé ayant de 1 à 3 hétéroatomes formant le cycle, choisis dans le groupe constitué par N, O et S, et au moins un atome d'azote en tant qu'hétéroatome formant le cycle, qui est lié au groupe alkylidène.

4. Composés de formule (I) ou leurs sels selon l'une quelconque des revendications 1 à 3, **caractérisés en ce que**
R² représente un atome d'hydrogène, un groupe alkyle en C₁-C₆, alcényle en C₂-C₆ ou alcynyle en C₂-C₆, chacun des trois derniers groupes nommés étant non substitué ou substitué par un ou plusieurs radicaux choisis dans le groupe qui est constitué par halogéno, alcoxy en C₁-C₄, alkyl(C₁-C₄)thio, halogénoalcoxy(C₁-C₄), alcoxy(C₁-C₂)-alcoxy(C₁-C₂) et cycloalkyle en C₃-C₆ éventuellement substitué par halogéno ou alkyle en C₁-C₄, ou
un groupe cycloalkyle en C₃-C₆ qui est non substitué ou substitué par un ou plusieurs radicaux choisis dans le groupe constitué par halogéno et alkyle en C₁-C₄,
R³ représente un groupe cyclopropyle ou cyclobutyle, chacun des deux derniers groupes nommés étant non substitué ou substitué par un ou plusieurs radicaux choisis dans le groupe constitué par halogéno, alkyle en C₁-C₄, alcényle en C₂-C₄ et alcynyle en C₂-C₄,
R⁴ et R⁵ représentent chacun, indépendamment l'un de l'autre,
un radical alkyle en C₁-C₄, alcényle en C₂-C₄ ou alcynyle en C₂-C₄, chacun des trois derniers radicaux nommés étant non substitué ou substitué par un ou plusieurs radicaux choisis dans le groupe constitué par halogéno, alcoxy en C₁-C₄, halogénoalcoxy(C₁-C₄), et alkyl (C₁-C₄) thio, ou
un groupe cyclopropyle, qui est non substitué ou substitué par un ou plusieurs radicaux choisis dans le groupe constitué par halogéno et alkyle en C₁-C₄, ou
R⁴ et R⁵ forment ensemble avec l'atome de carbone lié à ces groupes un cycle carbocyclique à 3-6 chaînons, qui est non substitué ou substitué par un ou plusieurs radicaux choisis dans le groupe constitué par halogéno et alkyle en C₁-C₄,
R⁶ représente un atome d'hydrogène ou un groupe alkyle en C₁-C₄, qui est non substitué ou substitué par un ou plusieurs radicaux choisis dans le groupe constitué par halogéno, alcoxy en C₁-C₄, halogénoalcoxy(C₁-C₄) et alkyl(C₁-C₄)thio,
R⁷ représente un atome d'hydrogène, un groupe méthyle, éthyle ou cyclopropyle, qui est non substitué ou substitué par un ou plusieurs radicaux choisis dans le groupe constitué par halogéno, alkyle en C₁-C₄ et halogénoalkyle(C₁-C₄).

5. Composés de formule (I) ou leurs sels selon l'une quelconque des revendications 1 à 4, **caractérisés en ce qu'**ils représentent des composés de formule (I-A) et leurs sels, 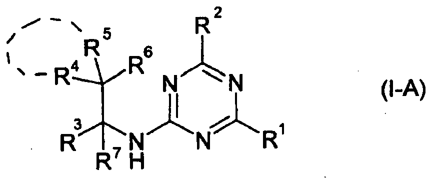 dans laquelle
R¹ à R⁷ sont tels que définis dans la formule (I) et les groupes R⁴ et R⁵ pouvant être liés en un cycle.

6. Procédé pour la préparation des composés de formule (I) ou de leurs sels selon l'une quelconque des revendications 1 à 5, **caractérisé en ce que**
a) on fait réagir un composé de formule (II),
R²-Fu (II)
dans laquelle Fu représente un groupe fonctionnel choisi dans le groupe constitué par ester d'acide carboxylique, orthoester d'acide carboxylique, chlorure de carbonyle, carboxamide, anhydride d'acide carboxylique et trichlorométhyle, avec un composé de formule (III) ou un sel d'addition avec un acide d'un tel composé 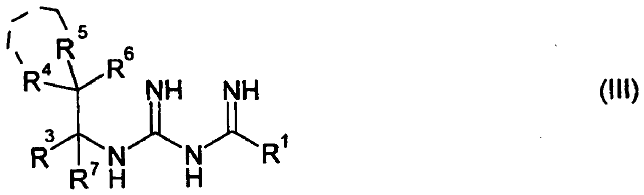 ou
b) on fait réagir un composé de formule (IV), 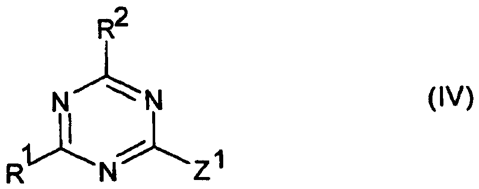 dans laquelle Z¹ représente un radical échangeable ou un groupe partant, par exemple chloro, trichlorométhyle, alkyl(C₁-C₄)sulfonyle, un groupe phényl-alkyl(C₁-C₄)-sulfonyle ou alkyl(C₁-C₄)-phénylsulfonyle substitué ou non substitué,
avec une amine de formule (V) ou un sel d'addition avec un acide de celle-ci 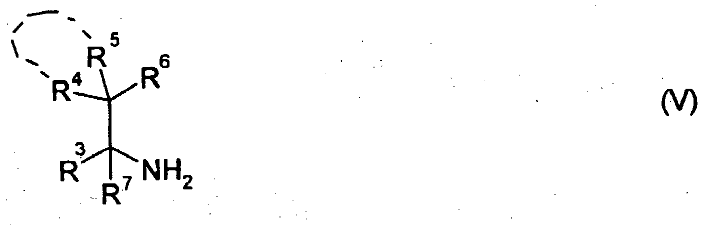 ou
c) on transforme en dérivé un composé de formule (I') ou un de ses sels, 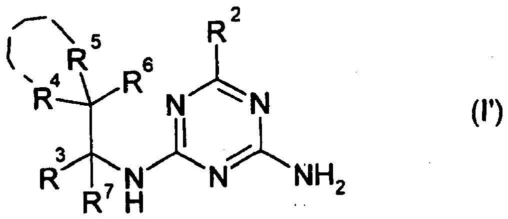 au niveau du groupe amino, pour aboutir au composé de formule (I),
les radicaux R¹, R², R³, R⁴, R⁵, R⁶ et R⁷ dans les formules (II), (III), (IV), (V) et (I') étant tels que définis dans la formule (I).

7. Composition herbicide ou régulatrice de croissance végétale, **caractérisée en ce qu'**elle contient un ou plusieurs composés de formule (I) ou sels de tels composés selon l'une quelconque des revendications 1 à 5 et des adjuvants de formulation usuels dans la protection des plantes.

8. Procédé pour la lutte contre des plantes nuisibles ou pour la régulation de la croissance de plantes, **caractérisé en ce qu'**on applique sur les plantes, les semences de plantes ou l'aire de culture une quantité efficace d'un ou de plusieurs composés de formule (I) ou sels de tels composés selon l'une quelconque des revendications 1 à 5.

9. Utilisation des composés de formule (I) ou de leurs sels selon l'une quelconque des revendications 1 à 5, en tant qu'herbicides ou régulateurs de croissance végétale.

10. Utilisation selon la revendication 9, **caractérisé en ce qu'**on utilise les composés de formule (I) ou leurs sels pour la lutte contre des plantes nuisibles ou pour la régulation de la croissance dans des cultures de plantes utiles ou ornementales.

11. Utilisation selon la revendication 10, **caractérisée en ce que** les plantes cultivées sont des plantes cultivées transgéniques.

12. Utilisation selon la revendication 10 ou 11, **caractérisée en ce que** les plantes cultivées sont choisies parmi des cultures agricoles.

13. Utilisation selon l'une quelconque des revendications 9 à 12, **caractérisée en ce qu'**on lutte contre des plantes nuisibles sélectivement dans des cultures de plantes utiles ou ornementales.

14. Composés de formule (III), tels qu'ils sont définis selon la revendication 6.
